# EUROPEAN PATENT APPLICATION

(11) **EP 4 834 898 A1**
(43) Date of publication of application: **04.11.2026**
(21) Application number: 24911553.6
(22) Date of filing: 27.12.2024
(51) Int. Cl.: C07D 405/04, C07D 307/00, C07D 307/02, C07D 307/77, C07D 333/00, A61K 31/335, A61K 31/38, A61K 31/558, A61P 35/00

(54) **TDG INHIBITOR COMPOUND AND USE THEREOF**

(30) Priority: 28.12.2023 CN 202311838444
(71) Applicant: Cytosinlab Therapeutics Co., Ltd., Hangzhou, Zhejiang 310020 (CN)
(72) Inventor: WU, Haiping, Hangzhou, Zhejiang 310020 (CN); MI, Yuan, Hangzhou, Zhejiang 310020 (CN); WANG, Meng, Hangzhou, Zhejiang 310020 (CN); GUO, Jiannan, Hangzhou, Zhejiang 310020 (CN); FU, Xingnian, Hangzhou, Zhejiang 310020 (CN); WANG, Long, Hangzhou, Zhejiang 310020 (CN); LIU, Yilin, Hangzhou, Zhejiang 310020 (CN); SHI, Hui, Hangzhou, Zhejiang 310020 (CN)
(74) Representative: Lavoix
(86) International application number: PCT/CN2024/143412
(87) International publication number: WO 2025/140637

(57) **Abstract**

The present invention provides a class of compounds that inhibit TDG activity. Specifically, the present invention provides a class of compounds having a novel structure as shown in Formula I. The small molecule inhibitors of the present invention exhibit excellent inhibitory effects on TDG.

## Description

### Technical Field

The present invention belongs to the field of medicinal chemistry, and specifically relates to TDG activity inhibitory compounds, as well as preparation and use thereof.

### Background of Invention

Human thymine DNA glycosylase (TDG), as a DNA glycosylase, can mediate DNA repair through the DNA base-excision repair (BER) pathway. When G·T or G·U mismatches occur in the DNA double-strand, TDG can recognize the mismatched bases, hydrolyze the N-glycosidic bond of U or T, forming an apurinic/apyrimidinic site (AP site). TDG can also promote the binding of apurinic/apyrimidinic endonuclease 1 (APE1) to the AP site, cleaving the phosphodiester bond at the 5' end of the abasic deoxynucleic acid, and other proteins and bases involved in repair are then added to ultimately repair the DNA.

TDG is a key enzyme in the DNA active demethylation pathway mediated synergistically by oxidation and the BER pathway. In the TET-TDG mediated DNA active demethylation mechanism, 5-methylcytosine (5mC) is oxidized to 5-carboxylcytosine (5caC) by a class of TET (ten-eleven-translocation protein) dioxygenases. TDG enzyme specifically recognizes and excises 5caC, thereby initiating the BER pathway to replace the original 5mC with cytosine, thus achieving DNA demethylation. DNA methylation is an important epigenetic modification method. TDG participates in epigenetic regulation by mediating DNA active demethylation and plays an important role in gene expression regulation, embryonic growth and development, hematopoietic stem cell differentiation, and other aspects.

TDG can promote tumorigenesis and may become a potential target for cancer therapy. Studies have shown that TDG is overexpressed in some human colorectal carcinomas (CRC). TDG acts as a positive regulator of the WNT signaling by serving as an adaptor protein for the transcription factor TCF4 and recruiting CBP/p300. Stable transfection of TDG shRNA into several CRC cell lines inhibited cell growth. Importantly, stable knockdown of TDG expression reduced the tumorigenic ability of CRC cells in xenograft assays, indicating that TDG is essential for CRC cell proliferation in vivo. Furthermore, knockdown of TDG can inhibit tumor formation of melanoma cell lines in xenograft models, suggesting that TDG activity is crucial for tumor initiation and/or progression. Therefore, developing specific small molecule inhibitors targeting TDG is very necessary and significant.

### Summary of the Invention

The purpose of the present invention is to provide an efficient small molecule inhibitor targeting TDG.

In the first aspect, the present invention provides a compound of Formula 1, or a deuterated derivative, stereoisomer, tautomer, or pharmaceutically acceptable salt thereof: 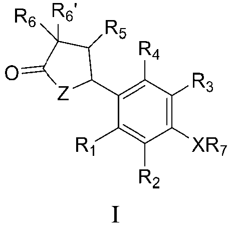
R₁ is selected from the group consisting of: OH, H, halogen, cyano, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted C₆-C₁₀ aryl, substituted or unsubstituted 3-12 membered heterocycle, substituted or unsubstituted 5-12 membered heteroaromatic ring, substituted or unsubstituted -O-3-12 membered heterocycle, substituted or unsubstituted -C₁-C₆ alkylphenyl, substituted or unsubstituted -O-phenyl, substituted or unsubstituted C₁-C₄ alkyl-C(O)-, substituted or unsubstituted C₁-C₄ alkyl-S(O)₂-, substituted or unsubstituted C₁-C₆ alkyl-NH-, (substituted or unsubstituted C₁-C₆ alkyl)₂-N-, -O(CH₂)ₛR₁₀, and -S(CH₂)ₛR₁₀; s is 0, 1, 2, or 3; R₁₀ is selected from the group consisting of: H, substituted or unsubstituted C₃₋₈ carbocycle, substituted or unsubstituted 3-8 membered heterocycle, substituted or unsubstituted C₆-C₁₀ aryl, and substituted or unsubstituted 5-12 membered heteroaromatic ring;
each R₂ is independently selected from the group consisting of: H, halogen, and substituted or unsubstituted C₁-C₆ alkyl;
R₃ is selected from the group consisting of: H, halogen, cyano, substituted or unsubstituted C₁-C₆ alkyl, substituted or unsubstituted C₁-C₆ alkoxy, substituted or unsubstituted C₃-C₁₂ cycloalkyl, or (C₁-C₆ alkyl)C(O)R₈, (C₁-C₆ alkyl)C(O)NHR₈, (C₁-C₆ alkyl)C(O)N(substituted or unsubstituted C₁-C₆ alkyl)R₈, (C₁-C₆ alkyl)C(O)OR₈; said R₈ is selected from the group consisting of: H, substituted or unsubstituted C₁-C₆ alkyl, substituted or unsubstituted C₆-C₁₀ aryl, -(OCH₂CH₂)ₘ-substituted or unsubstituted C₁-C₆ alkyl, or substituted or unsubstituted groups selected from the group consisting of: - (CH₂)ₘNHC(O)(CH₂)ₙR₁₃, -(CH₂)CHR₉NHC(O)(CH₂)ₙR₁₃, CHR₉(CH₂)NHC(O)(CH₂)ₙR₁₃; said R₉ is selected from the group consisting of: H, -COOH, -CONHR₁₂, -CONHCH₂R₁₂, - CONH(CH₂CH₂O)ₘ(CH₂)ₙCOOH, substituted or unsubstituted C₁-C₆ alkoxy, substituted or unsubstituted C₆-C₁₀ aryl, substituted or unsubstituted C₃-C₁₀ carbocycle, substituted or unsubstituted 3-12 membered heterocycle, and substituted or unsubstituted 5-12 membered heteroaromatic ring; R₁₂ is selected from the group consisting of: substituted or unsubstituted C₁-C₆ alkyl, substituted or unsubstituted C₃-C₈ cycloalkyl, substituted or unsubstituted C₆-C₁₀ aryl, and substituted or unsubstituted 5-12 membered heteroaromatic ring; said R₁₃ is selected from the group consisting of: substituted or unsubstituted C₃-C₁₀ carbocycle;
m and n are each independently 0, 1, 2, or 3;
R₄ is selected from the group consisting of H, halogen, cyano, substituted or unsubstituted C₁-C₆ alkoxy, substituted or unsubstituted C₁-C₆ amino, substituted or unsubstituted C₆-C₁₀ aryl, substituted or unsubstituted C₃-C₈ cycloalkyl, substituted or unsubstituted 3-12 membered heterocycle, substituted or unsubstituted 5-12 membered heteroaromatic ring, and substituted or unsubstituted -O-5-12 membered heteroaromatic ring,
or, R₃ and R₄ together with the carbon atoms to which they are attached form a structure selected from the group consisting of a substituted or unsubstituted C6-C10 aromatic ring, a substituted or unsubstituted 5-10 membered heteroaromatic ring, a substituted or unsubstituted C3-C8 carbocycle, and a substituted or unsubstituted 3-10 membered heterocycle;
R₅ and R₆ are each independently selected from the group consisting of H, substituted or unsubstituted C₁-C₆ alkyl, substituted or unsubstituted C6-C10 aromatic ring, substituted or unsubstituted 5-10 membered heteroaromatic ring, substituted or unsubstituted C3-C8 carbocycle, and substituted or unsubstituted 3-10 membered heterocycle; or said R₅ and R₆ together with the carbon atom to which they are attached form a substituted or unsubstituted 3-12 membered carbocycle;
R₆' is selected from the group consisting of: H, and substituted or unsubstituted C₁-C₆ alkyl; or R₆ and R₆' together form =CH₂;
X is selected from O or S;
Z is selected from O, S, or NR₁₄; wherein R₁₄ is H or C₁-C₄ alkyl;
R₇ is selected from the group consisting of: H, substituted or unsubstituted C₁-C₁₂ alkyl, C(O)R₁₁, C(O)OR₁₁, -CH₂OC(O)OR₁₁, and -S(O)₂NHR₁₁;
said R₁₁ is selected from the group consisting of: H, substituted or unsubstituted C₁-C₁₆ alkyl, substituted or unsubstituted C₆-C₁₀ aryl, -(OCH₂CH₂)ₘ-substituted or unsubstituted C₁-C₆ alkyl, substituted or unsubstituted 5-8 membered heterocyclyl;
unless otherwise specified, in the above formulas, said heteroaromatic ring, heteroaryl, heterocycle, or heterocyclyl contains 1, 2, or 3 heteroatoms selected from N, S, or O; said aromatic ring, aryl, heteroaromatic ring, or heteroaryl can be monocyclic or fused ring; said carbocycle, cycloalkyl, heterocycle, or heterocyclyl can be monocyclic, fused, bridged, or spiro; said carbocycle, heterocycle, or heterocyclyl can be saturated or partially unsaturated, but is not aromatic.

Said "substituted" means that a hydrogen atom on the corresponding group is replaced by one or more substituents selected from the group consisting of deuterium, halogen, hydroxy, carboxy, mercapto, benzyl, C₂-C₁₂ alkoxycarbonyl, C₁-C₆ aldehyde, (C₁-C₆ alkyl)₃Si, amino, C₁-C₆ amido, nitro, cyano, unsubstituted or halogenated C₁-C₆ alkyl, C₂-C₁₀ alkenyl, C₁-C₆ alkoxy, C₃-C₆ cycloalkyl, C₁-C₆ alkyl-amino, C₁-C₁₂ alkylaminocarbonyl, unsubstituted or halogenated C₂-C₁₀ acyl, unsubstituted or halogenated C₁-C₄ alkyl-S(O)₂-, unsubstituted or C₁-C₄ alkyl-substituted C₁-C₄ alkyl-OC(O)NH-, unsubstituted or halogenated C₁-C₄ alkyl-SO-, unsubstituted or C₁-C₄ alkyl-substituted 5-7 membered heterocycle, and phenyl (which may have 1-5 substituents selected from halogen, C₁-C₄ alkyl, C₁-C₄ alkoxy).

In another preferred embodiment, said R₁ is selected from the group consisting of: halogen, substituted or unsubstituted C₁-C₆ alkoxy, substituted or unsubstituted C₆-C₁₀ aryl, substituted or unsubstituted 3-12 membered heterocycle, substituted or unsubstituted 5-12 membered heteroaromatic ring, substituted or unsubstituted -O-3-12 membered heterocycle, substituted or unsubstituted C₁-C₄ alkyl-S(O)₂-, substituted or unsubstituted C₁-C₆ alkyl-NH-, (substituted or unsubstituted C₁-C₆ alkyl)₂-N-, and -O(CH₂)ₛR₁₀.

In another preferred embodiment, the compound of Formula I has the structure of Formula II below: 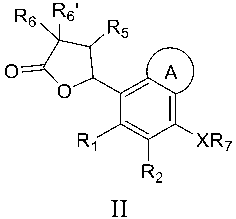 wherein ring A is a substituted or unsubstituted C6-C10 aromatic ring, a substituted or unsubstituted 5-10 membered heteroaromatic ring, a substituted or unsubstituted C3-C8 carbocycle, or a substituted or unsubstituted 3-10 membered heterocycle; preferably, ring A is a substituted or unsubstituted benzene ring, or a substituted or unsubstituted 5-7 membered heteroaromatic ring.

In another preferred embodiment, the compound has the structure of Formula IV below: 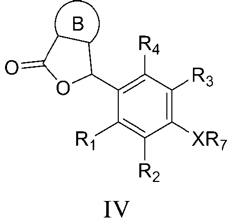 wherein ring B is a substituted or unsubstituted C3-C8 carbocycle, or a substituted or unsubstituted 3-10 membered heterocycle; preferably, ring B is a substituted or unsubstituted C3-C6 carbocycle, or a substituted or unsubstituted 3-8 membered heterocycle; wherein said carbocycle or heterocycle may be saturated or partially unsaturated.

In another preferred embodiment, the compound has the structure of Formula V below: 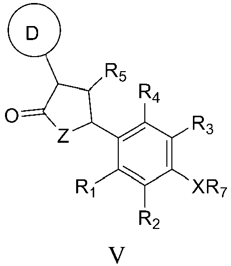 wherein ring D is a substituted or unsubstituted C6-C10 aromatic ring, a substituted or unsubstituted 5-10 membered heteroaromatic ring, a substituted or unsubstituted C3-C8 carbocycle, or a substituted or unsubstituted 3-10 membered heterocycle.

In another preferred embodiment, the compound has the structure of Formula VI or VII below: 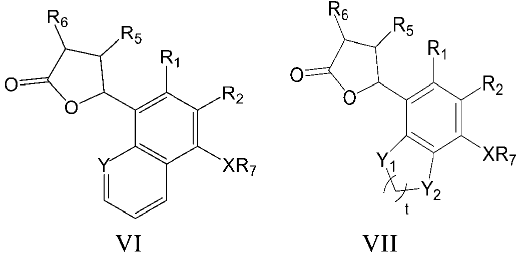 wherein Y is N or CH; Y₁ and Y₂ are each independently selected from the group consisting of CHR₁₅, NR₁₅, O and S; t is 1 or 2; wherein said R₁₅ is selected from the group consisting of: deuterium, halogen, hydroxy, carboxy, mercapto, amino, nitro, cyano, unsubstituted or halogenated C₁-C₆ alkyl, and C₁-C₆ alkoxy.

In another preferred embodiment, R₃ is selected from the group consisting of: H, halogen, substituted or unsubstituted C₁-C₆ alkyl, substituted or unsubstituted C₃-C₈ cycloalkyl, substituted or unsubstituted phenyl.

In another preferred embodiment, R₅ and R₆ are each independently selected from the group consisting of: H, substituted or unsubstituted C₁-C₆ alkyl; or said R₅ and R₆ together with the carbon atom to which they are attached form a substituted or unsubstituted 3-6 membered carbocycle.

In another preferred embodiment, R₃ is selected from the group consisting of: H, halogen, cyano, substituted or unsubstituted C₁-C₆ alkyl, substituted or unsubstituted C₃-C₁₂ cycloalkyl, or (C₁-C₆ alkyl)C(O)R₈, (C₁-C₆ alkyl)C(O)NHR₈, (C₁-C₆ alkyl)C(O)N(substituted or unsubstituted C₁-C₆ alkyl)R₈, (C₁-C₆ alkyl)C(O)OR₈; said R₈ is selected from the group consisting of: H, substituted or unsubstituted C₁-C₆ alkyl, substituted or unsubstituted C₆-C₁₀ aryl, -(OCH₂CH₂)ₘ-substituted or unsubstituted C₁-C₆ alkyl, and a substituted or unsubstituted group selected from the group consisting of: - (CH₂)ₘNHC(O)(CH₂)ₙR₁₃, -(CH₂)CHR₉NHC(O)(CH₂)ₙR₁₃, and CHR₉(CH₂)NHC(O)(CH₂)ₙR₁₃; said R₉ is selected from the group consisting of: H, -COOH, -CONHR₁₂, -CONHCH₂R₁₂, -CONH(CH₂CH₂O)ₘ(CH₂)ₙCOOH, substituted or unsubstituted C₁-C₆ alkoxy, substituted or unsubstituted C₆-C₁₀ aryl, substituted or unsubstituted C₃-C₁₀ carbocycle, substituted or unsubstituted 3-12 membered heterocycle, substituted or unsubstituted 5-12 membered heteroaromatic ring; R₁₂ is selected from the group consisting of: substituted or unsubstituted C₁-C₆ alkyl, substituted or unsubstituted C₃-C₈ cycloalkyl, substituted or unsubstituted C₆-C₁₀ aryl, and substituted or unsubstituted 5-12 membered heteroaromatic ring; said R₁₃ is selected from the group consisting of: substituted or unsubstituted C₃-C₁₀ carbocycle;
m and n are each independently 0, 1, 2, or 3;
R₄ is selected from the group consisting of: H, halogen, substituted or unsubstituted C₁-C₆ alkoxy, and substituted or unsubstituted -O-5-12 membered heteroaromatic ring;
or, R₃ and R₄ together with the carbon atoms to which they are attached form a substituted or unsubstituted C6-C10 aromatic ring, or a substituted or unsubstituted 5-10 membered heteroaromatic ring.

In another preferred embodiment, the compound has the structure of Formula III below: 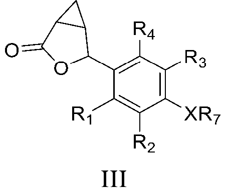
preferably, the compound has the structure of Formula III-A or III-B below: 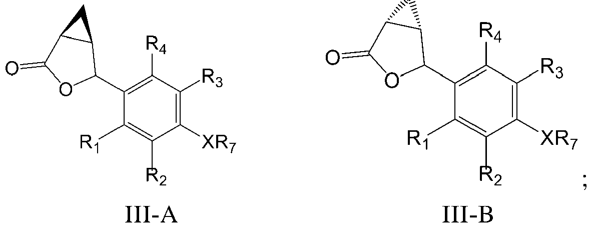
preferably, the compound has any one structure selected from the group consisting of:

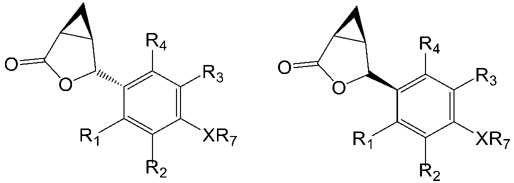

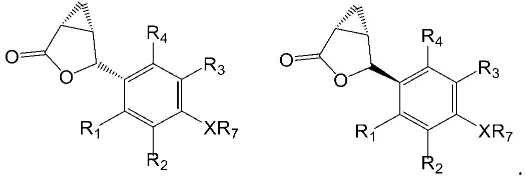

In another preferred embodiment, the compound has the structure of Formula IX below: 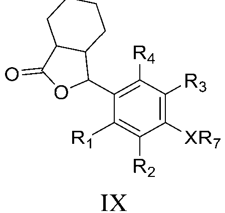
preferably, the compound has the structure of Formula IX-A or IX-B below: 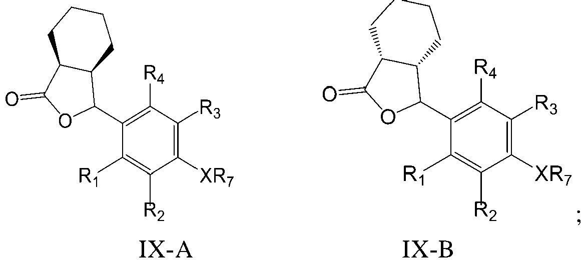
preferably, the compound has any one structure selected from the group consisting of:

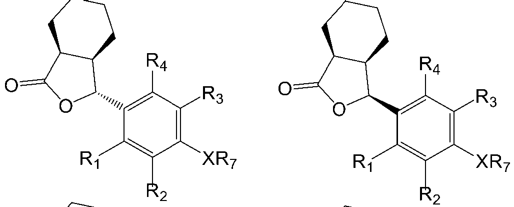

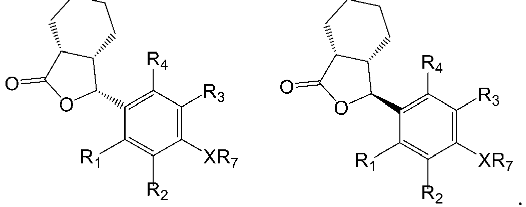

In another preferred embodiment, the compound has the structure of Formula X below: 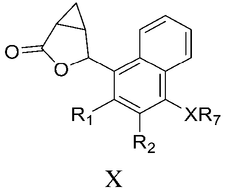
preferably, the compound has the structure of Formula X-A or X-B below: 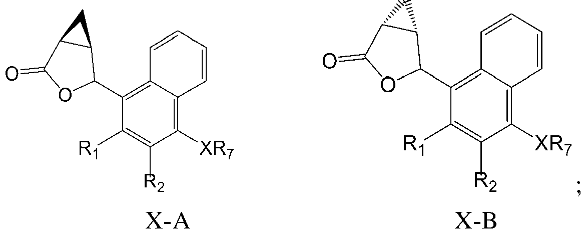
preferably, the compound has any one structure selected from the group consisting of:

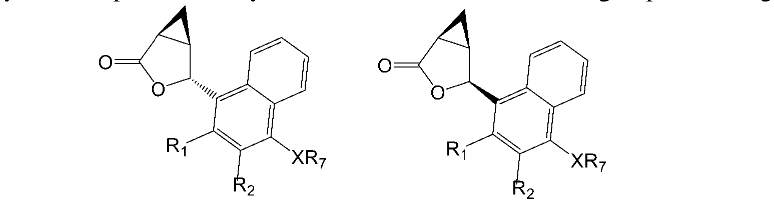

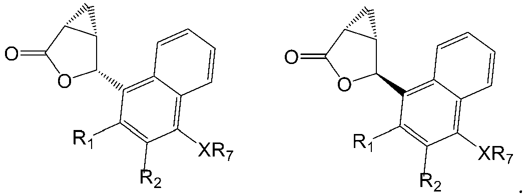

In the second aspect, the present invention provides a pharmaceutical composition, comprising (i) the compound according to the first aspect and (ii) a pharmaceutically acceptable carrier.

In the third aspect, the present invention provides use of the compound according to the first aspect, or a deuterated derivative, stereoisomer, tautomer, or pharmaceutically acceptable salt thereof, or the pharmaceutical composition according to the second aspect, for the preparation of a medicament for the treatment and/or prevention of diseases related to abnormal TDG expression.

In another preferred embodiment, the disease related to abnormal TDG expression is a tumor.

In another preferred embodiment, the tumor is selected from the group consisting of: lung cancer, acute leukemia, chronic leukemia, colorectal cancer, breast cancer, thyroid tumor, lymphoma, cholangiocarcinoma, liver cancer, pancreatic cancer, bronchial cancer, esophageal cancer, skin cancer, oral cancer, gastric cancer, urogenital tract tumors, central and peripheral nervous system tumors, and a combination thereof.

In another preferred embodiment, the tumor is selected from the group consisting of: melanoma, acute myeloid leukemia, small cell lung cancer, non-small cell lung cancer.

In the fourth aspect, the present invention provides a method for inhibiting TDG activity, said method comprises the step of:
contacting a subject with an effective amount of the compound according to the first aspect, or a stereoisomer or tautomer thereof, or a pharmaceutically acceptable salt, hydrate, crystalline form, or solvate thereof, or the pharmaceutical composition according to the second aspect, thereby inhibiting TDG activity.

In another preferred embodiment, the method is in vitro, non-therapeutic, and non-diagnostic.

In the fifth aspect, the present invention provides a method for treating and/or preventing a disease associated with TDG overexpression, said method comprises the step of:
administering to a subject an effective amount of the compound according to the first aspect, or a salt or isomer thereof, or the pharmaceutical composition according to the second aspect.

It should be understood that within the scope of the present invention, the above-mentioned technical features of the present invention and the technical features specifically described in the following description (e.g., in the Examples) can be combined with each other to constitute new or preferred technical solutions. Due to space limitations, they are not listed here individually.

### Description of the Drawings

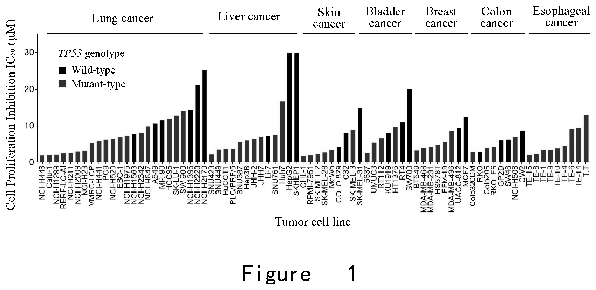

Figure 1 shows the IC₅₀ values of compound 14 for inhibiting the in vitro proliferation of p53 mutant and wild-type tumor cell lines.

### Detailed Description

The inventors, through extensive and in-depth research, have unexpectedly discovered for the first time a class of compounds with TDG inhibitory activity. The present invention was completed on this basis.

### Terms

In the present invention, halogen is F, Cl, Br, or I.

In the present invention, unless otherwise specified, the terms used have the general meanings known to those skilled in the art. In the present invention, unless otherwise specified, all chemical formulas are intended to cover any possible optical or geometric isomers (e.g., R-form, S-form, or racemates, or cis-trans isomers of alkenes, etc.).

In the present invention, the term "C1-C6 alkyl" refers to a straight or branched chain alkyl group having 1 to 6 carbon atoms, including, but not limited to, methyl, ethyl, propyl, isopropyl, butyl, isobutyl, sec-butyl, tert-butyl, pentyl, and hexyl; preferably ethyl, propyl, isopropyl, butyl, isobutyl, sec-butyl, and tert-butyl.

In the present invention, the term "C1-C6 alkoxy" refers to a straight or branched chain alkoxy group having 1 to 6 carbon atoms, including, but not limited to, methoxy, ethoxy, propoxy, isopropoxy, and butoxy.

In the present invention, the term C₁-C₆ amino refers to "C₁-C₆ alkyl-NH-" and "(C₁-C₆ alkyl)₂N-", or similar structures thereof.

In the present invention, the term "C2-C6 alkenyl" refers to a straight or branched chain alkenyl group having 2 to 6 carbon atoms and containing one double bond, including, but not limited to, vinyl, propenyl, butenyl, isobutenyl, pentenyl, and hexenyl.

In the present invention, the term "C2-C6 alkynyl" refers to a straight or branched chain alkynyl group having 2 to 6 carbon atoms and containing one triple bond, including, but not limited to, ethynyl, propynyl, butynyl, isobutynyl, pentynyl, and hexynyl.

In the present invention, the term "C3-C10 cycloalkyl" refers to a cyclic alkyl group having 3 to 10 carbon atoms in the ring, including, but not limited to, cyclopropyl, cyclobutyl, cyclopentyl, cyclohexyl, cycloheptyl, cyclooctyl, and cyclodecyl. The terms "C3-C8 cycloalkyl", "C3-C7 cycloalkyl", and "C3-C6 cycloalkyl" have similar meanings.

In the present invention, the term "C1-C12 alkoxycarbonyl" refers to an alkoxycarbonyl group having 1 to 12 carbon atoms in the alkyl chain, including, but not limited to, methoxycarbonyl, ethoxycarbonyl, propoxycarbonyl, isopropoxycarbonyl, tert-butoxycarbonyl, benzyloxycarbonyl, etc.

In the present invention, the term "C1-C12 alkylaminocarbonyl" refers to an alkylaminocarbonyl group having 1 to 12 carbon atoms in the alkyl chain, including, but not limited to, methylaminocarbonyl, ethylaminocarbonyl, propylaminocarbonyl, isopropylaminocarbonyl, tert-butylaminocarbonyl, benzylaminocarbonyl, dimethylaminocarbonyl, etc.

In the present invention, the terms "aromatic ring" and "aryl" have the same meaning, preferably "aryl" is "C6-C12 aryl" or "C6-C10 aryl". The term "C6-C12 aryl" refers to an aromatic ring group having 6 to 12 carbon atoms and containing no heteroatoms in the ring, such as phenyl, naphthyl, etc. The term "C6-C10 aryl" has a similar meaning.

In the present invention, the terms "heteroaromatic ring", "heteroaryl ring" or "heteroaryl" have the same meaning and refer to a heteroaromatic group containing one or more heteroatoms. The heteroatoms referred to herein include oxygen, sulfur, and nitrogen. For example, furyl, thienyl, pyridyl, pyrazolyl, pyrrolyl, N-alkylpyrrolyl, pyrimidinyl, pyrazinyl, imidazolyl, tetrazolyl, etc. The heteroaromatic ring may be fused to an aryl, heterocyclyl, or cycloalkyl ring, wherein the ring connected to the parent structure is the heteroaromatic ring. Heteroaryl groups may be optionally substituted or unsubstituted.

In the present invention, the term "3-12 membered heterocyclyl" refers to a saturated or unsaturated 3-12 membered cyclic group containing 1 to 3 heteroatoms selected from oxygen, sulfur, and nitrogen in the ring, such as dioxolanyl, etc. The term "3-7 membered heterocyclyl" has a similar meaning.

In the present invention, the term "substituted" means that one or more hydrogen atoms on a specific group are replaced by specific substituents. The specific substituents are those described in the preceding text, or those appearing in the various examples. Unless otherwise specified, a substituted group may have a substituent selected from a specific group at any substitutable position on the, and the substituents at each position may be the same or different. Cyclic substituents, such as heterocycloalkyl, may be attached to another ring, such as cycloalkyl, to form a spiro bicyclic system, for example, two rings share a common carbon atom. Those skilled in the art will understand that the combinations of substituents contemplated by the present invention are those that are stable or chemically achievable. Examples of such substituents include, but are not limited to: C1-8 alkyl, C2-8 alkenyl, C2-8 alkynyl, C3-8 cycloalkyl, 3- to 12-membered heterocyclyl, aryl, heteroaryl, halogen, hydroxy, carboxy (-COOH), C1-8 aldehyde, C2-10 acyl, C2-10 ester, C1-C12 alkoxycarbonyl, amino, alkoxy, C1-10 sulfonyl, etc.

### Compounds of Formula (I) as TDG Inhibitors

The present invention provides a class of compounds having TDG inhibitory activity: 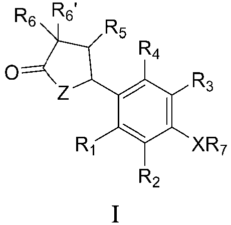 wherein each group has the definition as described above.

### Pharmaceutical Compositions and Methods of Administration

Since the compounds of the present invention have excellent biological activity, the compounds of the present invention and their various crystalline forms, pharmaceutically acceptable inorganic or organic salts, hydrates or solvates, as well as pharmaceutical compositions containing the compounds of the present invention as the main active ingredient, can be used for the treatment, prevention, and alleviation of related diseases caused by abnormal TDG activity or expression levels.

The pharmaceutical composition of the present invention comprises the compound of the present invention or a pharmacologically acceptable salt thereof in an amount within a safe and effective range, and a pharmacologically acceptable excipient or carrier. Wherein "safe and effective amount" refers to: an amount of the compound sufficient to significantly improve the condition without causing serious side effects. Typically, the pharmaceutical composition contains 1-2000 mg of the compound of the present invention per dose, more preferably, 5-200 mg of the compound of the present invention per dose. Preferably, said "one dose" is a capsule or tablet.

"Pharmaceutically acceptable carrier" refers to: one or more compatible solid or liquid fillers or gel substances, which are suitable for human use and must be of sufficient purity and sufficiently low toxicity. "Compatible" here refers to that the components of the composition can be mixed with the compound of the present invention and with each other, without significantly reducing the efficacy of the compound. Some examples of pharmaceutically acceptable carriers are cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agents (such as sodium lauryl sulfate), colorants, flavoring agents, stabilizers, antioxidants, preservatives, pyrogen-free water, etc.

The mode of administration of the compound or pharmaceutical composition of the present invention is not particularly limited, and representative modes of administration include, but are not limited to: oral, intratumoral, rectal, parenteral (intravenous, intramuscular, or subcutaneous), and topical administration.

Solid dosage forms for oral administration include capsules, tablets, pills, powders, and granules. In these solid dosage forms, the active compound is mixed with at least one conventional inert excipient (or carrier), such as sodium citrate or dicalcium phosphate, or with the following components: (a) fillers or extenders, for example, starch, lactose, sucrose, glucose, mannitol, and silicic acid; (b) binders, for example, hydroxymethyl cellulose, alginates, gelatin, polyvinylpyrrolidone, sucrose, and acacia; (c) humectants, for example, glycerol; (d) disintegrating agents, for example, agar, calcium carbonate, potato starch or tapioca starch, alginic acid, certain complex silicates, and sodium carbonate; (e) retarding agents, for example, paraffin; (f) absorption enhancers, for example, quaternary ammonium compounds; (g) wetting agents, for example, cetyl alcohol and glycerol monostearate; (h) adsorbents, for example, kaolin; and (i) lubricants, for example, talc, calcium stearate, magnesium stearate, solid polyethylene glycol, sodium lauryl sulfate, or mixtures thereof. In capsules, tablets, and pills, the dosage form may also contain buffering agents.

Solid dosage forms such as tablets, dragees, capsules, pills, and granules can be prepared with coatings and shells, such as enteric coatings and other materials well known in the art. They may contain opacifying agents, and the active compound or compounds in such compositions may be released in a delayed manner in a certain part in a certain part of the digestive tract. Examples of encapsulating components that can be used are polymeric substances and waxes. If necessary, the active compound can also be formulated into microcapsules with one or more of the above-mentioned excipients.

Liquid dosage forms for oral administration include pharmaceutically acceptable emulsions, solutions, suspensions, syrups, or tinctures. In addition to the active compound, the liquid dosage forms may contain inert diluents conventionally employed in the art, such as water or other solvents, solubilizers, and emulsifiers, for example, ethanol, isopropanol, ethyl carbonate, ethyl acetate, propylene glycol, 1,3-butylene glycol, dimethylformamide, and oils, particularly cottonseed oil, peanut oil, corn germ oil, olive oil, castor oil, and sesame oil or mixtures of these substances, etc.

In addition to these inert diluents, the composition may also contain adjuvants, such as wetting agents, emulsifiers and suspending agents, sweeteners, flavoring agents, and perfumes.

In addition to the active compound, suspensions may contain suspending agents, for example, ethoxylated isostearyl alcohols, polyoxyethylene sorbitol and sorbitan esters, microcrystalline cellulose, aluminium methoxide, and agar or mixtures thereof, etc.

Compositions for parenteral injection may contain physiologically acceptable sterile aqueous or non-aqueous solutions, dispersions, suspensions, or emulsions, and sterile powders for reconstitution into sterile injectable solutions or dispersions. Suitable aqueous and non-aqueous carriers, diluents, solvents, or excipients include water, ethanol, polyols, and suitable mixtures thereof.

Dosage forms for topical administration of the compounds of the present invention include ointments, powders, patches, sprays, and inhalants. The active ingredient is mixed under sterile conditions with a physiologically acceptable carrier and any preservatives, buffers, or propellants that may be required.

The compounds of the present invention may be administered alone or in combination with other pharmaceutically acceptable compounds. In some preferred embodiments, the compounds of the present invention may form PROTACs in combination with other small molecule compounds, or be administered as ADCs in combination with other macromolecular compounds such as monoclonal antibodies.

The present invention is further elaborated below in conjunction with specific examples. It should be understood that these examples are only for illustrating the present invention and are not intended to limit the scope of the present invention. The experimental methods in the following examples where specific conditions are not indicated are usually carried out under conventional conditions, or under the conditions recommended by the manufacturer. Unless otherwise indicated, percentages and parts are by weight.

### Acronyms:

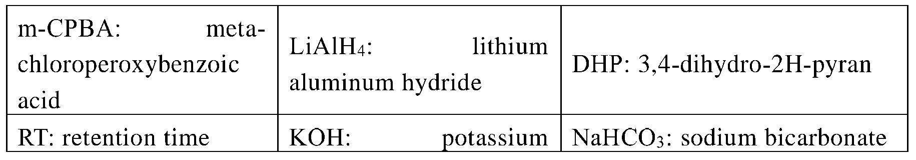

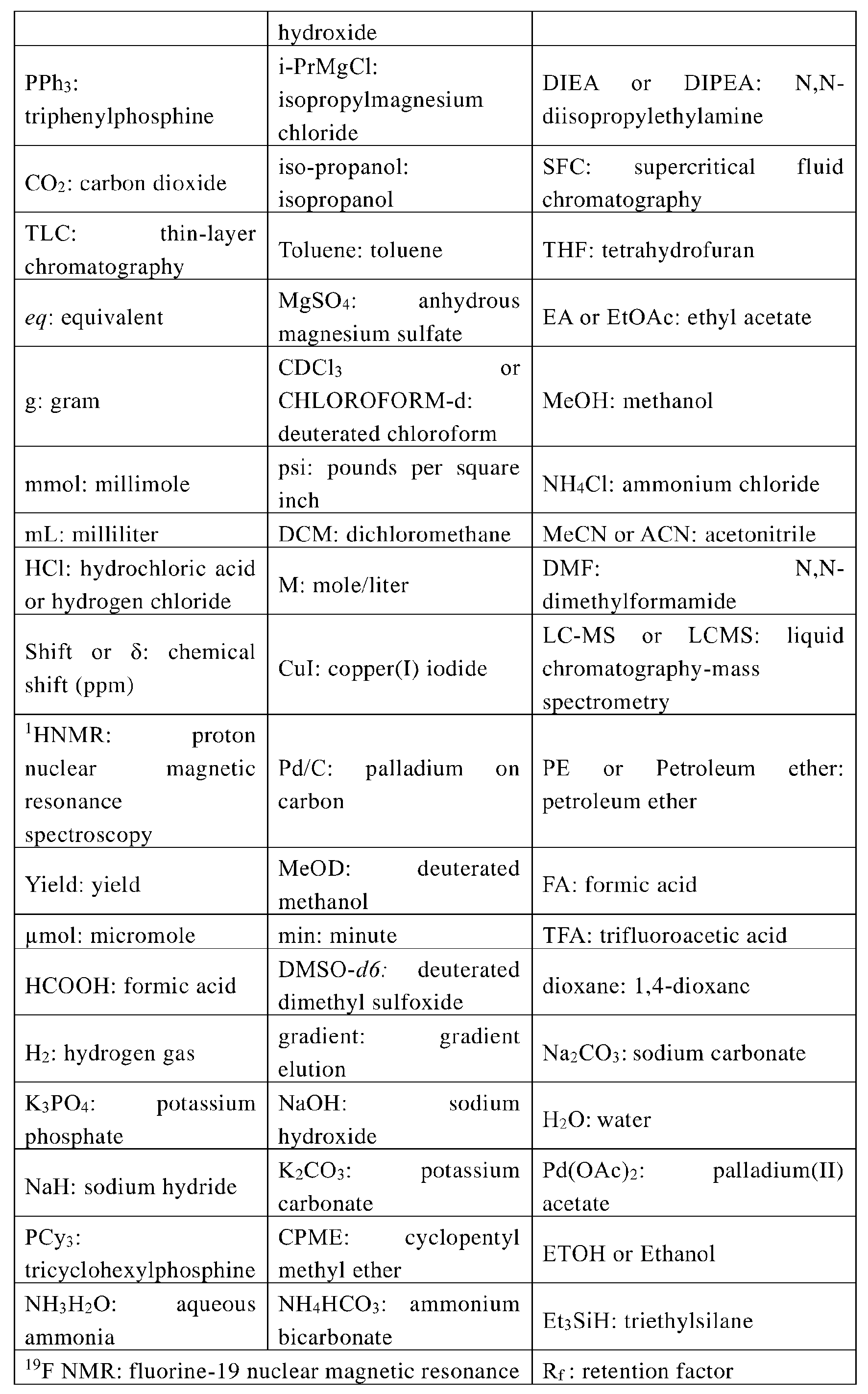

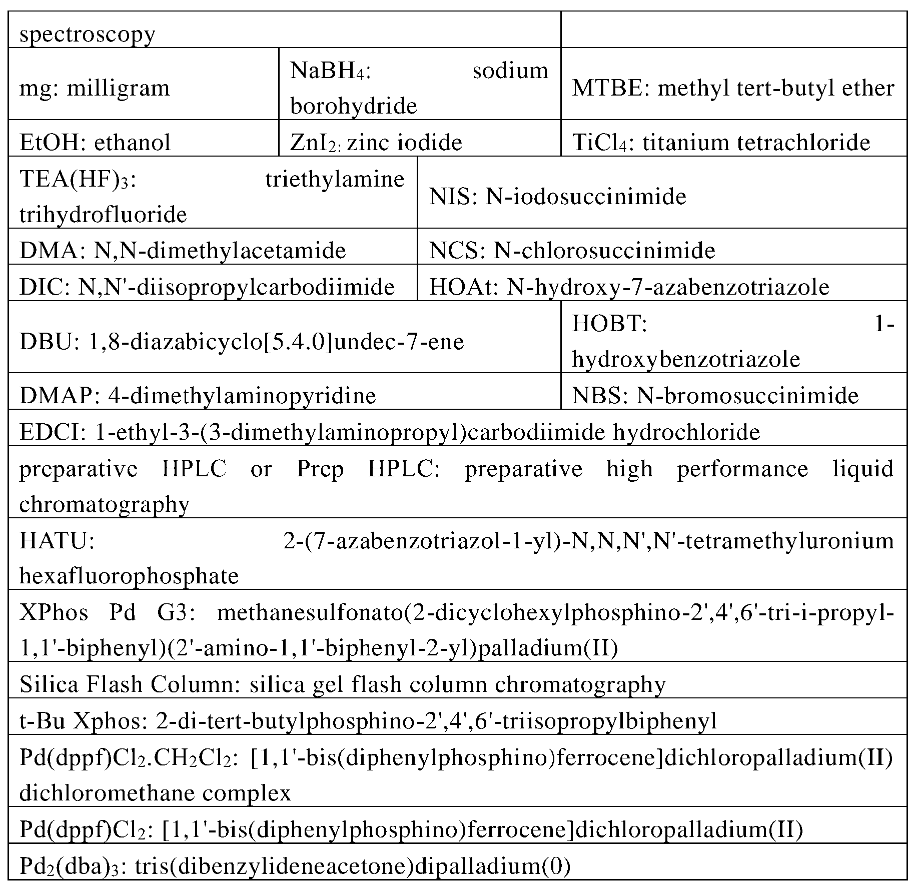

Starting materials can be obtained commercially or prepared by methods already known or disclosed in the art.

Purification of intermediates and compounds was carried out using normal-phase or reversed-phase chromatography or recrystallization and other conventional chemical experimental operations. Normal-phase chromatography used pre-packed silica gel columns or preparative thin-layer chromatography. Silica gel columns were mainly glass columns or flash column chromatography instruments, such as ISCO^{®} or other brands. The mobile phase for normal-phase chromatography was selected from petroleum ether/ethyl acetate, dichloromethane/methanol, or other suitable solvents and ratios for elution. Reversed-phase preparative liquid chromatography used C18 columns, performed on preparative liquid chromatographs or flash column chromatography instruments, detected at 214 nm and 254 nm or by preparative LC-MS, using water/acetonitrile containing 0.1% hydrochloric acid, water/acetonitrile, water/acetonitrile containing 0.1% ammonium bicarbonate, water/acetonitrile containing 0.1% formic acid, water/acetonitrile containing 0.1% ammonia water, water/acetonitrile containing 0.1% trifluoroacetic acid, or other suitable solvent systems as mobile phases for gradient elution.

Prep HPLC was performed on a Gilson preparative HPLC system (GX-281). Representative prep HPLC conditions include: 1. FA method: Column: Boston Prime C18 150*30mm*5um; mobile phase: [water (0.225%FA)-ACN]; 2. FA method -A: Phenomenex Gemini C18 250*50mm*10um; mobile phase: [water (0.225%FA)-ACN]; 3. FA method -B: Phenomenex C18 80*30mm*5um; mobile phase: [water(0.225FA)-ACN].

The conventional work-up procedure described herein is: combining the organic phases, drying the organic phase over anhydrous magnesium sulfate or anhydrous sodium sulfate, filtering, and concentrating the filtrate under reduced pressure to remove the solvent. The conventional work-up procedure 2 described herein is: combining the organic phases, washing the organic phase with saturated brine (in rare cases with 30% lithium chloride solution), drying over anhydrous magnesium sulfate or anhydrous sodium sulfate, filtering, and concentrating the filtrate under reduced pressure to remove the solvent. Unless otherwise specified, the reaction time indicated for the chemical reaction is the time when the reaction is complete or the starting material is completely consumed as shown by LCMS or TLC or other suitable detection methods.

Intermediate and compound structure characterization was performed using nuclear magnetic resonance (NMR) and liquid chromatography-mass spectrometry (LCMS). The NMR spectrometers used were Bruker Ascend TM-400 MHz or Bruker Ultrashield Plus 400 MHz or other models. The solvents used were deuterated dimethyl sulfoxide, deuterated chloroform, deuterated methanol, or other indicated deuterated solvents. Spectral data are reported in the format: chemical shift δ (peak splitting, coupling constant J (Hz), number of hydrogens). Tetramethylsilane was used as the internal standard for chemical shift, and its chemical shift was set to zero (δ, 0 ppm). The meanings of some abbreviations are: s (singlet), d (doublet), t (triplet), q (quartet), m (multiplet), br (broad).

Representative methods for liquid chromatography-mass spectrometry (LCMS) in intermediate and compound structure characterization are as follows:
LCMS was performed on an Agilent MSD mass spectrometer coupled to an Agilent 1260 system, with detection by UV (254 or 220 nm) and MS (ESI⁺).

Method 1: 5-95AB, Column: Agilent Pursult 5 C18 20*2.0mm, Solvent A: 0.0375% TFA in water, Solvent B: 0.1125% TFA in acetonitrile, gradient from 5% Solvent B to 95% Solvent B over 0.7 min, hold for 0.4 min, total 1.5 min; Flow rate: 1.5 mL/min; Column temperature: 50 °C.

Method 2: 5-95AB_3min_220&254_Agilent, Column: Agilent Poroshell 120 EC-C18 2.7um 3.0*30mm, Solvent A: 0.0375% TFA in water, Solvent B: 0.1125% TFA in acetonitrile, gradient from 5% Solvent B to 80% Solvent B over 1.2 min, then to 95% Solvent B over 1.3 min, hold for 0.5 min, total 3 min; Flow rate: 1.5 mL/min; Column temperature: 50 °C.

Method 3: 0-100AB_QC_220&254, Column: ACQUITY UPLC BEH C18 50*2.1mm,1.7um, Solvent A: 0.1% TFA in water, Solvent B: 0.1% TFA in acetonitrile, gradient from 0% Solvent B to 100% Solvent B over 1 min, hold for 0.2 min, total 1.5 min; Flow rate: 1.0 mL/min; Column temperature: 40 °C.

Method 4: 10-80AB_4min_220&254_Shimadzu, Column: Nano Chrom 120 C18 3.0*30mm, 3um, Solvent A: 0.0375% TFA in water, Solvent B: 0.1125% TFA in acetonitrile, gradient from 10% Solvent B to 80% Solvent B over 3 min, hold for 0.5 min, total 4 min; Flow rate: 0.8 mL/min; Column temperature: 50 °C.

Method 5: 5-95AB_1min_220&254_Agilent, Column: Agilent Poroshell 120 EC-C18 2.7um 3.0*30mm, Solvent A: 0.0375% TFA in water, Solvent B: 0.1125% TFA in acetonitrile, gradient from 5% Solvent B to 95% Solvent B over 0.4 min, hold for 0.3 min, total 1 min; Flow rate: 2 mL/min; Column temperature: 50 °C.

Method 6: 0-95AB_1.5min_220&254_Agilent, Column: Waters, Xbridge C18 30*2.1mm,3.5um, Solvent A: 0.0375% TFA in water, Solvent B: 0.1125% TFA in acetonitrile, gradient from 0% Solvent B to 95% Solvent B over 0.6 min, hold for 0.8 min, total 1.5 min; Flow rate: 1.2 mL/min; Column temperature: 40 °C.

Method 7: 10-80CD_3MIN_220&254_Shimadzu, Column: XBridge C18 3.5um 2.1*30mm, Solvent A: 0.025% aqueous aqueous ammonia, Solvent B: acetonitrile, gradient from 10% Solvent B to 80% Solvent B over 2 min, hold for 0.48 min, total 3 min; Flow rate: 1 mL/min; Column temperature: 50 °C.

Method 8: 10-80AB_7min_220&254_Shimadzu, Column: Xtimate C18 2.1*30mm,3um, Solvent A: 0.0375% TFA in water, Solvent B: 0.1125% TFA in acetonitrile, gradient from 10% Solvent B to 80% Solvent B over 6 min, hold for 0.5 min, total 7 min; Flow rate: 0.8 mL/min; Column temperature: 50 °C.

Representative procedure for catalytic hydrogenation: The compound was dissolved in a suitable solvent, such as EA or THF, and palladium on carbon (10% content, about 0.1 *eq*) or palladium hydroxide on carbon (20% content, about 0.1 *eq*) was added. The reaction mixture was thoroughly purged with hydrogen. The mixture was stirred at 25 °C under 15 psi hydrogen atmosphere until LCMS showed the reaction was complete. The reaction mixture was vacuum filtered, concentrated under reduced pressure to dryness, and the product was purified.

### Example 1: Synthesis of Compound 14, Method 1

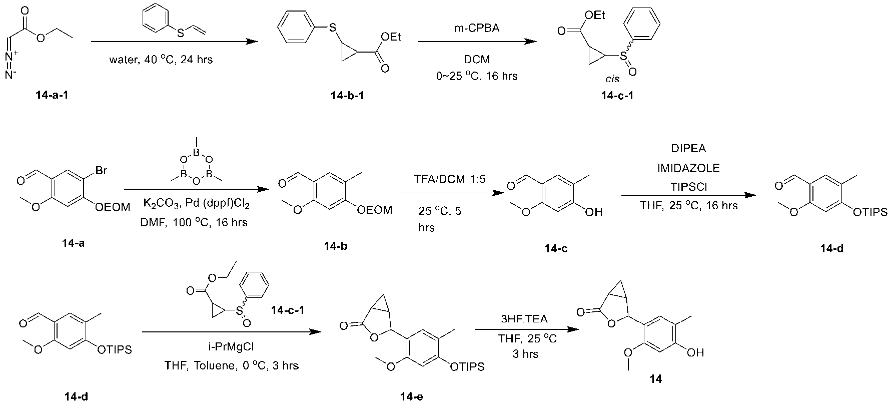

### Step 1:

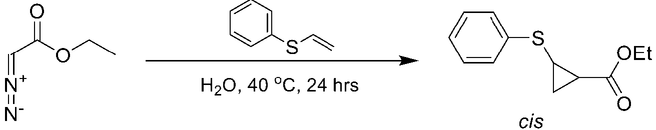

H₂O (25 mL), ethyl diazoacetate (**14-a-1**) (3.50 g, 24.54 mmol, 80% purity, 1 *eq*), and phenyl vinyl sulfide (5.01 g, 36.81 mmol, 1.5 *eq*) were added to a degassed and nitrogen-purged mixture of [(S,S)-(+)-N,N'-bis(3,5-di-tert-butylsalicylidene)-1,2-diaminocobalt] (CAS: 188264-84-8, 370 mg, 613.48 umol, 0.025 *eq*) and [(R,R)-(-)-N,N'-bis(3,5-di-tert-butylsalicylidene)-1,2-cyclohexanediaminocobalt] (CAS: 176763-62-5, 370 mg, 613.48 umol, 0.025 *eq*), and the mixture was stirred at 40 °C for 24 hours. TLC showed the reaction was completed, generating two products (PE: EA=8:1, Rf=0.4, 0.5). The mixture was cooled to 25 °C, degassed and purged with oxygen for several times, then stirred at 25 °C for 1 hour. The mixture was filtered through celite and washed with DCM (20 mL×2), and the aqueous phase was extracted with DCM (50 mL×2). The combined organic phases were dried over anhydrous magnesium sulfate, filtered, and concentrated under reduced pressure. The residue was purified by silica gel flash column chromatography (ISCO^{®}; 120 g SepaFlash^{®} Silica Flash Column, 0-0.1, 2, 3% EA/PE, 80 mL/min) to obtain **14-b-1** as a red oil (cis isomer, 3.08 g, 13.85 mmol, 56.46% yield). ¹H NMR (400 MHz, CDCl₃) Shift 7.35-7.42 (m, 2H), 7.26-7.30 (m, 2H), 7.13-7.21 (m, 1H), 4.07 (q, J=7.11 Hz, 2H), 2.72 (q, J=7.78 Hz, 1H), 2.22-2.35 (m, 1H), 1.45-1.53 (m, 2H), 1.12 (t, J=7.03 Hz, 3H).

### Step 2:

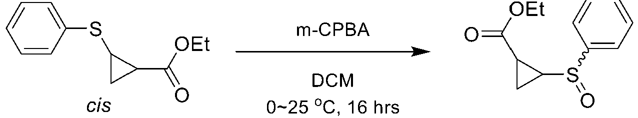

m-CPBA (21.9 g, 101.96 mmol, 80% purity, 1.48 *eq*) was added to a solution of compound (**14-b-1**) (15.3 g, 68.83 mmol, 1 *eq*) in DCM (400 mL) portionwise within 1 hour at 0 °C, and the mixture was stirred at 25 °C for 16 hours. After the reaction was completed, aqueous KOH solution (3M, 500 mL) was added. The aqueous phase was extracted with DCM (300 mL×2). The combined organic phases were washed with saturated brine (100 mL), dried over anhydrous magnesium sulfate, filtered, and concentrated under reduced pressure. The residue was purified by silica gel flash column chromatography (ISCO^{®}; 220 g SepaFlash^{®} Silica Flash Column, 50-80% EA/PE, 100 mL/min) to obtain **14-c-1** as a brown oil (9.8 g, 41.12 mmol, 59.75% yield).

(ESI) m/z=238.9 [M+H]⁺. ¹H NMR (400 MHz, CDCl₃) δ δ 7.62-7.72 (m, 2H), 7.50-7.55 (m, 3H), 4.16-4.28 (m, 2H), 2.62 (dt, J=6.60, 8.36 Hz, 1H), 2.14 (dt, J=6.49, 8.20 Hz, 1H), 2.03-2.11 (m, 1H), 1.64 (dt, J=5.72, 8.25 Hz, 1H), 1.31 (t, J=7.15 Hz, 3H).

### Step 3:

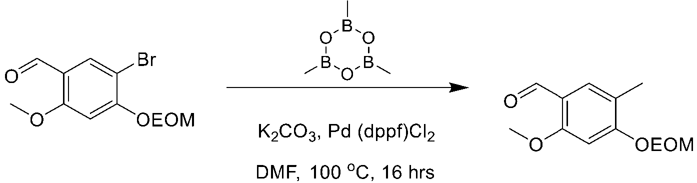

K₂CO₃ (2.29 g, 16.60 mmol, 3.0 *eq*) was added to a solution of compound (**14-a**) (1.60 g, 5.53 mmol, 1 *eq*) and trimethylboroxine (4.17 g, 16.60 mmol, 4.64 mL, 50% purity, 3.0 *eq*) in DMF (40 mL), and the mixture was purged with nitrogen several times. Pd(dppf)Cl₂ (202 mg, 276.70 umol, 0.05 *eq*) was added, and the mixture was stirred at 100 °C under a nitrogen atmosphere for 16 hours. After the reaction was completed, water (50 mL) was added, and the mixture was extracted with EtOAc (50 mL×3). The combined organic phases were dried over anhydrous magnesium sulfate, filtered, and concentrated under reduced pressure. The residue was purified by silica gel flash column chromatography (ISCO^{®}; 24 g SepaFlash^{®} Silica Flash Column, 0-25% EA/PE, 35 mL/min) to obtain **14-b** as a yellow solid (850 mg, 3.79 mmol, yield 68.49%). (ESI) m/z=225.1 (M+1) ⁺.

### Step 4:

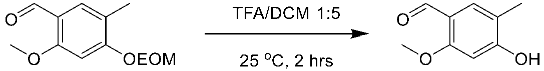

A solution of compound (**14-b**) (950 mg, 4.24 mmol, 1 *eq*) in DCM (10 mL) and TFA (2 mL) was stirred at 25 °C for 2 hours. After the reaction, the mixture was concentrated under reduced pressure to obtain **14-c** as a red oil (3.21 g, 13.47 mmol, 97.22% yield), which was used directly in the next step.

### Step 5:

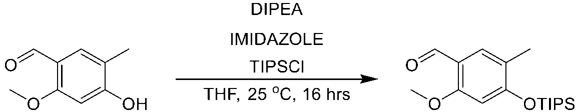

Imidazole (591 mg, 8.69 mmol, 2.0 *eq*) and DIEA (1.12 g, 8.69 mmol, 1.51 mL, 2.0 *eq*) were added to a solution of compound (**14-c**) (722 mg, 4.34 mmol, 1 *eq*) and triisopropylsilyl chloride (1.26 g, 6.52 mmol, 1.39 mL, 1.5 *eq*) in THF (10 mL), and the mixture was stirred at 25 °C for 2 hours. After the reaction, water (20 mL) was added, and the mixture was extracted with EtOAc (30 mL×3). The combined organic phases were dried over anhydrous magnesium sulfate, filtered, and concentrated under reduced pressure. The residue was purified by silica gel flash column chromatography (ISCO^{®}; 24 g SepaFlash^{®} Silica Flash Column, 0-10% EA/PE, 35 mL/min) to obtain **14-d** as a colorless oil (1.23 g, 3.81 mmol, 87.78% yield). ¹H NMR (400 MHz, CDCl₃) Shift 10.29 (s, 1H), 7.65 (s, 1H), 6.40 (s, 1H), 3.86 (s, 3H), 2.19 (s, 3H), 1.31-1.39 (m, 3H), 1.08 (s, 18H). (ESI) m/z=323.3 (M+1) ⁺.

### Step 6:

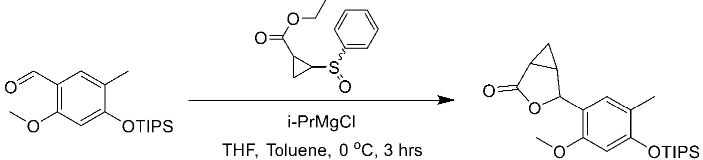

Isopropylmagnesium chloride solution (2 M, 2.00 mL, 1.5 *eq*) was added to a solution of compound (**14-c-1**) (635 mg, 2.67 mmol, 1 *eq*) in THF (25 mL) at -78 °C, and the mixture was stirred at -78 °C for 15 minutes. A solution of compound (**14-d**) (860 mg, 2.67 mmol, 1.0 *eq*) in toluene (3 mL) was added, and the mixture was stirred at 0 °C for 3 hours. After the reaction was completed, saturated aqueous NH₄Cl solution (20 mL) was added to quench, and the mixture was extracted with EtOAc (20 mL×3). The combined organic phases were dried over anhydrous sodium sulfate, filtered, and concentrated under reduced pressure. The residue was purified by silica gel flash column chromatography (ISCO^{®}; 24 g SepaFlash^{®} Silica Flash Column, 0-10% EA/PE, 35 mL/min) to obtain **14-e** as a colorless oil (220 mg, 563.25 umol, 21.12% yield). ¹H NMR (400 MHz, CDCl₃) 6.95-7.05 (m, 1H), 6.37-6.45 (m, 1H), 5.83 (d, J=4.77 Hz, 0.3H), 5.49 (s, 0.5H), 4.60 (s, 0.3H), 3.79-3.81 (m, 3H), 2.63-2.68 (m, 0.3H), 2.18-2.26 (m, 4.4H), 1.25-1.38 (m, 4H), 1.01-1.17 (m, 19H). (ESI) m/z= 391.2 (M+1) ⁺.

### Step 7:

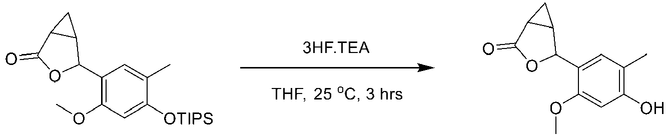

Triethylamine trihydrofluoride (59.4 mg, 368.68 umol, 60.09 uL, 0.8 *eq*) was added to a solution of compound (**14-e**) (180 mg, 460.84 umol, 1 *eq*) in THF (5 mL), and the mixture was stirred at 25 °C for 3 hours. After the reaction was completed, saturated aqueous NaHCO₃ solution (5 mL) was added to quench, and the mixture was extracted with EtOAc (5 mL×3). The combined organic phases were dried over anhydrous magnesium sulfate, filtered, and concentrated under reduced pressure. The residue was purified by silica gel flash column chromatography (ISCO^{®}; 12 g SepaFlash^{®} Silica Flash Column, 0-30% EA/PE, 35 mL/min) to obtain compound **14** as a pink solid (70 mg, 283.89 umol, 61.60% yield, 95% purity). (ESI) m/z=235.0 (M+1) ⁺.

According to steps 5 to 7 of the synthesis of compound **14** above, the following starting material was used to obtain the corresponding final product.

| Starting material | Final product | LCMS and NMR data |
|---|---|---|
| 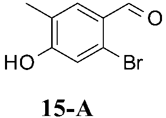 | 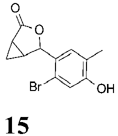 About 4.4% yield over three steps, white solid. | LCMS: (ESI) m/z=284.9(M+1)⁺; ¹H NMR (400 MHz, DMSO-d6) Shift 10.04 (br s, 1H), 6.99-7.11 (m, 2H), 5.74 (d, J=4.77 Hz, 1H), 5.47 (s, 1H), 2.58 (br s, 1H), 2.26-2.37 (m, 2H), 2.06-2.14 (m, 3H), 1.36 (m, 1H), 1.04-1.14 (m, 1H), 0.77-0.83 (m, 1H) |

### Example 2: Synthesis of Compound 14, Method 2

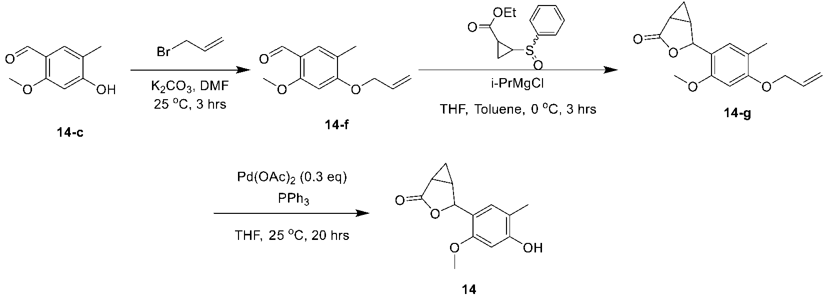

### Step 1:

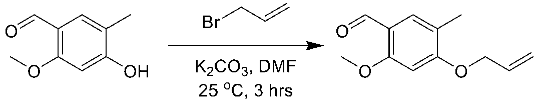

K₂CO₃ (12.5 g, 90.27 mmol, 3.0 *eq*) and 3-bromo-1-propene (4.73 g, 39.12 mmol, 1.3 *eq*) were added to a solution of compound (**14-c**) (5.0 g, 30.09 mmol, 1 *eq*) in DMF (75 mL), and the mixture was stirred at 25 °C for 3 hours. After the reaction, the mixture was filtered and concentrated under reduced pressure. The residue was purified by silica gel flash column chromatography (ISCO^{®}; 80 g SepaFlash^{®} Silica Flash Column, 0-10% EA/PE, 65 mL/min) to obtain **14-f** as a white solid (5.3 g, 25.70 mmol, 85.41% yield). ¹H NMR (400 MHz, CDCl₃) Shift 10.30 (s, 1H), 7.64 (d, J=0.61 Hz, 1H), 6.40 (s, 1H), 6.09 m, 1H), 5.48 (m, 1H), 5.36 (m, 1H), 4.65 (td, J=1.53, 5.01 Hz, 2H), 3.91 (s, 3H), 2.19 (s, 3H).

### Step 2:

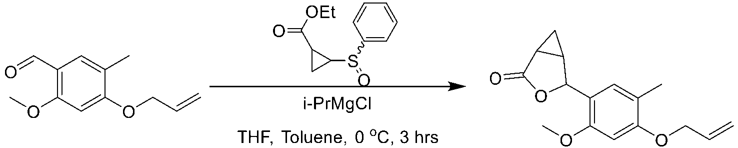

Isopropylmagnesium chloride solution (2 M, 25.46 mL, 1.5 *eq*) was added to a solution of compound (**14-c-1**) (8.09 g, 33.94 mmol, 1 *eq*) in THF (210 mL) at -78 °C, and the mixture was stirred at -78 °C for 60 minutes. A solution of compound (**14-f**) (7.0 g, 33.94 mmol, 1.0 *eq*) in toluene (20 mL) was added, and the mixture was stirred at 0 °C for 3 hours. After the reaction, saturated aqueous NH₄Cl solution (200 mL) was added to quench, and the mixture was extracted with EtOAc (200 mL×3). The combined organic phases were dried over anhydrous sodium sulfate, filtered, and concentrated under reduced pressure. The residue was purified by silica gel flash column chromatography (ISCO^{®}; 220 g SepaFlash^{®} Silica Flash Column, 0-15% EA/PE, 30 mL/min) to obtain **14-g** as a colorless oil (4.8 g, 16.8 mmol, 49.49% yield). ¹H NMR (400 MHz, CDCl₃) Shift 6.96-7.08 (m, 1H), 6.47 (s, 1H), 6.00-6.18 (m, 1H), 5.84 (d, J=4.62 Hz, 0.24H), 5.51 (s, 0.7H), 5.46 (qd, J=1.65, 17.28 Hz, 1H), 5.27-5.36 (m, 1H), 4.59 (td, J=1.68, 5.01 Hz, 2H), 3.82-3.90 (m, 3H), 2.61-2.72 (m, 0.2H), 2.12-2.30 (m, 4.8H), 1.20-1.40 (m, 1H), 0.96-1.10 (m, 1H), 0.80-0.90 (m, 0.28H). (ESI) m/z= 275.2 (M+1) ⁺.

### Step 3:

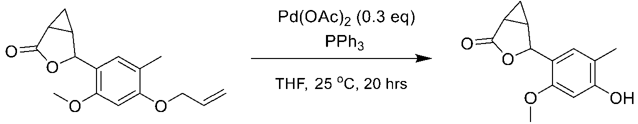

Pd(OAc)₂ (1.11 g, 4.96 mmol, 0.3 *eq*) and PPh₃ (5.2 g, 19.84 mmol, 1.2 *eq*) were added to a solution of compound (**14-g**) (4.53 g, 16.53 mmol, 1 *eq*) in THF (100 mL), and the mixture was stirred at 25 °C for 20 hours. After the reaction, the mixture was concentrated under reduced pressure. The residue was purified by silica gel flash column chromatography (ISCO^{®}; 120 g SepaFlash^{®} Silica Flash Column, 0-30% EA/PE, 60 mL/min), then triturated with MTBE:PE (1:1, 10 mL) to obtain compound **14** as an off-white solid (990 mg, 4.23 mmol, 25.64% yield). (ESI) m/z=235.1 (M+1) ⁺. ¹H NMR (400 MHz, DMSO-d6) Shift 9.19-9.67 (m, 1H), 6.82-6.98 (m, 1H), 6.44-6.54 (m, 1H), 5.73 (d, J=4.65 Hz, 0.2H), 5.39 (s, 0.8H), 3.64-3.79 (m, 3H), 2.12-2.28 (m, 2H), 1.98-2.11 (m, 3H), 1.29 (dt, J=4.46, 8.16 Hz, 0.8H), 1.00-1.10 (m, 0.2H), 0.96 (q, J=4.28 Hz, 0.8H), 0.71 (br d, J=3.30 Hz, 0.2H).

According to steps 2 and 3 of **Example 2,** the following starting material was used in place of (**14-c-1**) and reacted with (**14-f**) in two steps to obtain the corresponding final product.

| Starting material | Final product | LCMS and NMR data |
|---|---|---|
| 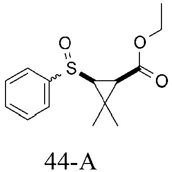 | 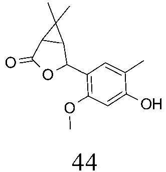 About 30.3% yield over two steps, as an off-white solid | ¹H NMR (400 MHz, DMSO-d6) δ 9.53 (s, 1H), 6.95 (s, 1H), 6.49 (s, 1H), 5.29 (s, 1H), 3.72 (s, 3H), 2.16 (d, J=6.11 Hz, 1H), 1.97-2.09 (m, 4H), 1.18 (s, 3H), 1.12 (s, 3H). LCMS: (ESI) m/z = 263.3 (M+1) ⁺ |

The synthesis of the starting material 44-A shown in the above table is as follows:

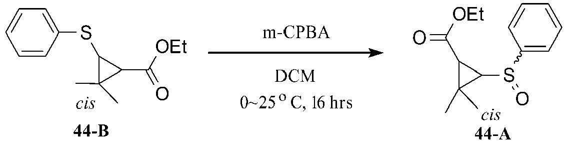

Following the procedure of step 2 in Example 1, 44-A was obtained as a colorless oil using 44-B (600 mg, 2.40 mmol) as the starting material (540 mg, yield 84.59%). ¹H NMR (400 MHz, CDCl₃) δ 7.63-7.85 (m, 2H), 7.38-7.61 (m, 3H), 4.10-4.37 (m, 2H), 2.38-2.55 (m, 1H), 1.90-2.15 (m, 1H), 1.52-1.71 (m, 3H), 1.22-1.42 (m, 4H), 1.13 (s, 2H).

According to the method of Example 2, the following starting material was used to obtain the corresponding final products through three-step reaction sequences.

| Starting material | Final product | LCMS and NMR data |
|---|---|---|
| 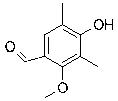 | 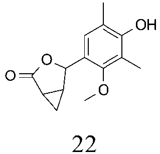 About 39% yield over three steps, as an off-white solid | LCMS:(ESI)m/z=249.1 (M+1)⁺; |
| | | ¹H NMR (400 MHz, DMSO-d6) δ 8.30-8.70 (m, 1H), 6.68-6.95 (m, 1H), 5.37-5.90 (m, 1H), 3.68 (s, 1H), 3.34 (s, 2H), 2.16-2.34 (m, 2H), 2.10-2.15 (m, 3H), 2.06-2.10 (m, 3H), 1.04-1.40 (m, 1H), 0.69-1.03 (m, 1H). |
| 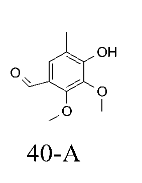 | 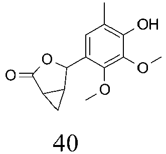 About 3.2% yield over two steps, as an off-white solid | ¹H NMR (400 MHz, DMSO-d6) δ 9.05 (br s, 1H), 6.60-6.84 (m, 1H), 5.29-5.90 (m, 1H), 3.77-3.86 (m, 3H), 3.73 (s, 3H), 2.14-2.36 (m, 2H), 2.02-2.12 (m, 3H), 1.07-1.38 (m, 1H), 0.71-1.05 (m, 1H). LCMS: (ESI) m/z = 265.0 (M+1) ⁺ |
| 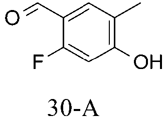 | 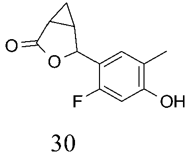 About 6.9% yield over two steps, white solid | ¹H NMR (400 MHz, DMSO-d6) Shift 10.06 (br s, 1H), 6.92-7.17 (m, 1H), 6.62 (d, J=12.32 Hz, 1H), 5.81 (d, J=4.84 Hz, 0.2H), 5.40 (s, 0.8H), 2.19-2.38 (m, 2H), 2.09 (s, 3H), 1.33 (dt, J=4.51, 8.20 Hz, 0.8H), 1.10-1.22 (m, 0.2H), 1.00 (q, J=4.25 Hz, 0.8H), 0.78-0.86 (m, 0.2H). LCMS: (ESI) m/z = 223.2 (M+1) ⁺ |
| 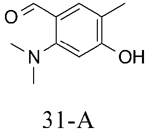 | 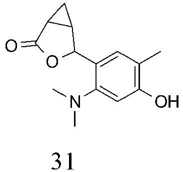 About 3.9% yield over two steps, yellow solid | 1H NMR (400 MHz, DMSO-d6) Shift 9.47 (s, 1H), 6.97 (s, 1H), 6.66 (s, 1H), 5.93 (d, J=4.52 Hz, 0.125H), 5.60 (s, 0.875H), 2.57 (s, 6H), 2.32 (m, 1H), 2.23 (m, 1H), 2.15 (m, 0.15H), 2.00-2.11 (m, 3H), 1.21-1.36 (m, 1H), 1.07 (m, 0.15H), 1.00 (q, J=4.28 Hz, 0.85H), 0.85-0.90 (m, 0.15H). LCMS: (ESI) m/z=248.3 (M+H)⁺ |
| 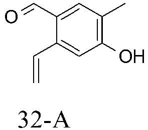 | 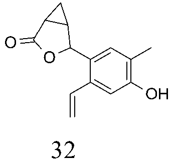 About 11% yield over three steps, as an off-white solid | ¹H NMR (400 MHz, DMSO-d6) δ 9.32-9.70 (m, 1H), 6.85-7.12 (m, 3H), 5.44-6.03 (m, 2H), 5.18-5.42 (m, 1H), 2.16-2.33 (m, 2H), 2.12 (s, 3H), 1.09-1.40 (m, 1H), 0.73-1.07 (m, 1H). LCMS:(ESI) m/z = 231.0 (M+1) ⁺ |
| 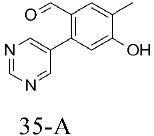 | 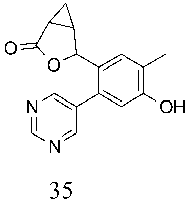 About 11% yield over three steps, white solid | LCMS:(ESI) m/z = 283.1 (M+1)⁺; ¹H NMR (400 MHz, DMSO-*d*₆) δ ppm 1.02 (s, 1 H) 1.10 - 1.30 (m, 1 H) 2.09 - 2.17 (m, 1 H) 2.18 - 2.24 (m, 3 H) 2.28 - 2.36 (m, 1 H) 2.38 - 2.46 (m, 1 H) 5.02 - 5.15 (m, 1 H) 5.61 (d,J=4.40 Hz, 1 H) 6.55 - 6.77 (m, 1 H) 7.17 - 7.37 (m, 1 H) 8.63 - 8.87 (m, 2 H) 9.10 - 9.35 (m, 1 H) 9.80 (br s, 1 H) |
| 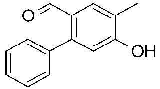 | 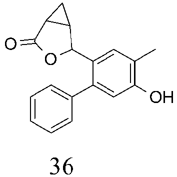 About 20.4% yield over three steps, white solid | LCMS: (ESI) m/z = 281.0 (M+1) ⁺; ¹H NMR (400 MHz, DMSO-*d*₆) δ ppm 0.82 - 0.97 (m, 1 H) 1.00 - 1.29 (m, 1 H) 1.97 - 2.10 (m, 1 H) 2.15 - 2.25 (m, 3 H) 2.30 - 2.39 (m, 2 H) 5.02 - 5.21 (m, 1 H) 5.45 - 5.67 (m, 1 H) 6.58 - 6.78 (m, 1 H) 7.08 - 7.22 (m, 1 H) 7.27 - 7.35 (m, 2 H) 7.36 - 7.43 (m, 1 H) 7.37 - 7.43 (m, 1 H) 7.43 - 7.51 (m, 2 H) 9.48 - 9.91 (m, 1 H) |
| 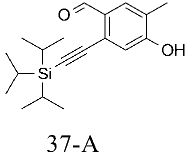 | 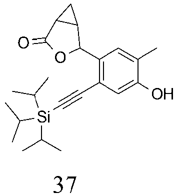 About 39.4% yield over three steps, white solid | LCMS: (ESI) m/z = 385.2 [M+H]⁺; ¹H NMR (400 MHz, DMSO-*d*₆) δ ppm 9.60 - 10.08 (m, 1 H) 6.98 - 7.06 (m, 1 H) 6.85 - 6.95 (m, 1 H) 5.87 (d, J=4.77 Hz, 0.24 H) 5.59 (s, 0.754 H) 2.23 - 2.32 (m, 2 H) 2.12 - 2.18 (m, 3 H) 1.36 (s, 1 H) 1.09 - 1.15 (m, 21 H) 0.96 (q, J=4.20 Hz, 1 H) |
| 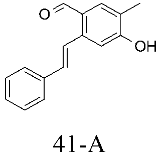 | 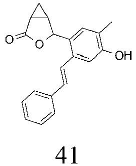 About 11.2% yield over three steps, white solid | LCMS: (ESI) m/z = 307.0 (M+1) ⁺; ¹H NMR (400 MHz, DMSO-d6) δ ppm 0.77 - 1.10 (m, 1 H) 1.14 (td, J=8.20, 4.73 Hz, 1 H) 2.13 - 2.18 (m, 3 H) 2.20 - 2.38 (m, 2 H) 2.57 - 2.62 (m, 1 H) 5.76 - 5.95 (m, 1 H) 6.15 (d, J=4.62 Hz, 1 H) 6.90 - 7.18 (m, 3 H) 7.22 - 7.33 (m, 1 H) 7.35 - 7.54 (m, 3 H) 7.60 - 7.75 (m, 2 H) 9.38 - 9.73 (m, 1 H) |
| 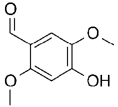 | 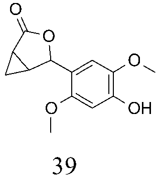 About 17.9% yield over three steps, white solid | ¹H NMR (400 MHz, DMSO-*d*₆) *δ* (ppm) 9.06-9.38 (m, 1H), 6.67-6.84 (m, 1H), 6.52-6.59 (m, 1H), 5.37-5.77 (m, 1H), 3.69-3.75 (m, 6H), 2.13-2.26 (m, 2H), 1.02-1.34 (m, 1H), 0.77-0.99 (m, 1H). LC-MS: (ESI) *m*/*z.* [M+H]⁺ 251.1 |
| 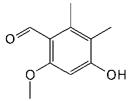 | 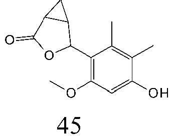 About 4.3% yield over three steps, white solid | ¹H NMR (400 MHz, DMSO-*d*₆) *δ* (ppm) 8.65 (s, 1H), 5.56 (s, 1H), 4.79 (s, 1H), 2.81 (s, 3H), 1.35 (s, 4H), 1.22-1.30 (m, 1H), 1.17 (s, 3H), 0.42-0.50 (m, 1H), 0.14 (q, *J* = 4.3 Hz, 1H). LC-MS: (ESI) m/z. [M+H]⁺ 249.3 |
| 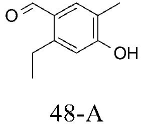 | 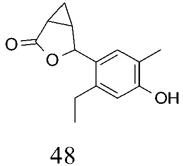 About 23.8% yield over three steps, as an off-white solid | LCMS: (ESI) m/z = 233.3 (M+1) ⁺, |
| | | ¹H NMR (400 MHz, DMSO-d6) δ 9.07-9.61 (m, 1H), 6.85-7.05 (m, 1H), 6.41-6.73 (m, 1H), 5.49 (s, 1H), 2.53-2.64 (m, 2H), 2.13-2.36 (m, 2H), 2.09 (s, 3H), 1.32 (dt, J=4.40, 8.19 Hz, 1H), 1.07-1.22 (m, 3H), 0.76-1.06 (m, 1H). |
| 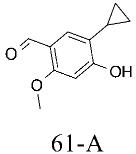 | 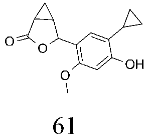 About 9% yield over three steps, as an off-white solid | LC-MS: (ESI) *m*/*z.* [M+H]⁺ 261.3; |
| | | ¹H NMR (400 MHz, DMSO-*d*₆) *δ* (ppm) 9.29-9.73 (m, 1H), 6.51-6.67 (m, 1H), 6.46-6.50 (m, 1H), 5.34 (s, 1H), 3.68-3.76 (m, 3H), 2.10-2.49 (m, 2H), 1.86-2.02 (m, 1H), 1.01-1.32 (m, 1H), 0.63-0.96 (m, 3H), 0.42-0.57 (m, 2H). |
| 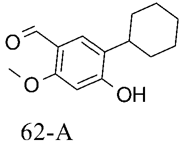 | 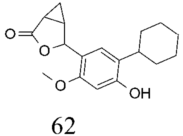 About 5.6% yield over three steps, white solid | LCMS: (ESI) m/z = 303.3 (M+1) ⁺; |
| | | ¹H NMR (400 MHz, DMSO-d6) δ ppm 0.63 - 0.69 (m, 1 H) 0.91 - 1.41 (m, 7 H) 1.63 - 1.81 (m, 5 H) 2.12 - 2.27 (m, 2 H) 2.69 - 2.83 (m, 1 H) 3.67 - 3.78 (m, 3 H) 5.30-5.46 (m, 1 H) 5.70-5.77 (m, 1 H) 6.42 - 6.56 (m, 1 H) 6.77 - 7.04 (m, 1 H) 9.32 - 9.80 (m, 1 H) |
| 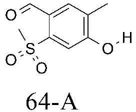 | 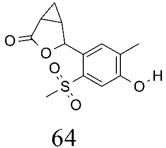 About 19% yield over three steps, yellow solid | LC-MS: (ESI) *m*/*z.* [M+H]⁺ 283.0; |
| | | ¹H NMR (400 MHz, DMSO-*d*₆) *δ* (ppm) 10.23-10.45 (m, 1H), 7.39-7.46 (m, 1H), 7.24-7.32 (m, 1H), 6.22-6.28 (m, 0.3H), 6.07 (s, 0.6H), 3.23-3.27 (m, 3H), 2.65-2.73 (m, 0.4H), 2.31-2.40 (m, 1.5H), 2.18-2.26 (m, 3H), 1.36 (dt, *J* = 4.8, 8.3 Hz, 0.68H), 1.04-1.14 (m, 1H), 0.95-1.00 (m, 0.32H) |
| 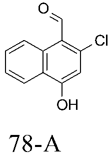 | 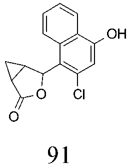 About 1.5% yield over three steps, yellow solid | LCMS: (ESI) m/z = 275.0 [M+H]⁺, |
| | | ¹H NMR (400 MHz, CDCl₃) δ ppm 8.28 (d, J=8.31 Hz, 1 H) 8.09 (d, J=8.68 Hz, 1 H) 7.62 (ddd, J=8.53, 6.94, 1.53 Hz, 1 H) 7.51 - 7.58 (m, 1 H) 6.87 - 6.93 (m, 1 H) 6.34 (s, 1 H) 6.00 (br s, 1 H) 2.50 - 2.60 (m, 1 H) 2.43 (br dd, J=5.87, 1.59 Hz, 1 H) 1.63 (br s, 1 H) 1.29 - 1.38 (m, 1 H) |
| 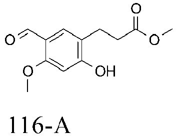 | 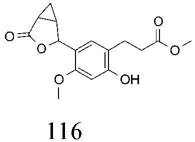 About 12.5% yield over three steps, white solid | LCMS: (ESI) m/z=307.4 (M+1)⁺, |
| | | ¹H NMR (400 MHz, DMSO-d6) Shift 9.51-9.86 (m, 1H), 6.76-6.99 (m, 1H), 6.43-6.55 (m, 1H), 5.73 (d, J=4.65 Hz, 0.24H), 5.38 (s, 0.78H), 3.70-3.80 (m, 3H), 3.53-3.63 (m, 3H), 2.68-2.80 (m, 2H), 2.53 (br s, 2H), 2.13-2.26 (m, 2H), 1.29 (dt, J=4.52, 8.13 Hz, 1H), 1.01-1.10 (m, 0.28 H), 0.67-0.99 (m, 1H) |
| 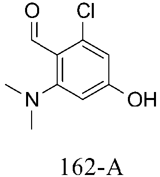 | 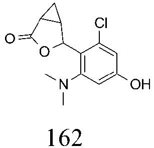 About 2.1% yield over three steps, white solid | ¹H NMR (400 MHz, DMSO-*d*₆) *δ* (ppm) 10.10 (br s, 1H), 6.52-6.77 (m, 2H), 5.84 (s, 1H), 2.58 (s, 6H), 2.20-2.37 (m, 2H), 1.34-1.45 (m, 1H), 1.12 (q, *J* = 4.2 Hz, 1H). LC-MS: (ESI) m/z. [M+H]⁺ 268.2 |
| 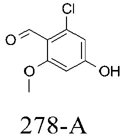 | 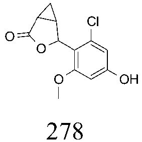 About 8.1% yield over three steps, grayish-white solid | LC-MS: (ESI) *m*/*z.* [M+H]⁺ 255.0; ¹H NMR (400 MHz, DMSO-*d*₆) *δ* (ppm) 10.22 (brs, 1H), 6.46-6.57 (m, 1H), 6.37-6.46 (m, 1H), 5.71 (s, 1H), 3.73 (s, 3H), 2.22-2.30 (m, 1H), 2.11-2.21 (m, 1H), 1.28-1.34 (m, 1H), 0.96-1.06 (m, 1H) |
| 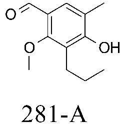 | 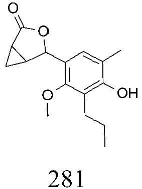 About 38% yield over three steps, yellow gum | LC-MS: (ESI) *m*/*z* [M+H]⁺ 277.0 |
| | | ¹H NMR (400 MHz, DMSO-*d*₆) *δ* (ppm) 8.49 (s, 1H), 6.89 (s, 1H), 5.81 (d, *J =* 4.6 Hz, 0.2H), 5.46 (s, 0.8H), 3.67 (s, 3H), 2.53-2.61 (m, 2H), 2.16-2.35 (m, 2H), 2.10-2.16 (m, 3H), 1.50 (m, 2H), 1.31 (td, *J =* 4.5, 8.2 Hz, 0.8H), 1.10 (td, *J* = 4.7, 8.2 Hz, 0.2H), 1.02 (q, *J =* 4.3 Hz, 0.8H), 0.94 (t, *J =* 7.3 Hz, 3H), 0.80-0.87 (m, 0.2H) |
| 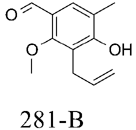 | 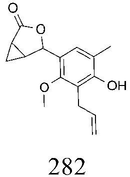 About 9.2% yield over three steps, colorless oil | LC-MS:(ESI) *m*/*z* [M+H]⁺ 275.0; |
| | | ¹H NMR (400 MHz, DMSO-*d*₆) *δ* (ppm) 8.60 (br s, 1H), 6.94 (s, 1H), 5.86-6.10 (m, 1H), 5.82 (br d, *J =* 4.2 Hz, 0.2H), 5.47 (s, 0.8H), 4.90-5.05 (m, 2H), 3.67 (s, 3H), 3.38 (br s, 2H), 2.53-2.62 (m, 0.2H), 2.19-2.36 (m, 1.8H), 2.16 (s, 3H), 2.08 (s, CH3CN, 0.4H), 1.32 (m, 0.8H), 1.07-1.14 (m, 0.2H), 1.02 (m, 0.8H), 0.83 (m, 0.2H) |
| 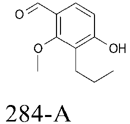 | 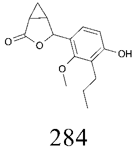 Yellow oil; about 5.5% yield over three steps | LC-MS: (ESI) *m*/*z.* [M+H]⁺ 263.1 |
| | | ¹H NMR (400 MHz, DMSO-*d*₆) *δ* (ppm) 9.43-9.67 (m, 1H), 6.85-7.04 (m, 1H), 6.54-6.71 (m, 1H), 5.81 (d, *J* = 4.6 Hz, 0.2H), 5.46 (s, 0.8H), 3.66-3.78 (m, 3H), 2.58 (br s, 0.2H), 2.51-2.55 (m, 2H), 2.15-2.32 (m, 1.8H), 1.44-1.58 (m, 2H), 1.28-1.32 (m, 0.8H), 1.07-1.14 (m, 0.2H), 0.99-1.04 (m, 0.8H), 0.89-0.96 (m, 3H), 0.78-0.82 (m, 0.2H) |
| 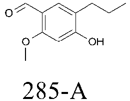 | 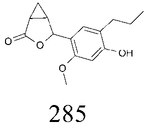 Grayish-white solid; about 8.3% yield over three steps | LC-MS: (ESI) *m*/*z* [M+H]⁺ 263.1 |
| | | ¹H NMR (400 MHz, DMSO-*d*₆) *δ* 9.30-9.57 (m, 1H), 6.78-6.96 (m, 1H), 6.44-6.54 (m, 1H), 5.73 (d, *J =* 0.2 Hz, 1H), 5.38 (s, 0.8H), 3.69-3.78 (m, 3H), 2.52-2.58 (m, 0.3H), 2.36-2.46 (m, 2H), 2.13-2.24 (m, 1.75H), 1.41-1.54 (m, 2H), 1.02-1.33 (m, 1H), 0.92-0.97 (m, 0.76H), 0.82-0.90 (m, 3H), 0.63-0.71 (m, 0.24H) |

The synthesis of the starting material 284-A and 285-A shown in the above table is as follows:

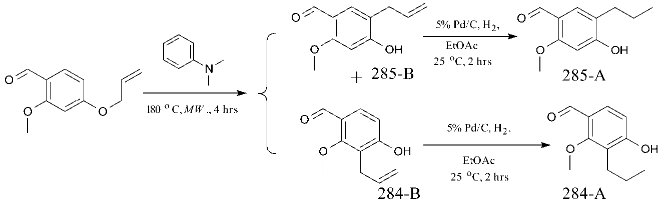

Step 1: Under microwave conditions, a mixture of 2-methoxy-4-(prop-2-en-1-yloxy)benzaldehyde (200 mg, 1.04 mmol, 1 *eq*) in N,N-dimethylaniline (2 mL) was stirred at 180 °C for 4 hours. After cooling to room temperature, the reaction was diluted with EtOAc (15 mL) and washed with 1M HCl (3 mL×3). The organic phase was subjected to conventional work-up procedure 2 to obtain the crude product, which was purified by silica gel column chromatography (EA/PE, EA from 7 to 15%) to obtain 284-B as an off-white solid (50 mg, yield 25%) and 285-B as a yellow solid (82.5 mg, yield 41.3%).

Compound 284-B: ¹H NMR (400 MHz, CDCl₃) *δ* (ppm) 10.22 (s, 1H), 7.74 (d, *J =* 8.6 Hz, 1H), 6.77 (d, *J* = 8.6 Hz, 1H), 6.39 (br s, 1H), 6.06 (ddt, *J* = 5.9, 10.5, 16.8 Hz, 1H), 5.09-5.25 (m, 2H), 3.91 (s, 3H), 3.47-3.60 (m, 2H).

Compound 285-B: ¹H NMR (400 MHz, CDCl₃) *δ* (ppm) 10.28 (s, 1H), 7.64 (s, 1H), 6.46 (s, 1H), 6.06-6.11 (m, 1H), 5.92-6.06 (m, 1H), 5.16-5.26 (m, 2H), 3.88 (s, 3H), 3.39 (d, *J =* 6.4 Hz, 2H).

Compound 284-A: Following the procedure for the synthesis of 281-A from 281-B, 284-B (200 mg) was reacted to obtain 284-A as a yellow oil (200 mg), which was used directly in the next step. LC-MS: (ESI) *m*/*z* [M+H]⁺ 195.2. ¹H NMR (400 MHz, CDCl₃) *δ* (ppm) 10.20 (s, 1H), 7.67 (d, *J =* 8.4 Hz, 1H), 6.70 (d, *J =* 8.6 Hz, 1H), 6.11 (br s, 1H), 3.92 (s, 3H), 2.55-2.74 (m, 2H), 1.52-1.65 (m, 2H), 0.96-1.16 (m, 3H).

Compound 285-A: Following the procedure for the synthesis of 281-A from 281-B, 285-B (330 mg) was reacted to obtain 285-A as an off-white solid (330 mg), which was used directly in the next step. LC-MS: (ESI) *m*/*z* [M+H]⁺ 195.2. ¹H NMR (400 MHz, CDCl₃) *δ* (ppm) 10.27 (s, 1H), 7.64 (s, 1H), 6.43 (s, 1H), 6.06 (s, 1H), 3.87 (s, 3H), 2.44-2.64 (m, 2H), 1.53-1.64 (m, 2H), 0.96 (t, *J =* 7.3 Hz, 3H).

The synthesis of the starting material 281-A and 281-B shown in the above table is as follows:

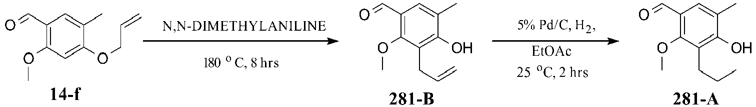

Step 1: A mixture of compound **14-f** (1.0 g, 4.85 mmol, 1 *eq*) in N,N-dimethylaniline (8 mL) was stirred at 180 °C for 8 hours. TLC showed only a small amount of starting material remained. After cooling to room temperature, the reaction was diluted with EtOAc (10 mL) and washed with 1M HCl (10 mL×3). The organic phase was subjected to conventional work-up procedure to obtain the crude product, which was purified by silica gel column chromatography (EA:PE, EA from 0 to 7%) to obtain compound 281-B as an off-white solid (640 mg, 3.10 mmol, yield 64%). LC-MS: (ESI) *m*/*z* [M+H]⁺ 207.0.

Step 2: Compound 281-B (300 mg, 1.45 mmol) was dissolved in EtOAc (30 mL), and palladium on carbon (5% content, 60 mg) was added. The reaction mixture was thoroughly purged with hydrogen and stirred at 25 °C under 15 psi hydrogen atmosphere for 2 hours. The mixture was filtered through celite, and the filtrate was concentrated under reduced pressure to obtain compound 281-A as an off-white solid (290 mg), which was used directly in the next step. LC-MS: (ESI) *m*/*z* [M+H]⁺ 209.1.

The synthesis method of the starting material 40-A shown in the above table is as follows:

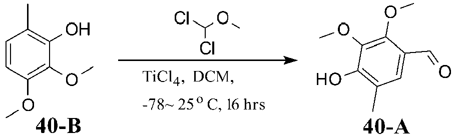

A solution of dichloromethyl methyl ether (410 mg, 3.57 mmol) and titanium tetrachloride (1.41 g, 7.43 mmol) in DCM (5 mL) was added to a solution of compound 40-B (500 mg, 2.97 mmol) in DCM (10 mL) at -78 °C. The mixture was allowed to warm to 25 °C and stirred for 16 hours. Water (50 mL) was added, and the mixture was extracted with EtOAc (50 mL×3). After conventional work-up procedure 2, the residue was purified by silica gel column chromatography (EA:PE, EA from 0 to 7%) to obtain compound 40-A as a white solid (440 mg, yield 75.44%). ¹H NMR (400 MHz, CDCl₃) δ 10.20 (s, 1H), 7.42 (s, 1H), 6.42 (s, 1H), 3.97 (s, 3H), 3.97 (s, 3H), 2.24 (d, J=0.72 Hz, 3H).

The synthesis method of the starting material 116-A shown in the above table is as follows:

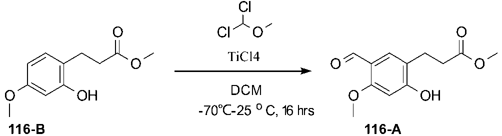

According to the synthetic method of 40-A, 116-B (3.0 g, 14.27 mmol) was reacted to obtain compound 116-A as a purple solid (2.2 g, yield 64.7%). ¹H NMR (400 MHz, DMSO-d6) Shift 10.80 (s, 1H), 10.11 (s, 1H), 7.45 (s, 1H), 6.54 (s, 1H), 3.82 (s, 3H), 3.57 (s, 3H), 2.71-2.78 (m, 2H), 2.53-2.60 (m, 2H).

The synthesis method of the starting material 48-A shown in the above table is as follows:

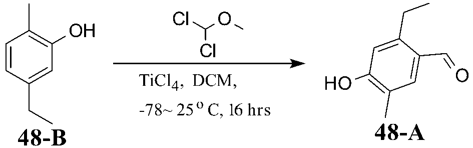

According to the synthetic method of 40-A, 48-B (870 mg, 6.39 mmol) was reacted to obtain compound 48-A as a white solid (720 mg, yield 68.64%). ¹H NMR (400 MHz, DMSO-d6) δ 10.41 (br s, 1H), 9.97 (s, 1H), 7.56 (s, 1H), 6.71 (s, 1H), 2.90 (q, J=7.46 Hz, 2H), 2.12 (s, 3H), 1.13 (t, J=7.46 Hz, 3H).

The synthesis method of the starting material 62-A shown in the above table is as follows:

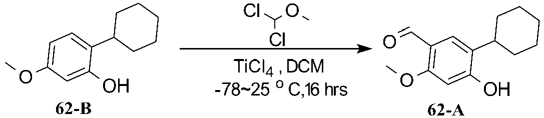

According to the synthetic method of 40-A, 62-B (1.06 g, 5.14 mmol) was reacted to obtain compound 62-A as a brown solid (432 mg, yield 19.3%, purity 73.3%). LCMS: (ESI) m/z = 235.0 (M+1)⁺.

The synthesis method of the starting material 31-A shown in the above table is as follows:

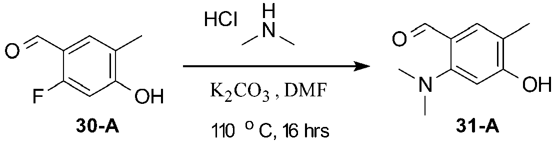

A solution of K₂CO₃ (5.11 g, 36.98 mmol) in DMF (50 mL) was added to a solution of compound **30-A** (1.9 g, 12.33 mmol) and dimethylamine hydrochloride (3.02 g, 36.98 mmol) in DMF (50 mL). The mixture was stirred at 110 °C for 16 hours, then cooled to room temperature. Water (30 mL) was added, and the mixture was extracted with EtOAc and THF (1:1, 50 mL×3). After conventional work-up procedure, the residue was purified by silica gel column chromatography (EA:PE, EA from 0 to 20%) to obtain compound **31-A** as a yellow solid (820 mg, yield 37.12%). ¹H NMR (400 MHz, CDCl₃) δ 10.00 (s, 1H), 7.59 (s, 1H), 6.50 (s, 1H), 2.85 (s, 6H), 2.22 (s, 3H).

The synthesis method of the starting material 162-A shown in the above table is as follows:

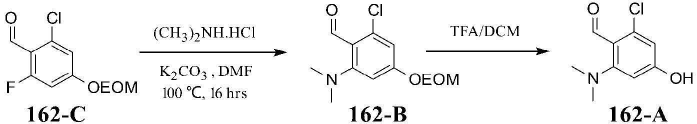

Step 1: According to the synthetic method of starting material 30-A, 162-C (1 g, 4.30 mmol) was reacted to obtain compound 162-B as a yellow oil (0.95 g, yield 85.7%). LC-MS: (ESI) *m*/*z*. [M+H]⁺ 258.1.

Step 2: According to the procedure of step 4 in Example 1, 162-B (0.80 g) was reacted to obtain compound 162-A as a yellow oil (0.62 g), which was used directly in the next step. LC-MS: (ESI) *m*/*z*. [M+H]⁺ 200.1.

The synthesis method of the starting material 32-A shown in the above table is as follows:

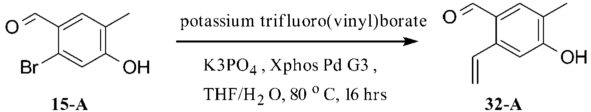

According to the procedure of step 4 in Example 8-b, 15-A (500 mg, 2.33 mmol) was reacted with potassium vinyltrifluoroborate (934 mg, 6.98 mmol) to obtain compound 32-A as an off-white solid (250 mg, yield 66.3%). ¹H NMR (CDCl₃, 400 MHz) δ 10.13 (s, 1H), 7.66 (s, 1H), 7.52 (dd, J=11.00, 17.36 Hz, 1H), 6.97 (s, 1H), 6.69 (s, 1H), 5.65 (d, J=17.36 Hz, 1H), 5.46 (d, J=11.00 Hz, 1H), 2.31 (s, 3H).

The synthesis method of the starting material 35-A shown in the above table is as follows:

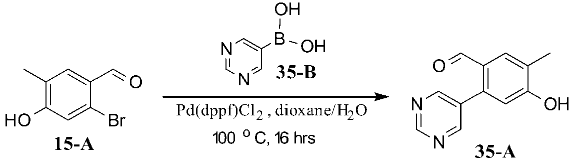

35-B (691 mg, 5.58 mmol) and K₃PO₄ (2.96 g, 13.95 mmol) were added to a mixture of compound 15-A (1 g, 4.65 mmol) in dioxane/water (20 mL, 9/1). After degassing and purging with nitrogen three times, Pd(dppf)Cl₂ (170 mg, 232.51 µmol) was added. The mixture was heated to 100 °C and stirred for 16 hours. After cooling to room temperature, the reaction was diluted with saturated brine (50 mL) and extracted with EtOAc (**20** mL×**3**) and THF (15 mL×3). After conventional work-up procedure, the crude product was triturated in DCM (**10** mL) and washed with DCM (**2** mL×**3**) to obtain compound 35-A as a white solid (767 mg, yield 77%). ¹H NMR (400 MHz, DMSO-d6) δ ppm 2.23 - 2.28 (m, 3 H) 6.77 - 6.84 (m, 1 H) 7.72 - 7.86 (m, 1 H) 8.80 - 8.90 (m, 2 H) 9.19 - 9.28 (m, 1 H) 9.66 - 9.75 (m, 1 H) 10.82 - 10.94 (m, 1 H).

The synthesis method of the starting material 41-A shown in the above table is as follows:

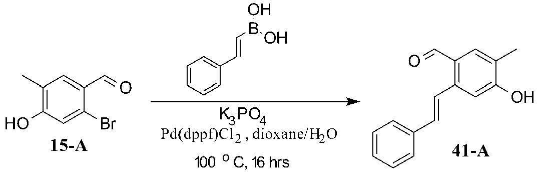

According to the synthetic method of compound 35-A, 15-A (1 g, 4.65 mmol) was reacted with (E)-styrylboronic acid (413 mg, 2.79 mmol, 1.2 *eq*) to obtain compound 41-A as a brown solid (302 mg, yield 54.51%). ¹H NMR (400 MHz, DMSO-d6) δ ppm 2.13 - 2.25 (m, 3 H) 7.14 - 7.21 (m, 1 H) 7.29 - 7.35 (m, 1 H) 7.37 - 7.47 (m, 2 H) 7.57 - 7.71 (m, 3 H) 8.03 - 8.20 (m, 1 H) 10.09 - 10.19 (m, 1 H).

The synthesis method of the starting material 37-A shown in the above table is as follows:

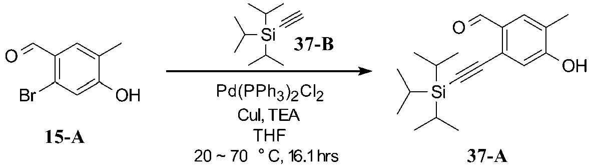

Pd(dppf)Cl₂ (326 mg, 465 µmol) was added to a mixture of compound 15-A (500 mg, 2.33 mmol) in THF (10 mL). After degassing and purging with nitrogen three times, TEA (1.18 g, 11.63 mmol), CuI (44.2 mg, 232.51 µmol), and 37-B (551 mg, 3.02 mmol) were added. The mixture was heated to 70 °C and stirred for 16 hours. After cooling to room temperature, the reaction was diluted with water (5 mL), and the organic phase was separated. After conventional work-up procedure, the crude product was purified by silica gel column chromatography (EA:PE, EA from 0 to 30%) to obtain compound **37-A** as a yellow solid (450 mg, yield 61.15%). ¹H NMR (CDCl₃, 400 MHz) δ ppm 10.44 (s, 1 H) 7.76 (s, 1 H) 6.98 (s, 1 H) 5.77 (s, 1 H) 2.30 (s, 3 H) 1.09 - 1.23 (m, 22 H).

The synthesis method of the starting material 61-A shown in the above table is as follows:

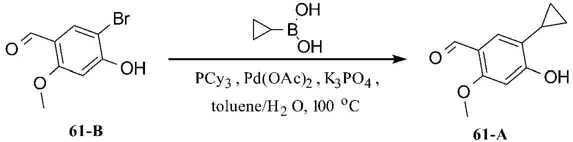

Cyclopropylboronic acid (473 mg, 5.51 mmol) and K₃PO₄ (2.70 g, 12.7 mmol) were added to a mixture of compound 61-B (980 mg, 4.24 mmol) in toluene (50 mL)/water (5 mL). The mixture was purged with nitrogen three times, then Pd(OAc) ₂ (50 mg, 223 µmol) and tricyclohexylphosphine (490 mg, 1.75 mmol) were added, and the mixture was stirred at 100 °C under a nitrogen atmosphere for 16 hours. After cooling to room temperature, the reaction was diluted with 2 mL of saturated brine and extracted with EA (5 mL×2). After conventional work-up procedure, the residue was purified by silica gel column chromatography (THF:PE, THF from 0 to 15%) to obtain compound 61-A as a yellow solid (330 mg, yield 40.48%). ¹H NMR (400 MHz, CDCl₃) *δ* (ppm) 10.26 (s, 1H), 7.64 (d, *J =* 0.7 Hz, 1H), 6.50 (s, 1H), 3.89 (s, 3H), 1.61-1.75 (m, 1H), 0.92-1.03 (m, 2H), 0.61-0.67 (m, 2H).

The synthesis method of the starting material 64-A shown in the above table is as follows:

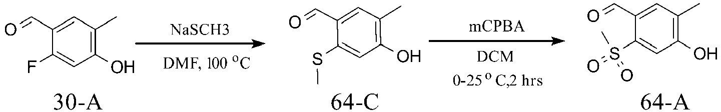

Step 1: NaSCH₃ (4.16 g, 59.4 mmol) was added to a solution of 30-A (2.50 g, 16.2 mmol) in DMF (50 mL). The mixture was stirred at 120 °C until LC-MS showed complete consumption of starting material. After cooling to room temperature, the reaction was poured into water (50 mL), adjusted to pH=5 with 50% acetic acid, and extracted with EA (3×50 mL). After conventional work-up procedure 2, the residue was purified by preparative TLC (EA:PE, EA from 0 to 25%) to obtain compound 47-A as a yellow oil (1.5 g, yield 50.7%). ¹H NMR (400 MHz, DMSO-*d*₆) *δ* (ppm) 9.87 (s, 1H), 7.62 (s, 1H), 6.80 (s, 1H), 2.38 (s, 3H), 2.13 (s, 3H).

Step 2: m-CPBA (2.79 g, 13.7 mmol, 85% purity) was added portionwise to a solution of compound 64-C (1.0 g, 5.49 mmol) in DCM (20 mL) at 0 °C. The mixture was stirred at 25 °C for 2 hours, then filtered and dried under vacuum to obtain compound 64-A as a white solid (3.2 g, 35% purity), which was used directly in the next step.

The synthesis method of the starting material 278-A shown in the above table is as follows:

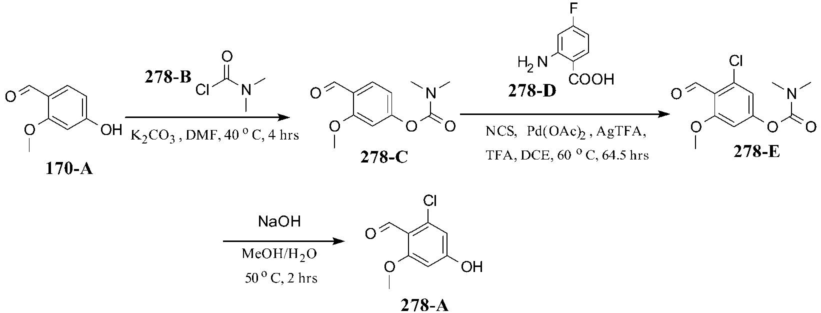

Step 1: K₂CO₃ (5.0 g, 36.2 mmol, 1.1 *eq*) and compound 278-B (4.24 g, 39.4 mmol, 1.2 *eq*) were added to a solution of 170-A (5.0 g, 32.9 mmol) in DMF (50 mL). The mixture was stirred at 40 °C for 3 hours. After cooled to room temperature, EA (100 mL) was added, and the mixture was filtered. The filtrate was concentrated under reduced pressure, and the crude product was purified by silica gel column chromatography (EA: PE, EA from 0 to 25%) to obtain compound 278-C as a white solid (7 g, yield 95%). LC-MS: (ESI) *m*/*z*. [M+H]⁺ 224.21.

Step 2: Pd(OAc)₂ (201 mg, 896 µmol), silver trifluoroacetate (AgTFA) (198 mg, 896 µmol), and TFA (10.2 g, 89.6 mmol) were added to a solution of compound 278-C (2 g, 8.96 mmol) and compound 278-D (695 mg, 4.48 mmol) in 1,2-dichloroethane (40 mL). After stirred at 25 °C for 0.5 hours, NCS (1.80 g, 13.5 mmol) was added. The mixture was stirred at 60 °C for 64.5 hours (LCMS showed 15% starting material remained). After cooled to room temperature, saturated NaHCO₃ solution (50 mL) was added, and the mixture was extracted with DCM (100 mL×3). After conventional work-up procedure 2, the residue was purified by silica gel column chromatography (EA:PE, EA from 0 to 21.5%) to obtain compound 278-E as a white solid (1.2 g, 4.66 mmol, yield 52%). LC-MS: (ESI) *m*/*z* [M+H]⁺ 258.1.

Step 3: A solution of NaOH (70 mg) in water (2.5 mL) was added to a solution of 278-E (300 mg) in MeOH (5 mL). The mixture was stirred at 50 °C for 2 hours, then concentrated under reduced pressure. Water (5 mL) was added, and the mixture was washed with MTBE (3 mL×2). The pH was adjusted to 2~3, and the precipitated solid was collected by filtration, washed with water (5 mL×2), and dried under vacuum to obtain compound 278-A as a yellow solid (200 mg, yield 90%). LCMS: (ESI) *m*/*z*. [M+H]⁺ 187.1.

According to the procedures of Step 2 and Step 3 in Example 2, the corresponding final products were prepared from the following starting materials via two-step reactions.

| Starting material | Final product | LCMS and NMR data |
|---|---|---|
| 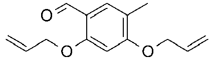 Note: In the second step, the equivalents of Pd(OAc)₂ and PPh₃ were doubled. | 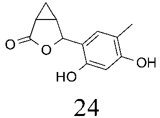 About 3.8% yield over two steps, white solid | LCMS: (ESI)m/z=221.1 (M+1)⁺; |
| | | ¹H NMR (400 MHz, DMSO-d6) δ ppm 0.90 - 0.97 (m, 1 H) 1.22 - 1.32 (m, 1 H) 1.94 - 2.03 (m, 3 H) 2.15 - 2.24 (m, 2 H) 5.33 - 5.39 (m, 1 H) 6.32 - 6.42 (m, 1 H) 6.70 - 6.89 (m, 1 H) 9.24 - 9.37 (m, 1 H) 9.40 - 9.49 (m, 1 H) |
| 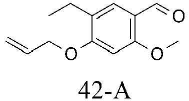 | 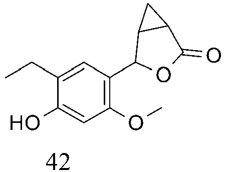 About 5.5% yield over two steps, white solid | ¹H NMR (400 MHz, DMSO-*d*₆) *δ* (ppm) 6.87-7.02 (m, 1H), 6.66-6.71 (m, 1H), 6.00-6.15 (m, 1H), 5.74-5.79 (m, 1H), 5.38-5.47 (m, 2H), 5.27 (br d, *J =* 10.6 Hz, 1H), 4.61-4.67 (m, 2H), 3.78-3.87 (m, 3H), 2.14-2.26 (m, 2H), 0.94-1.34 (m, 6H). LC-MS: (ESI) *m*/*z.* [M+H]⁺ 289.3 |
| 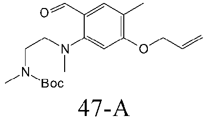 | 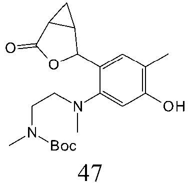 About 4.3% yield over two steps, as an off-white solid | LC-MS: (ESI) *m*/*z.* [M+H]⁺ 391.5; |
| | | ¹H NMR (400 MHz, CDCl₃) *δ* (ppm) 6.93-7.18 (m, 1H), 6.58-6.82 (m, 1H), 5.69-6.06 (m, 1H), 3.34 (br s, 2H), 2.98 (br s, 2H), 2.85 (s, 3H), 2.54-2.77 (m, 3H), 2.27 (br s, 1H), 2.21 (s, 4H), 1.45 (s, 9H), 1.33 (dt, J=5.07, 8.10 Hz, 1H), 1.07 (br s, 2H). |
| 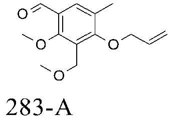 | 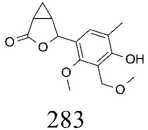 Yellow oil; about 9.2% yield over two steps | LC-MS: (ESI) *m*/*z* [M+H]⁺ 279.0 |
| | | ¹H NMR (400 MHz, DMSO-*d₆*) *δ* (ppm) 8.55-8.85 (m, 1H), 6.91-7.09 (m, 1H), 5.82 (d, *J* = 4.6 Hz, 0.2H), 5.46 (s, 0.8H), 4.45 (s, 2H), 3.68-3.82 (m, 3H), 3.26-3.32 (m, 3H), 2.55-2.60 (m, 0.2H), 2.18-2.35 (m, 1.8H), 2.10-2.17 (m, 3H), 1.08-1.34 (m, 1H), 0.80-1.05 (m, 1H) |

The synthesis method of the starting material 283-A shown in the above table is as follows:

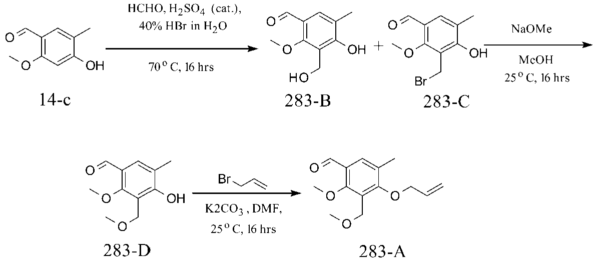

Step 1: Compound 14-c (500 mg, 3.01 mmol, 1 *eq*) was dissolved in aqueous hydrobromic acid solution (3 mL, 40% content). Paraformaldehyde (130 mg) and a catalytic amount of concentrated sulfuric acid (92 mg, 938 µmol, 50 µL) were added, and the mixture was stirred at 70 °C for 16 hours. After cooled to room temperature, the reaction was diluted with water (10 mL) and extracted with DCM (10 mL×3). The organic phase was subjected to conventional work-up procedure to obtain the crude product, which was triturated in a mixed solvent of PE and DCM (5/1) at 25 °C to obtain a brown solid (560 mg), which was a mixture of compounds 283-B and 283-C and was used directly in the next step. LC-MS: (ESI) *m*/*z* [M+H]⁺ 197.1.

Step 2: The mixture of compounds 283-B and 283-C (560 mg) was dissolved in MeOH (10 mL), and sodium methoxide solution in methanol (5.4 M, 2.00 mL) was added. The mixture was stirred at 25 °C for 16 hours, then concentrated under reduced pressure. The pH was adjusted to 4 with 1M HCl, and the mixture was extracted with DCM (5 mL×3). The organic phase was washed with saturated NaHCO₃ solution (5 mL) and subjected to conventional work-up procedure 2 to obtain compound 283-D as a brown oil (430 mg), which was used directly in the next step. LC-MS: (ESI) *m*/*z* [M+H]⁺ 211.2.

Step 3: 3-Bromo-1-propene (371 mg, 3.07 mmol) was added to a solution of compound 283-D (430 mg) and K₂CO₃ (707 mg, 5.11 mmol) in DMF (5 mL). The mixture was stirred at 25 °C for 16 hours. After the reaction was completed, saturated brine (5 mL) was added, and the mixture was extracted with EtOAc (3 mL×3). After conventional work-up procedure, the crude product was purified by silica gel column chromatography (EA:PE, EA from 0 to 5.5%) to obtain compound 283-A as a brown oil (482 mg, 64% yield over three steps). ¹H NMR (400 MHz, CDCl₃) *δ* (ppm) 10.29 (s, 1H), 7.71 (s, 1H), 6.12 (ddt, *J =* 5.3, 10.9, 16.7 Hz, 1H), 5.46 (dd, *J =* 0.7, 17.2 Hz, 1H), 5.31 (d, *J =* 10.4 Hz, 1H), 4.50 (s, 2H), 4.48 (d, *J* = 5.7 Hz, 2H), 3.98 (s, 3H), 3.47 (s, 3H), 2.30 (s, 3H).

The synthesis method of the starting material 42-A shown in the above table is as follows:

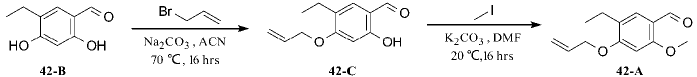

Step 1: 3-Bromo-1-propene (2.2 g, 18 mmol) and Na₂CO₃ (3.8 g, 36 mmol) were added to a mixture of 42-B (3 g, 18 mmol) in ACN (30 mL). The mixture was stirred at 70 °C for 16 hours. After the reaction, the solvent was evaporated, water (30 mL) was added, and the mixture was extracted with EA (30 mL×3). After conventional work-up procedure 2, the crude product was purified by preparative TLC (THF:PE, THF from 0 to 20%) to obtain compound 42-C as a yellow solid (1.3 g, yield 35%). ¹H NMR (400 MHz, DMSO-*d*₆) *δ* (ppm) 10.92 (br s, 1H), 9.98 (s, 1H), 7.41-7.51 (m, 1H), 6.50 (s, 1H), 6.06 (m, 1H), 5.27-5.48 (m, 2H), 4.59-4.74 (m, 2H), 2.51-2.55 (m, 2H), 1.12 (t, *J =* 7.5 Hz, 3H).

Step 2: Iodomethane (1.1 g, 8.00 mmol) and K₂CO₃ (1.5 g, 10.6 mmol) were added to a mixture of 42-C (1.1 g, 5 mmol) in DMF (10 mL). The mixture was stirred at 20 °C for 16 hours. Water (10 mL) was added, and the mixture was extracted with EA (15 mL×3). After conventional work-up procedure 2, the crude product was purified by preparative TLC (THF:PE, THF from 0 to 12%) to obtain compound 42-A as a white solid (990 mg, yield 84%). LCMS: (ESI) m/z=221.2 (M+1)⁺.

The synthesis method of the starting material 47-A shown in the above table is as follows:

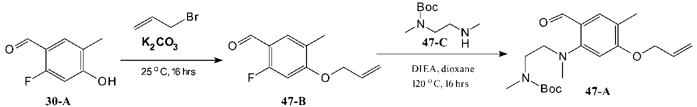

Step 1: According to the procedure of step 1 in Example 2, 30-A (268 mg, 1.74 mmol) was used as starting material to obtain compound 47-B as a white solid (260 mg, yield 77.00%). LCMS: (ESI) m/z=195.1 (M+1)⁺.

Step 2: A mixture of 47-B (1 g, 5.15 mmol), 47-C (1.94 g, 10.3 mmol), and DIEA (2.00 g, 15.5 mmol) in dioxane (12 mL) was stirred at 120 °C for 16 hours. LC-MS showed about 32.3% of starting material remained and about 48.0% of desired product was formed. The solvent was evaporated to obtain the crude product, which was purified by preparative TLC (EA:PE, EA from 0 to 30%) to obtain compound 47-A as a brown oil (900 mg, yield 48.2%). LCMS: (ESI) m/z=363.5 (M+1)⁺.

### Example 2-a: Synthesis of Compound 46

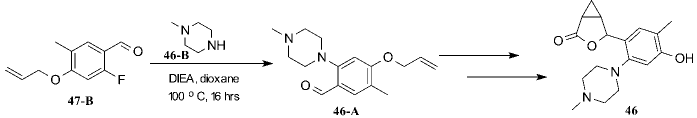

Step 1: According to the procedure of step 2 in the synthesis of 47-A, 46-B (1.55 g, 15.45 mmol) was reacted with 47-B (1 g, 5.15 mmol) to completion. After work-up, 46-A was obtained as a yellow solid (528 mg, yield 35.1%). LCMS: (ESI) m/z= 275.1 (M+1)⁺.

Steps 2 and 3: According to the procedure of steps 2 and 3 in Example 2, starting from 46-A, two-step reactions obtained compound 46 as a white solid in about 4.9% yield over two steps. LCMS: (ESI) m/z=303.0 (M+1)⁺; 1H NMR (400 MHz, DMSO-d6) δ ppm 0.75 - 0.88 (m, 1 H) 1.07 (br s, 1 H) 1.24 - 1.35 (m, 1 H) 2.04 - 2.11 (m, 3 H) 2.15 - 2.37 (m, 6 H) 2.55 (br s, 3 H) 2.70 - 2.80 (m, 2 H) 2.83 - 2.91 (m, 2 H) 5.58 (s, 1 H) 5.92 (d, J=4.62 Hz, 1 H) 6.62 - 6.78 (m, 1 H) 7.06 (s, 1 H) 8.14 (s, 1 H).

According to the synthetic method of Example 2-a, the following starting materials were reacted with compound 47-B through three steps to obtain the corresponding final products.

| Starting material | Final product | LCMS and NMR data |
|---|---|---|
| 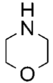 | 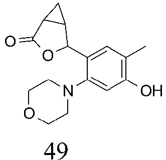 About 0.6% yield over three steps, yellow solid | LCMS: (ESI) m/z=290.1 (M+1)⁺; 1H NMR (400 MHz, DMSO-d6) δ ppm 9.23 - 9.69 (m, 1 H) 6.88 - 7.08 (m, 1 H) 6.62 - 6.75 (m, 1 H) 5.97 (d, J=4.63 Hz, 0.17 H) 5.61 (s, 0.76 H) 3.66 - 3.78 (m, 4 H) 2.77 - 2.89 (m, 2 H) 2.65 - 2.75 (m, 2 H) 2.31 - 2.39 (m, 1 H) 2.22 - 2.30 (m, 1 H) 2.04 - 2.12 (m, 3 H) 1.23 - 1.34 (m, 1 H) 0.81 - 1.06 (m, 1 H) |
| 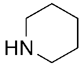 | 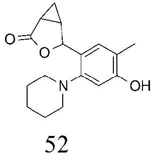 About 1.9% yield over three steps, gray solid | LCMS: (ESI) m/z=288.2 (M+1)⁺, ¹H NMR (400 MHz, CHLOROFORM-d) δ ppm 0.81 - 0.92 (m, 1 H) 0.97 - 1.14 (m, 1 H) 1.26 - 1.42 (m, 1 H) 1.46 - 1.72 (m, 6 H) 2.08 - 2.39 (m, 5 H) 2.56 - 3.21 (m, 4 H) 4.97 (br s, 1 H) 5.76 (br s, 1 H) 5.99 (br d, J=3.18 Hz, 1 H) 6.52 - 6.83 (m, 1 H) 6.95 - 7.20 (m, 1 H) |
| 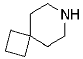 Hydrochloride salt Note: K₂CO₃ was used as the base in the first step instead of DIEA | 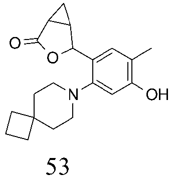 About 7.6% yield over three steps, as an off-white solid | LCMS: (ESI) m/z=328.4 (M+1)⁺, ¹H NMR (400 MHz, DMSO-*d*₆) *δ* (ppm) 9.20-9.57 (m, 1H), 6.82-7.10 (m, 1H), 6.60-6.75 (m, 1H), 5.58 (s, 1H), 2.67-2.79 (m, 2H), 2.56-2.65 (m, 2H), 2.29-2.38 (m, 1H), 2.24 (td, *J =* 5.0, 7.4 Hz, 1H), 2.08 (s, 3H), 1.85-1.95 (m, 2H), 1.75-1.83 (m, 4H), 1.69 (br t, *J* = 5.2 Hz, 4H), 1.22-1.37 (m, 1H), 0.97-1.07 (m, 1H) |
| 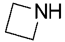 Note: K₂CO₃ was used as the base in the first step instead of DIEA | 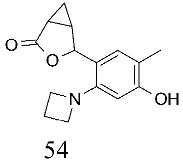 About 0.26% yield over three steps, gray solid | LCMS: (ESI) m/z = 260.0 (M+1) ⁺ |
| | | ¹H NMR (400 MHz, DMSO-d6) δ ppm 0.78 - 0.83 (m, 1 H) 1.02 (q, J=4.28 Hz, 1 H) 1.13 - 1.18 (m, 1 H) 1.26 - 1.34 (m, 1 H) 2.01 - 2.06 (m, 3 H) 2.18 - 2.32 (m, 4 H) 3.76 - 3.89 (m, 4 H) 5.38 (s, 1 H) 5.68 (d, J=4.28 Hz, 1 H) 6.00 - 6.08 (m, 1 H) 6.83 - 6.89 (m, 1 H) 9.05 - 9.49 (m, 1 H). |
| 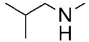 Note: DMSO was used as the solvant in the first step instead of dioxan | 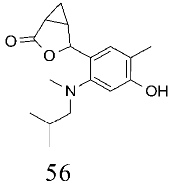 About 12% yield over three steps, yellow solid | LCMS:(ESI) m/z = 290.1(M+1)⁺, ¹H NMR (400 MHz, DMSO-*d*₆) δ ppm 0.82 - 0.94 (m, 6 H) 0.96 - 1.12 (m, 1 H) 1.22 - 1.34 (m, 1 H) 1.65 - 1.80 (m, 1 H) 2.02 - 2.11 (m, 3 H) 2.14 - 2.26 (m, 1 H) 2.27 - 2.37 (m, 1 H) 2.52 - 2.60 (m, 5 H) 5.75 (s, 1 H) 5.87 - 6.07 (m, 1 H) 6.58 - 6.74 (m, 1 H) 6.86 - 7.05 (m, 1 H) 9.44 (s, 1 H) |

### Example 2-b: Synthesis of Compound 55

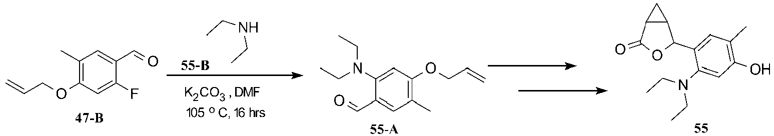

Step 1: According to the synthetic method of 31-A, 55-B (1.13 g, 15.45 mmol) was reacted with 47-B (1 g, 5.15 mmol) to obtain 55-A (250 mg, yield 19.63%). ¹H NMR (CDCl₃, 400 MHz) δ ppm 10.27 (s, 1 H) 7.63 - 7.75 (m, 1 H) 6.51 - 6.60 (m, 1 H) 6.01 - 6.18 (m, 1 H) 5.41 - 5.58 (m, 1 H) 5.35 (dd, J=10.64, 1.47 Hz, 1 H) 4.60 - 4.70 (m, 3 H) 3.17 (q, J=7.09 Hz, 4 H) 2.23 (s, 3 H) 1.07 (t, J=7.09 Hz, 6 H).

Steps 2 and 3: According to the procedure of steps 2 and 3 in Example 2, starting from 55-A, two-step reactions obtained compound 55 as a yellow solid in about 5.3% yield over two steps. LCMS: (ESI) m/z = 276.2 [M+H] ⁺, ¹H NMR (400 MHz, DMSO-d6) δ ppm 9.21 - 9.54 (m, 1 H) 6.83 - 7.04 (m, 1 H) 6.64 - 6.76 (m, 1 H) 5.95 (d, J=4.84 Hz, 0.201 H) 5.76 (s, 0.790 H) 2.80 - 2.97 (m, 4 H) 2.28 - 2.36 (m, 1 H) 2.14 - 2.21 (m, 1 H) 2.04 - 2.13 (m, 3 H) 1.23 - 1.34 (m, 1 H) 0.98 - 1.07 (m, 1 H) 0.86 - 0.97 (m, 6 H).

According to the synthetic method of Example 2-b, the following starting materials were reacted with compound 47-B through three steps to obtain the corresponding final products.

| | | | |
|---|---|---|---|
| 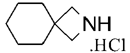 | 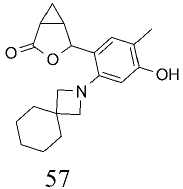 About 1.9% yield over three steps, yellow solid | LCMS: (ESI) m/z = 328.2 [M+H] ⁺, | |
| | | ¹H NMR (400 MHz, DMSO-*d*₆) δ ppm 9.05 - 9.34 (m, 1 H) 6.80 - 6.96 (m, 1 H) 6.01 (d, J=2.25 Hz, 1 H) 5.69 (d, J=4.38 Hz, 0.149 H) 5.38 (s, 0.848 H) 3.47 - 3.60 (m, 4 H) 2.33 (dd, J=3.56, 1.94 Hz, 1 H) 2.27 (ddd, J=8.76, 5.25, 3.13 Hz, 1 H) 2.01 (s, 3 H) 1.64 (br d, J=5.50 Hz, 4 H) 1.35 - 1.49 (m, 6 H) 1.25 - 1.32 (m, 1 H) 0.99 - 1.06 (m, 1 H) | |
| 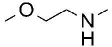 | 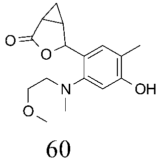 About 5.2% yield over three steps, white solid | ¹H NMR (400 MHz, DMSO-*d*₆) *δ* (ppm) 9.29-9.56 (m, 1H), 6.83-7.00 (m, 1H), 6.64-6.75 (m, 1H), 5.66-5.99 (m, 1H), 3.38 (q, *J* = 5.7 Hz, 2H), 3.19-3.25 (m, 3H), 2.62-3.00 (m, 2H), 2.56-2.62 (m, 3H), 2.19-2.36 (m, 2H), 2.03-2.18 (m, 3H), 0.99-1.35 (m, 1H), 0.74-0.98 (m, 1H). | |
| | | | LC-MS: (ESI) *m*/*z*. 292.1 [M+H]⁺; |
| 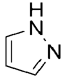 | 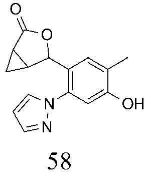 About 7.3% yield over three steps, white solid | ¹H NMR (400 MHz, DMSO-*d*₆) *δ* (ppm) 10.11 (br s, 1H), 7.99-8.08 (m, 1H), 7.75 (d, *J* = 1.5 Hz, 1H), 7.09-7.21 (m, 1H), 6.81 (s, 1H), 6.52 (t, *J* = 2.1 Hz, 1H), 5.15-5.71 (m, 1H), 2.00-2.30 (m, 5H), 0.95-1.31 (m, 1H), 0.78-0.94 (m, 1H). LC-MS: (ESI) *m*/*z*. [M+H]⁺ 271.2; | |

### Example 3-a: Synthesis of Compounds 26, 27, 28 and 29

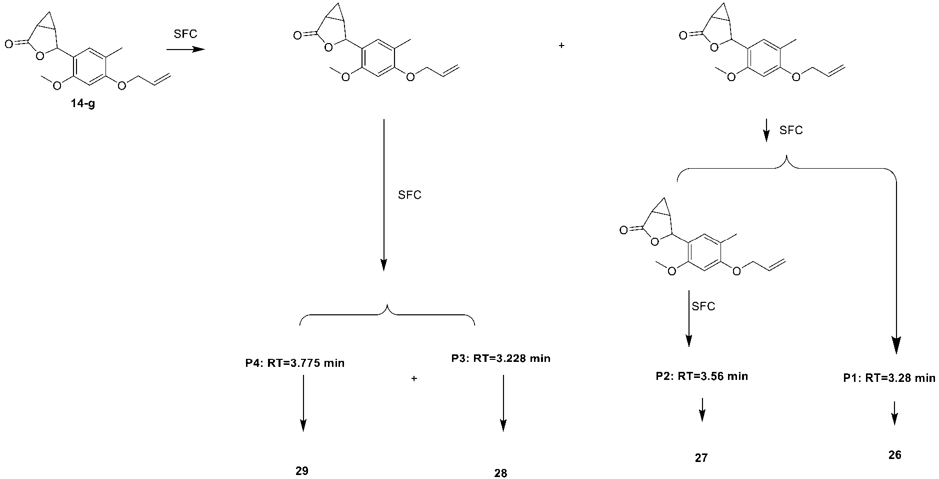

**Note: The four compounds P1, P2, P3, and P4 obtained from chiral resolution in the synthetic route each correspond to one of the following four structures; the absolute configurations have not been assigned.**

| | | |
|---|---|---|
| P1 | 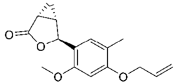 | 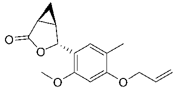 |
| P2 | | |
| P3 | 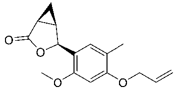 | 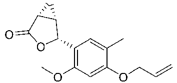 |
| P4 | | |

### Chiral Resolution of Compound 14-g

500 mg of compound **14-g** was resolved by SFC (Column: DAICEL CHIRALPAK AD (250mm×30mm, 10um); Mobile phase: [0.1% NH3H2O EtOH]; B%: 10%-10%) to obtain a colorless oil mixture containing four compounds (130 mg, 473.91 µmol, 26.00% yield) and a mixture containing P3 and P4. The P3/P4 mixture was further resolved by SFC (Column: REGIS(S,S) WHELK-O1 (250mm×25mm, 10um); Mobile phase: [0.1% NH3H2O EtOH]; B%: 30%-30%, 45 min) to obtain compound P3 and compound P4.

White solid (**P3**) (100 mg, 364.55 µmol, 20.00% yield). ¹H NMR (400 MHz, CHLOROFORM-d) Shift 6.97-7.09 (m, 1H), 6.47 (s, 1H), 6.02-6.17 (m, 1H), 5.84 (d, J=4.77 Hz, 0.1H), 5.41-5.58 (m, 1.9H), 5.32 (dd, J=1.41, 10.58 Hz, 1H), 4.59 (td, J=1.50, 4.95 Hz, 2H), 3.82-3.93 (m, 3H), 2.63-2.72 (m, 0.1H), 2.15-2.29 (m, 5H), 1.23-1.40 (m, 1.1H), 0.98-1.11 (m, 1H), 0.80-0.89 (m, 0.1H). (ESI) m/z=275.3 (M+1) +, RT= 0.94 min. SFC:RT= 3.227 min.

White solid (**P4**) (100 mg, 364.55 µmol, 20.00% yield). ¹H NMR (400 MHz, CHLOROFORM-d) Shift 7.05 (s, 1H), 6.47 (s, 1H), 5.98-6.20 (m, 1H),, lq 5.84 (d, J=4.65 Hz, 0.07H), 5.51 (s, 0.93H), 5.46 (qd, J=1.61, 17.29 Hz, 1H), 5.27-5.38 (m, 1H), 4.59 (td, J=1.54, 4.98 Hz, 2H), 3.85 (s, 3H), 2.11-2.30 (m, 5H), 1.22-1.40 (m, 1H), 0.97-1.13 (m, 1H), 0.78-0.91 (m, 0.11H).LCMS: (ESI) m/z=275.3 (M+1) ⁺, RT= 0.94 min. SFC: RT= 3.751 min.

130 mg of the colorless oil mixture containing four compounds was further resolved by SFC (Column: DAICEL CHIRALPAK IG (250mm×30mm,10um); Mobile phase: [CO₂-i-PrOH(0.1% NH3H2O)]; B%: 30%-30%, min) to obtain compound P1 and 100 mg of a mixture containing P2, P3, and P4.

White solid (**P1**) (20 mg, 72.91 µmol, 4.0% yield). LCMS: (ESI) m/z=275.3 (M+1) ⁺, RT= 0.85 min. SFC: RT= 3.28 min.

100 mg of the mixture containing P2, P3, and P4 was subjected to a third SFC resolution (Column: REGIS(S,S) WHELK-O1 (250mm×25mm,10um); Mobile phase: [CO₂-EtOH(0.1% NH3H2O)]; B%: 25%-25%, 45 min) to obtain compound P2.

White solid (**P2**) (18 mg, 65.62 µmol, 3.6% yield). LCMS: (ESI) m/z=275.3 (M+1) ⁺, RT= 1.06 min. SFC: RT= 3.56 min.

Note: The SFC RT results reported in the characterization data of the compounds obtained from chiral resolution of compound **14-g** were determined under the following analytical conditions: Column: (S,S) Whelk-01 100×4.6mm I.D., 5.0 um; Mobile phase: A: CO₂, B: Ethanol (0.05% DEA); Gradient: from 5% to 40% of B in 4.5 min and hold 40% for 2 min, then 5% of B for 1.5 min; Flow rate: 2.5 mL/min; Column temperature: 40 °C; ABPR: 100 bar.

### Synthesis of Compound 28

According to the final step of the synthetic method 2 for compound **14,** compound **P3** (90 mg, 328.09 µmol) was reacted to obtain compound **28** as a pale yellow solid (50 mg, 209.18 µmol, 63.76% yield). ¹H NMR (400 MHz, DMSO-d6) Shift 9.28-9.63 (m, 1H), 6.79-6.98 (m, 1H), 6.45-6.55 (m, 1H), 5.73 (d, J=4.52 Hz, 0.2H), 5.39 (s, 0.8H), 3.66-3.80 (m, 3H), 2.12-2.29 (m, 1.8H), 2.05 (s, 3H), 1.21-1.36 (m, 1H), 1.07 (td, J=4.39, 7.61 Hz, 0.2H), 0.96 (q, J=4.28 Hz, 0.8H), 0.71 (br d, J=3.42 Hz, 0.2H). LCMS: (ESI) m/z= 235.2 (M+1) ⁺, RT=0.76 min.

### Synthesis of Compound 29

According to the final step of the synthetic method 2 for compound **14,** compound **P4** (90 mg, 328.09 µmol) was reacted to obtain compound **29** as a pale yellow solid (60 mg, 251.02 µmol, 76.51% yield). LCMS: (ESI) m/z= 235.2 (M+1) ⁺, RT=0.76 min. ¹H NMR (400 MHz, DMSO-d6) Shift 9.26-9.66 (m, 1H), 6.77-7.03 (m, 1H), 6.45-6.62 (m, 1H), 5.73 (d, J=4.65 Hz, 0.2H), 5.39 (s, 0.8H), 3.62-3.83 (m, 3H), 2.10-2.28 (m, 1.8H), 2.05 (s, 3H), 1.14-1.34 (m, 0.2H), 0.96 (q, J=4.24 Hz, 0.8H), 0.65-0.76 (m, 0.2H).

### Synthesis of Compound 26

According to the final step of the synthetic method 2 for compound **14,** compound **P1** (18 mg, 65.62 µmol) was reacted and then purified by preparative HPLC (Column: Boston Prime C18 150×30mm×5um; Mobile phase: [water(FA)-ACN]; B%: 23%-43%, 16 min) to obtain compound **26** as a white solid (8 mg, 34.15 µmol, 52.05% yield). LCMS: (ESI) m/z= 235.2 (M+1) +, RT=0.890 min. ¹H NMR (400 MHz, DMSO-d6) Shift 9.23-9.72 (m, 1H), 6.74-7.07 (m, 1H), 6.40-6.63 (m, 1H), 5.73 (d, J=4.65 Hz, 1H), 5.39 (s, 1H), 3.60-3.80 (m, 3H), 2.13-2.28 (m, 2H), 2.05 (s, 3H), 1.02-1.36 (m, 1H), 0.66-1.00 (m, 1H).

### Synthesis of Compound 27

According to the final step of the synthetic method 2 for compound **14,** compound **P2** (18 mg, 65.62 µmol) was reacted and then purified by preparative HPLC (Column: Boston Prime C18 150×30mm×5um; Mobile phase: [water(HCl)-ACN]; B%: 25%-45%, 16 min) to obtain compound **27** as a pale yellow solid (5 mg, 19.64 µmol, 29.93% yield). LCMS: (ESI) m/z= 235.2 (M+1)⁺, RT=0.838 min. ¹H NMR (400 MHz, DMSO-d6) Shift 9.29-9.64 (s, 1H), 6.78-7.00 (s, 1H), 6.40-6.57 (s, 1H), 5.73 (d, J=4.52 Hz, 0.2H), 5.39 (s, 0.8 H), 3.70-3.77 (s, 3H), 2.11-2.26 (m, 1.8H), 2.05 (s, 3H), 1.23-1.34 (m, 1.2 H), 1.07 (dt, J=4.46, 8.28 Hz, 0.2H), 0.96 (q, J=4.28 Hz, 0.8H), 0.88 (br d, J=5.14 Hz, 0.2H), 0.68-0.74 (m, 0.2H).

### Example 3-b: Synthesis of Compounds 268 and 269

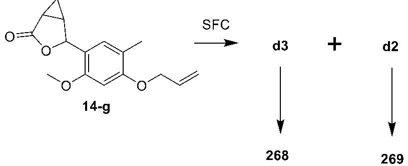

**Note: The two compounds d1 and d2 obtained from chiral resolution in the synthetic route each correspond to one of the following two structures; the absolute configurations have not been assigned.**

| | |
|---|---|
| d3 | 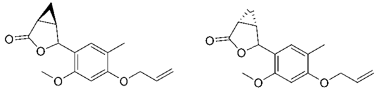 |
| d2 | |

### Chiral Resolution of Compound 14-g

10 g of compound **14-g** was resolved by SFC (Column: REGIS (s,s) WHELK-O1 (250 mm×50 mm, 10 um); Mobile phase: [CO₂-EtOH (0.1% NH₃H₂O)]; B%: 25%, isocratic elution mode; Column temperature: 35 °C; Flow rate: 200 mL/min) to obtain compound **d3** as an off-white solid (0.9 g, 3.28 mmol, 9.00% yield) and compound **d2** as an off-white solid (1.1 g, 4.01 mmol, 11.00% yield).

Compound **d3:** (ESI) m/z = 275.1 (M+1) ⁺, RT = 1.426 min. SFC: RT = 3.217 min, ~19.1% and RT = 3.547 min, ~80.9%. ¹H NMR (400 MHz, DMSO-d6) δ 6.91 (s, 1H), 6.66-6.73 (m, 1H), 5.98-6.17 (m, 1H), 5.77 (d, J=4.65 Hz, 1H), 5.37-5.55 (m, 1H), 5.28 (dd, J=1.41, 10.58 Hz, 1H), 4.56-4.70 (m, 2H), 3.79-3.89 (m, 3H), 2.57 (qd, J=4.92, 7.50 Hz, 1H), 2.14-2.30 (m, 1H), 2.10 (s, 3H), 1.02-1.10 (m, 1H), 0.65-1.01 (m, 1H).

Compound **d2:** (ESI) m/z = 275.1 (M+1) ⁺, RT = 1.423 min. SFC: RT = 3.264 min, ~63.3% and RT = 3.745 min, ~36.7%. ¹H NMR (400 MHz, DMSO-d6) δ 6.87-7.04 (m, 1H), 6.68 (d, J=4.28 Hz, 1H), 6.01-6.17 (m, 1H), 5.76 (d, J=4.52 Hz, 1H), 5.37-5.52 (m, 2H), 5.27 (td, J=1.50, 10.58 Hz, 1H), 4.48-4.77 (m, 2H), 3.82 (d, J=8.80 Hz, 3H), 2.57 (qd, J=4.89, 7.60 Hz, 1H), 2.14-2.27 (m, 2H), 2.10 (d, J=2.20 Hz, 3H), 1.03-1.31 (m, 1H), 0.66-1.01 (m, 1H).

Note: The SFC RT results reported in the characterization data of the compounds obtained from chiral resolution of compound **14-g** were determined under the following analytical conditions: Column: (S,S) Whelk-01 100×4.6mm I.D., 5.0um; Mobile phase: A: CO₂, B: Ethanol (0.05% DEA); Gradient: from 5% to 40% of B in 4.5 min and hold 40% for 2 min, then 5% of B for 1.5 min; Flow rate: 2.5 mL/min; Column temperature: 40 °C; ABPR: 100 bar.

### Synthesis of Compound 268

According to the final step of the synthetic method 2 for compound **14,** compound **d3** (850 mg, 3.10 mmol) was reacted to obtain compound **268** as a white solid (315 mg, 1.34 mmol, 43.4% yield). LCMS: (ESI) m/z= 235.0 (M+1)⁺, RT=1.062 min. SFC: RT = 3.790 min, 74.64% and RT = 4.125 min, 25.28%. ¹H NMR (400 MHz, DMSO-d6) δ 9.25-9.73 (m, 1H), 6.75-7.04 (m, 1H), 6.33-6.61 (m, 1H), 5.29-5.81 (m, 1H), 3.64-3.79 (m, 3H), 2.12-2.26 (m, 2H), 2.04 (s, 3H), 1.28 (dt, J=4.52, 8.07 Hz, 1H), 0.95 (q, J=3.95 Hz, 1H).

### Synthesis of Compound 269

According to the final step of the synthetic method 2 for compound **14,** compound **d2** (18 mg, 52.5 µmol) was reacted to obtain compound **269** as a white solid (8 mg, 34.15 µmol, 52.05% yield). LCMS: (ESI) m/z= 235.2 (M+1)⁺, RT=0.890 min. ¹H NMR (400 MHz, DMSO-d6) 9.23-9.72 (m, 1H), 6.74-7.07 (m, 1H), 6.40-6.63 (m, 1H), 5.73 (d, J=4.65 Hz, 1H), 5.39 (s, 1H), 3.60-3.80 (m, 3H), 2.13-2.28 (m, 2H), 2.05 (s, 3H), 1.02-1.36 (m, 1H), 0.66-1.00 (m, 1H). SFC: RT = 3.966 min, 20.82% and RT = 4.278 min, 79.98%.

Note: The SFC RT results reported for compounds 268 and 269 were determined under the following analytical conditions: Column: Chiralpak AD-3 150×4.6mm I.D., 3um; Mobile phase: A: CO₂, B: iso-propanol (0.05% DEA); Gradient: from 5% to 40% of B in 4 min and hold 40% for 2 min, then 5% of B for 2 min; Flow rate: 2.5 mL/min; Column temperature: 35 °C; ABPR: 1500 psi.

### Example 4: Synthesis of Compound 2

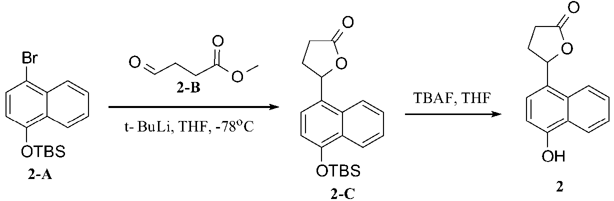

Step 1: n-Butyllithium solution in THF (0.71 mL, 2.4 mol/L, 0.85 mmol) was added to a solution of compound **2-A** (250 mg) in THF (10 mL) at -78 °C. The mixture was stirred under a nitrogen atmosphere for 0.5 hours. Then compound **2-B** (172 mg, 1.48 mmol) was added, and the mixture was stirred at -78 °C for 3 hours. After the reaction, water (20 mL) was added to quench, and the mixture was extracted with EA (25 mL×3). After conventional work-up procedure, **2-C** was obtained as a crude yellow oil (90 mg). LC-MS: (ESI) m/z [M+H]⁺ 343.1.

Step 2: Tetrabutylammonium fluoride (244 mg, 0.93 mmol) was added to a solution of compound **2-C** (90 mg, crude) in THF (10 mL) at -78 °C. The mixture was stirred at 25 °C for 1 hour. After the reaction, water (10 mL) was added to quench, and the mixture was extracted with EA (20 mL×3). After conventional work-up procedure, the residue was purified by silica gel column chromatography (EA:PE=0 to 10%:90%) to obtain compound **2** as a yellow solid (60 mg). ¹HNMR (400 MHz, DMSO-*d₆*) δ 8.19 - 8.06 (m, 2H), 7.56 - 7.43 (m, 3H), 7.22 - 7.18 (m, 1H), 6.89 - 6.87 (m, 1H), 6.16 - 6.11 (m, 1H), 3.30 - 3.27 (m, 4H). LC-MS: (ESI) m/z [M+H]⁺ 229.7.

### Example 4-a: Synthesis of Compound 1

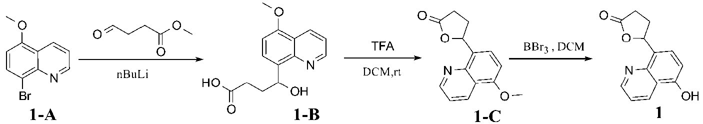

Step 1: According to the procedure of step 1 in Example 4, compound 1-A (400 mg, 1.7 mmol) was reacted to obtain compound 1-B (160 mg, yield 36%). LC-MS: (ESI) m/z [M+H]⁺ 262.

Step 2: A solution of compound 1-B (180 mg, 0.68 mmol) in TFA (1.02 mL) and DCM (15 mL) was stirred at room temperature for 2 hours. The mixture was concentrated under reduced pressure, and the residue was purified by silica gel column chromatography (EA:PE, EA from 0 to 25%) to obtain compound 1-C as a white solid (122 mg, yield 73%). LC-MS: (ESI) m/z [M+H]⁺ 244.1.

Step 3: Boron tribromide (2M in DCM, 1.25 mL) was added to a solution of compound 1-C (122 mg, 0.5 mmol) in DCM (10 mL) at -10 °C. The mixture was stirred at -10 °C for 2 hours. After the reaction, the mixture was diluted with EA (30 mL) and washed with saturated NaHCO₃ solution (15 mL×2). After conventional work-up procedure 2, the residue was purified by prep HPLC (column: YMC-Actus Triart C18 20×250 mm, 5 um, phase A: H₂O (0.1% FA), phase B: ACN, 10% -95%) to obtain compound 1 as a yellow solid (51.4 mg, yield 44%). LC-MS (ESI) m/z: 230.1 (M+H)⁺; ¹H NMR (DMSO-*d*₆, 400 MHz): *δ* (ppm) 10.66 (s, 1H), 8.90 (dd, *J =* 4.2, 1.8 Hz, 1H), 8.55 (dd, *J =* 8.4, 1.8 Hz, 1H), 8.15 (s, 1H), 7.58 (d, *J =* 8.0 Hz, 1H), 7.55-7.49 (m, 1H), 6.96 (d, *J =* 8.0 Hz, 1H), 6.42 (t, *J* = 7.2 Hz, 1H), 2.81-2.58 (m, 3H), 2.26-2.14 (m, 1H).

### Example 4-b: Synthesis of Compound 287

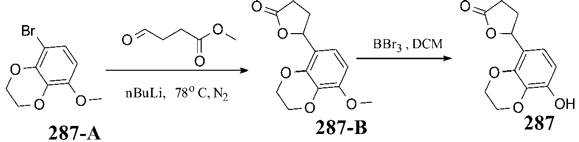

Step 1: n-Butyllithium solution in THF (1.6 M in hexane, 1.53 mL, 2.45 mmol) was added to a solution of compound 287-A (400 mg, 1.63 mmol) in THF (5 mL) at -78 °C. T he mixture was stirred under a nitrogen atmosphere for 0.5 hours. Then compound **2-B** (1.96 mmol) was added, and the mixture was stirred at -78 °C for 3 hours. After the reaction, saturated ammonium chloride solution (10 mL) was added to quench, and the mixture was extracted with EA (15 mL×3). After conventional work-up procedure 2, the residue was purified by silica gel column chromatography (EA:PE, EA from 0 to 16%) to obtain compound 287-B as a yellow solid (165 mg, yield 40.4%). LC-MS: (ESI) m/z [M+H]⁺ 251.1.

Step 2: According to the procedure of step 3 in Example 4-a, compound 287-B (160 mg) was reacted to obtain compound **1** as a yellow solid (7.1 mg, yield 4.7%). LC-MS: (ESI) m/z: 237 (M+H)⁺; ¹H NMR (DMSO-*d*₆, 400 MHz): *δ* (ppm) 9.28 (s, 1H), 6.67 (d, *J =* 8.4 Hz, 1H), 6.40 (d, *J* = 8.4 Hz, 1H), 5.57 (t, *J* = 7.5 Hz, 1H), 4.29-4.20 (m, 4H), 2.70-2.52 (m, 2H), 2.48-2.41 (m, 1H), 2.20-2.08 (m, 1H).

### Example 5: Synthesis of Compounds 3, 112, 107

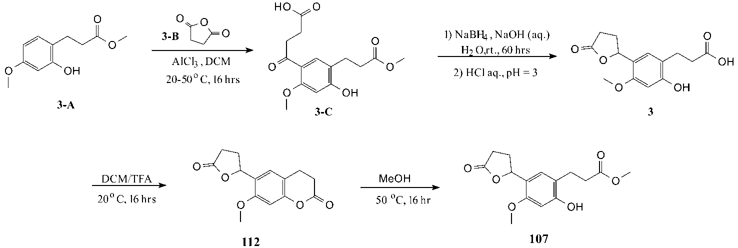

Step 1: Aluminum trichloride (6.98 g, 52.32 mmol) was added to a solution of compound **3-A** (5.0 g, 23.78 mmol) in DCM (125 mL) at 20 °C. After stirring for 10 minutes, compound **3-B** (3.57 g, 35.68 mmol) was added in one portion, and the mixture was stirred at 20 °C for 30 minutes. The solvent was removed under reduced pressure, the residue was diluted with water (100 mL), adjusted to pH 1-2 with 1M HCl, and extracted with EA (100 mL×3). After conventional work-up procedure 2, the residue was purified by silica gel column chromatography (EA:PE=0 to 70%:30%) to obtain compound **3-C** as a yellow solid (1.5 g, 4.59 mmol, yield 19.31%). LCMS: (ESI) m/z=310.9(M+1)^{+ 1}. ¹H NMR (400 MHz, DMSO-d6) Shift 12.02 (br s, 1H), 10.42 (s, 1H), 7.50 (s, 1H), 6.54 (s, 1H), 3.83 (s, 3H), 3.57 (s, 3H), 3.08 (t, J=6.40 Hz, 2H), 2.70-2.79 (m, 2H), 2.52-2.55 (m, 2H), 2.45-2.49 (m, 2H).

Step 2 (Representative procedure for constructing the five-membered lactone ring): NaOH (309.38 mg, 7.73 mmol) and NaBH₄ (292.60 mg, 7.73 mmol) were added to a mixture of compound **3-C** (800 mg, 2.58 mmol) in water (30 mL). The mixture was stirred at 20 °C for 60 hours. The pH was adjusted to 1-2 with 2M HCl, and a white solid precipitated. The mixture was extracted with EA (100 mL×3). After conventional work-up procedure, the residue was triturated with EA (10 mL), filtered, and washed with EA (3 mL×2) to obtain compound **3** as a white solid (520 mg, 1.76 mmol, yield 68.37%). LCMS: (ESI) m/z=280.9(M+1) ⁺; ¹HNMR (400 MHz, DMSO-d6) Shift 12.04 (br s, 1H), 9.61 (br s, 1H), 7.00 (s, 1H), 6.48 (s, 1H), 5.59 (t, J=7.53 Hz, 1H), 3.72 (s, 3H), 2.66-2.72 (m, 2H), 2.62 (t, J=9.41 Hz, 1H), 2.53-2.59 (m, 1H), 2.40-2.46 (m, 3H), 2.03-2.15 (m, 1H).

Step 3: A solution of compound **3** (100 mg, 0.36 mmol) in DCM (3.0 mL) and TFA (0.5 mL) was stirred at 20 °C for 16 hours. The solvent was removed under reduced pressure, and the residue was purified by silica gel column chromatography (EA:PE=0 to 5%:95%) to obtain compound **112** as a white solid (77 mg, 0.28 mmol, yield 78.17%). LCMS: (ESI) m/z=263.1(M+1)⁺, 285.1(M+Na)⁺; ¹H NMR (400 MHz, CHLOROFORM-d) Shift 7.17 (s, 1H), 6.63 (s, 1H), 5.73 (t, J=7.03 Hz, 1H), 3.85 (s, 3H), 2.92-3.03 (m, 2H), 2.78-2.85 (m, 2H), 2.68-2.77 (m, 1H), 2.61-2.67 (m, 2H), 2.04-2.17 (m, 1H).

Step 4: A solution of compound **112** (70 mg, 0.27 mmol) in MeOH (2 mL) was stirred at 50 °C for 16 hours. The solvent was removed under reduced pressure, and the residue was purified by silica gel column chromatography (EA:PE=0 to 35%:65%) to obtain compound **107** as a white solid (35 mg, 0.11 mmol, yield 42.33%). LCMS: (ESI) m/z=294.9 (M+1)⁺; ¹H NMR (400 MHz, CHLOROFORM-d) Shift 7.90 (s, 1H), 7.01 (s, 1H), 6.52 (s, 1H), 5.63-5.74 (m, 1H), 3.80 (s, 3H), 3.72 (s, 3H), 2.78-2.91 (m, 2H), 2.70-2.77 (m, 2H), 2.59-2.69 (m, 3H), 2.06-2.19 (m, 1H).

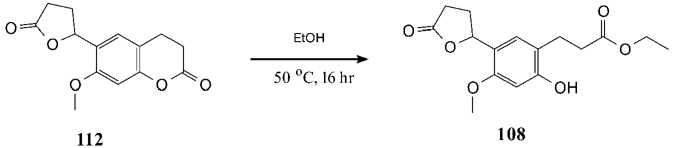

A solution of compound **112** (70 mg, 0.27 mmol) in EtOH (2 mL) was stirred at 50 °C for 16 hours. The solvent was removed under reduced pressure, and the residue was purified by silica gel column chromatography (EA:PE=0 to 35%:65%) to obtain compound 108 as a white solid (25 mg, 0.077 mmol, yield 28.86%). LCMS: (ESI) m/z=308.9 (M+1)⁺, 330.9 (M+Na)⁺; ¹H NMR (400 MHz, CHLOROFORM-d) Shift 8.04 (s, 1H), 7.01 (s, 1H), 6.52 (s, 1H), 5.65-5.72 (m, 1H), 4.17 (q, J=7.03 Hz, 2H), 3.80 (s, 3H), 2.79-2.87 (m, 2H), 2.69-2.74 (m, 2H), 2.59-2.68 (m, 3H), 2.06-2.19 (m, 1H), 1.27 (t, J=7.15 Hz, 3H).

### Example 5-a: Synthesis of Compound 109

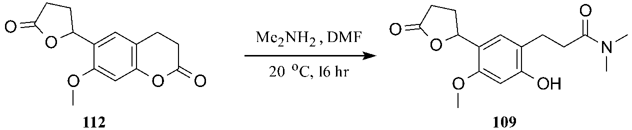

Dimethylamine solution in THF (2 M, 114.39 uL) was added to a solution of compound **112** (60 mg, 0.23 mmol) in DMF (2 mL). The mixture was stirred at 20 °C for 16 hours. LCMS showed only a small amount of starting material remained. The solvent was removed under reduced pressure, and the residue was purified by silica gel column chromatography (EA:PE, EA from 0 to 60%) to obtain compound **109** as a white solid (30 mg, yield 41%). LCMS: (ESI) m/z=308.2 (M+1)⁺, ¹H NMR (400 MHz, DMSO-d6) Shift 9.72 (s, 1H), 7.00 (s, 1H), 6.47 (s, 1H), 5.59 (t, J=7.65 Hz, 1H), 3.72 (s, 3H), 2.93 (s, 3H), 2.81 (s, 3H), 2.60-2.72 (m, 3H), 2.52-2.59 (m, 2H), 2.39-2.50 (m, 2H), 2.02-2.19 (m, 1H).

According to the synthetic method of Example 5-a, the following starting materials were reacted with compound 112 to obtain the corresponding final products.

| Starting material | Final product | LCMS and NMR data |
|---|---|---|
| Methylamine (33% solution in ethanol) | 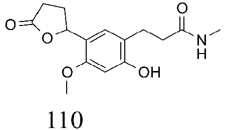 Yield 42%, white solid | LCMS:(ESI) m/z=394.1(M+1)⁺, |
| | | ¹H NMR (400 MHz, DMSO-d6) Shift 9.61 (s, 1H), 7.76 (br d, J=4.52 Hz, 1H), 6.97 (s, 1H), 6.47 (s, 1H), 5.59 (t, J=7.65 Hz, 1H), 3.72 (s, 3H), 2.89 (s, 1H), 2.62-2.71 (m, 3H), 2.56-2.61 (m, 1H), 2.55 (d, J=4.52 Hz, 3H), 2.39-2.48 (m, 1H), 2.28 (t, J=7.78 Hz, 2H), 2.00-2.14 (m, 1H) |
| 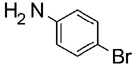 Stirred at 80 °C until the starting material was completely consumed (160 h) | 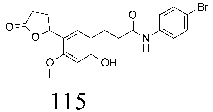 Yield 11.5%, yellow solid | LCMS: (ESI) m/z=435.8(M+1) ⁺; |
| | | ¹H NMR (400 MHz, DMSO-d6) Shift 10.02 (s, 1H), 9.63 (s, 1H), 7.51-7.60 (m, 2H), 7.42-7.50 (m, 2H), 7.02 (s, 1H), 6.49 (s, 1H), 5.59 (t, J=7.58 Hz, 1H), 3.72 (s, 3H), 2.73-2.82 (m, 2H), 2.58-2.67 (m, 1H), 2.52-2.57 (m, 3H), 2.37-2.46 (m, 1H), 1.95-2.09 (m, 1H) |
| 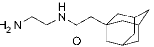 | 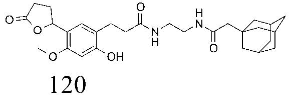 Yield 45%, white solid | LCMS: (ESI) m/z=499.3 (M+H) ⁺; |
| | | ¹H NMR (400 MHz, DMSO-d6) Shift 9.61 (s, 1H), 7.82 (br s, 1H), 7.69 (br s, 1H), 6.95 (s, 1H), 6.46 (s, 1H), 5.58 (t, J=7.65 Hz, 1H), 3.71 (s, 3H), 3.05 (br s, 4H), 2.58-2.70 (m, 3H), 2.53-2.57 (m, 1H), 2.39-2.46 (m, 1H), 2.21-2.30 (m, 2H), 2.00-2.11 (m, 1H), 1.89 (br s, 3H), 1.79 (s, 2H), 1.65 (br s, 1H), 1.62 (br s, 2H), 1.57 (br s, 2H), 1.53 (br s, 7H) |
| 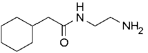 | 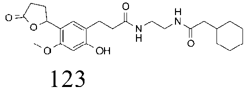 Yield 51.3%, white solid | LCMS: (ESI) m/z=447.3 (M+H) ⁺; |
| | | ¹H NMR (400 MHz, DMSO-d6) Shift 9.62 (s, 1H), 7.63-7.94 (m, 2H), 6.97 (s, 1H), 6.47 (s, 1H), 5.59 (t, J=7.53 Hz, 1H), 3.72 (s, 3H), 2.94-3.12 (m, 4H), 2.59-2.72 (m, 3H), 2.52-2.58 (m, 1H), 2.38-2.47 (m, 1H), 2.28 (br t, J=7.78 Hz, 2H), 1.98-2.13 (m, 1H), 1.92 (d, J=7.03 Hz, 2H), 1.62 (br d, J=7.03 Hz, 6H), 1.02-1.26 (m, 3H), 0.76-0.94 (m, 2H) |
| 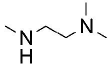 | 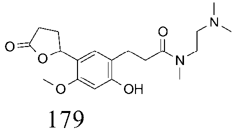 Yield 26%, grayish-white solid | LC-MS: (ESI) *m*/*z.* [M+1]⁺ 365.3; ¹H NMR (400 MHz, DMSO-*d*₆) *δ* (ppm) 8.20 (s, 1H), 6.99 (d, *J* = 5.9 Hz, 1H), 6.48 (s, 1H), 5.59 (t, *J* = 7.6 Hz, 1H), 4.70-4.80 (m, 0.1H), 3.67 (s, 3H), 3.28-3.47 (m, 2H), 2.77-2.97(s, 3H), 2.55-2.72 (m, 4H), 2.30-2.50 (m, 5H), 2.18-2.25 (m 7H) |
| 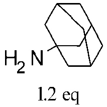 Stirred at 50 °C for 16 hours, some starting material remained unreacted | 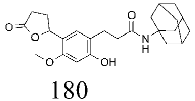 Yield 12%, white solid | LC-MS: (ESI) *m*/*z.* [M+1]⁺ 414.3; |
| | | ¹H NMR (400 MHz, DMSO-*d*₆) *δ* (ppm) 9.56 (br s, 1H), 7.25 (s, 1H), 6.98 (s, 1H), 6.47 (s, 1H), 5.59 (t, *J* = 7.6 Hz, 1H), 3.72 (s, 3H), 2.61-2.67 (m, 3H), 2.53-2.54 (m, 1H), 2.45-2.55 (m, 1H), 2.24 (t, *J =* 7.6 Hz, 2H), 2.05-2.13 (m, 1H), 1.99 (br s, 3H), 1.89 (s, 6H), 1.60 (br s, 6H) |
| 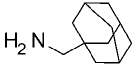 Stirred at 25 °C for 16 hours, some starting material remained unreacted | 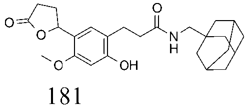 Yield 5.8%, white solid | LC-MS: (ESI) *m*/*z.* [M+1]⁺ 428.4; |
| | | ¹H NMR (400 MHz, DMSO-*d*₆) *δ* (ppm) 9.56 (s, 1H), 7.62 (br t, *J* = 6.2 Hz, 1H), 6.98 (s, 1H), 6.47 (s, 1H), 5.59 (t, *J=* 7.6 Hz, 1H), 3.71 (s, 3H), 2.74 (d, *J* = 6.2 Hz, 2H), 2.62-2.68 (m, 3H), 2.53-2.60 (m, 1H), 2.40-2.49 (m, 1H), 2.36 (t, *J =* 7.6 Hz, 2H), 2.0-2.15 (m, 1H), 1.88 (br s, 3H), 1.51-1.70 (m, 6H), 1.36 (d, *J* = 2.0 Hz, 6H) |

According to the synthetic method of Example 5-a, the following starting materials were reacted to obtain the corresponding final products.

| Starting material | Final product | LCMS and NMR data |
|---|---|---|
| 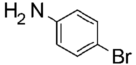 and 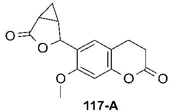 Stirred at 80 °C until the starting material was completely consumed (16 h) | 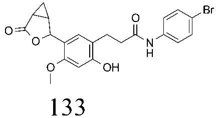 Yield 33%, white solid | LCMS:(ESI) m/z= 446.2 (M+1)⁺, |
| | | ¹H NMR (400 MHz, DMSO-d6) Shift 9.95-10.11 (m, 1H), 9.51-9.77 (m, 1H), 8.15 (s, 1H), 7.51-7.64 (m, 2H), 7.41-7.50 (m, 2H), 6.85-7.02 (m, 1H), 6.46-6.56 (m, 1H), 5.73 (d, J=4.84 Hz, 0.23H), 5.37 (s, 0.73H), 3.70-3.80 (m, 3H), 2.77 (br t, J=7.70 Hz, 2H), 2.52-2.64 (m, 2H), 2.11-2.24 (m, 2H),1.27 (dt, J=4.51, 8.20 Hz, 1H), 0.90-1.01 (m, 1H), 0.58-0.65 (m, 0.24H) |
| 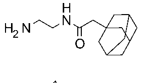 and 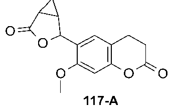 | 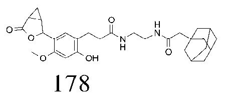 Yield 14%, white solid | LC-MS: (ESI) *m*/*z.* [M+H]⁺ 511.4 |
| | | ¹H NMR (400 MHz, DMSO-*d*₆) *δ* (ppm) 9.30-9.90 (m, 1H), 8.18 (s, 1H), 7.82 (br s, 1H), 7.68 (br s, 1H), 6.70-7.00 (s, 1H), 6.49 (s, 1H), 5.71-5.72 (d, *J* = 4.4 Hz, 0.22H), 5.37 (s, 0.75H), 3.72 (s, 3H), 3.06 (br s, 4H), 2.66 (br t, *J* = 7.6 Hz, 2H), 2.27 (br t, *J* = 7.8 Hz, 2H), 2.19 (br dd, *J =* 4.3, 7.6 Hz, 2H), 1.90 (br s, 3H), 1.79 (s, 2H), 1.5-1.7 (m, 12H), 1.28 (dt, *J =* 4.5, 8.0 Hz, 0.8H), 1.0-1.1 (m, 0.3H), 0.95 (q, *J =* 4.2 Hz, 0.7H), 0.6-0.7 (m, 0.25H) |
| 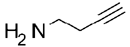 and 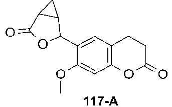 | 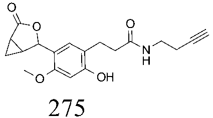 Yield 13.3%, white solid | ¹H NMR (400 HMz, DMSO-*d*₆) *δ* (ppm) 9.69 (br s, 1H), 7.90-8.08 (m, 1H), 6.75-6.97 (m, 1H), 6.37-6.56 (m, 1H), 5.72 (d, *J* = 4.6 Hz, 1H), 5.37 (s, 1H), 3.70-3.76 (m, 3H), 3.10-3.17 (m, 2H), 2.79-2.84 (m, 1H), 2.60-2.73 (m, 2H), 2.13-2.31 (m, 6H), 1.00-1.32 (m, 1H), 0.66-0.98 (m, 1H). LC-MS: (ESI) *m*/*z.* [M+H]⁺ 344.3; |
| 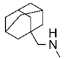 and 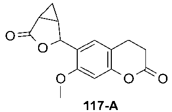 | 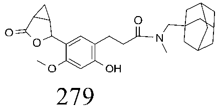 Yield 13%, white solid | ¹H NMR (400 MHz, DMSO-*d₆*) *δ* (ppm) 9.50-9.81 (m, 1H), 6.77-7.02 (m, 1H), 6.42-6.54 (m, 1H), 5.27-5.76 (m, 1H), 3.68-3.78 (m, 3H), 2.83 (s, 5H), 2.62-2.72 (m, 2H), 2.52-2.57 (m, 2H), 2.11-2.26 (m, 2H), 1.85-1.95 (m, 3H), 1.51-1.69 (m, 6H), 1.38-1.49 (m, 6H), 1.19-1.33 (m, 1H), 0.91-1.09 (m, 1H). LC-MS: (ESI) *m*/*z.* [M+H]⁺ 454.2. |

### Example 5-b: Synthesis of Compound 130

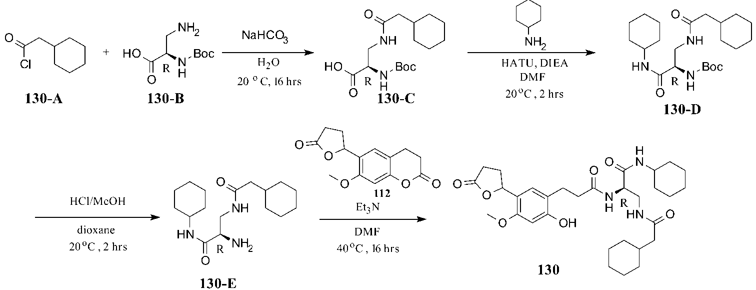

Step 1: Compound 130-A (3.70 g, 23.1 mmol) was added to a solution of compound 130-B (1.57 g, 7.69 mmol) in aqueous NaHCO₃ (646 mg, 7.69 mmol) solution (16 mL). NaHCO₃ (2.26 g, 26.9 mmol in 1.05 mL water) was added periodically to maintain the pH at 7.5, and the mixture was stirred at 20 °C for 16 hours. Phosphoric acid (5 mL) was added to adjust the pH to 4-5, and the mixture was extracted with EtOAc (15 mL×3). The organic phase was cooled to 0 °C, and the precipitated solid was collected by filtration. The crude product was recrystallized from EtOAc (4 mL) to obtain compound 130-C as a white solid (2.1 g, yield 83%). ¹H NMR (400MHz, DMSO-d6) Shift = 12.50 (br s, 1H), 7.88 (br t, J=5.4 Hz, 1H), 6.91 (br d, J=8.0 Hz, 1H), 4.08 - 3.94 (m, 1H), 3.45 - 3.24 (m, 2H), 2.00 - 1.86 (m, 2H), 1.62 (br d, J=10.0 Hz, 6H), 1.38 (s, 8H), 1.34 (br s, 1H), 1.26 - 1.09 (m, 3H), 0.97 - 0.77 (m, 2H).

Step 2: Cyclohexylamine (43.5 mg, 438 µmol), DIEA (142 mg, 1.10 mmol), and HATU (166 mg, 438 µmol) were added to a solution of compound 130-C (120 mg, 365 µmol) in DMF (2 mL). The mixture was stirred at 20 °C for 2 hours, diluted with water (20 mL), and extracted with EtOAc (10 mL×3). After conventional work-up procedure 2, the crude product was triturated and slurried in EtOAc at 20 °C for 3 hours, then filtered to obtain compound 130-D as a white solid (100 mg, yield 66.82%). ¹H NMR (400MHz, DMSO-d6) Shift = 7.84 - 7.71 (m, 1H), 7.61 (br d, J=7.8 Hz, 1H), 6.65 (br d, J=8.0 Hz, 1H), 4.02 - 3.89 (m, 1H), 3.49 (br d, J=7.3 Hz, 1H), 3.33 - 3.26 (m, 1H), 3.24 - 3.13 (m, 1H), 1.92 (br d, J=6.8 Hz, 2H), 1.63 (br d, J=10.8 Hz, 10H), 1.38 (s, 9H), 1.30 - 1.03 (m, 9H), 0.95 - 0.76 (m, 2H).

Step 3: HCl solution in dioxane (2M, 1 mL) was added to a mixture of compound 130-D (100 mg, 244 umol) in dioxane (1 mL). The mixture was stirred at 20 °C for 3 hours and concentrated under reduced pressure to obtain compound 130-E as a white solid (90.0 mg, HCl salt), which was used directly in the next step.

Step 4: TEA (40.8 mg, 0.4 mmol) and compound **112** (35.3 mg, 0.134 mmol) were added to a solution of compound **130-E** (50 mg, 0.134 mmol, HCl salt) in DMF (1 mL). The mixture was stirred at 40 °C for 16 hours, then concentrated under reduced pressure. The residue was purified by prep HPLC (FA method-B; B%: 35%-55%, 10 min) to obtain compound **130** as a white solid (25 mg, yield 29.32%). LCMS: (ESI) m/z= 572.3 [M+1] ⁺; ¹H NMR (400MHz, DMSO-d6) Shift = 9.61 (br s, 1H), 7.85 (dd, J=2.4, 8.2 Hz, 1H), 7.68 (br d, J=6.8 Hz, 2H), 6.99 (d, J=2.5 Hz, 1H), 6.48 (s, 1H), 5.59 (t, J=7.5 Hz, 1H), 4.30 (q, J=7.1 Hz, 1H), 3.72 (s, 3H), 3.55 - 3.44 (m, 1H), 3.29 - 3.17 (m, 2H), 2.71 - 2.62 (m, 3H), 2.38 - 2.31 (m, 2H), 2.17 - 2.02 (m, 1H), 1.91 (br d, J=5.3 Hz, 2H), 1.78 - 1.45 (m, 12H), 1.32 - 0.99 (m, 9H), 0.85 (br d, J=10.0 Hz, 2H).

According to the procedures of steps 2, 3, and 4 in Example 5-b, the following starting material was used in place of cyclohexylamine in step 2 to obtain the corresponding final product through three-step reactions.

| Starting material | Final product | LCMS and NMR data |
|---|---|---|
| 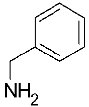 | 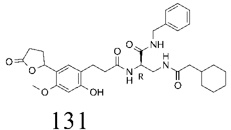 White solid, about 25% yield over three steps | LCMS:(ESI) m/z= 580.3 (M+1)⁺, |
| | | ¹H NMR (400MHz, DMSO-d6) Shift = 9.61 (s, 1H), 8.39 (t, J=6.0 Hz, 1H), 7.96 (d, J=8.0 Hz, 1H), 7.78 (br s, 1H), 7.36 - 7.15 (m, 5H), 6.99 (d, J=2.0 Hz, 1H), 6.48 (s, 1H), 5.59 (t, J=7.7 Hz, 1H), 4.44 - 4.33 (m, 1H), 4.26 (br d, J=6.0 Hz, 2H), 3.72 (s, 3H), 3.32 - 3.28 (m, 2H), 2.72 - 2.67 (m, 2H), 2.56 (br s, 2H), 2.36 - 2.30 (m, 2H), 2.17 - 2.02 (m, 1H), 1.92 (br d, J=4.5 Hz, 2H), 1.61 (br d, J=10.5 Hz, 6H), 1.28 - 0.97 (m, 4H), 0.85 (br d, J=10.5 Hz, 2H) |
| 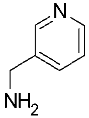 | 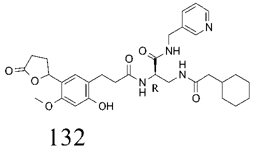 White solid, about 35.4% yield over three steps | LCMS:(ESI) m/z= 581.3 (M+1)⁺, |
| | | ¹H NMR (400MHz, DMSO-d6) Shift = 9.62 (s, 1H), 8.56 - 8.34 (m, 3H), 7.99 (d, J=7.8 Hz, 1H), 7.80 (br d, J=3.0 Hz, 1H), 7.63 (d, J=7.8 Hz, 1H), 7.33 (dd, J=4.5, 7.8 Hz, 1H), 6.99 (d, J=2.5 Hz, 1H), 6.48 (s, 1H), 5.59 (t, J=7.5 Hz, 1H), 4.47 - 4.32 (m, 1H), 4.32 - 4.22 (m, 2H), 3.72 (s, 3H), 3.30 (br d, J=5.5 Hz, 2H), 2.73 - 2.64 (m, 3H), 2.45 (br d, J=3.8 Hz, 1H), 2.42 - 2.31 (m, 3H), 2.14 - 2.00 (m, 1H), 1.91 (br d, J=4.5 Hz, 2H), 1.60 (br d, J=8.8 Hz, 6H), 1.22 - 1.04 (m, 3H), 0.85 (br s, 2H) |
| 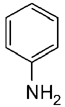 | 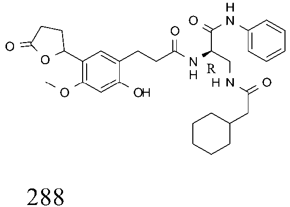 White solid, about 34% yield over three steps | LCMS: (ESI) m/z= 566.3 [M+1]⁺; |
| | | 1H NMR (400MHz, DMSO-d6) Shift = 9.99 (s, 1H), 9.61 (s, 1H), 8.06 (dd, J=4.3, 7.8 Hz, 1H), 7.83 (br t, J=5.0 Hz, 1H), 7.60 (d, J=7.5 Hz, 2H), 7.30 (t, J=7.9 Hz, 2H), 7.05 (t, J=7.1 Hz, 1H), 6.99 (d, J=4.6 Hz, 1H), 6.48 (s, 1H), 5.60 - 5.55 (m, 1H), 4.50 (q, J=6.9 Hz, 1H), 3.72 (s, 3H), 3.44 - 3.38 (m, 1H), 3.33 - 3.28 (m, 1H), 2.71 - 2.65 (m, 2H), 2.65 - 2.56 (m, 1H), 2.56 - 2.53 (m, 1H), 2.45 - 2.35 (m, 3H), 2.13 - 2.01 (m, 1H), 1.92 (d, J=6.8 Hz, 2H), 1.64 - 1.51 (m, 6H), 1.17 - 1.00 (m, 3H), 0.88 - 0.76 (m, 2H) |

### Example 6: Synthesis of Compound 111

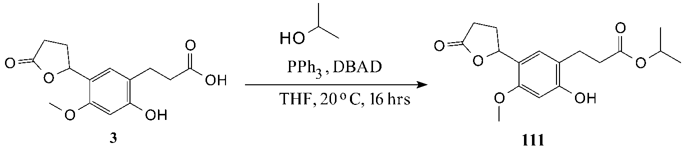

Triphenylphosphine (93.58 mg, 356.80 µmol) and di-tert-butyl azodicarboxylate (DBAD, 61.62 mg, 0.27 mmol) were added to a solution of compound 3 (50 mg, 0.18 mmol) and isopropanol (10.72 mg, 0.18 mmol) in THF (2 mL) at 20 °C. The mixture was stirred at 20 °C for 16 hours, then concentrated under reduced pressure. The residue was purified by silica gel column chromatography (EA:PE=0 to 40%:60%), followed by preparative HPLC (FA method-A, B%: 33%-53%, 10 min) to obtain compound **111** as a white solid (20 mg, 0.06 mmol, yield 33.74%). LCMS: (ESI) m/z=322.9(M+1)⁺; ¹HNMR (400 MHz, CHLOROFORM-d) Shift 8.17 (s, 1H), 7.00 (s, 1H), 6.52 (s, 1H), 5.64-5.74 (m, 1H), 5.04 (quin, J=6.21 Hz, 1H), 3.80 (s, 3H), 2.75-2.89 (m, 2H), 2.59-2.72 (m, 5H), 2.09-2.20 (m, 1H), 1.24 (dd, J=2.26, 6.27 Hz, 6H).

According to the synthetic method of compound **111,** the starting materials listed in the following table were reacted with compound **3** to obtain the corresponding final products.

| Starting material | Final product | LCMS and NMR data |
|---|---|---|
| 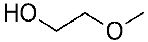 | 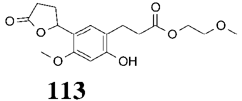 Yield 45%, white solid | LCMS:(ESI)m/z=338.9 (M+1)⁺; |
| | | ¹HNMR (400 MHz, DMSO-d6) Shift 9.67 (br s, 1H), 7.00 (s, 1H), 6.48 (s, 1H), 5.59 (t, J=7.53 Hz, 1H), 4.10 (dd, J=4.02, 5.52 Hz, 2H), 3.72 (s, 3H), 3.50 (dd, J=4.14, 5.40 Hz, 2H), 3.25 (s, 3H), 2.69-2.77 (m, 2H), 2.59-2.67 (m, 1H), 2.52-2.58 (m, 3H), 2.39-2.49 (m, 1H), 2.03-2.16 (m, 1H) |
| 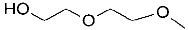 | 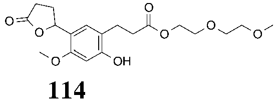 Yield 35%, white solid | LCMS:(ESI) m/z=383.1(M+1) ⁺; |
| | | ¹HNMR (400 MHz, DMSO-d6) Shift 9.68 (br s, 1H), 6.99 (s, 1H), 6.48 (s, 1H), 5.59 (t, J=7.65 Hz, 1H), 4.01-4.18 (m, 2H), 3.72 (s, 3H), 3.55-3.60 (m, 2H), 3.49-3.54 (m, 2H), 3.42-3.45 (m, 2H), 3.24 (s, 3H), 2.69-2.76 (m, 2H), 2.59-2.67 (m, 1H), 2.52-2.58 (m, 3H), 2.38-2.49 (m, 1H), 2.03-2.17 (m, 1H) |

### Example 7: Synthesis of Compound 4

Lithium aluminum hydride (360 mg, 9.49 mmol) was added to a solution of compound **4-A** (3.00 g, 11.8 mmol) in THF (60 mL) at 0 °C. The mixture was stirred at 0 °C for 1 hour, then quenched with 1M HCl solution and diluted with water (30 mL). The mixture was extracted with EA. After conventional work-up procedure, compound **4-B** was obtained as a purple solid (2.55 g, 9.57 mmol, yield 82%). ¹HNMR (400 MHz, CDCl₃) δ 7.24 (s, 1H), 6.51 (s, 1H), 3.86 (s, 3H), 3.68-3.66 (t, J=4, 2H), 2.73-2.70 (t, J=4, 2H), 1.89-1.83 (m, 2H).

Step 2: DHP (2.05 g, 24.4 mmol) and pyridinium p-toluenesulfonate (PPTS, 245 mg, 976 umol) were added to a solution of compound **4-B** (2.55 g, 9.77 mmol) in DCM (50 mL). The mixture was stirred at room temperature for 16 hours, then water was added, and the mixture was extracted with DCM (20 mL×3). After conventional work-up procedure 2 and purification by silica gel column chromatography (EA:PE, EA from 0 to 11.5%), compound 4-C was obtained as a yellow oil (3.3 g, 6.15 mmol, purity 80%). LCMS: (ESI) m/z=453.1 (M+Na)⁺.

Step 3: It was ensured that all glassware used in this operation was dry and anhydrous. n-Butyllithium solution in THF (2.5 M, 2.5 mL) was added to a solution of compound **4-C** (2.00 g, purity 80%) in THF (50 mL) at -78 °C. After stirring for 1 hour, succinic anhydride (932 mg, 9.32 mmol) was added, and stirring was continued for 2 hours. The reaction was quenched with 10% citric acid solution (30 mL), and the mixture was extracted with EA (30 mL×3). After conventional work-up procedure 2 and purification by silica gel column chromatography (THF:PE, THF from 0 to 40%), compound **4-C** was obtained as a colorless oil (0.65 g, 1.3 mmol, LCMS purity 89.9%). LCMS: (ESI) m/z= 473.2 (M+Na)⁺. ¹HNMR showed residual succinic anhydride (2 equivalents).

Step 4: NaBH₄ (75.70 mg, 2.00 mmol) was added to a mixture of compound **4-D** (601 mg, purity 89.9%) in aqueous NaOH (190 mg, 4.75 mmol) solution (5.5 mL). The mixture was stirred at 25 °C for 16 hours, then carefully acidified to pH 2 with 10% citric acid solution (30 mL) and extracted with EA (20 mL×3). After conventional work-up procedure 2, 508 mg of a pale-yellow gum was obtained. LCMS showed the product was a mixture of 20.3% compound 4-F [(ESI) m/z=457.4(M+Na)⁺] and 54.8% compound 4-E [(ESI) m/z=475.1(M+Na)⁺].

Step 5: p-Toluenesulfonic acid monohydrate (40 mg, 210.28 µmol) was added to a solution of the mixture of compound **4-E** and **4-F** (190 mg) in THF (2 mL) and water (1 mL). The mixture was stirred at 25 °C for 16 hours. Purification by Prep HPLC (FA method-A, B%: 15%-35%, 10 min) obtained compound **4** as an off-white solid (35 mg, 122.24 µmol). LCMS: (ESI) m/z=267.1(M+1)⁺; ¹HNMR (400MHz, CHLOROFORM-d) Shift = 7.02 (s, 1H), 6.49 (s, 1H), 5.74 - 5.67 (m, 1H), 3.81 (s, 3H), 3.74 - 3.63 (m, 2H), 2.81 - 2.70 (m, 2H), 2.69 - 2.59 (m, 3H), 2.22 - 2.11 (m, 1H), 1.91 - 1.83 (m, 2H).

### Example 8-a: Synthesis of Compounds 5 and 6

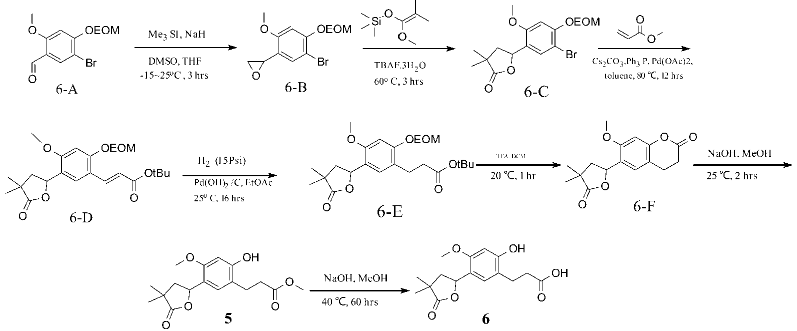

Step 1: Under a nitrogen atmosphere, a suspension of sodium hydride (304 mg, 7.61 mmol, 60% purity) in DMSO (20 mL) was heated at 65 °C for 1 hour. After cooling to room temperature, THF (20 mL) was added to the clear solution. The reaction mixture was cooled to -15 °C, and a solution of trimethylsulfoxonium iodide (1.41 g, 6.92 mmol) in DMSO (20 mL) was added dropwise. After 3 minutes, a solution of compound 6-A (2 g, 6.92 mmol) in THF (20 mL) was added dropwise. The mixture was stirred at 25 °C for 2 hours, then quenched with saturated NH₄Cl solution (50 mL) at 0-10 °C, diluted with 100 mL of water, and extracted with EA (100 mL×2). After conventional work-up procedure 2, compound 6-B was obtained as a yellow gum (2 g, crude), which was used directly in the next step.

Step 2: A yellow mixture of compound 6-B (2 g, crude), (1-methoxy-2-methyl-prop-1-enyloxy)trimethylsilane (5.75 g, 33 mmol), and tetrabutylammonium fluoride trihydrate (1.04 g, 3.30 mmol) was stirred at 60 °C for 12 hours. The reaction mixture was concentrated to dryness under reduced pressure, and the residue was purified by silica gel column chromatography (EA:PE, EA from 0 to 3%) to obtain compound 6-C as a yellow gum (920 mg, 2.46 mmol, about 32% yield over two steps). ¹H NMR (400 MHz, CDCl₃) δ (ppm) 7.53 (s, 1H), 6.82 (s, 1H), 5.61 (dd, *J =* 6.5, 9.5 Hz, 1H), 5.29-5.34 (m, 2H), 3.85 (s, 3H), 3.78-3.83 (m, 2H), 2.52 (dd, *J* = 6.4, 12.9 Hz, 1H), 1.99 (dd, *J* = 9.8, 12.8 Hz, 1H), 1.38 (s, 3H), 1.32 (s, 3H), 1.26 (t, *J =* 7.03 Hz, 3H).

Step 3: Compound 6-C (760 mg, 2.04 mmol) and methyl acrylate (438 mg, 5.09 mmol) were dissolved in 5 mL of anhydrous toluene. Cesium carbonate (995 mg, 3.05 mmol), triphenylphosphine (427 mg, 1.63 mmol), and palladium acetate (183 mg, 814 mmol) were added. The mixture was degassed, purged with nitrogen, and stirred at 80 °C under a nitrogen atmosphere for 12 hours. After cooling to room temperature, the reaction was diluted with 30 mL of water and extracted with EA (30 mL×2). After conventional work-up procedure, the residue was purified by silica gel column chromatography (EA:PE, EA from 0 to 15%) to obtain compound 6-D as a white gum (560 mg, 1.48 mmol, yield 72.68%). ¹H NMR (400 MHz, CDCl₃) δ (ppm) 7.95-8.01 (m, 1H), 7.55 (s, 1H), 6.81 (s, 1H), 6.39-6.47 (m, 1H), 5.63 (dd, *J* = 6.5, 9.5 Hz, 1H), 5.33 (s, 2H), 3.89 (s, 3H), 3.81 (s, 3H), 3.78 (d, *J* = 7.03 Hz, 2H), 2.53 (dd, *J =* 6.5, 12.8 Hz, 1H), 2.02 (dd, *J* = 9.8, 12.8 Hz, 1H), 1.39 (s, 3H), 1.33 (s, 3H), 1.23-1.27 (m, 3H).

Step 4: According to the representative procedure for catalytic hydrogenation, compound 6-D (528 µmol) was reacted to obtain 6-E as a yellow gum (342 µmol, 64.66% yield), purified by silica gel column chromatography (EA:PE, EA from 0 to 15%). ¹H NMR (400 MHz, CDCl₃) *δ* (ppm) 7.13 (s, 1H), 6.77 (s, 1H), 5.64 (dd, *J* = 6.4, 9.6 Hz, 1H), 5.27 (s, 2H), 3.83 (s, 3H), 3.72-3.81 (m, 2H), 3.69 (s, 3H), 2.86-2.94 (m, 2H), 2.55-2.62 (m, 2H), 2.48 (dd, *J =* 6.5, 12.8 Hz, 1H), 1.99-2.05 (m, 1H), 1.37 (s, 3H), 1.32 (s, 3H), 1.23-1.28 (m, 3H).

Step 5: A solution of 6-E (130 mg, 342 µmol) in DCM (1.5 mL) and TFA (0.1 mL) was stirred at 20 °C for 1 hour. The reaction mixture was concentrated to dryness to obtain compound 6-F as a pink gum (130 mg, crude), which was used directly in the next step. LC-MS: (ESI) *m*/*z*. [M+H]⁺ 290.9.

Step 6: Compound 6-F (130 mg) was dissolved in MeOH (1 mL), and aqueous NaOH solution (238 mg, 895 µmol) was added. The mixture was stirred at 25 °C for 30 minutes, then acidified to pH 3 with 1M HCl and stirred at 25 °C for another 30 minutes. The mixture was concentrated under reduced pressure, diluted with 10 mL of water, and extracted with EA (10 mL×2). After conventional work-up procedure, the residue was purified by silica gel column chromatography to obtain compound 5 as a white solid (60 mg, 186 µmol, 54.4% yield over two steps). LC-MS: (ESI) *m*/*z*. [M+H]⁺ 323.1. ¹H NMR (400 MHz, DMSO-d₆) *δ* (ppm) 9.65 (s, 1H), 7.02 (s, 1H), 6.48 (s, 1H), 5.58 (dd, *J* = 6.5, 9.8 Hz, 1H), 3.72 (s, 3H), 3.57 (s, 3H), 3.34 (s, 2H), 2.67-2.75 (m, 2H), 2.30-2.37 (m, 1H), 2.08 (dd, *J* = 10.0, 12.5 Hz, 1H), 1.25 (s, 3H), 1.21 (s, 3H).

Step 7: Compound 5 (18 mg) was dissolved in MeOH (10 mL), and aqueous NaOH solution (1 M, 111 uL) was added. The mixture was stirred at 40 °C for 60 hours. LCMS showed 18% starting material remained and 30% target product was formed. The mixture was concentrated, diluted with EA and water (5 mL), acidified to pH 3 with 1M HCl, and extracted with EA (5 mL×2). After conventional work-up procedure, the residue was purified by prep-HPLC (column: YMC Triart C18 150×25mm×5um; mobile phase: [water (0.225% FA)-ACN]; B%: 30%-50%, 10 min) to obtain compound 6 as a white solid (2 mg, 6.16 µmol, yield 11%). LCMS: ESI) m/z=309.2 (M+H) ⁺; 1H NMR (400MHz, DMSO-d6) Shift = 12.03 (br s, 1H), 9.62 (br s, 1H), 7.03 (s, 1H), 6.47 (s, 1H), 5.57 (dd, J=6.5, 10.0 Hz, 1H), 3.71 (s, 3H), 2.68 (t, J=7.7 Hz, 2H), 2.42 (t, J=7.7 Hz, 2H), 2.31 (dd, J=6.4, 12.7 Hz, 1H), 2.12 - 2.05 (m, 1H), 1.24 (s, 3H), 1.20 (s, 3H).

### Example 8-b: Synthesis of Compounds 7 and 8

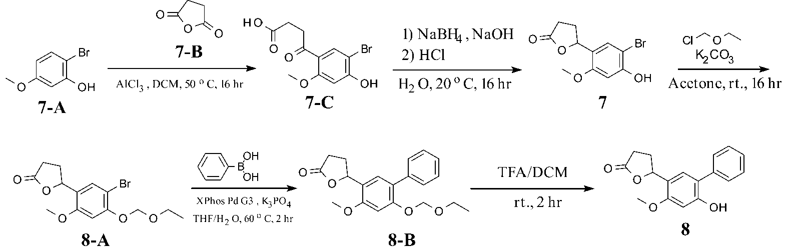

Steps 1 and 2: According to the procedure of steps 1 and 2 in Example 5, compound 7-A was used as starting material to obtain compound 7 as a white solid in about 2.4% yield over two steps. LCMS: (ESI) m/z=288.6(M+1) ⁺;¹H NMR (400 MHz, DMSO-d6) Shift 10.44 (s, 1H), 7.36 (s, 1H), 6.63 (s, 1H), 5.59 (t, J=7.65 Hz, 1H), 3.76 (s, 3H), 2.53-2.70 (m, 2H), 2.41-2.50 (m, 1H), 1.98-2.18 (m, 1H).

Step 3: K₂CO₃ (317.72 mg, 2.30 mmol) was added to a solution of compound 7 (220 mg, 766.27 umol) and chloromethyl ethyl ether (86.93 mg, 919.52 umol) in acetone (8 mL). The mixture was stirred at 20 °C for 16 hours, filtered, and the filtrate was concentrated to dryness. The residue was purified by silica gel column chromatography (EA:PE, EA from 0 to 35%) to obtain 8-A as a colorless oil (250 mg, 706.14 umol). (ESI) m/z=346.9 (M+1) ⁺. ¹H NMR (400 MHz, CDCl₃) Shift 7.48 (s, 1H), 6.83 (s, 1H), 5.61-5.77 (m, 1H), 5.32 (s, 2H), 3.86 (s, 3H), 3.81 (q, J=7.09 Hz, 2H), 2.53-2.81 (m, 3H), 2.02-2.28 (m, 1H), 1.26 (t, J=7.03 Hz, 3H).

Step 4 (Representative Suzuki coupling procedure): K₃PO₄ (129 mg, 608.37 umol) and XPhos Pd G3 (17.17 mg, 20.28 umol) were added to a mixture of compound 8-A (70 mg, 202.79 umol) and phenylboronic acid (74.18 mg, 608.37 umol) in THF/water (3 mL, 4/1). The mixture was degassed and purged with nitrogen twice, then heated to 60 °C and stirred for 1 hour. The reaction mixture was concentrated to dryness, diluted with ethyl acetate (3 mL), and subjected to conventional work-up procedure. The crude product was purified by preparative TLC (PE:EA=1:1) to obtain compound 8-B as a yellow oil (60 mg, yield 82.09%). LCMS: (ESI) m/z=343.2(M+1)⁺;¹H NMR (400 MHz, CHLOROFORM-d) Shift 7.44-7.52 (m, 2H), 7.35-7.43 (m, 2H), 7.27-7.33 (m, 2H), 6.89 (s, 1H), 5.67-5.82 (m, 1H), 5.18 (s, 2H), 3.89 (s, 3H), 3.66 (dq, J=1.16, 7.07 Hz, 2H), 2.56-2.74 (m, 3H), 2.13-2.28 (m, 1H), 1.20 (t, J=7.09 Hz, 3H).

Step 5: A solution of compound **8-B** (80 mg, 0.23 mmol) in DCM (3.5 mL) and TFA (0.35 mL) was stirred at 20 °C for 2 hours. The reaction was quenched with saturated NaHCO₃ solution (2 mL), and the mixture was extracted with DCM (3 mL×3). After conventional work-up procedure 2, the residue was purified by silica gel column chromatography (EA:PE, EA from 0 to 30%) to obtain compound **8** as a white solid (35 mg, yield 50%). LCMS: (ESI) m/z=284.9(M+1)⁺. ¹H NMR (400 MHz, DMSO-d6) Shift 9.81 (s, 1H), 7.51 (d, J=7.03 Hz, 2H), 7.38 (t, J=7.65 Hz, 2H), 7.22-7.30 (m, 1H), 7.19 (s, 1H), 6.63 (s, 1H), 5.67 (t, J=7.53 Hz, 1H), 3.80 (s, 3H), 2.54-2.73 (m, 2H), 2.43-2.50 (m, 1H), 2.12-2.25 (m, 1H).

According to the synthetic method of compound 8, the starting materials listed in the following table were reacted with compound 8-A to obtain the corresponding final products.

| Starting material | Final product | LCMS and NMR data |
|---|---|---|
| 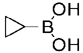 | 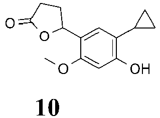 About 8% yield over two steps, white solid | LCMS: (ESI) m/z=248.9(M+1)⁺; ¹H NMR (400 MHz, DMSO-d6) Shift 9.51 (s, 1H), 6.69 (s, 1H), 6.47 (s, 1H), 5.57 (t, J=7.78 Hz, 1H), 3.71 (s, 3H), 2.54-2.70 (m, 2H), 2.38-2.46 (m, 1H), 2.04-2.18 (m, 1H), 1.89-1.99 (m, 1H), 0.80 (dd, J=2.26, 8.53 Hz, 2H), 0.46-0.60 (m, 2H) |
| 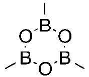 | 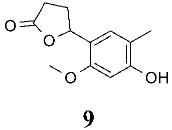 About 21% yield over two steps, white solid | LCMS: (ESI) m/z=223.0(M+1) ⁺; ¹H NMR (400 MHz, DMSO-d6) Shift 9.48 (s, 1H), 6.98 (s, 1H), 6.47 (s, 1H), 5.60 (t, J=7.65 Hz, 1H), 3.71 (s, 3H), 2.56-2.73 (m, 2H), 2.38-2.47 (m, 1H), 2.06-2.16 (m, 1H), 2.05 (s, 3H) |

### Example 9: Synthesis of Compound 11

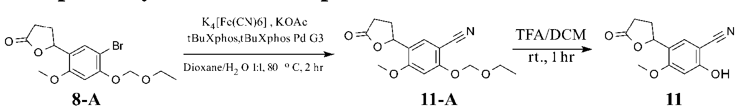

Step 1: Potassium ferrocyanide (53.35 mg, 144.85 umol), potassium acetate (3.55 mg, 36.21 umol), t-Bu Xphos (2.46 mg, 5.79 umol, 0.02 *eq*), and t-Bu Xphos Pd G3 (2.30 mg, 2.90 umol) were added to a mixture of compound 8-A (100 mg, 0.29 mmol) in dioxane/water (4 mL, 1/1). The mixture was degassed and purged with nitrogen, then heated to 80 °C and stirred for 2 hours. LCMS showed a small amount of starting material remained, but the desired product was formed. The reaction mixture was cooled to room temperature and extracted with EA (5 mL×3). After conventional work-up procedure 2, the crude product was purified by preparative TLC (EA:PE, EA from 0 to 30%) to obtain compound 11-A as a colorless oil (60 mg, yield 35.5%). LCMS: (ESI) m/z=292.2(M+1)⁺.

Step 2: A solution of compound **11-A** (50 mg, 0.17 mmol) in DCM (1 mL) and TFA (0.5 mL) was stirred at 25 °C for 1 hour. The reaction was quenched with saturated NaHCO₃ solution (1 mL), and the mixture was extracted with EA (3 mL×3). The aqueous phase was acidified to pH 3-4 with 1M HCl and extracted again with EA (5 mL×3). After conventional work-up procedure 2, the residue was washed with DCM (1 mL) to obtain compound **11** as a white solid (35 mg, yield 50%). LCMS: (ESI) m/z=234.1(M+1)⁺. ¹H NMR (400 MHz, DMSO-d6) Shift 7.44 (s, 1H), 6.54 (s, 1H), 4.74 (dd, J=4.39, 7.40 Hz, 1H), 3.80 (s, 3H), 2.16-2.25 (m, 2H), 1.77-1.90 (m, 1H), 1.62-1.75 (m, 1H).

### Example 10: Synthesis of Compound 12

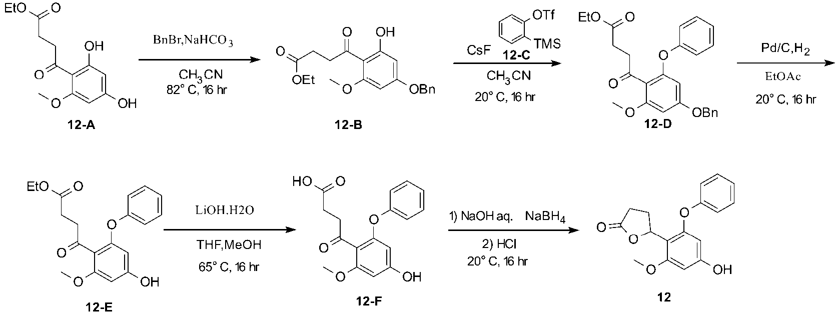

Step 1: NaHCO₃ (3.91 g, 46.50 mmol) and benzyl bromide (3.98 g, 23.25 mmol) were added to a mixture of compound 12-A (5.76 g, 21.1 mmol) in acetonitrile (60 mL). The mixture was heated to 82 °C and stirred for 16 hours. After cooling to room temperature, water (80 mL) was added, and the mixture was extracted with EA (100 mL). After conventional work-up procedure 2, the crude product was purified by preparative TLC (EA:PE, EA from 0 to 29%) to obtain compound 12-B as a yellow solid (4.6 g, yield 60.73%).

Step 2: Compound 12-C (811 mg, 2.72 mmol) and cesium fluoride (826 mg, 5.44 mmol) were added to a mixture of compound 12-B (650 mg, 1.81 mmol) in acetonitrile (26 mL). The mixture was stirred at 20 °C for 16 hours, then saturated brine (20 mL) was added, and the mixture was extracted with EA (20 mL). After conventional work-up procedure 2, the crude product was purified by preparative TLC (EA:PE, EA from 0 to 20%) to obtain compound 12-D as a yellow oil (238 mg, yield 30.22%). ¹H NMR (400 MHz, DMSO-*d*₆) *δ* ppm 1.10 - 1.14 (m, 3 H) 2.44 - 2.47 (m, 2 H) 2.95 - 3.02 (m, 2 H) 3.77 - 3.82 (m, 3 H) 3.97 - 4.01 (m, 2 H) 5.02 - 5.09 (m, 2 H) 6.07 - 6.12 (m, 1 H) 6.54 - 6.59 (m, 1 H) 7.11 - 7.38 (m, 10 H).

Step 3: According to the representative procedure for catalytic hydrogenation, compound 12-D (235 mg, 541 µmol) was reacted to obtain 12-E as a yellow gum (120 mg, 349 µmol). Purification was performed by silica gel column chromatography (EA:PE, EA from 0 to 15%). ¹H NMR (CDCl₃, 400 MHz) δ 7.95 (d, J=8.53 Hz, 1H), 7.76 (s, 1H), 7.29-7.57 (m, 9H), 6.96 (d, J=8.53 Hz, 1H), 6.23 (br t, J=5.52 Hz, 1H), 4.75 (d, J=5.52 Hz, 2H), 4.17-4.30 (m, 2H), 3.89 (s, 3H), 3.68 (s, 3H), 2.76 (t, J=7.53 Hz, 2H), 2.41 (t, J=7.53 Hz, 2H), 2.01 (t, J=7.40 Hz, 2H), 1.77-1.88 (m, 4H), 1.63-1.77 (m, 3H), 1.32-1.44 (m, 1H), 1.15-1.29 (m, 3H), 0.94-1.09 (m, 2H).

Step 4 (Representative ester hydrolysis procedure): Lithium hydroxide monohydrate (36.7 mg, 0.87 mmol, about 2.5 *eq*) was added to a mixture of compound 12-E (120 mg, 0.35 mmol) in THF (1 mL) and MeOH (1 mL). The mixture was stirred at 65 °C for 16 hours (LCMS showed complete reaction). After cooling to room temperature, the pH was adjusted to 1-2 with 2M HCl, and the mixture was extracted with EA (4 mL×3). After conventional work-up procedure, the crude product was purified by preparative TLC (EA:PE, EA from 0 to 24%-73%) to obtain compound 12-F as a yellow oil (65 mg, yield 58.82%). ¹H NMR (400 MHz, DMSO-*d*₆) δ ppm 2.46 - 2.48 (m, 2 H) 2.95 - 3.03 (m, 2 H) 3.53 - 3.53 (m, 1 H) 3.71 - 3.78 (m, 3 H) 5.78 - 5.87 (m, 1 H) 6.20 - 6.26 (m, 1 H) 6.97 - 7.03 (m, 2 H) 7.12 - 7.19 (m, 1 H) 7.34 - 7.44 (m, 2 H) 9.95 - 10.01 (m, 1 H).

Step 5: According to the representative procedure for constructing the five-membered lactone ring, compound **12-F** (65 mg, 205 µmol) was reacted to obtain compound **12** as a white solid (10 mg, yield 16.2%). ¹H NMR (400 MHz, DMSO-*d*₆) *δ* ppm 1.54 - 1.60 (m, 2 H) 2.06 - 2.11 (m, 2 H) 2.41 - 2.46 (m, 2 H) 3.65 - 3.72 (m, 6 H) 5.98 - 6.07 (m, 2 H) 9.21 - 9.30 (m, 1 H) 11.78 - 11.91 (m, 1 H). LCMS: (ESI) m/z = 301.0 (M+1)⁺.

### Example 11: Synthesis of Compound 13

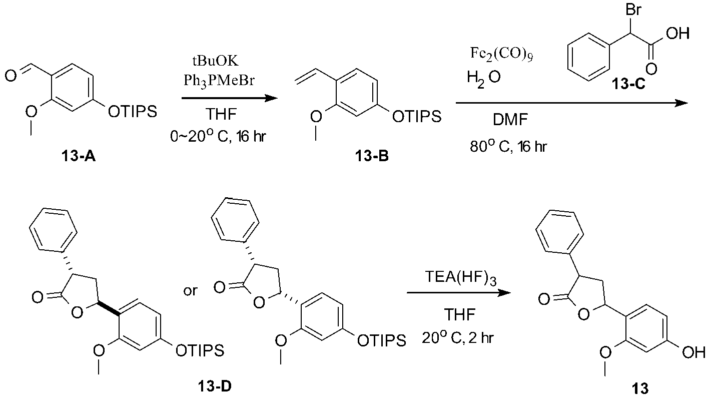

Step 1: Under a nitrogen atmosphere at 0 °C, potassium tert-butoxide solution in THF (1 M, 25.93 mL) was added dropwise to a mixture of methyltriphenylphosphonium bromide (Ph₃PMeBr, 10.65 g, 29.82 mmol) in THF (80 mL). The mixture was stirred at 0 °C for 1 hour. At 0 °C, a solution of compound 13-A (4 g, 12.97 mmol) in THF (60 mL) was added to the above mixture. The mixture was allowed to warm to room temperature (20 °C) and stirred for 16 hours. At 0-10 °C, the reaction was quenched with saturated NH₄Cl solution (5 mL), water (8 mL) was added, and the mixture was extracted with MTBE (4 mL×3). After conventional work-up procedure 2, the crude product was purified by preparative TLC (EA:PE, EA from 0 to 4%) to obtain compound 13-B as a yellow solid (2.03 g, yield 51%).¹H NMR (400 MHz, DMSO-*d*₆) δ ppm 1.02 - 1.12 (m, 18 H) 1.21 - 1.30 (m, 3 H) 3.72 - 3.77 (m, 3 H) 5.07 - 5.14 (m, 1 H) 5.59 - 5.66 (m, 1 H) 6.39 - 6.48 (m, 2 H) 6.77 - 6.90 (m, 1 H) 7.34 - 7.39 (m, 1 H).

Step 2: 13-C (584 mg, 2.72 mmol), H₂O (97.9 mg, 5.44 mmol), and diiron nonacarbonyl (CAS: 15321-51-4, 49.4 mg, 135.94 umol) were added to a mixture of 13-B (1 g, 3.26 mmol, 1.2 *eq*) in DMF (10 mL). The mixture was stirred at 80 °C for 16 hours. After cooling to room temperature, water (4 mL) was added, and the mixture was extracted with EA (12 mL). After conventional work-up procedure 2, the crude product was purified by preparative TLC (EA:PE, EA from 0 to 7% to 11.5%) to obtain compound 13-D as a white solid (157 mg, yield 13.11%), which may be either cis or trans isomer, and was not further identified. ¹H NMR (400 MHz, CHLOROFORM-*d*) δ ppm 1.11 - 1.14 (m, 18 H) 1.25 - 1.32 (m, 3 H) 2.66 - 2.85 (m, 2 H) 3.79 - 3.83 (m, 3 H) 3.91 - 3.98 (m, 1 H) 5.78 - 5.87 (m, 1 H) 6.44 - 6.54 (m, 2 H) 7.12 - 7.22 (m, 1 H) 7.30 - 7.41 (m, 5 H).

Step 3: TEA(HF)₃ (14.6 mg, 90.78 umol) was added to a mixture of 13-D (50 mg, 113.47 umol) in THF (1 mL). The mixture was stirred at 20 °C for 2 hours. After the reaction, saturated aqueous NaHCO₃ solution was added to adjust the pH to 7-8, and the mixture was extracted with EA (2 mL×3).After conventional work-up procedure 2, the crude product was purified by preparative TLC (EA:PE, EA from 0 to 62%) to obtain compound 13 as a yellow solid (9 mg, yield 27.9%). ¹H NMR (400 MHz, CHLOROFORM-*d*) δ ppm 2.40 (m, 0.6H), 2.66-2.75 (m, 0.4H), 2.76-2.87 (m, 0.4H), 3.09 (m, 0.6H), 3.95 (m, 0.4H), 4.04 (m, 0.6H), 5.00 (br s, 1H) 5.73 (dd, J=10.5, 5.8 Hz, 0.6H), 5.85 (dd, J=7.8, 4.8 Hz, 0.4H), 6.38-6.57 (m, 2H), 7.09-7.25 (m, 1H), 7.31-7.60 (m, 5H).

### Example 12: Synthesis of Compound 16

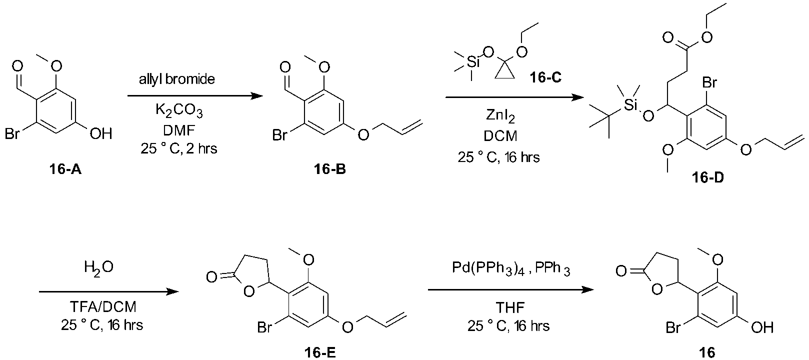

Step 1: 3-Bromo-1-propene (235 mg, 1.95 mmol) and K₂CO₃ (358 mg, 2.60 mmol) were added to a mixture of 16-A (300 mg, 1.3 mmol) in DMF (6 mL). The mixture was stirred at 25 °C for 2 hours, then water (6 mL) was added, and the mixture was extracted with EA (8 mL×3).After conventional work-up procedure, the crude product was purified by preparative TLC (EA:PE, EA from 0 to 30%) to obtain compound 16-B as a yellow oil (220 mg, yield 62.5%). LCMS: (ESI) m/z=273.0 (M+1)⁺.

Step 2: 16-C (655 mg, 3.76 mmol) and ZnI₂ (360 mg, 1.13 mmol) were added to a solution of 16-B (170 mg, 0.63 mmol) in DCM (4 mL). The mixture was stirred at 25 °C for 16 hours, then water (5 mL) was added, and the mixture was extracted with DCM (3 mL×3). After conventional work-up procedure, compound 16-D was obtained as a brown oil (280 mg), which was used directly in the next step.

Step 3: Water (33.9 mg, 1.89 mmol) was added to a solution of compound 16-D (280 mg) in DCM (4 mL) and TFA (0.4 mL). The mixture was stirred at 25 °C for 16 hours. After the reaction, saturated aqueous NaHCO₃ solution was added at 5 °C-10 °C to adjust the pH to 7-8, and the mixture was extracted with DCM (2 mL×3). After conventional work-up procedure, the crude product was purified by preparative TLC (EA:PE, EA from 0 to 50%) to obtain compound 16-E as a yellow oil (17 mg, purity 74%). LCMS: (ESI) m/z = 327.0 [M+H]⁺.

Step 4: Under a nitrogen atmosphere, to a solution of compound 16-E (17 mg) in THF (0.5 mL) were added Pd(PPh₃)₄ (6 mg, 5.2 µmol) and PPh₃ (14.99 mg, 57.16 µmol). The mixture was stirred at 25 °C for 16 hours, then concentrated under reduced pressure. The residue was purified by prep-HPLC (FA method; B%: 23%-53%, 8 min) to obtain compound 16 as a white solid (1 mg, 3.36 umol, about 5.3% yield over three steps). (ESI) m/z = 286.9[M+H]⁺). ¹H NMR (400 MHz, CHLOROFORM-*d*) δ ppm 6.70 (d, J=2.32 Hz, 1 H) 6.43 (d, J=2.32 Hz, 1 H) 6.12 (dd, J=8.86, 6.91 Hz, 1 H) 5.90 (br s, 1 H) 3.75 (s, 3 H) 2.65 - 2.82 (m, 2 H) 2.51 - 2.64 (m, 1 H) 2.35 - 2.46 (m, 1 H).

According to the procedures of Steps 2 to 4 in Example 12 (Representative procedure A for constructing the five-membered lactone ring), the corresponding final products were obtained from the starting materials listed in the following table via three-step reactions.

| Starting material | Final product | LCMS and NMR data |
|---|---|---|
| 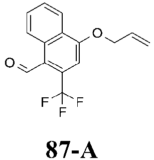 | 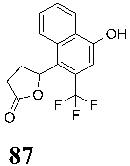 About 19% yield over three steps, white solid | ¹H NMR (400 MHz, DMSO-d6) Shift 11.15 (br s, 1H), 8.26-8.40 (m, 1H), 7.93-8.08 (m, 1H), 7.57-7.75 (m, 2H), 7.16 (s, 1H), 6.19 (t, J=8.80 Hz, 1H), 2.95-3.11 (m, 1H), 2.78-2.93 (m, 1H), 2.53-2.61 (m, 2H) |

The synthesis of the above starting material 87-A is as follows:

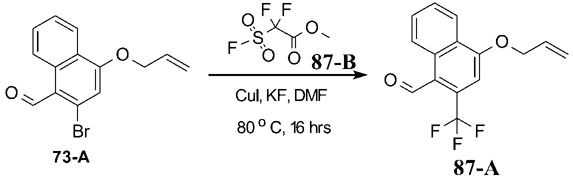

Compound 87-B (395 mg, 2.06 mmol), potassium fluoride (59.8 mg, 1.03 mmol), and CuI (196 mg, 1.03 mmol) were added to a solution of compound 73-A (300 mg, 1.03 mmol) in DMF (5 mL). The mixture was heated to 80 °C under light-protected conditions and stirred for 16 hours. After cooling to room temperature, the mixture was filtered and washed with EA. The filtrate was subjected to conventional work-up procedure 2, and the crude product was purified by silica gel column chromatography (EA:PE, EA from 0 to 10%) to obtain compound 87-A as a gray solid (280 mg, yield 97%). LCMS: (ESI) m/z=280.9 (M+1) ⁺.

### Example 12-a: Synthesis of Compound 65

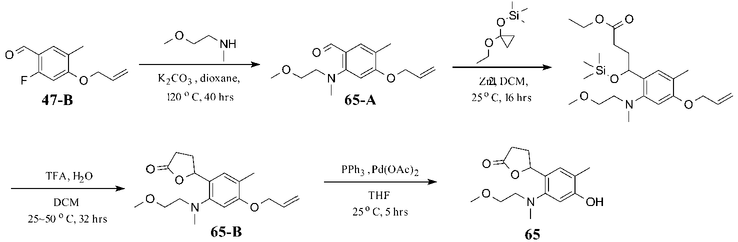

Step 1: A mixture of 47-B (3.6 g, 18.5 mmol), N-(2-methoxyethyl)methylamine (3.30 g, 37.1 mmol), and K₂CO₃ (5.12 g, 37.1 mmol, 2 *eq*) in dioxane (80 mL) was stirred at 120 °C for 40 hours. LC-MS showed about 32% of starting material remained and about 65% of desired product was formed. The reaction was diluted with EA (100 mL), filtered, and concentrated under reduced pressure. The crude product was purified by preparative TLC (EA:PE, EA from 0 to 15%) to obtain compound 65-A as a yellow oil (3.7 g, yield 75.8%). LCMS: (ESI) m/z=264.3 (M+1)⁺.

Steps 2 and 3: According to the procedures of steps 2 and 3 in Example 12, compound 65-A was subjected to two-step reactions to obtain 65-B as an orange oil in about 20% yield over two steps. LC-MS: (ESI) *m*/*z*. [M+H]⁺ 320.3.

Step 4: According to the procedure of step 3 in Example 2, compound 65-A was reacted to obtain compound 65 as a yellow oil in 63% yield. LC-MS: (ESI) *m*/*z*. [M+H]⁺ 280.3; ¹H NMR (400 MHz, DMSO-*d*₆) *δ* (ppm) 9.39 (s, 1H), 7.05 (s, 1H), 6.66 (s, 1H), 5.87 (dd, *J =* 6.8, 9.0 Hz, 1H), 3.37 (t, *J* = 5.9 Hz, 2H), 3.22 (s, 3H), 2.84-2.98 (m, 2H), 2.63-2.75 (m, 1H), 2.53-2.62 (m, 4H), 2.44 (m, 1H), 2.08-2.16 (m, 1H), 2.07 (s, 3H).

### Example 12-b: Synthesis of Compound 66

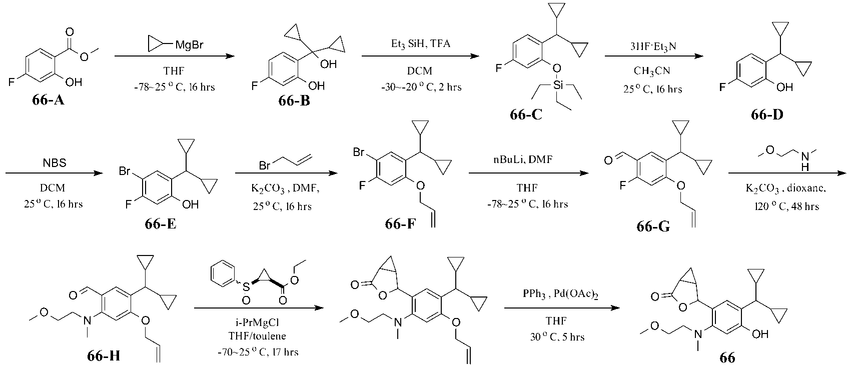

Step 1: Cyclopropylmagnesium bromide solution (0.5 M, 205 mL) was added dropwise to a solution of compound 66-A (5 g, 29.4 mmol) in THF (50 mL) at -78 °C. The mixture was allowed to warm to room temperature (25 °C) and stirred for 16 hours. After the reaction, saturated aqueous NH₄Cl solution (200 mL) was added to quench, and the mixture was extracted with EtOAc (200 mL×3). After conventional work-up procedure 2, compound 66-B was obtained as a crude yellow solid (7.6 g). LC-MS: (ESI) *m*/*z*. [M-OH]⁺ 205.1.

Step 2: Et₃SiH (5.23 g, 45 mmol) and TFA (10.2 g, 90.0 mmol) were added dropwise to a solution of 66-B (5 g) in DCM (100 mL) at -30 °C. The mixture was stirred for 2 hours while maintaining the temperature at -30 °C to -20 °C. After the reaction, water (100 mL) was added, the pH was adjusted to 8-9 with saturated sodium carbonate solution, and the mixture was extracted with DCM (50 mL×3). After conventional work-up procedure 2, the crude product was purified by silica gel column chromatography (EA:PE, EA from 0 to 3%) to obtain compound 66-C as a colorless oil (5.14 g, about 83% yield over two steps). ¹H NMR (400 MHz, CDCl₃) *δ* (ppm) 7.23 (dd, *J* = 7.3, 8.4 Hz, 1H), 6.64 (dt, *J* = 2.6, 8.4 Hz, 1H), 6.49 (dd, *J* = 2.5, 10.6 Hz, 1H), 1.95 (t, *J* = 8.6 Hz, 1H), 1.03-1.12 (m, 2H), 0.95-1.03 (m, 9H), 0.73-0.82 (m, 6H), 0.48-0.58 (m, 2H), 0.22-0.34 (m, 4H), 0.04-0.12 (m, 2H).

Step 3: Triethylamine trihydrofluoride (5.17 g, 32.1 mmol) was added to a solution of compound 66-C (5.14 g, 16 mmol) in ACN (60 mL). The mixture was stirred at 25 °C for 16 hours, then concentrated under reduced pressure. The residue was dissolved in EtOAc (100 mL) and subjected to conventional work-up procedure 2. The crude product was purified by silica gel column chromatography (EA:PE, EA from 0 to 3.5%) to obtain compound 66-D as a colorless oil (3.2 g, yield 88%). ¹H NMR (400 MHz, CDCl₃) *δ* (ppm) 7.19 (dd, *J* = 6.7, 8.50 Hz, 1H), 6.63 (dt, *J* = 2.5, 8.4 Hz, 1H), 6.53 (dd, *J* = 2.5, 10.0 Hz, 1H), 5.22 (s, 1H), 1.97 (t, *J =* 8.0 Hz, 1H), 1.12 (tq, *J =* 5.1, 8.0 Hz, 2H), 0.53-0.65 (m, 2H), 0.36-0.46 (m, 2H), 0.31 (qd, *J =* 4.8, 9.4 Hz, 2H), 0.06-0.19 (m, 2H).

Step 4: NBS (949 mg, 5.33 mmol) was added to a solution of compound 66-D (1 g, 4.85 mmol) in DCM (30 mL). The mixture was stirred at 25 °C for 16 hours, then concentrated under reduced pressure. The residue was purified by silica gel column chromatography (EA:PE, EA from 0 to 3%) to obtain compound 66-E as a colorless oil (610 mg, yield 44%). ¹H NMR (400 MHz, CDCl₃) *δ* (ppm) 7.38 (d, *J =* 7.8 Hz, 1H), 6.61 (d, *J =* 9.4 Hz, 1H), 5.24 (s, 1H), 1.92 (t, *J =* 8.1 Hz, 1H), 1.09 (tq, *J =* 5.1, 8.1 Hz, 2H), 0.54-0.68 (m, 2H), 0.38-0.50 (m, 2H), 0.32 (qd, *J =* 4.8, 9.57 Hz, 2H), 0.08-0.19 (m, 2H).

Steps 5 and 6: According to the procedures of steps 2 and 3 in Example 21, compound 66-E was used as starting material to obtain 66-G as a yellow oil in about 46.74% yield over two steps. ¹H NMR (400 MHz, CDCl₃) *δ* (ppm) 10.25 (s, 1H), 7.82 (d, *J =* 8.2 Hz, 1H), 6.60 (d, *J =* 12.5 Hz, 1H), 6.04 (tdd, *J* = 5.1, 10.5, 17.3 Hz, 1H), 5.43 (qd, *J =* 1.5, 17.3 Hz, 1H), 5.35 (qd, *J =* 1.3, 10.6 Hz, 1H), 4.61 (td, *J =* 1.5, 5.1 Hz, 2H), 1.90 (t, *J =* 8.9 Hz, 1H), 1.06-1.23 (m, 2H), 0.53-0.64 (m, 2H), 0.26-0.37 (m, 4H), 0.01-0.11 (m, 2H).

Step 7: According to the procedure of step 1 in Example 12-a, compound 66-G (210 mg) was used as starting material to obtain 66-H as a yellow oil (200 mg, yield 76.07%). LC-MS: (ESI) *m*/*z*. [M+H]⁺ 344.2.

Steps 8 and 9: According to the procedures of steps 2 and 3 in Example 2, compound 66-H was subjected to two-step reactions to obtain compound 66 as an off-white solid in about 51% yield over two steps. ¹H NMR (400 MHz, DMSO-*d*₆) *δ* (ppm) 9.15-9.43 (m, 1H), 6.94-7.20 (m, 1H), 6.60-6.80 (m, 1H), 5.63-6.02 (m, 1H), 3.42-3.45 (m, 2H), 3.19-3.29 (m, 3H), 2.89-3.04 (m, 2H), 2.57-2.69 (m, 3H), 2.28-2.36 (m, 1H), 2.11-2.27 (m, 1H), 1.67-1.79 (m, 1H), 1.28 (dt, J = 4.7, 8.35 Hz, 1H), 1.03-1.15 (m, 2H), 0.74-0.99 (m, 1H), 0.41-0.54 (m, 2H), 0.17-0.30 (m, 4H), -0.09-0.03 (m, 2H). LC-MS: (ESI) *m*/*z*. [M+H]⁺ 372.4.

### Example 13: Synthesis of Compound 17

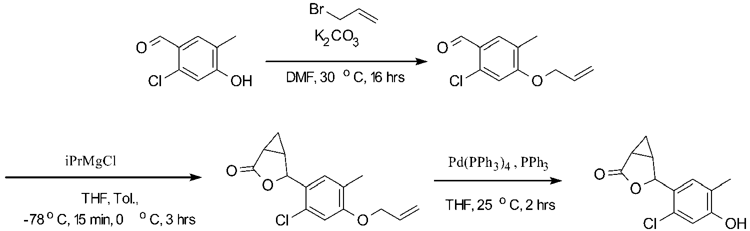

Step 1 was performed according to the procedure of Step 1 in Example 2, Step 2 according to the procedure of Step 2 in Example 2, and Step 3 according to the procedure of Step 4 in Example 12. The starting materials listed in the following table were subjected to the three-step reactions to obtain compound 17.

| Starting material | Final product | LCMS and NMR data |
|---|---|---|
| 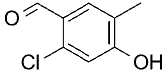 | 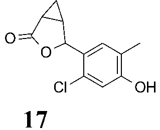 About 6% yield over three steps, off-white solid | (ESI) m/z=239.0 (M+1) ⁺ |
| | | ¹H NMR (400 MHz, CHLOROFORM-d) δ ppm 0.80 - 0.85 (m, 1 H) 1.02 - 1.16 (m, 1 H) 1.33 - 1.43 (m, 1 H) 2.16 - 2.21 (m, 1 H) 2.21 - 2.24 (m, 3 H) 2.27 - 2.32 (m, 1 H) 2.76 - 2.82 (m, 1 H) 5.11 (br s, 1 H) 5.19 (br s, 1 H) 5.60 (s, 1 H) 5.85 (d, J=4.77 Hz, 1 H) 6.87 (s, 1 H) 7.03 (s, 1 H) 7.11 (s, 1 H) |

According to the procedures of steps 2 and 3 in Example 13, the starting materials listed in the following table were subjected to two-step reactions to obtain the corresponding final products.

| Starting material | Final product | LCMS and NMR data |
|---|---|---|
| 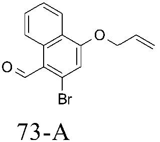 | 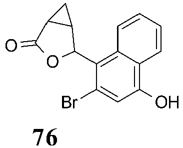 About 5.2% yield over two steps, off-white solid | LCMS: (ESI) m/z= 318.8 (M+1)⁺, |
| | | ¹H NMR (400 MHz, DMSO-d6) Shift 11.02 (br s, 1H), 8.24 (d, J=8.31 Hz, 1H), 8.17 (d, J=8.68 Hz, 1H), 7.62-7.71 (m, 1H), 7.53-7.61 (m, 1H), 7.12 (s, 1H), 6.28 (s, 1H), 2.62 (m, 1H), 2.46-2.49 (m, 1H), 1.57 (m, 1H), 1.43 (m, 1H) |

### Example 14: Synthesis of Compound 18

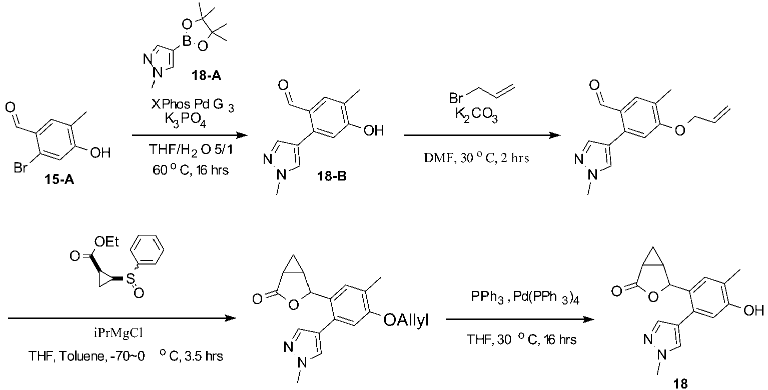

Step 1: According to the procedure of step 4 in Example 8-b, 15-A (2.0 g, 9.30 mmol) was reacted with 18-A (2.90 g, 13.95 mmol) to obtain compound 18-B as an off-white solid (1.96 g, yield 84.17%). LCMS: (ESI) m/z=217.0 (M+1)⁺.

Steps 2 to 4: According to the procedures in Example 13, the starting materials listed in the following table were subjected to three-step reactions to obtain the corresponding final products.

| Starting material | Final product | LCMS and NMR data |
|---|---|---|
| 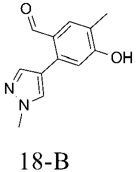 | 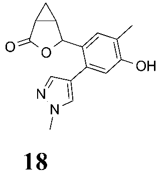 About 4.8% yield over three steps, white solid | (ESI) m/z=285.3 (M+1) ⁺ |
| | | ¹H NMR (400 MHz, DMSO-d6) Shift 9.61 (br s, 1H), 8.16 (s, 0.116H), 7.73-7.91 (s, 1H), 7.45-7.59 (s, 1H), 7.06-7.17 (s, 1H), 6.69-6.81 (s, 1H), 5.73 (d, J=4.40 Hz, 0.16H), 5.35 (s,0.84H), 3.87-3.94 (s, 3H), 2.34 (dd, J=3.19, 6.05 Hz, 2H), 2.11-2.19 (s, 3H), 1.29 (m, 0.8H), 1.11 (m, 0.2H), 1.00 (m, 1H) |
| 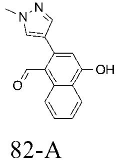 | 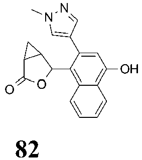 About 1.4% yield over three steps, off-white solid | ¹H NMR (400 MHz, DMSO-d6) Shift 10.59 (s, 1H), 8.25 (d, J=8.03 Hz, 1H), 8.13 (d, J=8.53 Hz, 1H), 7.84 (s, 1H), 7.57-7.65 (m, 1H), 7.47-7.55 (m, 2H), 6.81 (s, 1H), 5.90 (s, 1H), 3.91-3.96 (m, 3H), 2.56-2.65 (m, 2H),1.53 (m, 1H), 1.22-1.31 (m, 1H) |
| | | LCMS: (ESI) m/z=321.0 (M+1)⁺ |
| 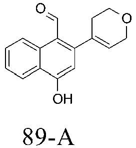 | 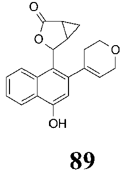 About 4.6% yield over three steps, off-white solid | ¹H NMR (400 MHz, DMSO-d6) δ 10.52 (br s, 1H), 8.22 (br d, J=6.82 Hz, 1H), 8.08 (br d, J=6.60 Hz, 1H), 7.37-7.67 (m, 3H), 6.64 (br d, J=2.64 Hz, 1H), 5.95 (br s, 1H), 5.65 (br s, 1H), 4.19 (br s, 2H), 3.87 (br s, 3H), 2.11-2.39 (m, 2H), 1.52 (br s, 1H), 1.35 (br s, 1H). |
| | | LCMS: (ESI) m/z = 323.3 (M+1) ⁺ |

The starting material 82-A listed in the above table was synthesized as follows:

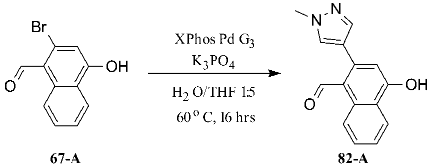

According to the procedure of Step 4 in Example **8-b,** compound **67-A** (750 mg, 2.99 mmol) was reacted with 18-A (932 mg, 1.5 *eq*) to obtain compound **82-A** as a yellow solid (750 mg, yield 99%). LCMS: (ESI) m/z= 253.0 (M+1)⁺.

According to the method for starting material 82-A, the starting materials listed in the following table were used instead of 18-A and reacted with compound **67-A** to obtain the corresponding intermediate compounds.

| Starting material | Final product | LCMS and NMR data |
|---|---|---|
| 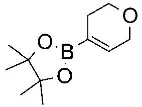 | 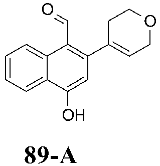 Yield 87.4%, brown solid | (ESI) m/z=255.1 (M+1) ⁺ |

### Example 15: Synthesis of Compound 19

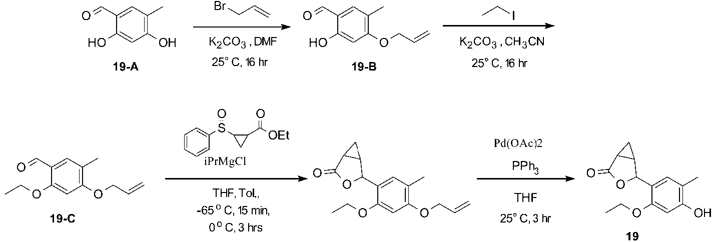

Step 1: According to the procedure of step 1 in Example 2, 19-A (5.1 g, 33.52 mmol) was used to prepare 19-B as a yellow oil (1.6 g, 24.83% yield). ¹H NMR (400 MHz, DMSO-*d*₆) δ ppm 2.10 (s, 3 H) 4.64 (d, *J*=5.00 Hz, 2 H) 5.30 (dd, *J*=10.63, 1.38 Hz, 1 H) 5.42 (dd, *J*=17.26, 1.63 Hz, 1 H) 6.00 - 6.11 (m, 1 H) 6.50 (s, 1 H) 7.45 (s, 1 H) 9.96 (s, 1 H) 10.89 (s, 1 H).

Step 2: K₂CO₃ (575 mg, 4.16 mmol) and iodoethane (486 mg, 3.12 mmol) were added to a solution of compound 19-B (400 mg, 2.08 mmol) in acetonitrile (8 mL). The mixture was stirred at 25 °C for 16 hours, then concentrated under reduced pressure. The residue was purified by silica gel column chromatography (EA:PE, EA from 0 to 10%) to obtain compound 19-C as a white solid (210 mg, 45.81%). (ESI) m/z=245.2 (M+Na)⁺.

Steps 3 and 4: According to the procedures of steps 2 and 3 in Example 2, compound 19-C (573 mg, 2.41 mmol) was subjected to two-step reactions to obtain compound 19 as a yellow solid (45 mg, about 15.8% yield over two steps). ¹H NMR (400 MHz, CHLOROFORM-*d*) δ ppm 0.78 - 0.86 (m, 1 H) 0.95 - 1.07 (m, 1 H) 1.28 - 1.36 (m, 1 H) 1.38 - 1.44 (m, 3 H) 2.10 - 2.20 (m, 4 H) 2.21 - 2.28 (m, 1 H) 2.64 - 2.72 (m, 1 H) 3.90 - 4.13 (m, 2 H) 4.75 - 5.10 (m, 1 H) 5.35 - 5.54 (m, 1 H) 5.83 (d, *J*=4.63 Hz, 1 H) 6.33 - 6.46 (m, 1 H) 6.87 - 7.02 (m, 1 H). LCMS: (ESI) m/z = 249.1 (M+1)⁺.

### Example 16: Synthesis of Compound 21

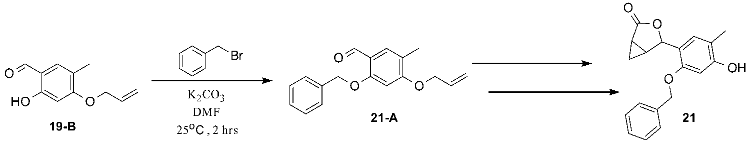

According to the procedure of Example 2, 19-B and benzyl bromide were used as starting materials in a three-step reaction to obtain compound 21 as a white solid (45 mg, about 1.4% yield over three steps). LCMS: (ESI) m/z = 311.1 [M+H] ⁺, ¹H NMR (400 MHz, CHLOROFORM-*d*) δ ppm 7.32 - 7.48 (m, 5 H) 6.94 - 7.09 (m, 1 H) 6.46 - 6.56 (m, 1 H) 5.52 - 5.91 (m, 1 H) 5.02 - 5.16 (m, 2 H) 4.71 - 4.87 (m, 1 H) 2.23 - 2.70 (m, 1 H) 2.20 (s, 3 H) 2.00 - 2.17 (m, 1 H) 1.30 (m, 1 H) 0.97 - 1.07 (m, 1 H).

According to the method of Example 16, the starting materials listed in the following table were used in place of benzyl bromide and subjected to three-step reactions to obtain the corresponding final products.

| Starting material | Final product | LCMS and NMR data |
|---|---|---|
| 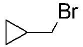 | 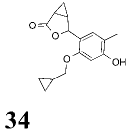 About 14% yield over three steps, white solid | LCMS:(ESI) m/z=275.0 (M+H) ⁺; ¹H NMR (400 MHz, DMSO-d6) Shift 9.50 (br s, 1H), 6.92 (s, 1H), 6.44 (s, 1H), 5.35 (s, 1H), 3.69-3.84 (m, 2H), 2.15-2.27 (m, 2H), 2.02-2.09 (m, 3H), 1.13-1.33 (m, 2H), 0.89-1.09 (m, 1H), 0.51-0.63 (m, 2H), 0.29-0.38 (m, 2H) |
| 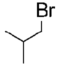 | 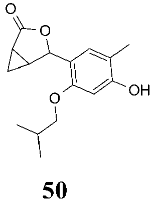 About 17.7% yield over three steps, white solid | LCMS: (ESI) m/z = 277.1 (M+1) ⁺, |
| | | ¹H NMR (400 MHz, DMSO-*d*6) δ ppm 0.72 (br d, *J*=3.42 Hz, 1 H) 0.93 - 1.04 (m, 7 H) 1.06 - 1.38 (m, 1 H) 1.92 - 2.12 (m, 4 H) 2.15 - 2.33 (m, 2 H) 2.53 - 2.59 (m, 1 H) 3.59 - 3.85 (m, 2 H) 5.41 (s, 1 H) 5.78 (d, *J*=4.52 Hz, 1 H) 6.40 - 6.56 (m, 1 H) 6.74 - 6.99 (m, 1 H) 9.55 (br s, 1 H). |
| 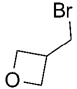 | 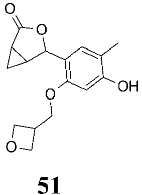 About 4.3% yield over three steps, white solid | LCMS: (ESI) m/z = 291.0 (M+1) ⁺, |
| | | ¹H NMR (400 MHz, DMSO-d6) δ ppm 0.94 (br d, J=3.79 Hz, 1 H) 1.00 - 1.35 (m, 1 H) 2.06 (s, 3 H) 2.13 - 2.28 (m, 2 H) 2.55 - 2.64 (m, 1 H) 3.98 - 4.25 (m, 2 H) 4.34 - 4.60 (m, 2 H) 4.67 - 4.84 (m, 2 H) 5.32 (s, 1 H) 5.77 (d, J=4.65 Hz, 1 H) 6.44 - 6.62 (m, 1 H) 6.79 - 7.02 (m, 1 H) 9.61 (br s, 1 H) |
| Iodomethane-d3 | 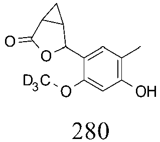 About 20% yield over three steps, white solid | LCMS: (ESI) *m*/*z*. [M+H]⁺ 238.1 |
| | | ¹H NMR (400 MHz, DMSO-*d*₆) *δ* (ppm) 9.51 (br s, 1H), 6.76-7.01 (m, 1H), 6.48 (s, 1H), 5.73 (br d, *J* = 3.5 Hz, 1H), 5.38 (s, 1H), 2.53-2.57 (m, 1H), 2.12-2.25 (m, 2H), 2.00-2.08 (m, 3H), 1.01-1.36 (m, 1H), 0.67-0.99 (m, 1H) |

### Example 17: Synthesis of Compound 20

Step 1: Under ice-bath condition, compound 19-B (1 g, 5.20 mmol) was added to a solution of potassium tert-butoxide (1 M in THF, 7.80 mL) in THF (20 mL) while maintaining at 0 °C. After stirring for 6 minutes, 20-A (CAS: 66003-76-7, 2.91 g, 6.76 mmol, 1.3 *eq*) was added. After stirring for 6 minutes, 20-A (CAS: 66003-76-7, 2.91 g, 6.76 mmol, 1.3 *eq*) was added. The mixture was allowed to warm to room temperature (25 °C) and stirred for 2 hours. The reaction mixture was poured into ice water (20 mL) and extracted with EtOAc (15 mL×3). After conventional work-up procedure, the crude product was purified by preparative TLC (EA:PE, EA from 0 to 3%) to obtain compound 20-B as a yellow oil (290 mg, yield 21%).¹H NMR (CDCl₃, 400 MHz) δ ppm 10.31 (s, 1 H) 7.75 (s, 1 H) 7.35 - 7.48 (m, 2 H) 7.13 - 7.21 (m, 1 H) 7.00 - 7.10 (m, 2 H) 6.34 (s, 1 H) 5.91 - 6.03 (m, 1 H) 5.23 - 5.39 (m, 2 H) 4.41 - 4.53 (m, 2 H) 2.25 (s, 3 H).

Steps 2 and 3: According to the procedures of steps 3 and 4 in Example 2, compound 20-B was subjected to two-step reactions to obtain compound 20 as a white solid (6 mg, about 3.5% yield over two steps). LCMS: (ESI) m/z = 297.1[M+H]⁺, ¹H NMR (CDCl₃, 400 MHz) δ ppm 8.25 - 8.31 (m, 1 H) 8.09 (d, J=8.56 Hz, 1 H) 7.58 - 7.67 (m, 1 H) 7.49 - 7.57 (m, 1 H) 6.88 - 6.93 (m, 1 H) 6.34 (s, 1 H) 2.51 - 2.61 (m, 1 H) 2.38 - 2.47 (m, 1 H) 1.63 (br s, 1 H) 1.32 - 1.37 (m, 1 H).

### Example 18: Synthesis of Compound 23

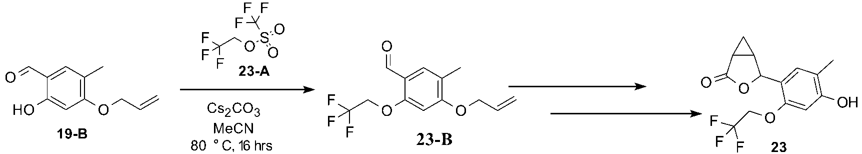

Step 1: Compound 23-A (664 mg, 2.86 mmol) and cesium carbonate (1.70 g, 5.20 mmol) were added to a solution of 19-B (500 mg, 2.60 mmol) in MeCN (10 mL). The mixture was stirred at 80 °C for 16 hours. The reaction was diluted with water (10 mL) and extracted with EtOAc (10 mL×3). After conventional work-up procedure, the crude product was purified by preparative TLC (EA:PE, EA from 0 to 30%) to obtain compound 23-B as a yellow solid (300 mg, yield 42%). ¹H NMR (CDCl₃, 400 MHz) δ ppm 10.32 (s, 1 H) 7.70 (s, 1 H) 6.37 (s, 1 H) 6.00 - 6.18 (m, 1 H) 5.48 (dd, J=17.17, 1.32 Hz, 1 H) 5.38 (dd, J=10.56, 1.32 Hz, 1 H) 4.62 - 4.68 (m, 2 H) 4.47 (q, J=8.00 Hz, 2 H) 2.23 (s, 3 H).

Steps 2 and 3: According to the procedures of steps 3 and 4 in Example 2, compound 23-B was subjected to two-step reactions to obtain compound 23 as a white solid (10 mg, about 2.5% yield over two steps). LCMS: (ESI) m/z = 303.1[M+H] ⁺, ¹H NMR (DMSO-d₆, 400 MHz) δ ppm 9.29 - 10.19 (m, 1 H) 6.83 - 7.09 (m, 1 H) 6.48 - 6.61 (m, 1 H) 5.30 - 5.80 (m, 1 H) 4.61 - 4.82 (m, 2 H) 2.15 - 2.28 (m, 2 H) 2.02 - 2.11 (m, 3 H) 1.03 - 1.36 (m, 1 H) 0.66 - 1.01 (m, 1 H).

### Example 19: Synthesis of Compound 33

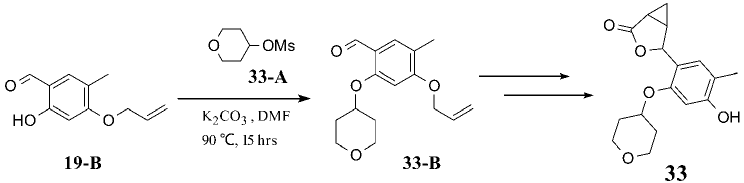

Step 1: K₂CO₃ (1.08 g, 7.8 mmol) was added to a solution of compound **19-B** (500 mg, 2.60 mmol) and compound **33-A** (562 mg, 3.12 mmol) in DMF (10 mL). The mixture was stirred at 90 °C for 15 hours. After cooling to room temperature, the reaction was diluted with saturated brine (50 mL) and extracted with EtOAc (50 mL×2). After conventional work-up procedure, the crude product was purified by silica gel column chromatography (EA:PE, EA from 0 to 10%) to obtain compound 33-B as a yellow solid (580 mg, yield 80.7%).

Steps 2 and 3: According to the procedures of steps 2 and 3 in Example 2, two-step reactions were performed to obtain compound 33 as a white solid (about 46% yield over two steps). LCMS: (ESI) m/z=305.1 (M+H) ⁺; ¹H NMR (400 MHz, DMSO-d6) Shift 9.48 (s, 1H), 6.94 (s, 1H), 6.51-6.57 (m, 1H), 5.36 (s, 1H), 4.37-4.51 (m, 1H), 3.86 (td, J=4.22, 11.32 Hz, 2H), 3.39-3.55 (m, 2H), 2.13-2.33 (m, 2H), 2.04 (s, 3H), 1.92-2.00 (m, 2H), 1.50-1.71 (m, 2H), 1.30 (dt, J=4.38, 8.13 Hz, 1H), 0.90 (br s, 1H).

According to the method of Example 19, the starting materials listed in the following table were used instead of 33-A and subjected to three-step reactions to obtain the corresponding final products.

| Starting material | Final product | LCMS and NMR data |
|---|---|---|
| 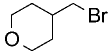 | 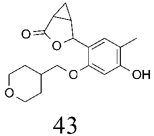 About 16.8% yield over three steps, white solid | LCMS: (ESI) m/z= 319.0 (M+1) ⁺; ¹H NMR (400MHz, DMSO-d6) Shift = 9.61 - 9.29 (m, 1H), 7.03 - 6.77 (m, 1H), 6.57 - 6.44 (m, 1H), 5.82 - 5.32 (m, 1H), 3.97 - 3.69 (m, 4H), 3.35 - 3.30 (m, 2H), 2.22 (br s, 2H), 2.05 (br s, 4H), 1.68 (br s, 2H), 1.42 - 1.27 (m, 3H), 1.11 - 0.95 (m, 1H), 0.71 (br s, 1H) |
| 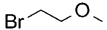 | 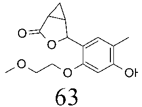 About 4.8% yield over three steps, white solid | ¹H NMR (400 MHz, DMSO-*d₆*) *δ* (ppm) 9.33-9.60 (m, 1H), 6.79-6.98 (m, 1H), 6.31-6.60 (m, 1H), 5.72 (d, *J* = 4.6 Hz, 1H), 5.34 (s, 1H), 3.94-4.10 (m, 2H), 3.59-3.72 (m, 2H), 3.31 (s, 3H), 2.11-2.24 (m, 2H), 2.04 (s, 3H), 0.99-1.35 (m, 1H), 0.64-0.96 (m, 1H). LC-MS: (ESI) *m*/*z.* [M+H]⁺ 279.3 |

### Example 20: Synthesis of Compound 38

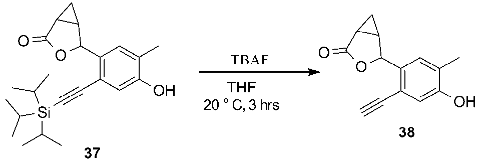

Tetrabutylammonium fluoride (122.37 mg, 468.04 µmol) was added to a solution of compound **37** (120 mg, 312.03 µmol) in THF (2 mL) at 20 °C (room temperature). The mixture was stirred at 20 °C for 3 hours. After the reaction, water (3 mL) was added to quench, and the mixture was extracted with EA (3 mL×3). After conventional work-up procedure 2 and purification by prep HPLC (FA method, B: 20%-50% over 8 min), compound **2** was obtained as a white solid (15 mg, yield 19.6%). ¹HNMR (400 MHz, DMSO-*d₆*) δ 9.65 - 10.02 (m, 1 H) 6.96 - 7.10 (m, 1 H) 6.89 - 6.94 (m, 1 H) 5.86 (d, J=4.84 Hz, 0.28 H) 5.53 (s, 0.81 H) 4.38 - 4.48 (m, 1 H) 2.28 (dd, J=8.36, 3.74 Hz, 2 H) 2.10 - 2.18 (m, 3 H) 1.34 (td, J=8.36, 4.62 Hz, 1 H) 1.03 - 1.17 (m, 1 H). LC-MS: (ESI) m/z = 229.0 [M+H]⁺.

### Example 21: Synthesis of Compound 59

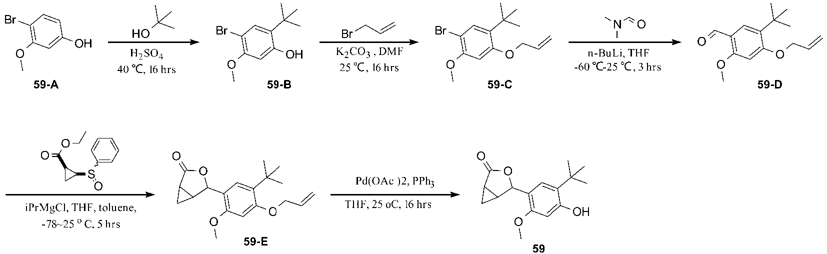

Step 1: A solution of compound **59-A** (6 g, 29 mmol) in tert-butanol (60 mL) and concentrated sulfuric acid (7.5 mL) was stirred at 40 °C for 16 hours. After cooling to room temperature, the reaction was quenched with water and extracted with EtOAc (50 mL×3). After conventional work-up procedure 2, the crude product was purified by silica gel column chromatography (EA:PE, EA from 0 to 10%) to give compound 59-B as a white solid (2.2 g, yield 28.73%). ¹H NMR (400 MHz, DMSO-*d*₆) *δ* (ppm) 9.70 (s, 1H), 7.16 (s, 1H), 6.48-6.56 (m, 1H), 3.74 (s, 3H), 1.29 (s, 9H).

Step 2: According to the procedure of step 1 in Example 12, compound 59-B (2.2 g) was used as starting material to obtain compound 59-C as a white solid (2.2 g, yield 86%). LCMS: (ESI) m/z=273.0 (M+1)⁺. ¹H NMR (400 MHz, DMSO-*d*₆) *δ* (ppm) 7.25 (s, 1H), 6.74 (s, 1H), 6.04-6.18 (m, 1H), 5.46 (dd, *J* = 1.8, 17.39 Hz, 1H), 5.30 (dd, *J =* 1.7, 10.7 Hz, 1H), 4.67 (d, *J =* 5.1 Hz, 2H), 3.83 (s, 3H), 1.28-1.34 (m, 9H).

Step 3: n-Butyllithium solution in THF (2.5 M, 3.2 mL) was added to a solution of compound 59-C (2.00 g, 6.7 mmol) in THF (20 mL) at -60 °C. After stirring for 0.5 hours, DMF (977 mg, 13 mmol) was added. The mixture was warmed to 25 °C and stirred for 3 hours. The reaction was quenched with NH₄Cl solution (20 mL) and extracted with EA (30 mL×3). After conventional work-up procedure 2 and purification by silica gel column chromatography (THF:PE, THF from 0 to 30%), compound **59-D** was obtained as a white solid (0.61 g, yield 37%). LCMS: (ESI) m/z= 249.2 (M+ H)⁺.

Steps 4 and 5: According to the procedures of steps 2 and 3 in Example 2, compound 59-D (585 mg, 2.5 mmol) was subjected to two-step reactions to obtain compound 59 as a white solid in about 4.5% yield over two steps. ¹H NMR (400 MHz, DMSO-*d*₆) *δ* (ppm) 9.48-9.66 (m, 1H), 6.90-7.03 (m, 1H), 6.43-6.56 (m, 1H), 5.33-5.80 (m, 1H), 3.69-3.78 (m, 3H), 2.13-2.26 (m, 2H), 1.07-1.37 (m, 10H), 0.66-0.98 (m, 1H). LC-MS: (ESI) *m*/*z*. [M+H]⁺ 277.3.

### Example 22: Synthesis of Compound 67

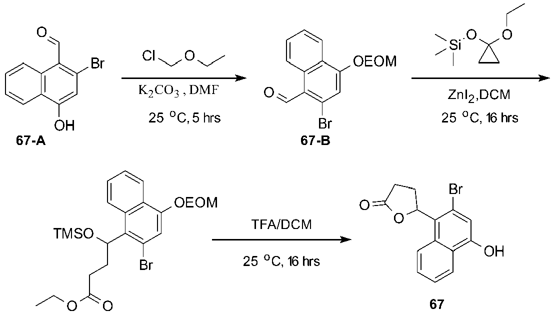

Step 1: Chloromethyl ethyl ether (1.58 g, 16.73 mmol) and K₂CO₃ (3.47 g, 25.09 mmol) were added to a solution of 16-A (2.1 g, 8.36 mmol) in DMF (40 mL). The mixture was stirred at 25 °C for 5 hours. Water (50 mL) was added, and the mixture was extracted with EA (50 mL×3). After conventional work-up procedure 2, the crude product was purified by silica gel column chromatography (EA:PE, EA from 0 to 3%) to obtain compound 67-B as a white solid (2.3 g, yield 89%). LCMS: (ESI) m/z=308.9 (M+1)⁺.

Steps 2 and 3: According to the procedures of steps 2 and 3 in Example 12 (Representative procedure B for constructing the five-membered lactone ring), compound 67-B was subjected to two-step reactions to obtain compound 67 as a white solid in about 17.2% yield over two steps. LCMS: (ESI) m/z= 306.9 [M+H] ⁺, ¹H NMR (400 MHz, CDCl₃) 8.32 (d, J=7.19 Hz, 1H), 7.98 (d, J=7.43 Hz, 1H), 7.51-7.60 (m, 2H), 7.32 (s, 1H), 6.50 (t, J=8.50 Hz, 1H), 5.43-5.50 (m, 2H), 3.82 (q, J=7.00 Hz, 2H), 2.83-2.94 (m, 2H), 2.61-2.71 (m, 2H), 1.28 (t, J=7.07 Hz, 3H).

According to the method of Example 22 (Representative procedure C for constructing the five-membered lactone ring), the starting materials listed in the following table were subjected to three-step reactions to give the corresponding final products.

| Starting material | Final product | LCMS and NMR data |
|---|---|---|
| 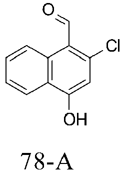 | 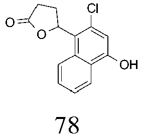 About 14.1% yield over three steps, yellow solid | LCMS: (ESI) m/z= 263.0 (M+1)⁺; ¹H NMR (400MHz, DMSO-d6) δ ppm 8.22 (dd, J=8.28, 1.00 Hz, 1 H) 7.99 (d, J=8.53 Hz, 1 H) 7.58 - 7.67 (m, 1 H) 7.47 - 7.57 (m, 1 H) 6.90 (s, 1 H) 6.48 (dd, J=10.04, 7.53 Hz, 1 H) 2.85 - 2.99 (m, 1 H) 2.73 - 2.84 (m, 1 H) 2.55 - 2.62 (m, 1 H) 2.42 - 2.48 (m, 1 H) |

The starting material 78-A listed in the above table was synthesized as follows:

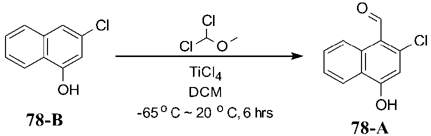

According to the synthetic method of intermediate 40-A, compound 78-B (440 mg, 2.46 mmol) was used as starting material to give compound 78-A as a yellow solid (400 mg, yield 78%). LCMS: (ESI) m/z= 207.0 (M+1)⁺.

According to the procedures of steps 2 and 3 in Example 22 (Representative procedure B for constructing the five-membered lactone ring), the starting materials listed in the following table were reacted to give the corresponding final products.

| Starting material | Final product | LCMS and NMR data |
|---|---|---|
| 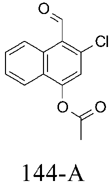 | 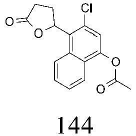 About 31% yield over two steps, yellow oil | LCMS: (ESI) m/z= 305.1 (M+1)⁺; ¹H NMR (400MHz, CDCl₃) δ ppm 8.08 (dd, J=7.64, 1.53 Hz, 1 H) 7.93 - 7.99 (m, 1 H) 7.54 - 7.66 (m, 2 H) 7.35 (s, 1 H) 6.54 (dd, J=10.21, 7.40 Hz, 1 H) 2.85 - 2.95 (m, 2 H) 2.61 - 2.76 (m, 2 H) 2.50 (s, 3 H) |

The starting material 78-A mentioned in the above table was synthesized as follows:

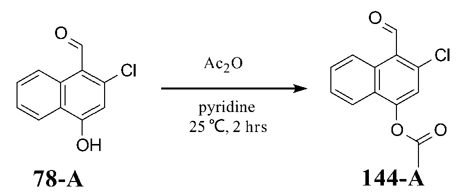

According to the method of Example 37, compound 78-A (60 mg) was used as starting material to give compound 144-A as a yellow solid (50 mg, yield 69%). LCMS: (ESI) m/z = 248.9 [M+H]⁺.

### Example 23: Synthesis of Compounds 71 and 72

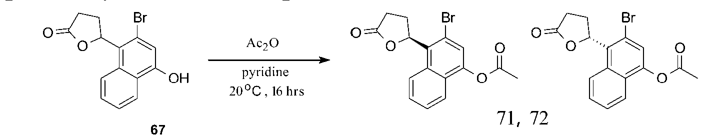

Acetic anhydride (22.4 mg, 219.77 µmol) was added to a solution of compound 67 (45 mg, 146.51 µmol) in pyridine (1 mL). The mixture was stirred at 25 °C for 16 hours. Water (3 mL) was added, and the mixture was extracted with EA (2 mL×3). After conventional work-up procedure, the crude product was purified by silica gel column chromatography (EA:PE, EA from 0 to 50%) followed by SFC chiral resolution to obtain compound 71 (5 mg) and compound 72 as white solid (5 mg).

Chiral separation conditions: column: DAICEL CHIRALPAK AD (250mm*30mm, 10um); mobile phase: 0.1% NH₃H₂O IPA; B%: 15 % - 15 %, flowrate: 100 mL/min.

First compound: LCMS: (ESI) m/z = 349.0 [M+H] ⁺, ¹H NMR (400 MHz, DMSO-*d*₆) δ ppm 8.13 - 8.19 (m, 1 H) 8.02 - 8.08 (m, 1 H) 7.66 - 7.76 (m, 3 H) 6.54 (dd, J=10.38, 7.38 Hz, 1 H) 2.90 - 3.03 (m, 1 H) 2.78 - 2.88 (m, 1 H) 2.62 - 2.68 (m, 1 H) 2.54 (br d, J=2.00 Hz, 1 H) 2.48 (s, 3 H). SFC chiral analysis: RT = 2.703 min.

Second compound: LCMS: (ESI) m/z = 349.0 [M+H] ⁺, ¹H NMR (400 MHz, DMSO-*d*₆) δ ppm 8.13 - 8.19 (m, 1 H) 8.01 - 8.09 (m, 1 H) 7.64 - 7.75 (m, 3 H) 6.54 (dd, J=10.32, 7.44 Hz, 1 H) 2.90 - 3.03 (m, 1 H) 2.78 - 2.89 (m, 1 H) 2.62 - 2.68 (m, 1 H) 2.54 (br s, 1 H) 2.48 (s, 3 H). SFC chiral analysis: RT = 2.974 min.

Chiral separation conditions: Column: ChiralPak AS-3 150×4.6mm I.D., 3um; Mobile phase: A: CO2 B:Ethanol (0.05% DEA); Gradient: from 5% to 40% of B in 4.5min , then 5% of B for 1.5 min; Flow rate: 2.5mL/min Column temp.:40°C; Back pressure: 100 bar.

### Example 24: Synthesis of Compound 68

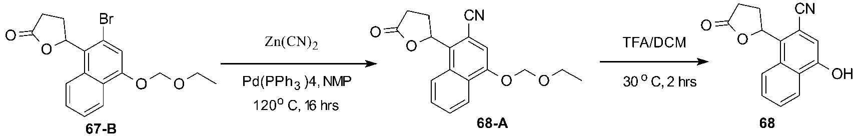

Step 1: Under a nitrogen atmosphere, Zn(CN)2 (68.8 mg, 0.59 mmol) and Pd(PPh3)4 (22.5 mg, 0.2 mmol) were added to a solution of compound 67-B (60 mg, 0.17 mmol) in NMP (1.2 mL). The mixture was heated to 120 °C and stirred for 16 hours. After cooling to room temperature, water (5 mL) was added, and the mixture was extracted with EA (5 mL×3). After conventional work-up procedure 2, the crude product was purified by prep-HPLC (FA method; B%: 28%-48%, 13 min) to obtain compound 68-A as a yellow solid (17 mg, yield 33%). LCMS: (ESI) m/z=312.1(M+1)⁺.

Step 2: According to the procedure of step 3 in Example 12, compound 68-A (50 mg) was reacted to obtain compound 68 as a white solid (12 mg, yield 29.2%). LCMS:(ESI) m/z= 253.9 [M+1] ⁺;¹H NMR (400 MHz, DMSO-d6) 11.24 (br s, 1H), 8.25-8.34 (m, 1H), 8.18 (br d, J=8.28 Hz, 1H), 7.67-7.80 (m, 2H), 7.06 (s, 1H), 6.43-6.52 (m, 1H), 2.83-2.98 (m, 1H), 2.70-2.82 (m, 2H), 2.28-2.42 (m, 1H).

### Example 25: Synthesis of Compound 73

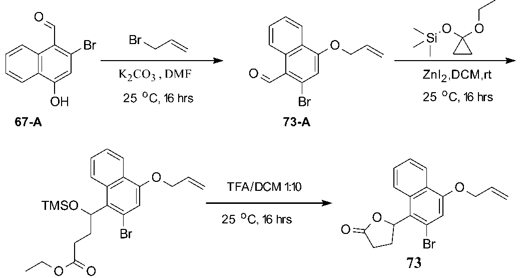

Step 1: 3-Bromo-1-propene (1.95 g, 16.13 mmol) and K₂CO₃ (4.46 g, 32.26 mmol) were added to a solution of 67-A (2.7 g, 10.75 mmol) in DMF (54 mL). The mixture was stirred at 25 °C for 16 hours, filtered, and concentrated under reduced pressure. Water (60 mL) was added, and the mixture was extracted with EA (60 mL×3). After conventional work-up procedure 2, the crude product was purified by silica gel column chromatography (EA:PE, EA from 0 to 4%) to obtain compound 73-A as an off-white solid (yield 98%). ¹H NMR (400 MHz, CDCl₃) 10.68 (s, 1H), 9.32 (d, J=8.78 Hz, 1H), 8.33 (d, J=8.52 Hz, 1H), 7.68-7.74 (m, 1H), 7.58 (t, J=7.64 Hz, 1H), 7.06 (s, 1H), 6.19 (ddt, 1H), 5.54-5.61 (m, 1H), 5.45 (dd, J=1.25, 10.54 Hz, 1H), 4.80-4.86 (m, 2H).

Steps 2 to 3: According to the procedures of steps 2 to 3 in Example 12, compound 73-A was subjected to two-step reactions to obtain compound 73 as a yellow oil (about 69% yield over two steps). ¹H NMR (400 MHz, CDCl₃) 8.37 (d, J=7.53 Hz, 1H), 7.97 (d, J=7.62 Hz, 1H), 7.51-7.60 (m, 2H), 6.99 (s, 1H), 6.48 (t, J=8.91 Hz, 1H), 6.13-6.23 (m, 1H), 5.55 (dd, J=1.25, 17.32 Hz, 1H), 5.41 (dd, J=1.25, 10.54 Hz, 1H), 4.74 (d, J=5.27 Hz, 2H), 2.86-2.93 (m, 2H), 2.64-2.70 (m, 2H).

### Example 26: Synthesis of Compound 80

According to the procedure of step 4 in Example **8-b,** compound 67 (70 mg, 227.91 µmol) was reacted with 80-A (1.2 *eq*) to obtain compound 80 as a white solid (25 mg, yield 27%). LCMS: (ESI) m/z= 371.0 [M+1] ⁺; ¹H NMR (400 MHz, DMSO-d6) 10.57 (s, 1H), 8.67 (s, 1H), 8.27 (d, J=8.03 Hz, 1H), 7.89-7.98 (m, 4H), 7.49-7.59 (m, 4H), 7.34-7.39 (m, 1H), 6.85 (s, 1H), 6.14 (t, J=8.78 Hz, 1H), 2.85-2.98 (m, 1H), 2.72-2.80 (m, 1H), 2.63-2.70 (m, 2H).

According to the method of Example 26, the starting materials listed in the following table were used in place of 80-A and reacted to give the corresponding final products.

| Starting material | Final product | LCMS and NMR data |
|---|---|---|
| 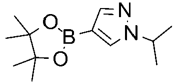 | 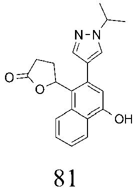 Yield 12%, pale yellow solid | LCMS: (ESI) m/z= 337.0 (M+1) ⁺; ¹H NMR (400 MHz, DMSO-d6) 10.45 (br s, 1H), 8.23 (d, J=7.53 Hz, 1H), 7.94 (s, 1H), 7.87 (d, J=8.53 Hz, 1H), 7.51-7.57 (m, 2H), 7.45-7.50 (m, 1H), 6.78 (s, 1H), 6.12 (dd, J=8.03, 9.79 Hz, 1H), 4.58 (td, J=6.65, 13.30 Hz, 1H), 2.85-2.97 (m, 1H), 2.72-2.80 (m, 1H), 2.59-2.65 (m, 2H), 1.49 (d, J=6.53 Hz, 6H) |
| 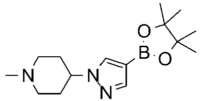 | 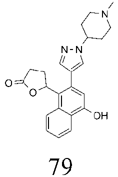 (containing 0.6 equivalents of formic acid); yield 5.5%, yellow solid | LCMS: (ESI) m/z= 392.0 (M+1) ^{+ 1}H NMR (400 MHz, DMSO-d6) Shift 10.48 (br s, 1H), 8.20-8.26 (m, 1H), 8.18 (s, 1H), 7.96 (s, 1H), 7.87 (d, J=8.53 Hz, 1H), 7.58 (s, 1H), 7.52-7.57 (m, 1H), 7.45-7.51 (m, 1H), 6.78 (s, 1H), 6.04-6.18 (m, 1H), 4.10-4.31 (m, 1H), 2.71-2.97 (m, 4H), 2.63 (br d, J=8.78 Hz, 1H), 2.24 (s, 3H), 1.97-2.12 (m, 7H) |
| 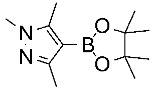 | 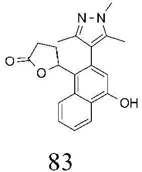 Yield 13.2%, gray solid | LCMS: (ESI) m/z= 337.1 (M+1) ⁺, ¹H NMR (400 MHz, DMSO-d6) Shift 10.49 (br s, 1H), 8.25 (d, J=8.28 Hz, 1H), 7.85 (dd, J=4.77, 8.28 Hz, 1H), 7.40-7.63 (m, 2H), 6.56 (d, J=5.02 Hz, 1H), 5.64-5.86 (m, 1H), 3.73 (s, 3H), 2.74-2.86 (m, 1H), 2.64-2.74 (m, 1H), 2.58 (br s, 1H), 2.23-2.34 (m, 1H), 2.05 (s, 3H), 1.96 (d, J=7.28 Hz, 3H) |
| 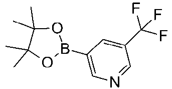 | 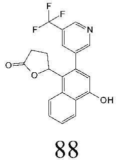 Yield 10.3%, white solid | LCMS:(ESI) m/z= 374.0 [M+1]⁺; ¹H NMR (400 MHz, DMSO-d6) 10.79 (br s, 1H), 9.06 (br s, 1H), 8.86 (br s, 1H), 8.30 (br d, J=8.03 Hz, 1H), 8.23 (br s, 1H), 7.96 (br d, J=8.53 Hz, 1H), 7.50-7.73 (m, 2H), 6.69 (s, 1H), 5.68-5.86 (m, 1H), 2.57-2.89 (m, 4H) |
| 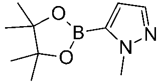 | 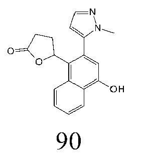 Yield 11.4%, white solid | LCMS:(ESI) m/z= 309.0 [M+1]⁺; ¹H NMR (400 MHz, DMSO-d6) 10.71 (br s, 1H), 8.29 (d, J=7.81 Hz, 1H), 7.95 (br d, J=8.28 Hz, 1H), 7.52-7.65 (m, 3H), 6.65 (br s, 1H), 6.34 (br s, 1H), 5.49-5.94 (m, 1H), 3.62 (br s, 3H), 2.79 (br s, 1H), 2.53-2.59 (m, 2H), 2.37-2.47 (m, 1H) |
| 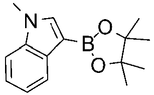 | 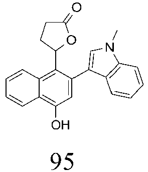 Yield 8.6%, brown solid | LCMS: (ESI) m/z= 358.0 [M+1]⁺;¹H NMR (400 MHz, DMSO-d6) 10.46 (br s, 1H), 8.27 (d, J=7.70 Hz, 1H), 7.89 (d, J=8.80 Hz, 1H), 7.46-7.60 (m, 4H), 7.44 (s, 1H), 7.25 (br t, J=7.70 Hz, 1H), 7.08-7.14 (m, 1H), 6.90 (s, 1H), 6.06-6.14 (m, 1H), 3.89 (s, 3H), 2.73-2.84 (m, 2H), 2.62-2.71 (m, 2H) |
| 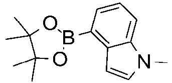 | 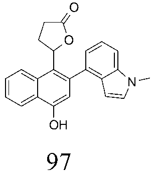 Yield 11%, white solid | LCMS:(ESI) m/z= 358.0[M+1]⁺; ¹H NMR (400 MHz, DMSO-d6) 10.53 (br s, 1H), 8.29 (br d, J=8.14 Hz, 1H), 7.87 (dd, J=8.47, 19.70 Hz, 1H), 7.49-7.62 (m, 3H), 7.33-7.38 (m, 1H), 7.24-7.31 (m, 1H), 6.93 (t, J=6.82 Hz, 1H), 6.76 (d, J=10.12 Hz, 1H), 6.12 (d, J=3.08 Hz, 1H), 5.61-5.70 (m, 1H), 3.85 (s, 3H), 2.57-2.74 (m, 2H), 2.28-2.38 (m, 1H), 2.20 (br d, J=7.26 Hz, 1H) |
| 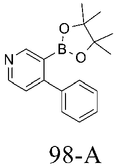 | 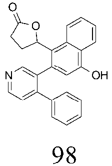 Yield 1.6%, white solid | LCMS:(ESI) m/z= 382.0 (M+H)⁺; ¹H NMR (400 MHz, DMSO-d6) Shift 10.34-10.73 (m, 1H), 8.71 (t, J=4.77 Hz, 1H), 8.46 (d, J=14.31 Hz, 1H), 8.16-8.27 (m, 1H), 7.74 (br d, J=8.03 Hz, 1H), 7.46-7.64 (m, 3H), 7.28 (d, J=8.03 Hz, 5H), 6.25-6.72 (m, 1H), 5.43-5.83 (m, 1H), 2.64-2.83 (m, 2H), 1.51 (br d, J=10.54 Hz, 1H), 0.86 (br s, 1H) |
| 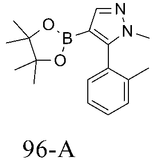 | 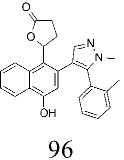 Yield 12.4%, white solid | LCMS: (ESI) m/z=399.0 (M+H) ⁺; ¹H NMR (400 MHz, DMSO-d6) Shift 10.37 (br s, 1H), 8.11-8.17 (m, 1H), 7.78 (d, J=8.50 Hz, 1H), 7.57 (d, J=4.98 Hz, 1H), 7.38-7.54 (m, 3H), 7.28-7.36 (m, 2H), 7.11-7.23 (m, 1H), 6.56 (s, 1H), 5.91-6.03 (m, 1H), 3.59-3.70 (m, 3H), 2.64-2.91 (m, 2H), 2.54-2.63 (m, 1H), 1.86-2.25 (m, 4H) |
| 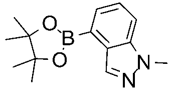 | 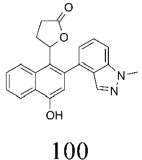 Yield 53%, white solid | LCMS: (ESI) m/z= 359.0[M+1]⁺; ¹H NMR (400 MHz, DMSO-d6) 10.60 (s, 1H), 8.31 (d, J=8.14 Hz, 1H), 7.90 (dd, J=8.58, 16.07 Hz, 1H), 7.69-7.74 (m, 2H), 7.49-7.64 (m, 3H), 7.05 (d, J=6.82 Hz, 1H), 6.77 (d, J=12.32 Hz, 1H), 5.59-5.68 (m, 1H), 4.11 (s, 3H), 2.55-2.76 (m, 4H) |
| 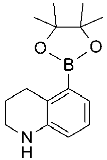 | 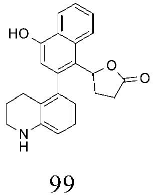 Yield 87%, off-white solid | LCMS:(ESI) m/z= 360.0[M+1]⁺; ¹H NMR (400 MHz, DMSO-d6) 10.47 (br s, 1H), 8.25 (d, J=8.36 Hz, 1H), 7.82 (dd, J=3.74, 8.36 Hz, 1H), 7.45-7.59 (m, 2H), 6.92 (t, J=7.71 Hz, 1H), 6.56 (s, 1H), 6.46-6.51 (m, 1H), 6.24 (t, J=7.30 Hz, 1H), 5.85 (br s, 1H), 5.55 (br d, J=3.08 Hz, 1H), 3.17 (br s, 2H), 2.66-2.84 (m, 2H), 2.54-2.60 (m, 1H), 2.27 (br d, J=6.82 Hz, 3H), 1.71 (br d, J=5.06 Hz, 2H) |
| 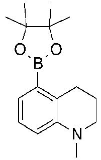 | 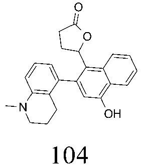 Yield 18.3%, white solid | LCMS:(ESI) m/z=374.1 [M+1] ⁺; ¹H NMR (400 MHz, DMSO-d6) 10.49 (br s, 1H), 8.26 (d, J=8.36 Hz, 1H), 7.83 (br d, J=8.36 Hz, 1H), 7.53-7.59 (m, 1H), 7.48-7.53 (m, 1H), 7.07 (t, J=7.81 Hz, 1H), 6.62 (t, J=7.91 Hz, 1H), 6.55 (d, J=3.30 Hz, 1H), 6.37 (t, J=7.26 Hz, 1H), 5.52 (dd, J=7.26, 10.34 Hz, 1H), 3.16-3.24 (m, 2H), 2.89 (s, 3H), 2.66-2.84 (m, 2H), 2.54-2.62 (m, 1H), 2.15-2.36 (m, 3H), 1.75-1.83 (m, 2H) |
| 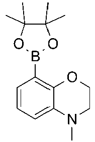 | 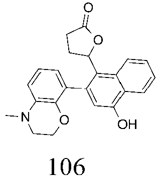 Yield 16%, white solid | LCMS:(ESI) m/z= 376.0[M+1]⁺; ¹H NMR (400 MHz, DMSO-d6) 10.34-10.42 (m, 1H), 8.24 (br d, J=7.70 Hz, 1H), 7.79-7.86 (m, 1H), 7.52-7.57 (m, 1H), 7.47-7.52 (m, 1H), 6.86 (q, J=8.07 Hz, 1H), 6.75 (d, J=7.92 Hz, 1H), 6.60-6.63 (m, 1H), 6.40-6.48 (m, 1H), 5.62-5.71 (m, 1H), 4.09-4.23 (m, 2H), 3.23-3.29 (m, 2H), 2.90 (s, 3H), 2.56-2.86 (m, 4H) |
| 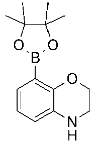 | 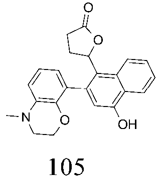 Yield 26%, white solid | LCMS:(ESI) m/z= 362.0[M+1]⁺;¹H NMR (400 MHz, DMSO-d6) 10.34-10.43 (m, 1H), 8.21-8.27 (m, 1H), 7.79-7.87 (m, 1H), 7.46-7.57 (m, 2H), 6.75 (q, J=7.78 Hz, 1H), 6.60-6.65 (m, 2H), 6.29-6.38 (m, 1H), 5.95 (s, 1H), 5.64-5.73 (m, 1H), 4.00-4.12 (m, 2H), 3.32 (s, 1H), 3.29 (br d, J=3.08 Hz, 1H), 2.59-2.91 (m, 3H), 2.27-2.49 (m, 1H) |
| 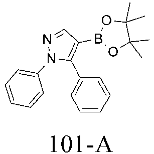 | 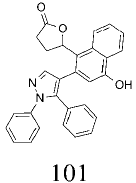 Yield 34%, white solid | LCMS: (ESI) m/z= 447.1[M+1]⁺; ¹H NMR (400 MHz, DMSO-d6) 10.46 (br s, 1H), 8.21 (br d, J=8.14 Hz, 1H), 7.85 (s, 1H), 7.82 (br d, J=8.14 Hz, 1H), 7.45-7.56 (m, 2H), 7.35-7.45 (m, 3H), 7.32 (br d, J=7.26 Hz, 2H), 7.21-7.28 (m, 3H), 7.08 (dd, J=2.20, 7.04 Hz, 2H), 6.65 (br s, 1H), 5.96 (br s, 1H), 2.53-2.82 (m, 3H), 2.23-2.50 (m, 1H) |
| 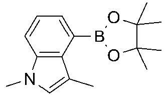 | 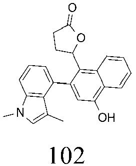 Yield 17.5%, pale yellow solid | LCMS:(ESI) m/z= 372.0 [M+1]⁺; ¹H NMR (400 MHz, DMSO-d6) 10.48 (br s, 1H), 8.29 (br d, J=8.14 Hz, 1H), 7.82-7.93 (m, 1H), 7.48-7.63 (m, 2H), 7.43 (br d, J=8.14 Hz, 1H), 7.19 (q, J=7.70 Hz, 1H), 7.07 (br s, 1H), 6.79 (br dd, J=7.04, 18.49 Hz, 1H), 6.69 (br d, J=4.62 Hz, 1H), 5.50-5.65 (m, 1H), 3.77 (s, 3H), 2.56-2.75 (m, 3H), 2.25 (br d, J=8.80 Hz, 1H), 1.52-1.66 (m, 3H) |
| 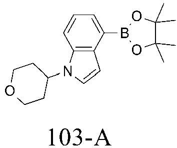 | 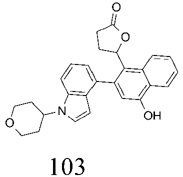 Yield 40%, white solid | LCMS:(ESI) m/z=428.1 [M+1]⁺; ¹H NMR (400 MHz, DMSO-d6) 10.51 (br s, 1H), 8.30 (d, J=8.19 Hz, 1H), 7.83-7.92 (m, 1H), 7.66 (dd, J=2.69, 8.31 Hz, 1H), 7.50-7.62 (m, 3H), 7.22-7.29 (m, 1H), 6.89-6.96 (m, 1H), 6.77 (d, J=6.14 Hz, 1H), 6.17 (d, J=3.06 Hz, 1H), 5.67-5.74 (m, 1H), 4.66-4.76 (m, 1H), 3.98-4.08 (m, 2H), 3.55-3.68 (m, 2H), 2.58-2.79 (m, 3H), 2.16-2.46 (m, 1H), 1.91-2.12 (m, 4H) |

The starting material 103-A listed in the above table was synthesized as follows:

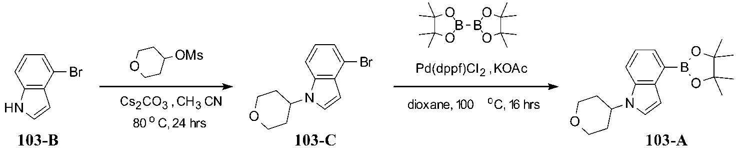

Step 1: 4-Tetrahydropyranyl methanesulfonate (4 *eq*) and cesium carbonate (6 *eq*) were added to a solution of 103-B (0.7 g, 3.57 mmol) in MeCN (14 mL). The mixture was stirred at 80 °C for 24 hours. LCMS showed incomplete reaction, but the desired product was formed. The mixture was filtered and concentrated under reduced pressure. The crude product was purified by silica gel column chromatography (EA:PE, EA from 0 to 5%) to give compound 103-C as a yellow oil (560 mg, yield 39%). LCMS: (ESI) m/z=281.9 [M+1]⁺.

Step 2: Under a nitrogen atmosphere, Pd(dppf)Cl₂ (104 mg, 0.1 eq) was added to a mixture of compound 103-C (400 mg, 1.43 mmol), bis(pinacolato)diboron (2 *eq*), and potassium acetate (3 *eq*) in dioxane (8 mL). The mixture was stirred at 100 °C under a nitrogen atmosphere for 16 hours. After the reaction, the mixture was cooled to room temperature, water (10 mL) was added, and the mixture was extracted with EtOAc (10 mL×3). After conventional work-up procedure 2, the crude product was purified by silica gel column chromatography (EA:PE, EA from 0 to 14%) to give compound 103-A as a yellow solid (420 mg, 84% yield). (ESI) m/z=328.1 (M+1)⁺.

The starting material 101-A listed in the above table was synthesized as follows:

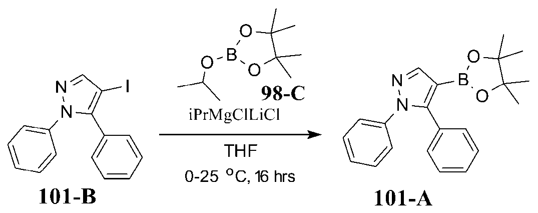

At 0 °C, a solution of isopropylmagnesium chloride lithium chloride complex in THF (1.3 M, 4.44 mL) was added dropwise to a solution of compound 101-B (1 g, 2.89 mmol) in THF (20 mL). After stirring for 1 hour, a solution of compound 98-C (1.07 g, 5.78 mmol) in THF (1.18 mL) was added dropwise. The mixture was warmed to 25 °C and stirred for an additional 15 hours. The reaction was quenched with saturated ammonium chloride solution (20 mL), and the mixture was extracted with EA (20 mL×2). After conventional work-up procedure 2, the crude product was purified by silica gel column chromatography (EA:PE, EA from 0 to 5%) to give compound **101-A** as an off-white solid (600 mg, yield 56%). LCMS: (ESI) m/z=347.1 (M+ H)⁺.

The starting material 98-A listed in the above table was synthesized as follows:

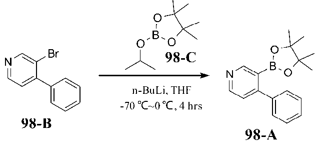

At -70 °C, n-butyllithium solution in THF (2.5 M, 1.71 mL) was added dropwise to a solution of compound **98-B** (500 mg, 2.14 mmol) in THF (20 mL) under a nitrogen atmosphere. The mixture was stirred for 1 hour. Then compound **98-C** (794 mg, 4.27 mmol) was added dropwise, and the mixture was warmed to 0 °C and stirred for 3 hours. After the reaction, the mixture was quenched with saturated NH4Cl solution, concentrated under reduced pressure, and diluted with EtOAc (20 mL). The mixture was filtered, and the filtrate was concentrated again under reduced pressure. The residue was purified by silica gel column chromatography (EA:PE, EA from 0 to 30%) to give a yellow gum (220 mg, yield 51.76%).

According to the method for starting material 98-A, the starting materials listed in the following table were used in place of 98-B and reacted to give the corresponding boronic ester starting materials.

| Starting material | Final product | LCMS and NMR data |
|---|---|---|
| 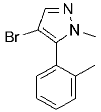 | 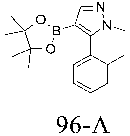 Yield 56%, white solid | LCMS: ESI) m/z=299.2 (M+H) ⁺ |

### Example 27: Synthesis of Compound 69

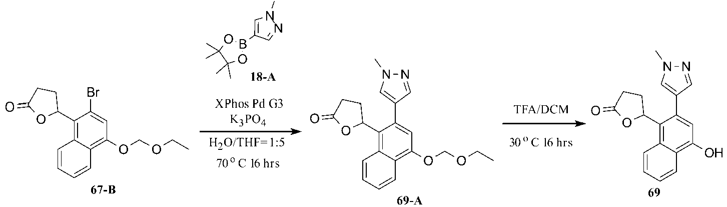

Step 1: According to the procedure of step 4 in Example **8-b,** 67-B (100 mg) was reacted with 18-A (74 mg) to obtain compound 69-A as a brown solid (50 mg, yield 47%). LCMS: ES19974-299-P1B1, (ESI) m/z= 367.5 [M+1]⁺.

Step 2: According to the procedure of step 3 in Example 12, compound 69-A (50 mg) was reacted to obtain compound 69 as a white solid (1.4 mg, yield 3.28%). LCMS: (ESI) m/z= 309.0 [M+1] ⁺, ¹H NMR (400MHz, DMSO-d6) Shift = 10.50 (br s, 1H), 8.23 (d, J=8.0 Hz, 1H), 7.91 - 7.84 (m, 2H), 7.58 - 7.51 (m, 2H), 7.51 - 7.45 (m, 1H), 6.76 (s, 1H), 6.09 (dd, J=7.8, 10.0 Hz, 1H), 3.93 (s, 3H), 2.95 - 2.73 (m, 2H), 2.70 - 2.57 (m, 2H).

According to the method of Example 27, the starting materials listed in the following table were used in place of 18-A and reacted to give the corresponding final products.

| Starting material | Final product | LCMS and NMR data |
|---|---|---|
| 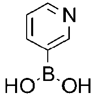 | 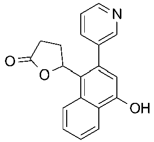 Compound 70 About 2.4% yield over two steps, white solid | LCMS: (ESI) m/z= 306.3 (M+1) ⁺; ¹H NMR (400MHz, DMSO-d6) Shift = 10.73 (br s, 1H), 8.66 (dd, J=1.6, 4.9 Hz, 1H), 8.57 (d, J=1.8 Hz, 1H), 8.29 (dd, J=1.1, 8.3 Hz, 1H), 7.92 (d, J=8.5 Hz, 1H), 7.80 (td, J=1.9, 7.8 Hz, 1H), 7.66 - 7.46 (m, 3H), 6.69 (s, 1H), 5.72 (dd, J=7.6, 9.8 Hz, 1H), 2.86 - 2.69 (m, 2H), 2.69 - 2.55 (m, 2H) |

### Example 28: Synthesis of Compound 84

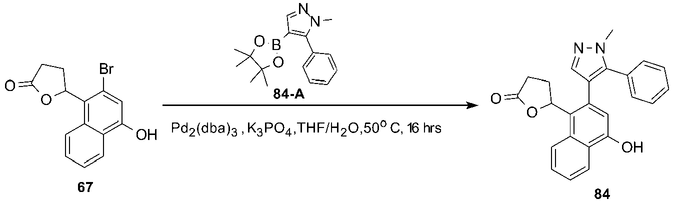

Under a nitrogen atmosphere, K₃PO₄ (93.3 mg, 439 µmol) and Pd₂(dba)₃ (26.8 mg, 29.30 µmol) were added to a mixture of compound 67 (90 mg, 293 µmol) and 84-A (100 mg, 352 µmol) in THF/water (2.4 mL, 5/l). The mixture was heated to 60 °C and stirred for 16 hours. The reaction was diluted with water (5 mL) and extracted with EA (5 mL×3). After conventional work-up procedure, the crude product was purified by preparative TLC (PE:EA, EA from 0 to 15%), followed by prep HPLC (FA method; B%: 35% -55%, 9 min) to obtain compound 84 as a white solid (18 mg, yield 16%). LCMS: (ESI) m/z= 385.1 [M+1]⁺.

### Example 28-a: Synthesis of Compound 85

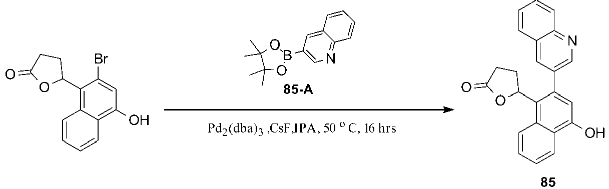

Under a nitrogen atmosphere, to a mixture of compound 67 (80 mg, 0.26 mmol), 85-A (80 mg, 312 µmol), 3-(tert-butyl)-4-(2,6-dimethoxyphenyl)-2,3-dihydrobenzo[d][1,3]oxaphosphole (CAS:1246888-90-3, 17.2 mg, 52.09 umol), and CsF (59.3 mg, 390.70 umol, 14.41 uL, 1.5 eq) in isopropanol (2 mL) was added Pd2(dba)3 (23.8 mg, 26 µmol). The mixture was heated to 50 °C and stirred for 16 hours. After cooling to room temperature, the reaction was diluted with water (5 mL) and extracted with EA (5 mL×3). After conventional work-up procedure 2, the crude product was purified by preparative TLC (PE:EA, EA from 0 to 25%), followed by prep HPLC (FA method, B%: 30%-50%, 9 min) to obtain compound 85 as a white solid (10 mg, yield 10.6%). LCMS: (ESI) m/z= 356.0 [M+1]⁺. ¹H NMR (400 MHz, DMSO-d6) 10.77 (br s, 1H), 8.89 (s, 1H), 8.39 (s, 1H), 8.32 (d, J=7.48 Hz, 1H), 8.11 (dd, J=4.73, 7.81 Hz, 2H), 7.95 (d, J=8.36 Hz, 1H), 7.85 (t, J=7.59 Hz, 1H), 7.68-7.74 (m, 1H), 7.55-7.66 (m, 2H), 6.80 (s, 1H), 5.80 (br t, J=8.47 Hz, 1H), 2.58-2.81 (m, 4H).

### Example 29: Synthesis of Compound 74

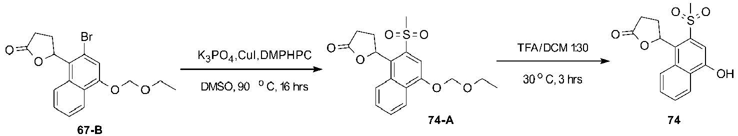

Step 1: Under a nitrogen atmosphere, sodium methanesulfinate (29.3 mg, 287.50 µmol), K₃PO₄ (81.4 mg, 383.33 µmol), CuI (1.83 mg, 9.58 µmol), and (2S,4R)-N-(2,6-dimethylphenyl)-4-hydroxypyrrolidine-2-carboxamide (2.25 mg, 9.58 µmol) were added to a solution of compound 67-B (70 mg, 0.19 mmol) in DMSO (1.4 mL). The mixture was heated to 90 °C and stirred for 16 hours. After cooling to room temperature, the reaction was diluted with EA and water (5 mL), and extracted with EA (10 mL×3). After conventional work-up procedure, the crude product was purified by silica gel column chromatography (EA:PE, EA from 0 to 20%) to obtain compound 74-A as a yellow gum (35 mg, yield 25%). LCMS: (ESI) m/z=365.0 (M+1)⁺.

Step 2: According to the procedure of step 3 in Example 12, compound 74-A (30 mg) was reacted to obtain compound 74 as a white solid (10 mg, yield 40%). LCMS: (ESI) m/z= 307.0 [M+1] ⁺, ¹H NMR (400 MHz, DMSO-d6) 11.23 (br s, 1H), 8.29-8.35 (m, 1H), 8.05-8.12 (m, 1H), 7.67-7.73 (m, 2H), 7.51 (s, 1H), 6.74 (dd, J=7.53, 10.04 Hz, 1H), 3.44 (br s, 3H), 2.95 (td, J=10.48, 17.69 Hz, 1H), 2.81 (br dd, J=8.28, 17.82 Hz, 1H), 2.61 (br d, J=10.54 Hz, 2H).

### Example 30: Synthesis of Compound 75

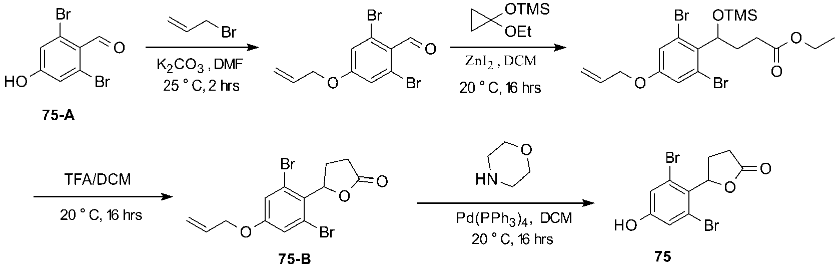

According to the procedures of steps 1 to 3 in Example 12: 75-A was used as starting material to obtain compound 75-B as a yellow solid in about 66.6% yield over three steps. ¹H NMR (400MHz, CHLOROFORM-d) Shift = 7.21 - 7.12 (m, 2H), 6.19 (t, J=8.8 Hz, 1H), 6.07 - 5.91 (m, 1H), 5.47 - 5.30 (m, 2H), 4.54 - 4.52 (m, 2H), 2.78 - 2.71 (m, 2H), 2.59 - 2.54 (m, 2H).

Step 4: Under a nitrogen atmosphere, a mixture of compound 75-B (300 mg, 0.8 mmol), Pd(PPh₃)₄ (92.2 mg, 79.8 µmol), and morpholine (139.0 mg, 1.60 mmol) in THF (16 mL) was stirred at 20 °C for 16 hours. Water (40 mL) was added, and the mixture was extracted with EA (30 mL×3). After conventional work-up procedure 2, the crude product was purified by silica gel column chromatography (EA:PE, EA from 0 to 30%) to obtain compound 75 as a white solid (125 mg, yield 47%). LCMS: (ESI) m/z= 336.8 [M+1] ⁺; ¹H NMR (400MHz, DMSO-d6) Shift = 10.58 (s, 1H), 7.20 - 7.03 (m, 2H), 6.09 (t, J=8.8 Hz, 1H), 2.90 - 2.74 (m, 1H), 2.70 - 2.59 (m, 1H), 2.49 - 2.43 (m, 1H), 2.42 - 2.25 (m, 1H).

### Example 31: Synthesis of Compound 77

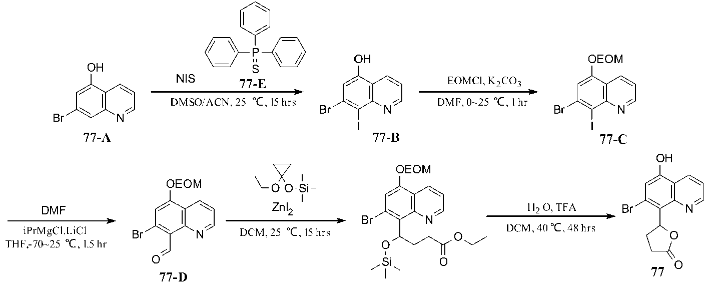

Step 1: 77-E (65.6 mg, 223.16 µmol) was added to a solution of compound 77-A (1 g, 4.46 mmol) and NIS (1.14 g, 5.07 mmol) in DMSO/ACN (100 mL, 1:1). The mixture was stirred at 25 °C for 15 hours. Water (50 mL) was added, and the mixture was extracted with EA (50 mL×2). After conventional work-up procedure, 1 g of a gray solid was obtained, which was used directly in the next step. LCMS: (ESI) m/z=351.8 (M+1)⁺.

Step 2: According to the procedure of step 1 in Example 22, compound 77-B (1 g, crude) was reacted to obtain compound 77-C as a yellow solid (1.1 g, purity 58.65%). LCMS: (ESI) m/z=409.9 (M+1)⁺.

Step 3: At -70 °C, isopropylmagnesium chloride lithium chloride complex solution in THF (1.3 M, 1.27 mL) was added dropwise to a solution of compound 77-C (560 mg) in THF (30 mL). The mixture was warmed to 25 °C and stirred for 1 hour. At -70 °C, a solution of DMF (120 mg, 1.65 mmol) in THF (1 mL) was added. The mixture was warmed to 25 °C and stirred for 0.5 hours. At 0 °C, water (10 mL) was added to quench, and the mixture was extracted with EA (10 mL×2). After conventional work-up procedure, the residue was purified by silica gel column chromatography (EA:PE, EA from 0 to 5%) to obtain compound **77-D** as a yellow solid (240 mg, purity 80.4%). LCMS: (ESI) m/z=311.9 (M+ H)⁺.

Steps 4 and 5: According to the procedures of steps 2 and 3 in Example 12, compound 77-D was subjected to two-step reactions to obtain compound 77 as a yellow solid in 8.8% yield over five steps. LCMS: (ESI) m/z=310.0 (M+H)⁺; ¹H NMR (400 MHz, DMSO-d6) Shift 11.25 (s, 1H), 8.91 (d, J=4.49 Hz, 1H), 8.54 (d, J=8.56 Hz, 1H), 7.56 (dd, J=4.14, 8.41 Hz, 1H), 7.11-7.14 (m, 1H), 6.58 (dd, J=7.53, 8.78 Hz, 1H), 2.71-2.91 (m, 2H), 2.57-2.68 (m, 2H).

### Example 32: Synthesis of Compound 86

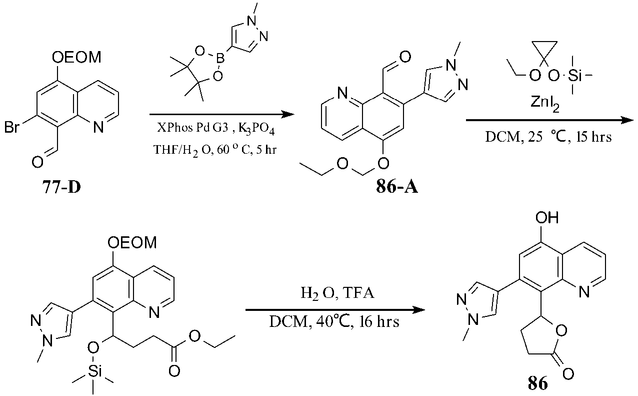

Step 1: According to the procedure of step 4 in Example **8-b,** 77-D (200 mg, 644.86 µmol) was reacted with 18-A (147 mg, 709.35 µmol) to obtain compound 86-A as a yellow solid (130 mg, yield 64.75%). ¹H NMR (400 MHz, CHLOROFORM-d) δ 11.03 (s, 1H), 9.02 (dd, J=1.83, 4.28 Hz, 1H), 8.58 (dd, J=1.83, 8.44 Hz, 1H), 7.87 (s, 1H), 7.76 (s, 1H), 7.44 (dd, J=4.16, 8.44 Hz, 1H), 7.23 (s, 1H), 5.52 (s, 2H), 3.99 (s, 3H), 3.82 (q, J=7.09 Hz, 2H), 1.26 (t, J=7.09 Hz, 3H).

Steps 2 and 3: According to the procedures of steps 2 and 3 in Example 12, compound 86-A (130 mg, 417.56 umol) was subjected to two-step reactions to obtain compound 86 as a yellow solid (4 mg) in about 2.7% yield over two steps. LCMS: (ESI) m/z = 309.9 [M+H] ⁺. ¹H NMR (400 MHz, DMSO-d6) δ 10.79 (s, 1H), 8.85 (dd, J=1.76, 4.02 Hz, 1H), 8.52 (dd, J=1.76, 8.28 Hz, 1H), 7.92 (s, 1H), 7.59 (s, 1H), 7.47 (dd, J=4.27, 8.28 Hz, 1H), 6.84 (s, 1H), 6.10 (t, J=7.53 Hz, 1H), 3.94 (s, 3H), 2.85-2.98 (m, 1H), 2.61-2.76 (m, 3H).

### Example 33: Synthesis of Compounds 92, 93, 94

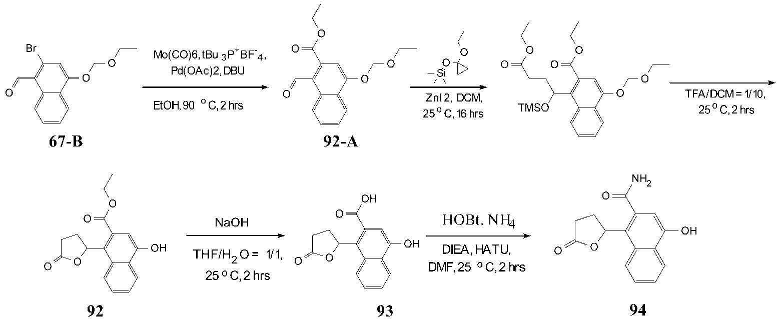

Step 1: Under a nitrogen atmosphere, Pd(OAc)₂ (26.8 mg, 119.68 µmol), molybdenum hexacarbonyl (Mo(CO)₆, 173 mg, 658.25 µmol), and tri-tert-butylphosphonium tetrafluoroborate (34.7 mg, 119.68 µmol) were added to a solution of compound 67-B (370 mg, 1.20 mmol) and DBU (273 mg, 1.80 mmol) in EtOH (10 mL). The mixture was stirred at 90 °C Under a nitrogen atmosphere for 16 hours. After filtration and concentration, the residue was purified by silica gel column chromatography (EA:PE, EA from 0 to 2%) to obtain compound 92-A as a yellow oil (170 mg, yield 47%). LCMS: (ESI) m/z = 303.0 (M+1)⁺.

Steps 2 and 3: According to the procedures of steps 2 and 3 in Example 12, compound 92-A (260 mg, 860 µmol) was subjected to two-step reactions to obtain compound 92 as a white solid (40 mg) in about 15.5% yield over two steps. LCMS: (ESI) m/z = 301.3 (M+1)⁺. ¹H NMR (400 MHz, CDCl₃) δ 8.35-8.50 (m, 1H), 7.90-8.06 (m, 1H), 7.59-7.77 (m, 2H), 7.36 (br s, 1H), 7.30 (s, 1H), 5.90 (dd, J=2.31, 8.47 Hz, 1H), 3.91-4.26 (m, 2H), 2.77-2.93 (m, 1H), 2.59-2.73 (m, 1H), 2.46 (td, J=6.27, 16.95 Hz, 1H), 2.03 (dt, J=6.71, 14.36 Hz, 1H), 1.25 (t, J=7.15 Hz, 3H).

Step 4: Compound 92 (20 mg) was dissolved in THF (0.3 mL), and aqueous NaOH solution (10.7 mg, 266 µmol in 0.3 mL water) was added. The mixture was stirred at 25 °C for 2 hours, then acidified to pH 2 with 1M HCl and extracted with EA (1 mL×3). The extract was concentrated to obtain a crude product, which was triturated and slurried in DCM (3 mL) for 10 minutes, then filtered to obtain compound 93 as a white solid (15 mg, yield 82.48%). ¹H NMR (400 MHz, DMSO-d6) δ 12.25 (br s, 1H), 10.95 (br s, 1H), 8.22-8.42 (m, 1H), 7.99-8.16 (m, 1H), 7.54-7.88 (m, 2H), 7.04 (s, 1H), 5.97 (dd, J=2.53, 8.25 Hz, 1H), 2.55-2.71 (m, 1H), 2.33-2.47 (m, 1H), 2.14-2.29 (m, 1H), 1.83-1.98 (m, 1H). LCMS: (ESI) m/z = 273.2 (M+1)⁺.

Step 5: DIEA (56.96 mg, 440.77 umol) and HATU (139.66 mg, 367.31 µmol) were added to a solution of compound 93 (40 mg, 146.92 µmol) and HOBT ammonium salt (CAS: 63307-62-0, 111.77 mg, 734.61 µmol) in DMF (1 mL). The mixture was stirred at 25 °C for 2 hours, diluted with water (2 mL) and acetonitrile (1 mL), and filtered. The filtrate was concentrated and purified by prep HPLC (column: C18-1 150×30 mm×5 um; mobile phase: [water (FA)-ACN]; B%: 5%-45%, 9 min) to obtain compound 94 as a white solid (20 mg, yield 50.18%). ¹H NMR (400 MHz, DMSO-d6) δ 10.95 (s, 1H), 8.25-8.40 (m, 1H), 8.04-8.17 (m, 1H), 7.64-7.83 (m, 2H), 7.35 (br s, 1H), 7.03 (s, 1H), 6.82 (br s, 1H), 5.95 (dd, J=2.64, 7.92 Hz, 1H), 2.55-2.72 (m, 1H), 2.18-2.33 (m, 1H), 1.95-2.06 (m, 1H), 1.80-1.95 (m, 1H). LCMS: (ESI) m/z = 272.0 (M+1)⁺.

### Example 34: Synthesis of Compound 117

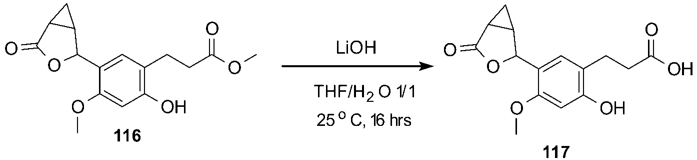

To a mixture of compound 116 (100 mg, 0.33 mmol) in THF (1.5 mL) and water (1.5 mL) was added lithium hydroxide monohydrate (20.5 mg, 0.49 mmol, about 1.5 *eq*). The mixture was stirred at 25 °C for 16 hours. THF was removed under reduced pressure, and the pH was adjusted to 6-7 with AcOH. Purification by prep HPLC (FA method, B: 18%-38%, 10 min) obtained compound 117 as a white solid (25 mg, yield 25.68%). ¹H NMR (400 MHz, DMSO-d6) Shift 10.84 (m, 1H ), 9.38-12.31 (m, 1H), 6.82-7.05 (m, 1H), 6.42-6.56 (m, 1H), 5.73 (d, J=4.65 Hz, 0.24H), 5.37 (s, 0.76H), 3.67-3.82 (m, 3H), 2.64-2.75 (m, 2H), 2.38-2.43 (m, 2H), 2.21 (dt, J=2.45, 5.69 Hz, 2H), 1.29 (dt, J=4.46, 8.16 Hz, 1H), 1.05 (br d, J=4.52 Hz, 1H), 0.68-0.99 (m, 1H). LCMS: (ESI) m/z=293.3 (M+1)⁺.

### Example 35: Synthesis of Compounds 118 and 119

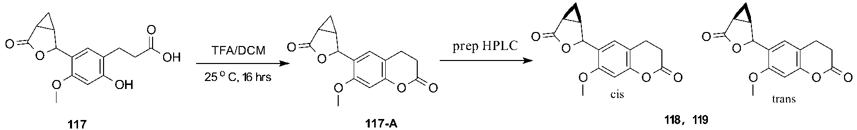

A solution of compound 117 (90 mg, 307.92 µmol) in TFA (0.2 mL) and DCM (2 mL) was stirred at 25 °C for 1 hour. The reaction was quenched with saturated NaHCO₃ solution (5 mL) and extracted with DCM (5 mL×3). After conventional work-up procedure, compound 117-A was obtained as a yellow oil (60 mg, yield 71.05%). LCMS: (ESI) m/z=275.3 (M+1)⁺.

Compound 117-A (15 mg) was purified by prep HPLC (column: Boston Prime C18 150*30mm*5um; mobile phase: [water (ammonia hydroxide v/v)-ACN]; gradient: 33%-53% B over 10 min) to obtain compound 118 as a white solid (8 mg, purity 80%) and compound 119 as a white solid (5 mg, purity 90%).

Compound 118: ¹H NMR (400 MHz, DMSO-d6) Shift 7.15 (s, 1H), 6.86 (s, 1H), 5.48 (s, 1H), 3.83 (s, 3H), 2.89-2.98 (m, 2H), 2.75-2.83 (m, 2H), 2.16-2.38 (m, 3H), 1.30-1.37 (m, 1H), 1.00-1.06 (m, 1H). LCMS: (ESI) m/z=275.3 (M+1)⁺.

Compound 119: ¹H NMR (400 MHz, DMSO-d6) Shift 7.06 (s, 1H), 6.77-6.86 (m, 1H), 6.83 (s, 1H), 5.80 (d, J=4.52 Hz, 1H), 3.84 (s, 3H), 2.89-2.95 (m, 2H), 2.73-2.81 (m, 2H), 2.58-2.66 (m, 1H), 2.21 (ddd, J=3.14, 5.58, 8.85 Hz, 1H), 1.06 (dt, J=4.89, 8.22 Hz, 1H), 0.70 (q, J=4.27 Hz, 1H). LCMS: (ESI) m/z=275.3 (M+1)⁺.

### Example 36: Synthesis of Compound 25

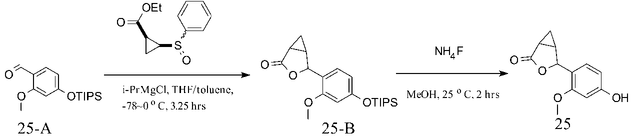

**Step 1:** According to the procedure of step 6 in Example 1, (14-c-1) (1.5 g, 6.29 mmol) and compound 25-A (860 mg, 2.67 mmol) were reacted to obtain compound 25-B as a white solid (70 mg, yield 2.9%). (ESI) m/z= 377.1 (M+1) ⁺.

**Step 2:** Ammonium fluoride (11.8 mg, 318.67 umol, 2 *eq*) was added to a solution of compound 25-B (60 mg, 159 µmol) in MeOH (2 mL). The mixture was stirred at 25 °C for 2 hours, then concentrated under reduced pressure. EtOAc (20 mL) was added, and the mixture was subjected to conventional work-up procedure 2. The residue was purified by silica gel column chromatography (EA/PE, EA from 0 to 50%) to obtain compound **25** as a white solid (5 mg, yield 14.25%). (ESI) m/z=221.0 (M+1)⁺. ¹H NMR (400 MHz, CDCl₃) δ 7.11 (d, *J*=8.03 Hz, 1H), 6.46 (d, *J*=2.26 Hz, 1H), 6.39-6.44 (m, 1H), 5.50 (s, 1H), 3.82 (s, 3H), 2.25 (m, 1H), 2.14-2.21 (m, 1H), 1.33 (m, 1H), 1.02-1.10 (m, 1H).

### Example 37: Synthesis of Compound 134

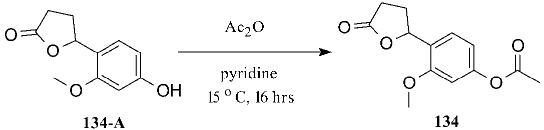

Acetic anhydride (14.7 mg, 144.09 µmol) was added to a solution of compound 134-A (20 mg, 96 µmol) in pyridine (0.5 mL). The mixture was stirred at room temperature (15 °C) for 16 hours, then concentrated under reduced pressure. Water (2 mL) was added, and the mixture was extracted with EA (1 mL×2). After conventional work-up procedure, the crude product was purified by silica gel column chromatography (EA:PE, EA from 0 to 30%) to obtain compound 134 as a yellow oil (15 mg, yield 60.28%). LCMS: ESI m/z = 251.0 [M+H]⁺. ¹H NMR (400 MHz, CDCl₃) δ ppm 7.34 (d, J=8.44 Hz, 1 H) 6.73 (dd, J=8.31, 2.08 Hz, 1 H) 6.68 (d, J=2.08 Hz, 1 H) 5.74 (t, J=7.03 Hz, 1 H) 3.85 (s, 3 H) 2.67 - 2.76 (m, 1 H) 2.60 - 2.66 (m, 2 H) 2.33 (s, 3 H) 2.09 - 2.20 (m, 1 H).

According to the method in Example 37, the starting materials listed in the following table were used in place of 134-A and reacted to give the corresponding final products.

| Starting material | Final product | LCMS and NMR data |
|---|---|---|
| Compound 109 | 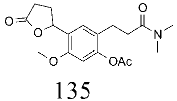 Yield 55.6%, colorless gum | LCMS: (ESI) m/z= 350.0 (M+1) ⁺; ¹H NMR (400 MHz, CDCl₃) δ 7.21 (s, 1H), 6.59 (s, 1H), 5.70 (t, J=7.15 Hz, 1H), 3.81 (s, 3H), 2.95 (d, J=3.26 Hz, 6H), 2.74-2.89 (m, 2H), 2.59-2.73 (m, 3H), 2.48-2.56 (m, 2H), 2.35 (s, 3H), 2.06-2.19 (m, 1H) |
| Compound 25 | 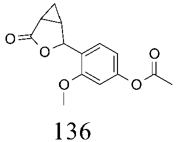 Yield 70.5%, colorless oil | LCMS: (ESI) m/z= 263.0 (M+1) ⁺; ¹H NMR (400 MHz, CDCl₃) Shift 7.31 (d, J=8.19 Hz, 1H), 7.23 (d, J=8.31 Hz, 0.08 H), 6.74 (dd, J=2.14, 8.25 Hz, 1H), 6.68-6.71 (m, 1H), 5.85 (d, J=4.77 Hz, 0.08H), 5.55 (s, 1H), 3.81-3.96 (m, 3H), 2.68-2.76 (m, 0.08H), 2.33 (s, 3H), 2.29 (m, 1H), 2.11-2.20 (m, 1H), 1.35 (m, 1H), 1.06-1.16 (m, 1H), 0.98-1.05 (m, 0.88H) |
| Compound 69 | 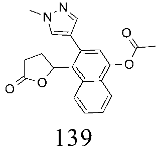 Yield 35%, white solid | LCMS: (ESI) m/z=351.1 (M+1) ⁺, ¹H NMR (400 MHz, DMSO-d6) Shift 12.25 (s, 1H), 7.92-8.13 (m, 3H), 7.51-7.77 (m, 3H), 7.28 (s, 1H), 6.09-6.31 (m, 1H), 3.93 (s, 3H), 2.78-3.02 (m, 2H), 2.64-2.76 (m, 2H), 2.47 (s, 3H) |
| Compound 85 | 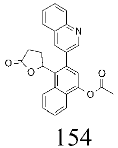 Yield 55%, yellow solid | LCMS:(ESI) m/z=398.1[M+1]⁺; ¹H NMR (400 MHz, DMSO-d6) 8.92 (d, J=2.00 Hz, 1H), 8.44 (d, J=1.88 Hz, 1H), 8.07-8.16 (m, 4H), 7.85 (t, J=7.20 Hz, 1H), 7.67-7.77 (m, 3H), 7.41 (s, 1H), 5.95 (br t, J=8.38 Hz, 1H), 2.59-2.86 (m, 4H), 2.47-2.49 (m, 3H) |
| Compound 76 | 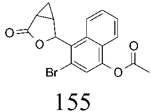 Yield 69%, white solid | ¹H NMR (400 MHz, CHLOROFORM-d) Shift 8.12-8.25 (m, 1H), 7.88-8.02 (m, 1H), 7.58-7.70 (m, 2H), 7.56 (s, 1H), 6.38 (s, 1H), 2.57 (m, 1H), 2.40-2.53 (m, 4H), 1.64 (m, 1H), 1.34-1.45 (m, 1H) |
| Compound 91 | 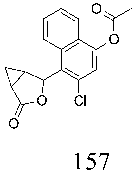 Yield 19.6%, yellow solid | ¹H NMR (400 MHz, CDCl₃) δ ppm 8.17 (d, J=8.68 Hz, 1 H) 7.97 (dd, J=8.31, 0.98 Hz, 1 H) 7.63 - 7.69 (m, 1 H) 7.57 - 7.62 (m, 1 H) 7.39 (s, 1 H) 6.38 (s, 1 H) 2.52 - 2.58 (m, 1 H) 2.51 (s, 3 H) 2.42 - 2.48 (m, 1 H) 1.61 - 1.66 (m, 1 H) 1.33 - 1.38 (m, 1 H); LCMS:(ESI) m/z=317.0 [M+1]⁺ |
| Compound 14 | 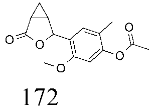 Yield 19.3%, white solid | LCMS: (ESI) m/z = 277.0 (M+1)⁺.¹H NMR (DMSO-d6, 400 MHz) δ 7.0-7.1 (m, 1H), 6.8-6.9 (m, 1H), 5.47 (s, 1H), 3.7-3.8 (m, 3H), 2.3-2.4 (m, 3H), 2.2-2.3 (m, 2H), 2.0-2.1 (m, 3H), 1.3-1.4 (m, 1H), 1.0-1.0 (m, 1H). |

According to the method in Example 37, the starting materials listed in the following table were reacted to give the corresponding final products.

| Starting material | Final product | LCMS and NMR data |
|---|---|---|
| Compound 67 and 5 eq. of 148-A 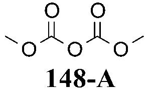 | 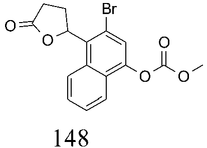 Yield 59%, white solid | ¹H NMR (400 MHz, DMSO-*d*₆) δ ppm 2.78 - 2.88 (m, 2 H) 2.90 - 3.02 (m, 2 H) 3.93 (s, 3 H) 6.54 (dd, *J*=10.42, 7.40 Hz, 1 H) 7.69 - 7.76 (m, 2 H) 7.86 (s, 1 H) 8.01 (d, *J*=6.95 Hz, 1 H) 8.16 - 8.20 (m, 1 H). LCMS: (ESI) m/z = 365.0 (M+1) ⁺ |
| Compound 76 and 5 eq. of 148-A 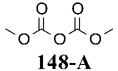 | 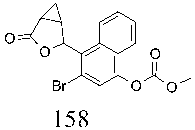 Yield 76%, yellow solid | LCMS: (ESI) m/z=377.2 (M+1)⁺; ¹H NMR (400 MHz, CHLOROFORM-d) Shift 8.18 (dd, J=1.77, 7.40 Hz, 1H), 8.02-8.13 (m, 1H), 7.58-7.72 (m, 3H), 6.38 (s, 1H), 4.01 (s, 3H), 2.58 (m, 1H), 2.39-2.52 (m, 1H), 1.63-1.70 (m, 1H), 1.34-1.44 (m, 1H) |

### Example 38: Synthesis of Compound 138

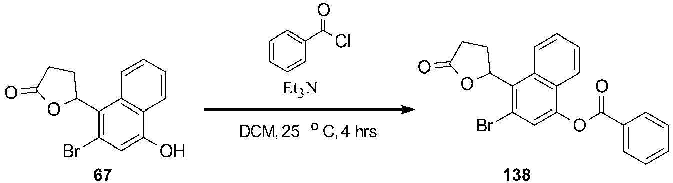

TEA (24.7 mg, 212 µmol) and benzoyl chloride (29.7 mg, 212 µmol) were added to a solution of compound 67 (50 mg, 163 µmol) in DCM (2 mL). The mixture was stirred at 25 °C for 4 hours, then concentrated under reduced pressure. The residue was purified by silica gel column chromatography (EA/PE, EA from 0 to 30%) to obtain compound **138** as a white solid (20 mg, yield 28.6%). LCMS: (ESI) m/z =411.0 [M+H]⁺; ¹H NMR (400 MHz, DMSO-d6) δ 8.28 (d, J=7.28 Hz, 2H), 8.20 (d, J=8.53 Hz, 1H), 8.00 (d, J=8.03 Hz, 1H), 7.91 (s, 1H), 7.81-7.87 (m, 1H), 7.66-7.77 (m, 4H), 6.58 (dd, J=7.53, 10.29 Hz, 1H), 2.80-3.06 (m, 2H), 2.64-2.75 (m, 1H), 2.55-2.62 (m, 1H).

According to the method of Example 38, the starting materials listed in the following table were used in place of benzoyl chloride and reacted with compound 67 to give the corresponding final products.

| Starting material | Final product | LCMS and NMR data |
|---|---|---|
| 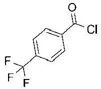 | 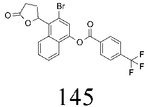 Yield 26%, colorless oil | LCMS: (ESI) m/z= 481.0 (M+1) ⁺; ¹H NMR (400 MHz, DMSO-*d*₆) δ ppm 2.55 - 2.63 (m, 2 H) 2.84 - 2.88 (m, 1 H) 2.93 - 3.01 (m, 1 H) 6.57 (dd, *J*=10.45, 7.37 Hz, 1 H) 7.66 - 7.76 (m, 2 H) 7.95 (s, 1 H) 8.03 - 8.07 (m, 3 H) 8.20 (d, *J*=8.80 Hz, 1 H) 8.46 (d, *J*=8.14 Hz, 2 H) |

### Example 39: Synthesis of Compounds 140 and 141

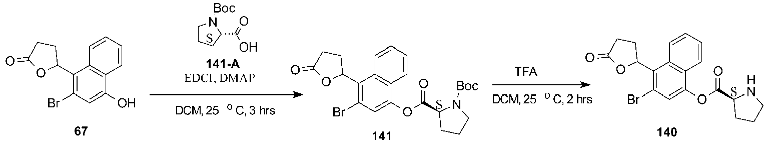

Step 1: EDCI (40.5 mg, 211.63 µmol, 1.3 *eq*) and DMAP (1.99 mg, 16.28 µmol, 0.1 eq) were added to a solution of compound 67 (50 mg, 163 µmol) and compound 141-A (38.5 mg, 179 µmol, 1.1 *eq*) in DCM (2 mL). The mixture was stirred at 25 °C for 3 hours, then concentrated under reduced pressure. The crude product was purified by silica gel column chromatography (EA/PE, EA from 0 to 30%) to obtain compound **141** as a white solid (70 mg, yield 82%). ¹H NMR (400 MHz, DMSO-d6) δ 8.09-7.95(m, 2H), 7.60-7.81 (m, 3H), 6.54 (br dd, J=7.64, 9.96 Hz, 1H), 4.52-4.77 (m, 1H), 3.46-3.56 (m, 2H), 2.62-3.06 (m, 3H), 2.44 (br d, J=5.14 Hz, 3H), 1.90-2.16 (m, 2H), 1.44 (d, J=18.46 Hz, 9H). (ESI) m/z =403.9 [M-Boc+H]⁺.

Step 2 (Representative Boc deprotection procedure): A solution of compound **141** (20 mg, 0.397 mmol) in DCM (2 mL) and TFA (0.4 mL) was stirred at 25 °C for 2 hours, then concentrated under reduced pressure. The residue was purified by prep HPLC (FA method; B%: 5%-35%, 8 min) to obtain compound **140** as a white solid (2 mg, yield 12%). LCMS: (ESI) m/z = 404.1 [M+H] ⁺. ¹H NMR (400 MHz, DMSO-d6) δ 8.22 (d, J=7.78 Hz, 1H), 7.96 (d, J=8.53 Hz, 1H), 7.50-7.69 (m, 2H), 7.11 (s, 1H), 6.43 (dd, J=7.53, 10.29 Hz, 1H), 3.67 (br dd, J=6.02, 8.53 Hz, 1H), 2.74-3.07 (m, 4H), 1.65-2.13 (m, 4H).

According to the method of step 1 in Example 39, the starting materials listed in the following table were used in place of 141-A and reacted with compound 67 to give the corresponding final products.

| Starting material | Final product | LCMS and NMR data |
|---|---|---|
| 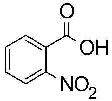 | 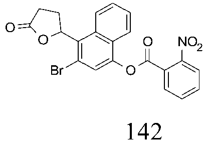 Yield 79%, white solid | LCMS: (ESI) m/z= 457.9 (M+1) ⁺; ¹H NMR (400 MHz, DMSO-d6) δ 8.36 (dd, J=1.38, 7.65 Hz, 1H), 8.18-8.30 (m, 2H), 7.96-8.13 (m, 3H), 7.92 (s, 1H), 7.70-7.83 (m, 2H), 6.58 (dd, J=7.53, 10.29 Hz, 1H), 2.95 (br d, J=10.04 Hz, 1H), 2.80-2.90 (m, 1H), 2.69 (br d, J=9.54 Hz, 1H), 2.57 (br s, 1H). |
| 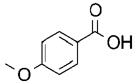 | 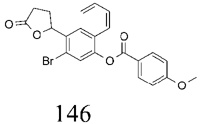 Yield 33%, white solid | LCMS: (ESI) m/z= 488.2 (M+2Na) ⁺; ¹H NMR (400 MHz, CDCl₃) δ 8.23-8.30 (m, 2H), 8.08 (d, J=7.70 Hz, 1H), 7.97-8.02 (m, 1H), 7.59-7.62 (m, 1H), 7.51-7.59 (m, 2H), 7.02-7.09 (m, 2H), 6.53 (dd, J=7.15, 10.45 Hz, 1H), 3.94 (s, 3H), 2.83-2.97 (m, 2H), 2.58-2.78 (m, 2H) |
| 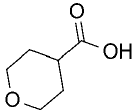 | 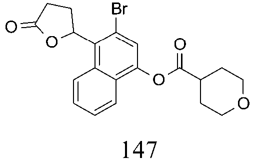 Yield 51%, white solid | LCMS: (ESI) m/z= 419.1 (M+H) ⁺; ¹H NMR (400 MHz, CDCl₃) δ ppm 2.01 - 2.17 (m, 4 H) 2.57 - 2.76 (m, 2 H) 2.86 - 2.93 (m, 2 H) 2.97 - 3.05 (m, 1 H) 3.58 (td, *J*=11.29, 2.56 Hz, 2 H) 4.11 (dt, *J*=11.51, 3.44 Hz, 2 H) 6.50 (dd, *J*=10.51, 7.13 Hz, 1 H) 7.49 (s, 1 H) 7.55 - 7.62 (m, 2 H) 7.87 - 7.91 (m, 1 H) 8.05 - 8.10 (m, 1 H) |
| 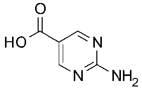 | 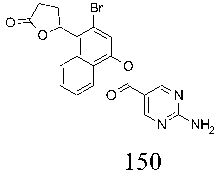 Yield 71%, white solid | LCMS: (ESI) m/z= 429.9 (M+H) ⁺; ¹H NMR (400 MHz, DMSO-d6) 8.98 (s, 2H), 8.18 (d, J=8.58 Hz, 1H), 8.04 (d, J=8.36 Hz, 1H), 7.84 (d, J=9.02 Hz, 3H), 7.64-7.75 (m, 2H), 6.56 (dd, J=7.48, 10.12 Hz, 1H), 2.91-3.04 (m, 1H), 2.79-2.89 (m, 1H), 2.63-2.73 (m, 1H), 2.53-2.61 (m, 1H) |
| 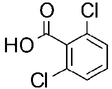 | 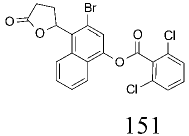 Yield 12.5%, white solid | LCMS:(ESI) m/z= 480.8 [M+1]⁺; ¹H NMR (400 MHz, DMSO-d6) 8.24 (br d, J=8.80 Hz, 1H), 8.14-8.20 (m, 1H), 7.77 (br d, J=7.82 Hz, 4H), 7.67-7.74 (m, 2H), 6.54-6.63 (m, 1H), 2.91-3.06 (m, 1H), 2.78-2.91 (m, 1H), 2.63-2.76 (m, 1H), 2.55-2.63 (m, 1H) |
| 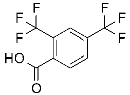 | 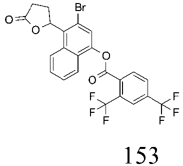 Yield 62%, white solid | ¹H NMR (400 MHz, DMSO-*d*₆) δ ppm 2.59 (br s, 1 H) 2.70 (br s, 1 H) 2.87 (br d, *J*=9.29 Hz, 1 H) 2.95 - 3.01 (m, 1 H) 6.58 (dd, *J*=10.16, 7.40 Hz, 1 H) 7.70 - 7.78 (m, 2 H) 8.00 - 8.04 (m, 2 H) 8.22 (d, *J*=8.28 Hz, 1 H) 8.37 - 8.43 (m, 2 H) 8.66 (d, *J*=8.28 Hz, 1 H). LCMS: (ESI) m/z = 547.0 (M+1)⁺ |
| 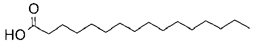 | 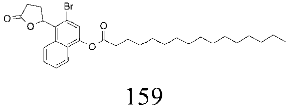 Yield 68%, white solid | ¹H NMR (400 MHz, DMSO-*d*₆) *δ* (ppm) 8.15 (d, *J* = 7.9 Hz, 1H), 7.93-8.01 (m, 1H), 7.62-7.76 (m, 3H), 6.53 (dd, *J =* 7.4, 10.3 Hz, 1H), 2.90-3.03 (m, 1H), 2.74-2.82 (m, 2H), 2.60-2.70 (m, 1H), 1.71 (quin, *J* = 7.4 Hz, 2H), 1.15-1.39 (m, 26H), 0.81-0.87 (m, 3H). LCMS: (ESI) m/z = 568.9 (M+Na)⁺ |
| 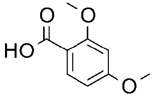 | 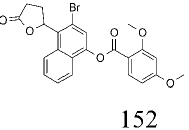 Yield 25.5%, yellow solid | LCMS:(ESI) m/z = 473.0 [M+H]⁺; |
| | | ¹H NMR (400 MHz, DMSO-*d*₆) δ ppm 8.14 - 8.23 (m, 1 H) 8.10 (d, J=8.63 Hz, 1 H) 8.00 - 8.06 (m, 1 H) 7.78 (s, 1 H) 7.66 - 7.75 (m, 2 H) 6.79 (d, J=2.13 Hz, 1 H) 6.75 (dd, J=8.76, 2.25 Hz, 1 H) 6.56 (dd, J=10.32, 7.44 Hz, 1 H) 3.92 (d, J=5.88 Hz, 6 H) 2.92 - 3.05 (m, 1 H) 2.80 - 2.90 (m, 1 H) 2.63 - 2.74 (m, 1 H) 2.53 - 2.61 (m, 1 H) |

According to the method of step 1 in Example 39, the starting materials listed in the following table were reacted to give the corresponding final products.

| Starting material | Final product | LCMS and NMR data |
|---|---|---|
| Compound 76 and cyclopropanecarboxylic acid | 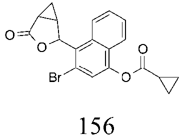 Compound 36%, white solid | LCMS: (ESI) m/z= 387.2 (M+1) ⁺; ¹H NMR (400 MHz, DMSO-d6) Shift 8.33 (d, J=7.82 Hz, 1H), 7.86-8.03 (m, 1H), 7.67-7.84 (m, 3H), 6.40 (s, 1H), 2.63-2.71 (m, 1H), 2.57-2.61 (m, 1H), 2.05-2.19 (m, 1H), 1.54-1.65 (m, 1H), 1.44-1.54 (m, 1H), 1.12-1.25 (m, 4H) |

### Example 40: Synthesis of Compound 161

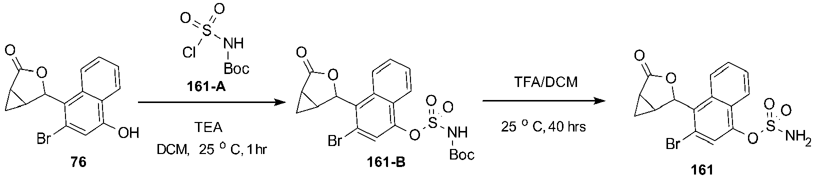

Step 1: According to the procedure of Example 38, compound 76 (60 mg) and compound 161-A (5 *eq*) were used as starting materials to obtain compound 161-B as a yellow solid (100 mg). The crude product was used directly in the next step.

Step 2: According to the procedure of step 2 in Example 39, compound 161-B (100 mg) was reacted to obtain compound 161 as a white solid (25 mg, about 29% yield over two steps). LCMS: (ESI) m/z= 398.1 (M+1) ⁺, ¹H NMR (400 MHz, DMSO-d6) Shift 8.46 (br s, 2H), 8.30-8.36 (m, 1H), 8.24 (dd, J=2.87, 7.03 Hz, 1H), 8.16 (d, J=8.44 Hz, 1H), 7.52-7.83 (m, 4H), 6.41 (s, 0.86H), 6.28 (s, 0.12H), 2.54-2.70 (m, 2H), 1.39-1.68 (m, 2H).

### Example 41: Synthesis of Compound 143

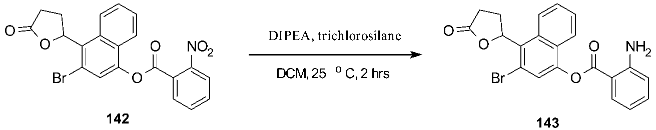

DIEA (84.9 mg, 657 µmol) and trichlorosilane (89.0 mg, 657 µmol) were added to a solution of compound **142** (100 mg, 0.22 mmol) in DCM (2 mL). The mixture was stirred at 25 °C for 2 hours, then quenched with saturated NaHCO-3- solution (10 mL) and extracted with DCM (15 mL×3). After conventional work-up procedure 2, the residue was purified by silica gel column chromatography (EA:PE, EA from 0 to 40%) to obtain compound **143** as a white solid (30 mg, yield 28%). LCMS: (ESI) m/z =427.9 [M+H]⁺; ¹H NMR (400 MHz, DMSO-d6) δ 8.19 (d, J=8.53 Hz, 1H), 8.09-8.15 (m, 1H), 7.93-7.97 (m, 1H), 7.81 (s, 1H), 7.65-7.75 (m, 2H), 7.39-7.45 (m, 1H), 6.90 (d, J=8.53 Hz, 1H), 6.78-6.84 (m, 2H), 6.69 (t, J=7.53 Hz, 1H), 6.57 (dd, J=7.28, 10.29 Hz, 1H), 2.79-3.06 (m, 2H), 2.65-2.74 (m, 1H), 2.57-2.61 (m, 1H).

### Example 42: Synthesis of Compound 149

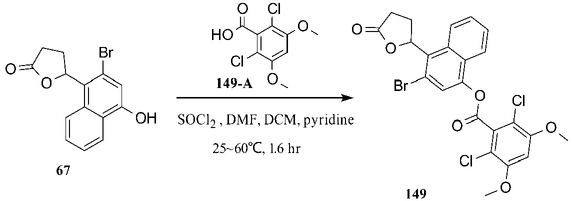

1 drop of DMF was added to a solution of compound 149-A (36.7 mg, 146 µmol) and thionyl chloride (20.9 mg, 176 µmol) in DCM (0.2 mL). The mixture was stirred at 40 °C for 40 minutes, then cooled to 25 °C and the solvent was blown off with nitrogen. A solution of compound 67 (30 mg, 97.68 µmol) in pyridine (2 mL) was added, and the mixture was stirred at 60 °C for 1 hour. EtOAc (4 mL) was added, and after conventional work-up procedure 2, the crude product was purified by silica gel column chromatography (EA/PE, EA from 8% to 30%) to obtain compound 149 as a white solid (25 mg, yield 47%). ¹H NMR (400 MHz, DMSO-*d*₆) *δ* ppm 2.54 - 2.59 (m, 1 H) 2.68 - 2.74 (m, 1 H) 2.83 - 2.89 (m, 1 H) 2.92 - 3.00 (m, 1 H) 4.03 (s, 6 H) 6.57 (dd, J=10.27, 7.46 Hz, 1 H) 7.15 (s, 1 H) 7.68 (s, 1 H) 7.74 - 7.81 (m, 2 H) 8.15 - 8.19 (m, 1 H) 8.20 - 8.26 (m, 1 H). LCMS: (ESI) m/z = 541.0 (M+1)⁺.

### Example 43: Synthesis of Compound 160

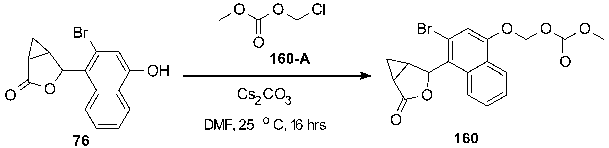

Compound 160-A (38 mg, 306 µmol) was added to a solution of compound 76 (65 mg, 204 µmol) and cesium carbonate (99.5 mg, 305.50 µmol) in DMF (2 mL). The mixture was stirred at 25 °C for 16 hours. After the reaction, saturated brine (10 mL) was added, and the mixture was extracted with EtOAc (5 mL×3). The combined organic phases were dried over anhydrous magnesium sulfate, filtered, and concentrated under reduced pressure. The residue was purified by silica gel column chromatography (EA/PE, EA from 0 to 20%), followed by prep HPLC (FA method; B%: 43% -63%, 16 min) to obtain compound 160 as a white solid (10 mg, yield 12%). (ESI) m/z=407.2 (M+1)⁺. ¹H NMR (400 MHz, CDCl₃), δ 8.22-8.40 (m, 1H), 8.09 (d, *J*=8.58 Hz, 1H), 7.48-7.69 (m, 2H), 7.33 (s, 1H), 6.33 (s, 1H), 5.99 (s, 2H), 3.87 (s, 3H), 2.50-2.67 (m, 1H), 2.42 (dt, *J*=4.62, 6.05 Hz, 1H), 1.62-1.66 (m, 1H), 1.37 (q, *J*=4.03 Hz, 1H).

### Example 44: Synthesis of Compound 163

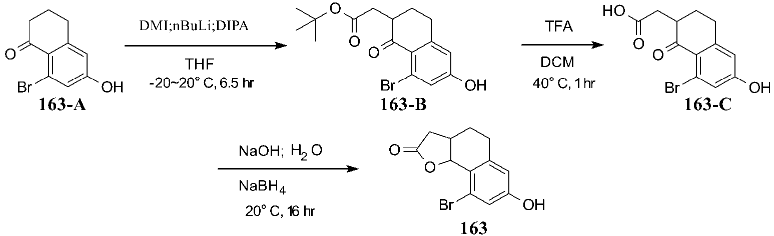

### Step 1:

At -78 °C, n-butyllithium solution in THF (2.5 M, 1.46 mL) was added dropwise to a solution of diisopropylamine (402 mg, 3.98 mmol) in THF (8 mL).The mixture was stirred at 20 °C for 0.5 hours.

At -20 °C, a solution of compound 163-A (400 mg, 1.66 mmol) in THF (2 mL) was added to the above LDA solution, and the mixture was stirred at 0 °C for 1 hour. Then tert-butyl bromoacetate (356 mg, 1.83 mmol) and 1,3-dimethyl-2-imidazolidinone (189 mg, 1.66 mmol) were added, and the mixture was stirred at 0 °C for 5 hours. The reaction was quenched with saturated NH-4-Cl solution (6 mL). After stirring at 20 °C for 0.5 hours, the mixture was extracted with EtOAc (8 mL×3). After conventional work-up procedure 2, the crude product was purified by silica gel column chromatography (EA/PE, EA from 8 to 32%) to obtain compound 163-B as a yellow oil (161 mg, yield 27.32%). ¹H NMR (400 MHz, DMSO-*d*₆) δ ppm 1.36 - 1.40 (m, 9 H) 1.75 - 1.85 (m, 1 H) 2.00 - 2.06 (m, 1 H) 2.34 - 2.43 (m, 1 H) 2.54 - 2.57 (m, 1 H) 2.81 - 2.92 (m, 2 H) 2.96 - 3.04 (m, 1 H) 6.66 - 6.70 (m, 1 H) 6.94 - 7.00 (m, 1 H) 10.63 - 10.70 (m, 1 H).

A solution of compound 163-B (140 mg, 394.12 µmol) in DCM (2 mL) and TFA (224 mg, 1.97 mmol, 150 uL, 5 *eq*) was stirred at 40 °C for 1 hour. The mixture was concentrated under reduced pressure, and the crude product was purified by silica gel column chromatography (EA/PE, EA from 0 to 57%) to obtain compound 163-C as a yellow oil (51 mg, yield 43%). LCMS: (ESI) m/z = 300.9 [M+H]⁺.

Step 3: According to the procedure of step 2 in Example 5, compound 163-C (45 mg, 150.4 µmol) was reacted to obtain compound 163 as a white solid (12 mg, yield 28.2%). ¹H NMR (400 MHz, CDCl₃) δ ppm 1.86 (br dd, *J*=13.27, 3.97 Hz, 1 H) 2.43 (d, *J*=17.36 Hz, 1 H) 2.65 - 2.85 (m, 4 H) 2.97 (dd, *J*=17.18, 7.52 Hz, 1 H) 5.03 - 5.22 (m, 1 H) 5.54 (d, *J*=5.26 Hz, 1 H) 6.61 (d, *J*=2.45 Hz, 1 H) 7.04 (d, *J*=2.45 Hz, 1 H). LCMS: (ESI) m/z = 284.9 (M+1)⁺.

### Example 45: Synthesis of Compounds 164 and 165

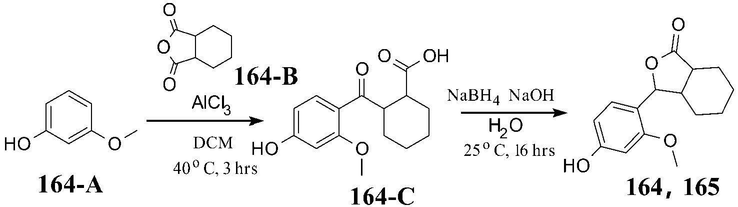

Step 1: Aluminum trichloride (3.24 g, 24.32 mmol) was added to a solution of compound 164-A (2.01 g, 16.22 mmol) in DCM (50 mL). After stirring at 10 °C for 10 minutes, a solution of 164-B (2.5 g, 16.22 mmol) in DCM (5 mL) was added dropwise. The mixture was stirred at 10 °C for 10 minutes, then heated to reflux for 3 hours. The mixture was concentrated under reduced pressure, water (100 mL) was added, and the pH was adjusted to 1-2 with concentrated HCl. The mixture was extracted with DCM (100 mL×3). After conventional work-up procedure, the crude product was purified by silica gel column chromatography (EA/PE, EA from 16 to 53%) to obtain compound 164-C as a yellow solid (180 mg, yield 4%). ¹H NMR (400 MHz, DMSO-d6) 11.95 (br s, 1H), 10.18 (s, 1H), 7.36 (d, J=7.96 Hz, 1H), 6.44 (s, 1H), 6.40 (dd, J=2.26, 8.53 Hz, 1H), 3.82 (s, 3H), 3.67 (br d, J=4.02 Hz, 1H), 2.68 (br s, 1H), 1.62-1.93 (m, 6H), 1.49 (br s, 1H), 1.34 (br d, J=15.06 Hz, 4H).

Step 2: According to the procedure of step 2 in Example 5, compound 164-C (100 mg, 359.3 µmol) was reacted (NaOH at 5 *eq,* NaBH₄ at 40 *eq*) to obtain compound 164 as a white solid (18 mg, yield 19.1%) and compound 165 as a white solid (8 mg, yield 8.5%).

Compound 164: LCMS: (ESI) m/z=263.0 [M+1]⁺; ¹H NMR (400 MHz, DMSO-d6) 9.56 (s, 0.6H), 9.47 (s, 0.3H), 7.02 (d, *J =* 8.0 Hz, 0.3H), 6.93 (d, *J =* 8.3 Hz, 0.6H), 6.45 (d, *J* = 2.0 Hz, 0.6H), 6.42 (d, *J =* 2.0 Hz, 0.3H), 6.36 (dd, *J =* 8.3, 2.0 Hz, 1H), 5.53 (d, *J* = 4.5 Hz, 0.3H), 5.29 (d, *J =* 3.5 Hz, 0.6H), 3.77 (s, 2H), 3.75 (s, 1H), 3.10 (br t, *J =* 5.6 Hz, 0.3H), 2.65-2.72 (m, 1.2H), 2.35-2.43 (m, 0.6H), 1.98 (br d, *J =* 14.1 Hz, 0.4H), 1.77-1.88 (m, 1.3H), 1.42-1.63 (m, 3.2H), 1.16-1.30 (m, 2H), 0.83-1.05 (m, 1.2H), 0.58-0.69 (m, 0.3H).

Compound 165: LCMS: (ESI) m/z=263.0 [M+1]⁺; ¹H NMR (400 MHz, DMSO-d6) 9.65 (s, 1H), 7.15 (d, *J =* 8.5 Hz, 1H), 6.44 (s, 1H), 6.39 (br d, *J =* 8.3 Hz, 1H), 5.33 (d, *J =* 10.3 Hz, 1H), 3.74 (s, 3H), 2.31-2.39 (m, 1H), 1.92-2.04 (m, 2H), 1.66-1.82 (m, 2H), 1.61 (br d, *J* = 12.0 Hz, 1H), 1.14-1.30 (m, 4H).

### Example 46: Synthesis of Compound 166

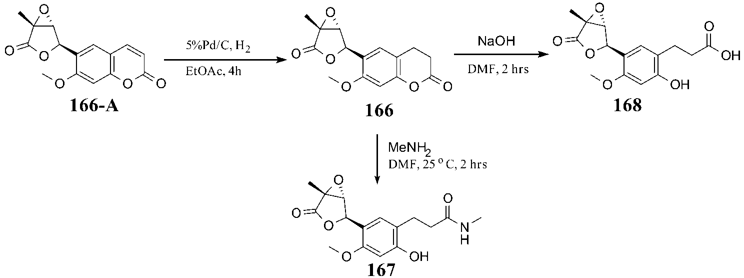

Step 1: According to the representative procedure for catalytic hydrogenation, compound 166-A (35 mg) was reacted to obtain compound 166 as a white solid (28 mg, yield 39%), purified by silica gel column chromatography (EA:PE, EA from 0 to 25%). LCMS: (ESI) m/z= 291.1 [M+1]⁺; ¹H NMR (400 MHz, CDCl₃) 6.99 (s, 1H), 6.58 (s, 1H), 5.40 (s, 1H), 3.92 (s, 1H), 3.77 (s, 3H), 2.84-2.93 (m, 2H), 2.67-2.80 (m, 2H), 1.60 (s, 3H).

Synthesis of compound 168: NaOH (0.5 M, 1.5 *eq*) was added to a solution of compound 166 in DMF (0.5 mL). The mixture was stirred at 25 °C for 2 hours. LCMS showed 20% starting material remained and 35% desired product was formed. The pH was adjusted to 6 with formic acid, and the mixture was directly purified by prep HPLC (FA method-B; B%: 23%-43%, 10 min) to obtain compound 168 as a white solid (yield 54%). LCMS:(ESI) m/z= 309.0 [M+1]⁺; ¹H NMR (400 MHz, DMSO-d6) 9.67-11.99 (m, 1H), 7.06 (br s, 1H), 6.50 (br s, 1H), 5.37 (br s, 1H), 4.29 (br s, 1H), 3.76 (br s, 1H), 3.69 (br s, 3H), 2.61-2.79 (m, 2H), 2.44 (br d, J=7.03 Hz, 2H), 1.49-1.63 (m, 3H).

Synthesis of compound 167: According to the synthetic method of Example 5-a, methylamine (33% in ethanol) was reacted with compound 166 to obtain compound 167 as a white solid in 28.4% yield (reaction time 2 hours, 44% starting material remained). LCMS: (ESI) m/z= 322.0 [M+1] ⁺; ¹H NMR (400 MHz, DMSO-d6) 9.84 (br s, 1H), 7.75 (br s, 1H), 7.02 (s, 1H), 6.49 (s, 1H), 5.37 (s, 1H), 4.29 (s, 1H), 3.70 (s, 3H), 2.63-2.71 (m, 2H), 2.56 (br d, J=4.28 Hz, 3H), 2.24-2.32 (m, 2H), 1.58 (s, 3H).

### Example 47: Synthesis of Compound 169

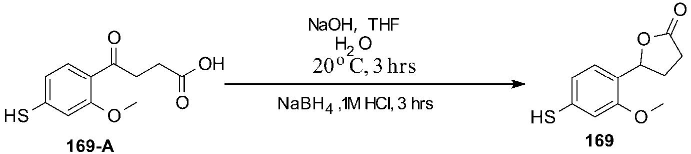

NaOH (49.9 mg, 1.25 mmol) and NaBH₄ (94.5 mg, 2.5 mmol) were added to a mixture of compound 169-A (100 mg, 416 µmol) in THF (2 mL) and water (5 mL). The mixture was stirred at 20 °C for 3 hours. At 10 °C, 1M HCl (5 mL) and EtOAc (5 mL) were added, and the mixture was extracted with EA (5 mL×2). The combined organic phases were blown dry with nitrogen (note: do not concentrate under reduced pressure) to obtain the crude product, which was purified by silica gel column chromatography (EA/PE, EA from 0 to 50%) to obtain compound 169 as a white solid (20 mg, yield 21%). LCMS: (ESI) m/z =224.9 [M+H] ⁺, ¹H NMR (400 MHz, DMSO-d6) δ 7.23 (d, J=7.78 Hz, 1H), 6.88 (d, J=1.51 Hz, 1H), 6.85 (dd, J=1.51, 7.78 Hz, 1H), 5.03 (br s, 1H), 4.79 (dd, J=4.64, 7.15 Hz, 1H), 3.74 (s, 3H), 2.15-2.23 (m, 2H), 1.64-1.85 (m, 2H).

### Example 48: Synthesis of Compounds 171 and 267

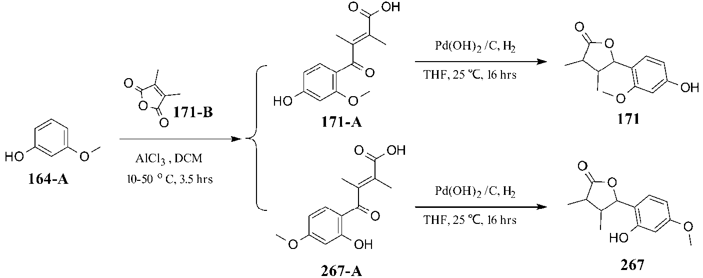

Step 1: According to the procedure of step 1 in Example 45, compound 164-A (9.5 g, 76.53 mmol) was reacted with 171-B (1 *eq)* to obtain compound 171-A as a yellow solid (130 mg, yield 0.68%) and compound 267-A as a yellow oil (14 g, 55.9 mmol, yield 73.1%).

Compound 171-A: ¹H NMR (400 MHz, DMSO*-d₆*) *δ* (ppm) 9.63 (s, 1H), 7.46 (s, 1H), 7.43-7.45 (m, 1H), 6.32-6.39 (m, 2H), 3.5 (s, 3H), 1.75 (s, 3H), 1.58 (s, 3H).

Synthesis of compound 171: Compound 171-A (50 mg, 199 µmol) was dissolved in THF (5 mL), and palladium hydroxide on carbon (20% content, 100 mg) was added. The reaction mixture was thoroughly purged with hydrogen and stirred at 25 °C under 15 psi hydrogen atmosphere for 16 hours. The mixture was filtered, concentrated under reduced pressure to dryness, and purified by silica gel column chromatography (EA/PE, EA from 0 to 35%) to obtain compound 171 as a yellow solid (15 mg, yield 32%). ¹H NMR (400 HMz, DMSO*-d₆*) *δ* (ppm) 9.59 (s, 1H), 6.96-7.01 (m, 1H), 6.35-6.44 (m, 2H), 5.22-5.39 (m, 1H), 3.75 (s, 3H), 2.68-2.85 (m, 1H), 2.49-2.55 (m, 1H), 1.05-1.18 (m, 3H), 0.94-1.03 (m, 2H), 0.36-0.38 (m, 0.5H). LC-MS: (ESI) *m*/*z*. [M+H]⁺ 237.0.

Synthesis of compound 267: According to the synthetic method of compound 171, compound 267-A (100 mg, 399 µmol, 1 *eq*) was reacted to obtain compound 267 as a white solid (30 mg, yield 30.5%). ¹H NMR (400HMz, DMSO-*d₆*) *δ* (ppm) 9.72-9.92 (m, 1H), 6.97-7.13 (m, 1H), 6.42 (dt, *J =* 2.2, 4.7 Hz, 2H), 5.20-5.69 (m, 1H), 3.70 (s, 3H), 2.82-3.20 (m, 1H), 2.56-2.80 (m, 1H), 1.07 (dd, *J* = 7.4, 15.7 Hz, 3H), 0.36-0.98 (m, 3H). LC-MS: (ESI) *m*/*z*. [M+H]⁺ 237.0.

### Example 50: Synthesis of Compound 174

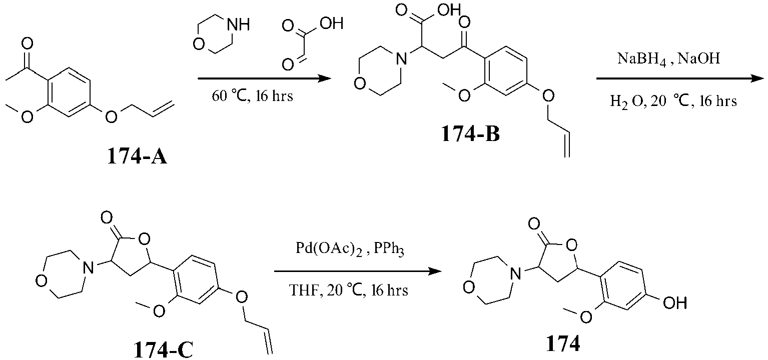

Step 1: A mixture of compound 174-A (100 mg, 485 µmol), morpholine (123 mg, 1.45 mmol, 3 *eq*), and glyoxylic acid (179 mg, 1.21 mmol, 135 µL, 2.5 *eq*) was stirred at 60 °C for 16 hours. After cooling to room temperature, the mixture was diluted with ACN (1 mL) and directly purified by prep HPLC (FA method, B: 15%-35% over 38 min) to obtain compound 174-B as a brown solid (38 mg, yield 22.4%). ¹H NMR (400 MHz, CDCl₃) *δ* (ppm) 7.87 (d, *J* = 8.6 Hz, 1H), 6.58 (dd, *J* = 2.4, 8.8 Hz, 1H), 6.48 (d, *J* = 2.2 Hz, 1H), 6.03-6.20 (m, 1H), 5.47 (dd, *J =* 1.3, 17.4 Hz, 1H), 5.38 (dd, *J =* 0.9, 10.6 Hz, 1H), 4.66 (d, *J* = 5.7 Hz, 2H), 4.21 (t, *J* = 6.2 Hz, 1H), 3.88 (s, 3H), 3.80-3.85 (m, 4H), 3.65-3.74 (m, 1H), 3.47-3.57 (m, 1H), 2.81-2.88 (m, 4H).

Step 2: According to the procedure of step 2 in Example 5 (representative procedure for constructing the five-membered lactone ring), compound 174-B (310 mg, 887 µmol, 1 *eq*) was reacted to obtain compound 174-C as a yellow oil (77 mg, 230 µmol, yield 26%). LC-MS: (ESI) *m*/*z*. [M+H]⁺ 334.1.

Step 3: According to the procedure of step 3 in Example 2, compound 174-C (77 mg, 230 µmol) was reacted to obtain compound 174 as a white solid (15 mg, 51 µmol, yield 22.1%). ¹H NMR (400 MHz, DMSO-*d₆*) *δ* (ppm) 9.68-10.17 (m, 1H), 7.05-7.22 (m, 1H), 6.42 (dd, *J =* 2.1, 4.5 Hz, 2H), 5.61 (dd, *J =* 5.0, 8.5 Hz, 0.3H), 5.51 (dd, *J =* 5.7, 10.8 Hz, 0.7H), 3.91 (dd, *J =* 8.4, 12.1 Hz, 0.7H), 3.69-3.76 (s, 3H), 3.67 (dd, *J =* 2.3, 4.7 Hz, 0.3H), 3.59 (t, *J* = 4.5 Hz, 4H), 2.76-2.91 (m, 2H), 2.42-2.50 (m, 3H), 2.18-2.39 (m, 1H). LC-MS: (ESI) *m*/*z*. [M+H]⁺ 294.1.

### Example 51: Synthesis of Compounds 271 and 272

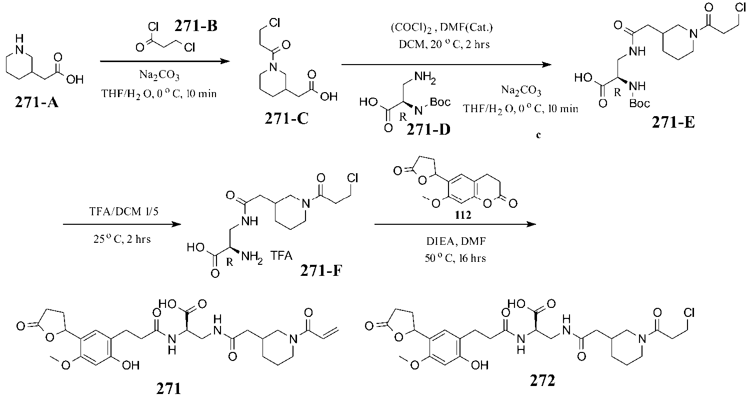

Step 1: 271-B (500 mg, 3.94 mmol) was added to a mixture of compound 271-A (500 mg, 3.49 mmol) and Na₂CO₃ (740 mg, 6.98 mmol) in THF/water (10 mL, 1:1) at 0 °C. The mixture was stirred at 0 °C for 10 minutes. After removing the solvent under reduced pressure, the crude product was purified by Prep HPLC (column: C18 150×40mm; mobile phase: [water (FA)-ACN]; gradient: 1%-41% B over 9 min) to obtain compound 271-C as a colorless oil (520 mg, yield 64%). LCMS: (ESI) m/z=234.0 (M+1)⁺.

Step 2: Preparation of acyl chloride: Thionyl chloride (349 mg, 2.01 *eq*) and DMF (1.00 mg, 0.01 *eq*) were added to a solution of compound 271-C (320 mg, 1.37 mmol) in DCM (5 mL). The mixture was stirred at 25 °C for 2 hours, then concentrated under reduced pressure and dissolved in THF (2 mL) for later use.

Na₂CO₃ (218 mg, 2.06 mmol) was added to a mixture of compound 271-D (140 mg, 687 µmol) in THF/water (10 mL, 1:1), and the mixture was cooled to 0 °C. The above acyl chloride solution was added, and the mixture was stirred at 0 °C for 10 minutes. After concentration under reduced pressure, the crude product was directly purified by prep HPLC (column: Boston Prime C18 150*30mm*5um; mobile phase: [Water (NH₃H₂O-NH₄HCO₃)-MeCN]; gradient: 10%-30% B over 10 min) to obtain compound 271-E as a yellow solid (110 mg, yield 38.29%). LC-MS: (ESI) *m*/*z*. [M+H]⁺ 420.3.

Step 3: According to the procedure of step 2 in Example 39 (representative Boc deprotection procedure), compound 271-E (110 mg) was reacted to obtain compound 271-F as a white solid (80 mg, 184 µmol, yield 70.40%, TFA salt). LC-MS: (ESI) *m*/*z*. [M+H]⁺ 320.0.

Step 4: DIEA (104 mg, 806 µmol, 5 *eq*) and compound 112 (42.3 mg, 161 µmol, 1.0 *eq*) were added to a solution of compound 271-F (70 mg, 161.36 µmol, 1 *eq,* TFA) in DMF (1 mL). The mixture was stirred at 50 °C for 16 hours. The reaction mixture was directly purified by prep HPLC (FA method, B: 18%-38% over 11 min) to obtain compound 271 as a white solid (15 mg, 26.4 µmol, yield 16.36%) and compound 272 as a white solid (45 mg, 74.2 µmol, yield 46.00%).

Compound 271: ¹H NMR (400MHz, DMSO-*d₆*) *δ* (ppm) 9.45-9.78 (m, 1H), 7.74-8.05 (m, 2H), 6.99 (d, *J =* 3.9 Hz, 1H), 6.77 (br dd, *J =* 10.7, 16.3 Hz, 1H), 6.48 (s, 1H), 6.06 (dd, *J =* 2.3, 16.8 Hz, 1H), 5.48-5.73 (m, 2H), 3.78-4.50 (m, 4H), 3.72 (s, 3H), 2.78-3.13 (m, 3H), 2.63-2.75 (m, 3H), 2.40-2.46 (m, 1H), 2.31-2.39 (m, 2H), 1.89-2.24 (m, 4H), 1.42-1.89 (m, 4H), 1.04-1.38 (m, 2H). LC-MS: (ESI) *m*/*z*. [M+H]⁺ 546.3.

Compound 272: ¹H NMR (400MHz, DMSO-*d*₆) *δ* (ppm) 9.35-9.86 (m, 1H), 7.97-8.11 (m, 1H), 7.80-7.96 (m, 1H), 6.99 (d, *J* = 4.0 Hz, 1H), 6.49 (s, 1H), 5.59 (t, *J =* 7.5 Hz, 1H), 4.72-4.81 (m, 1H), 4.00-4.39 (m, 2H), 3.64-3.86 (m, 6H), 2.61-3.06 (m, 8H), 2.29-2.48 (m, 4H), 1.88-2.28 (m, 4H), 1.42-1.87 (m, 4H), 1.08-1.41 (m, 2H). LC-MS: (ESI) *m*/*z*. [M+H]⁺ 582.5.

According to the synthetic method of compound 271, the starting materials listed in the following table were subjected to four-step reactions to give the corresponding final products.

| Starting material | Final product | LCMS and NMR data |
|---|---|---|
| 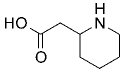 | 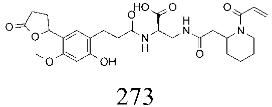 Yield 0.2% over four steps, white solid | ¹HNMR (400MHz, DMSO-*d*₆) *δ* (ppm) 9.12-9.84 (m, 1H), 7.72-8.15 (m, 2H), 6.99 (d, *J =* 3.2 Hz, 1H), 6.64-6.86 (m, 1H), 6.48 (s, 1H), 6.01 (dd, *J =* 2.0, 16.8 Hz, 1H), 5.34-5.72 (m, 2H), 3.78-4.61 (m, 4H), 3.72 (s, 3H), 2.57-2.73 (m, 6H), 2.30-2.48 (m, 5H), 2.01-2.16 (m, 1H), 1.26-1.70 (m, 6H) LC-MS:(ESI) *m*/*z* [M+H]⁺ 546.3 |

According to the procedures of steps 3 and 4 in the synthetic method of compound 271, the starting materials listed in the following table were subjected to two-step reactions to give the corresponding final products.

| Starting material | Final product | LCMS and NMR data |
|---|---|---|
| 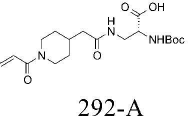 | 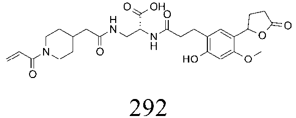 Yield 0.46% over two steps, white solid | ¹HNMR (400MHz, DMSO-*d*₆) *δ* 9.63 (s, 1H), 8.04-7.66 (m, 2H), 7.00 (d, *J* = 4.0 Hz, 1H), 6.80-6.73 (m, 1H), 6.49 (s, 1H), 6.06 (dd, *J =* 16.6, 2.4 Hz, 1H), 5.64-5.58 (m, 2H), 4.35 (d, *J =* 12.4 Hz, 1H), 4.26-4.14 (m, 1H), 3.98 (d, *J* = 13.2 Hz, 1H), 3.72 (s, 3H), 3.00 (t, *J* = 12.8 Hz, 1H), 2.69-2.59 (m, 5H), 2.44-2.40 (m, 1H), 2.36-2.31 (m, 2H), 2.15-2.06 (m, 1H), 2.03-1.96 (m, 1H), 1.94-1.87 (m, 1H), 1.70-1.60 (m, 2H), 1.23-1.13 (m, 2H), 1.02-0.90 (m, 3H).LC-MS:(ESI) *m*/*z* [M+H]⁺ 546.4 |

**The synthesis of 292-A listed in the above table is as follows:**

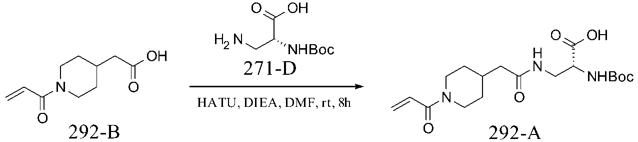

According to the procedure of step 2 in Example 5-b, 292-B (500 mg, 2.54 mmol) was reacted with 271-D (517.7 mg, 2.54 mmol) to obtain compound 292-A as a white solid (70 mg, yield 7.2%). LC-MS (ESI) m/z: 384.4 (M+H)⁺.

### Example 52: Synthesis of Compound 274

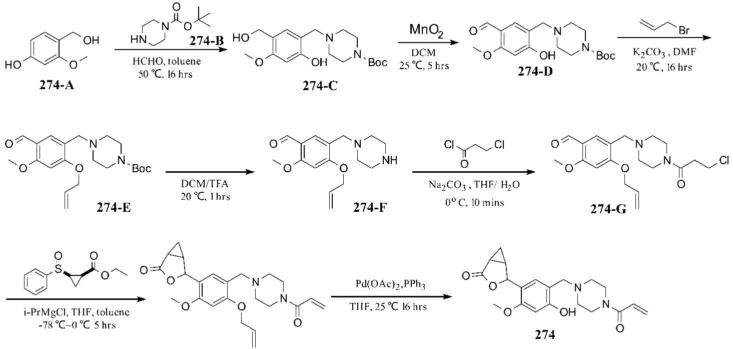

Step 1: 274-B (6.04 g, 32.4 mmol) and paraformaldehyde (1.95 g, 64.8 mmol, 2 *eq*) were added to a solution of compound 274-A (5 g, 32.4 mmol) in toluene (50 mL). The mixture was stirred at 50 °C for 16 hours, then concentrated under reduced pressure. The crude product was purified by silica gel column chromatography (EA:PE, EA from 0 to 30%) to obtain compound 274-C as a white solid (9 g, yield 79%). LC-MS: (ESI) *m*/*z* [M+H]⁺ 353.2.

Step 2: MnO₂ (16 g, 184 mmol, 10 eq) was added to a solution of compound 274-C (6.5 g, 18.4 mmol) in DCM (60 mL). The mixture was stirred at 25 °C for 5 hours, then filtered and concentrated under reduced pressure to obtain compound 274-D as a white solid (2 g), which was used directly in the next step. LC-MS: (ESI) m/z [M+H]⁺ 351.1.

Step 3: According to the procedure of step 1 in Example 2, compound 274-D (2 g) was reacted to obtain compound 274-E as a yellow oil (2 g, about 28% yield over two steps). LC-MS: (ESI) *m*/*z* [M+H]⁺ 391.2.

Step 4: According to the procedure of step 2 in Example 39 (representative Boc deprotection procedure), compound 274-E (2.5 g) was reacted to obtain compound 274-F as a yellow oil (1.8 g), which was used directly in the next step. LC-MS: (ESI) *m*/*z* [M+H]⁺ 291.1.

Step 5: According to the procedure of step 1 in Example 51, 274-E (500 mg, 1.24 mmol, TFA salt) was reacted to obtain compound 274-G as a yellow oil (230 mg, about 34% yield over two steps). LC-MS: (ESI) *m*/*z* [M+H]⁺ 381.1.

Steps 6 and 7: According to the procedures of steps 2 and 3 in Example 2, compound 274-G (143 mg, 603 µmol) was subjected to two-step reactions to obtain compound 274 as a white solid (1 mg, about 1.4% yield over two steps). ¹H NMR (400 MHz, DMSO-*d₆*) *δ* (ppm) 6.89 (s, 1H), 6.73-6.85 (m, 1H), 6.46-6.53 (m, 1H), 6.05-6.15 (m, 1H), 5.73-5.78 (m, 1H), 5.63-5.71 (m, 1H), 5.36-5.43 (m, 1H), 3.73-3.80 (m, 3H), 3.48-3.61 (m, 6H), 2.41 (br s, 4H), 2.12-2.26 (m, 2H), 1.29 (td, *J* = 4.4, 8.3 Hz, 1H), 0.93-1.12 (m, 1H), 0.63-0.70 (m, 1H). LC-MS: (ESI) *m*/*z* [M+H]⁺ 373.3.

### Example 53: Synthesis of Compound 276

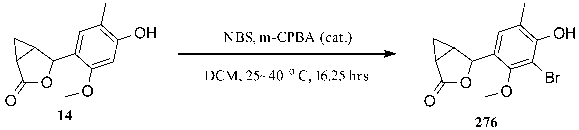

m-CPBA (4.33 mg, 21.3 µmol, 85% purity, 0.05 *eq*) was added to a solution of compound 14 (100 mg, 427 µmol) in DCM (5 mL). After stirring at 25 °C for 15 minutes, NBS (76 mg, 427 µmol) was added, and the mixture was stirred at 40 °C for 16 hours. The mixture was concentrated under reduced pressure, and the residue was purified by silica gel column chromatography (EA:PE, EA from 0 to 6%) to obtain compound 276 as a white solid (100 mg, yield 75%). ¹H NMR (400 MHz, DMSO-*d₆*) *δ* (ppm) 9.13-9.45 (m, 1H), 6.94-7.12 (m, 1H), 5.84 (d, *J* = 4.8 Hz, 0.22H), 5.47 (s, 0.75H), 3.69-3.85 (m, 3H), 2.56-2.63 (m, 0.27H), 2.26-2.37 (m, 1.61H), 2.17-2.21 (m, 3H), 1.07-1.37 (m, 1H), 0.78-1.06 (m, 1H). LC-MS: (ESI) *m*/*z*. [M+H]⁺ 313.0.

### Example 54: Synthesis of Compound 277

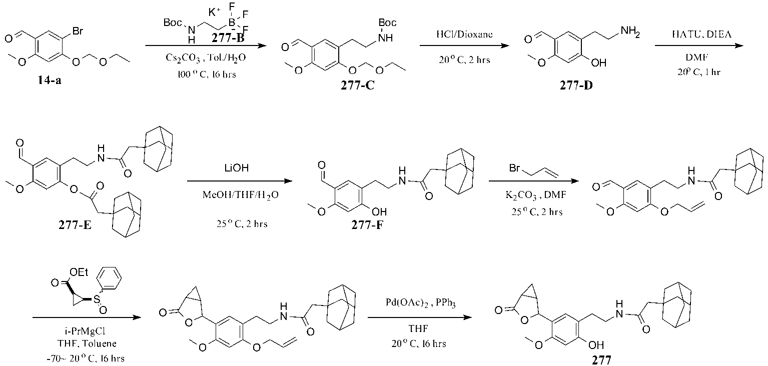

Step 1: Cesium carbonate (2.7 g, 8.3 mmol) was added to a mixture of compound 14-a (800 mg, 2.77 mmol) and 277-B (900 mg, 3.58 mmol) in toluene (15 mL) and H₂O (5 mL). After thorough purging with nitrogen, RuPhos Pd G3 catalyst (CAS: 1445085-77-7, 231 mg, 276 µmol) was added, and the mixture was stirred at 100 °C under a nitrogen atmosphere for 16 hours. After cooling to room temperature, the mixture was extracted with EA (10 mL×3). After conventional work-up procedure, the residue was purified by silica gel column chromatography (EA:PE, EA from 0 to 20%) to obtain compound 277-C as a white solid (960 mg, yield 98%). LC-MS: Rt = 0.983 min, (ESI) *m*/*z* [M+H]⁺ 354.2.

Step 2: A solution of compound 277-C (870 mg, 2.46 mmol) in HCl in dioxane (2M, 10 mL) was stirred at 20 °C for 2 hours. The mixture was concentrated under reduced pressure to obtain compound 277-D as a green solid (570 mg, HCl salt), which was used directly in the next step. LC-MS: (ESI) *m*/*z* [M+H]⁺ 196.1.

Step 3: According to the procedure of step 2 in Example 5-b, 277-D (570 mg, HCl salt) was reacted with 1-adamantylacetic acid (956 mg, 2 *eq*) to obtain compound 277-E as a white solid (1.0 g, about 74% yield over two steps). LC-MS: (ESI) *m*/*z* [M+H]⁺ 548.4.

Step 4: According to the procedure of Example 34, compound 277-E (1.0 g) was reacted. After the reaction, the mixture was concentrated under reduced pressure, and the pH was adjusted to 1 with 1M HCl. The precipitated solid was collected by filtration to obtain compound 277-F as a white solid (700 mg), which was used directly in the next step. LC-MS: (ESI) *m*/*z* [M+H]⁺ 372.2.

Steps 5 to 7: According to the procedure of Example 2, compound 277-F (645 mg) was used as starting material in a three-step reaction to obtain compound 277 as a white solid (17 mg, 12.6% yield over four steps). LC-MS: (ESI) *m*/*z* [M+H]⁺ 440.2. ¹H NMR (400 MHz, DMSO-*d₆*) *δ* (ppm) 9.48-9.88 (m, 1H), 7.60-7.79 (m, 1H), 6.78-6.98 (m, 1H), 6.45-6.56 (m, 1H), 5.72 (d, *J =* 4.6 Hz, 0.2H), 5.37 (s, 0.8H), 3.64-3.81 (m, 3H), 3.10-3.23 (m, 2H), 2.58 (br t, *J* = 7.4 Hz, 2H), 2.21 (m, 1.8H), 1.89 (br s, 3H), 1.73-1.83 (m, 2H), 1.44-1.71 (m, 13H), 1.24-1.35 (m, 1H), 0.92-1.09 (m, 1H), 0.71-0.78 (m, 0.2H).

The synthesis of compound 14-a is as follows:

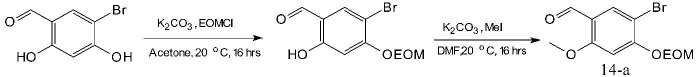

Step 1: Chloromethyl ethyl ether (1.96 g, 20.74 mmol) and K₂CO₃ (8.60 g, 62.21 mmol) were added to a solution of 5-bromo-2,4-dihydroxybenzaldehyde (4.5 g, 20.74 mmol) in acetone (100 mL). The mixture was stirred at 20 °C for 16 hours. Saturated aqueous NH-4-Cl solution (50 mL) was added, and the mixture was extracted with EA (50 mL×3). After conventional work-up procedure 2, the crude product was purified by silica gel column chromatography (EA:PE, EA from 0 to 9%) to obtain 5-bromo-4-[(ethoxymethyl)oxy]-2-hydroxybenzaldehyde as a white solid (3.22 g, yield 56%).

Step 2: Iodomethane (2.23 g, 15.70 mmol) and K₂CO₃ (3.62 g, 26.17 mmol) were added to a mixture of 5-bromo-4-[(ethoxymethyl)oxy]-2-hydroxybenzaldehyde (3.6 g, 13.09 mmol) in DMF (2 mL). The mixture was stirred at 20 °C for 16 hours. Water (50 mL) was added, and the mixture was extracted with EA (50 mL×2). After conventional work-up procedure 2, the crude product was purified by preparative TLC (EA:PE, EA from 6 to 9%) to obtain compound 14-a as a white solid (3.58 g, yield 94%).

### Example 55: Synthesis of Compound 129

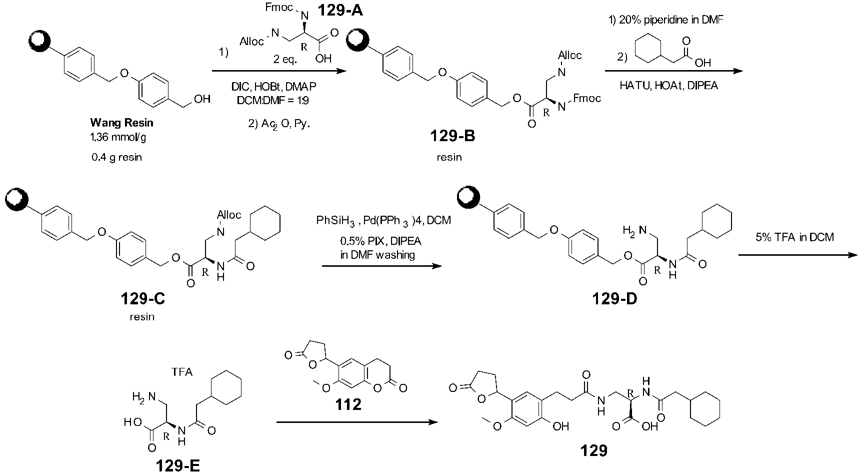

Step 1: Wang resin (400 mg, 0.544 mmol) was placed in a 40 mL reaction vessel and suspended in 10 mL of DCM/DMF (1:9). In a separate vial, compound 129-A (440 mg, 1.07 mmol) and HOBt (146 mg, 1.08 mmol) were dissolved in DMF (1 mL), and the solution was added to the resin. DMAP (8 mg, 65.5 umol) was added to the reaction vessel, followed by DIC (145 mg, 1.15 mmol), and the mixture was shaken at 25 °C for 6 hours. Acetic anhydride (120 mg, 1.17 mmol) and pyridine (108 mg, 1.36 mmol) were added to the reaction vessel, and the mixture was shaken at 25 °C for an additional 30 minutes to cap any unreacted hydroxyl groups on the resin. The resin was filtered, washed with 20 mL of DMF twice and 20 mL of DCM five times to obtain wet resin 129-B for the next step.

Step 2: Resin 129-B (0.544 mmol) was placed in a solid-phase synthesis reactor and 15 mL of DMF was added. After swelling with stirring for 30 seconds, the solvent was removed by vacuum filtration. 15 mL of 20% piperidine/DMF was added, and the mixture was shaken for 15 minutes, then the solvent was removed by vacuum filtration. This was repeated with another 15 mL of 20% piperidine/DMF for 15 minutes, followed by washing with 15 mL of DMF four times. 2-Cyclohexylacetic acid (315 mg, 2.22 mmol), HATU (833 mg, 2.19 mmol), and HOAt (301 mg, 2.21 mmol) were dissolved in DMF (15 mL), and the solution was added to the resin with stirring for 30 seconds. DIPEA (643 mg, 4.98 mmol) was added. The reaction mixture was stirred at 25 °C for 2 hours, then the solvent was removed by vacuum filtration and the resin was washed with 15 mL of DMF four times. The resulting resin 129-C was washed with 15 mL of DCM four times and collected in a reaction vessel for the next step.

Some resin beads were transferred to a small glass tube, and 1 mL of 5% TFA in DCM was added and left for 3 hours. The solvent was removed, diluted with ACN/water (1:1, 1 mL), filtered, and monitored by LCMS to confirm the target compound. LC-MS: (ESI) *m*/*z*. [M+H]⁺313.1.

Step 3: Resin 129-C (544 µmol) was placed in a reaction vessel, and DCM (10 mL) and phenylsilane (482 mg, 4.46 mmol) were added. The mixture was gently stirred for 1 minute and allowed to swell for 15 minutes. The reaction suspension was degassed under vacuum and purged with nitrogen five times. Pd(PPh₃)₄ (150 mg, 130 µmol) was added to the suspension, and after degassing and purging with nitrogen five times, the suspension was shaken at 25 °C under a nitrogen atmosphere for 1.5 hours. The resin was filtered and washed with 15 mL of DMF five times. A solution of 0.5% PIX (potassium isopropylxanthate) and 0.5% DIEA in DMF (10 mL) was added, gently stirred for 5 minutes, and the solvent was removed by vacuum filtration. This was repeated twice, followed by washing with 15 mL of DMF five times. The resin was then washed with 15 mL of DCM five times to obtain resin 129-D as an off-white solid for the next step.
Step 4: Resin 129-D (544 µmol) was placed in a reaction vessel, and 5% TFA/DCM solution (15 mL) was added. The mixture was shaken at 25 °C for 4 hours. The suspension was filtered, and the resin was washed with 15 mL of DCM three times. The combined filtrates were concentrated under reduced pressure. The crude product was dissolved in 5 mL of deionized water and filtered to remove insoluble materials. This was repeated three times, and the combined filtrates were lyophilized to obtain compound 129-E as a colorless syrup (156 mg, yield 83.77%, TFA salt), which was used directly in the next step. LC-MS: (ESI) *m*/*z*. [M+H]⁺ 229.1;
Step 5: TFA (51 mg, 503 µmol) was added to a solution of 129-E (62 mg, 181 µmol, 1.2 eq, TFA salt) in DMF (1.5 mL), followed by compound 112 (40 mg, 153 µmol, 1.0 *eq*)*.* The reaction mixture was stirred at 55 °C for 12 hours, then concentrated under reduced pressure and purified by prep HPLC (FA method-A; B%: 25%-45%, 10 min) to obtain compound 129 as an off-white solid (32 mg, 41.5% yield). LC-MS: (ESI) *m*/*z*. [M+H]+491.4; ¹H NMR (400 MHz, CDCl₃) δ (ppm) 7.36-7.55 (m, 2H) , 6.93 (s, 1H) , 6.46 (s, 1H), 5.61-5.69 (m, 1H), 4.42(br s, 1H), 3.74(s, 3H) , 3.43-3.70(m, 2H), 2.66-2.90 (m, 2H), 2.38-2.66 (m, 5H), 1.97-2.22 (m, 3H), 1.53-1.76 (m, 6H), 0.99-1.28 (m, 3H), 0.82-0.99 (m, 2H).

According to the method of **Example 55,** the starting materials listed in the following table were used in place of compound 129-A in the first step and subjected to five-step reactions to give the corresponding final products.

| Starting material | Final product | LCMS and NMR data |
|---|---|---|
| 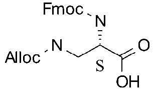 | 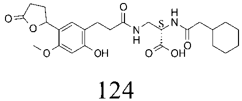 Off-white solid | LC-MS: (ESI) *m*/*z.* [M+H]⁺ 491.4; |
| | | ¹H NMR (400 MHz, CDCl₃) *δ* (ppm) 7.35-7.43 (m, 1H), 7.29-7.35 (m, 1H), 6.94 (s, 1H), 6.46 (s, 1H), 5.66 (br t, *J* = 6.02 Hz, 1H), 4.42 (br s, 1H), 3.75 (s, 3H), 3.62-3.75 (m, 1H), 3.39-3.62 (m, 1H), 2.67-2.90 (m, 2H), 2.49-2.67 (m, 5H), 2.11-2.25 (m, 1H), 2.09 (d, *J* = 6.53 Hz, 2H), 1.55-1.78 (m, 6H), 1.02-1.34 (m, 3H), 0.82-1.00 (m, 2H) |

### Example 56: Synthesis of Compound 125

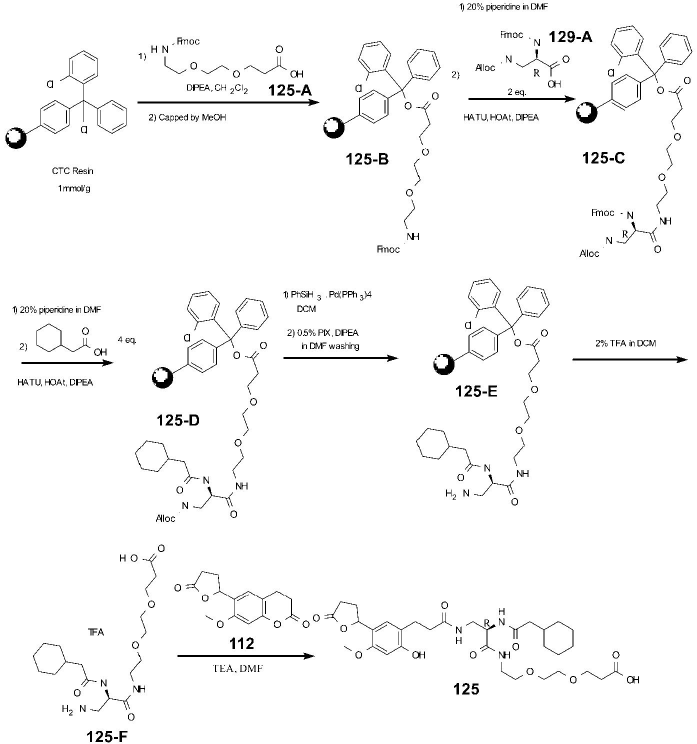

Step 1: Compound 125-A (780 mg, 1.95 mmol) was dissolved in DCM (15 mL), and DIPEA (964.57 mg, 7.46 mmol) was added to the solution. 2-Chlorotrityl chloride resin (CTC resin) (1.3 g, 1.0 mmol/g, 1.30 mmol) was added to the solution, and the mixture was shaken at 25 °C for 2 hours. Methanol (2 mL) was added to the solution, and the mixture was shaken at 25 °C for 30 minutes. The mixture was filtered, and the resin was washed with 20 mL of DCM five times and dried under vacuum to obtain resin 125-B as a yellow solid (1.78 g).

Step 2: Resin 125-B (1.54 g, 1.0 mmol) was placed in a solid-phase synthesis reactor, and 20 mL of DCM was added. After swelling for 15 minutes, the solvent was removed by vacuum filtration, and the resin was washed once with 20 mL of DMF. 20 mL of 20% piperidine/DMF was added, and the mixture was shaken for 30 minutes, then the solvent was removed by vacuum filtration. This was repeated with another 20 mL of 20% piperidine/DMF for 30 minutes, followed by washing with 20 mL of DMF five times. Compound 129-A (827 mg, 2.02 mmol), HATU (768 mg, 2.02 mmol), and HOAt (275 mg, 2.02 mmol) were dissolved in DMF (10 mL), and the solution was added to the resin with stirring for 30 seconds. DIPEA (890 mg, 6.89 mmol) was added. The reaction mixture was stirred at 25 °C for 2 hours, then the solvent was removed by vacuum filtration and the resin was washed with 15 mL of DMF five times. The resulting resin 125-C was washed with 15 mL of DCM five times for the next step.

Step 3: Resin 125-C (0.75 g, -0.5 mmol) was placed in a solid-phase synthesis reactor, and 15 mL of DCM was added. After swelling for 15 minutes, the solvent was removed by vacuum filtration, and the resin was washed once with 20 mL of DMF. 10 mL of 20% piperidine/DMF was added, and the mixture was shaken for 15 minutes, then the solvent was removed by vacuum filtration. This was repeated with another 20 mL of 20% piperidine/DMF for 15 minutes, followed by washing with 15 mL of DMF five times. 2-Cyclohexylacetic acid (285 mg, 2.00 mmol), HATU (760 mg, 2.00 mmol), and HOAt (273 mg, 2.01 mmol) were dissolved in DMF (10 mL), and the solution was added to the resin with stirring for 30 seconds. DIPEA (649 mg, 5.02 mmol) was added. The reaction mixture was stirred at 25 °C for 2 hours, then the solvent was removed by vacuum filtration and the resin was washed with 15 mL of DMF five times. The resulting resin 125-D was washed with 15 mL of DCM five times and collected in a reaction vessel for the next step.

Step 4: According to the procedure of step 3 in Example 55, resin 125-D (0.5 mmol, *1.0 eq)* was reacted to obtain yellow resin 125-E for the next step.

Some resin beads were transferred to a small glass tube, and 1 mL of 2% TFA in DCM was added and left for 15 minutes. The solvent was removed, diluted with acetonitrile/water (1:1, 1 mL), filtered, and monitored by LCMS to confirm the target compound. LC-MS: (ESI) *m*/*z*. [M+H]⁺ 388.2;
Step 5: According to the procedure of step 4 in Example 55, resin 125-E (0.5 mmol, *1.0 eq)* was reacted to obtain compound 125-F as an off-white solid (232 mg, 92.52% yield, TFA salt). LC-MS: (ESI) *m*/*z* [M+H]⁺ 388.3;
Step 6: According to the procedure of step 5 in Example 55 (reaction temperature at 25 °C), 125-F (71 mg, 183 µmol, TFA salt) was reacted to obtain compound 125 as a white solid (72 mg, 69.07% yield). LC-MS: (ESI) *m*/*z*. [M+H]⁺650.6; ¹H NMR (400 MHz, CDCl₃) *δ* (ppm) 7.03-7.21 (m, 3H), 6.89-7.01 (m, 1H), 6.47 (s, 1H), 5.62-5.69 (m, 1H), 4.59-4.74 (m, 1H), 3.77 (s, 3H), 3.70-3.86 (m, 2H), 3.41-3.67 (m, 9H), 3.23-3.39 (m, 1H), 2.73-2.94 (m, 2H), 2.48-2.70 (m, 7H), 1.98-2.22 (m, 3H), 1.55-1.83 (m, 6H), 1.05-1.33 (m, 3H), 0.77-1.02 (m, 2H).

According to the method of **Example 56,** the starting materials listed in the following table were used in place of compound 129-A in the second step and reacted with 125-B via five-step reactions to give the corresponding final products.

| Starting material | Final product | LCMS and NMR data |
|---|---|---|
| 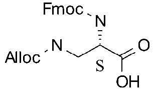 | 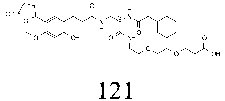 Off-white solid | LC-MS: (ESI) *m*/*z*. [M+H]+ 650.5; |
| | | ¹H NMR (400 MHz, CDCl₃) *δ* (ppm) 7.05-7.20 (m, 3H), 6.96 (s, 1H), 6.47 (s, 1H), 5.62-5.69 (m, 1H), 4.61-4.74 (m, 1H), 3.77 (s, 3H), 3.69-3.84 (m, 2H), 3.39-3.68 (m, 9H), 3.23-3.39 (m, 1H), 2.71-2.91 (m, 2H), 2.44-2.70 (m, 7H), 1.97-2.21 (m, 3H), 1.59-1.78 (m, 6H), 1.06-1.30 (m, 3H), 0.80-1.01 (m, 2H) |

### Example 57: Synthesis of Compound 128

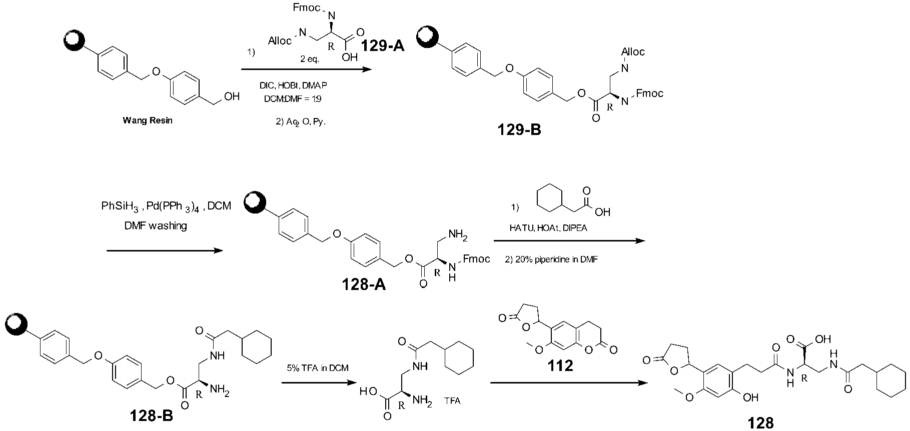

Step 1: Resin 129-B (0.544 mmol) was placed in a reaction vessel, and DCM (10 mL) and phenylsilane (482 mg, 4.46 mmol) were added. The mixture was gently stirred for 1 minute and allowed to swell for 15 minutes. The reaction suspension was degassed under vacuum and purged with nitrogen five times. Pd(PPh₃)₄ (140 mg, 121 umol) was added to the suspension, and after degassing and purging with nitrogen five times, the suspension was shaken at 25 °C under a nitrogen atmosphere for 1.5 hours. The resin was filtered and washed with 15 mL of DCM five times, then with 15 mL of DMF three times to obtain yellow resin 128-A for the next step.

Step 2: Resin 128-A (0.544 mmol) was placed in a solid-phase synthesis reactor and washed once with 20 mL of DMF. 2-Cyclohexylacetic acid (315 mg, 2.22 mmol), HATU (834 mg, 2.19 mmol), and HOAt (302 mg, 2.22 mmol) were dissolved in DMF (10 mL), and the solution was added to the resin with stirring for 30 seconds. DIPEA (649 mg, 5.02 mmol) was added. The reaction mixture was stirred at 25 °C for 2 hours, then the solvent was removed by vacuum filtration and the resin was washed with 15 mL of DMF twice. A solution of 0.5% PIX and 0.5% DIPEA in DMF (10 mL) was added, stirred for 5 minutes, and the solvent was removed by vacuum filtration. This was repeated twice, followed by washing with 15 mL of DMF three times. 15 mL of 20% piperidine/DMF was added, and the mixture was shaken for 30 minutes, then the solvent was removed by vacuum filtration. This was repeated with another 15 mL of 20% piperidine/DMF for 30 minutes, followed by washing with 15 mL of DMF four times.

Steps 3 and 4: According to the procedures of steps 4 and 5 in Example 55, resin 128-B was subjected to two-step reactions to obtain compound 128 as a white solid in 36% yield over two steps. LCMS: (ESI) m/z=491.5 (M+1)⁺; ¹H NMR (400 MHz, CDCl₃) δ 7.51-7.68 (m, 1H), 6.88-7.11 (m, 1H), 6.96 (s, 1H), 6.46 (s, 1H), 5.61-5.72 (m, 1H), 4.50 (br s, 1H), 3.74 (s, 3H), 3.42-3.68 (m, 2H), 2.69-2.96 (m, 2H), 2.41-2.69 (m, 5H), 2.07-2.24 (m, 1H), 1.92-2.07 (m, 2H), 1.53-1.76 (m, 6H), 1.00-1.28 (m, 3H), 0.77-0.97(m, 2H).

According to the method of **Example 57,** the starting materials listed in the following table were used in place of compound 129-A in the first step and subjected to five-step reactions to give the corresponding final products.

| Starting material | Final product | LCMS and NMR data |
|---|---|---|
| 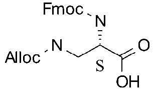 | 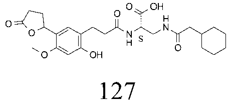 Off-white solid | LC-MS: (ESI) *m*/*z.* [M+H]⁺ 491.3; |
| | | ¹H NMR (400 MHz, CDCl₃)*δ* (ppm)7.55-7.72 (m, 1H), 7.04-7.21 (m, 1H), 6.95 (s, 1H), 6.46 (s, 1H), 5.59-5.73 (m, 1H), 4.52 (br s, 1H), 4.33-4.62 (m, 1H), 3.73 (s, 3H), 3.30-3.67 (m, 2H), 2.71-2.90 (m, 2H), 2.40-2.71 (m, 5H), 2.07-2.23 (m, 1H), 1.86-2.07 (m, 2H), 1.49-1.74 (m, 6H), 0.98-1.30 (m, 3H), 0.75-0.95 (m, 2H) |

### Example 58: Synthesis of Compound 126

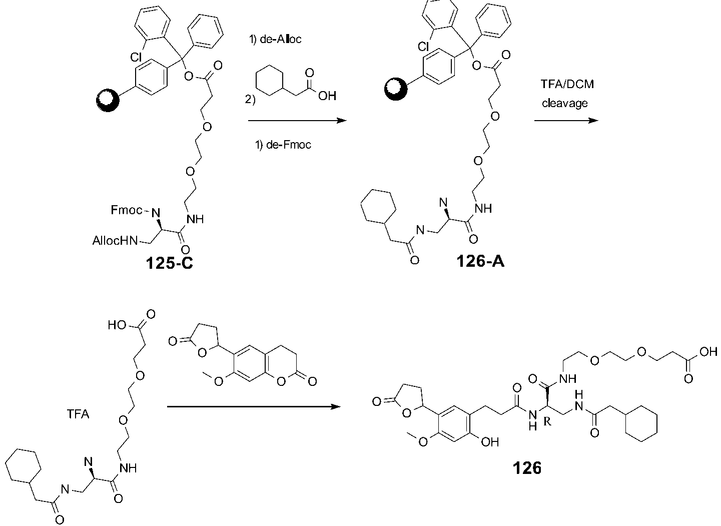

Step 1: Resin 125-C (0.75 g, 0.5 mmol) was placed in a reaction vessel, and DCM (15 mL) and phenylsilane (438 mg, 4.05 mmol) were added. The mixture was gently stirred for 1 minute and allowed to swell for 15 minutes. The reaction suspension was degassed under vacuum and purged with nitrogen five times. Pd(PPh-3-)-4- (150 mg, 130 umol) was added to the suspension, and after degassing and purging with nitrogen five times, the suspension was shaken at 25 °C under a nitrogen atmosphere for 1.5 hours. The resin was filtered and washed with 15 mL of DMF five times. The resulting resin was placed in a solid-phase synthesis reactor. 2-Cyclohexylacetic acid (285 mg, 2.00 mmol), HATU (761 mg, 2.00 mmol), and HOAt (273 mg, 2.01 mmol) were dissolved in DMF (15 mL), and the solution was added to the resin with stirring for 30 seconds. DIPEA (742 mg, 5.74 mmol) was added. The reaction mixture was stirred at 25 °C for 2 hours, then the solvent was removed by vacuum filtration and the resin was washed with 15 mL of DMF four times. 15 mL of 20% piperidine/DMF was added, and the mixture was shaken for 15 minutes, then the solvent was removed by vacuum filtration. This was repeated with another 15 mL of 20% piperidine/DMF for 15 minutes, followed by washing with 15 mL of DMF four times.

Steps 2 and 3: According to the procedures of steps 4 and 5 in Example 55 (reaction temperature in step 5 at 25 °C), resin 126-A was subjected to two-step reactions to obtain compound 126 as a white solid in 31% yield over two steps.

LC-MS: (ESI) *m*/*z* [M+H]⁺650.6; ¹H NMR (400 MHz, CDCl₃)*δ* (ppm)7.64-7.78 (m, 1H), 7.13-7.27 (m, 1H), 6.97 (s, 1H), 6.70-6.88 (m, 1H), 6.47 (s, 1H), 5.57-5.79 (m, 1H), 4.60-4.72 (m, 1H), 3.76 (s, 3H), 3.70-3.84 (m, 2H), 3.43-3.67 (m, 10H), 3.23-3.37 (m, 1H), 3.23-3.37 (m, 1H), 2.47-2.93 (m, 9H), 2.09-2.22 (m, 1H), 1.90-2.09 (m, 2H), 1.55-1.79 (m, 6H), 1.04-1.31 (m, 3H), 0.78-0.99 (m, 2H).

### Example 59: Synthesis of Compound 122

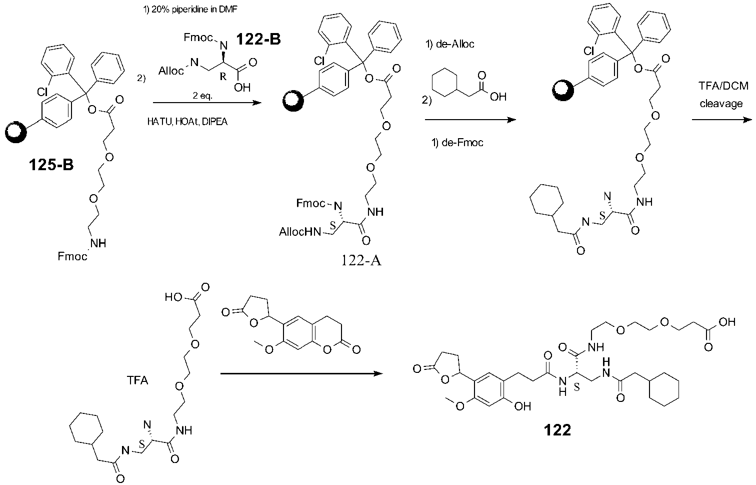

Step 1: According to the procedure of step 2 in Example 56, 122-B was used in place of compound 129-A to react with 125-B to obtain 122-A. Then, According to the procedure of Example 58, 122-A was used as starting material in a three-step reaction to obtain compound 122 as a white solid. LC-MS: (ESI) *m*/*z*. [M+H]⁺ 650.4; ¹H NMR (400 MHz, CDCl₃)*δ* (ppm)7.57-7.79 (m, 1H), 7.10-7.27 (m, 1H), 6.97 (s, 1H), 6.60-6.78 (m, 1H), 6.47 (s, 1H), 5.55-5.75 (m, 1H), 4.62-4.73 (m, 1H), 3.77 (s, 3H), 3.69-3.87 (m, 2H), 3.38-3.69 (m, 9H), 3.19-3.37 (m, 1H), 2.72-2.97 (m, 2H), 2.35-2.72 (m, 7H), 2.08-2.22 (m, 1H), 1.88-2.08 (m, 2H), 1.47-1.80 (m, 6H), 1.04-1.33 (m, 3H), 0.80-0.98 (m, 2H).

### Example 60: Synthesis of Compound 286

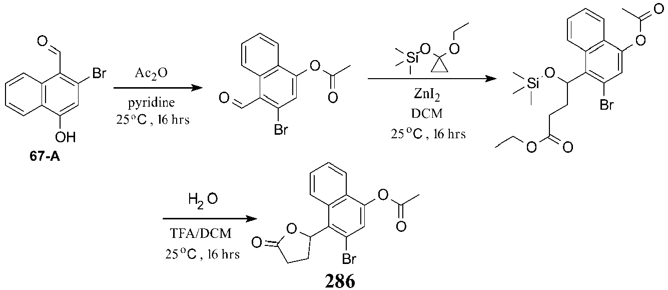

Step 1: Acetic anhydride (2.03 g, 19.91 mmol) was added to a solution of compound 67-A (500 mg, 1.99 mmol) in pyridine (10 mL). The mixture was stirred at 25 °C for 16 hours, then concentrated under reduced pressure. Water (10 mL) was added, and the mixture was extracted with EA (5 mL×3). After conventional work-up procedure, the crude product was purified by silica gel column chromatography (EA:PE, EA from 0 to 50%) to obtain a yellow solid (560 mg, yield 95.94%). ¹H NMR (400 MHz, DMSO-d6) δ ppm 10.60 (s, 1 H) 8.97 (d, J=8.53 Hz, 1 H) 8.10 (d, J=8.03 Hz, 1 H) 7.86 (s, 1 H) 7.79 - 7.84 (m, 1 H) 7.71 - 7.76 (m, 1 H) 2.49 - 2.50 (m, 3 H).

Steps 2 and 3: According to the procedures of steps 2 and 3 in Example 12, product (510 mg, 1.74 mmol) of the above step was used as starting material in a two-step reaction to obtain compound 286 as a yellow solid in 73.6% yield over two steps. LCMS: m/z = 349.0 [M+H]⁺; ¹H NMR (400 MHz, CDCl₃)*δ* ppm 8.03 - 8.12 (m, 1 H) 7.90 - 7.98 (m, 1 H) 7.56 - 7.63 (m, 2 H) 7.52 (s, 1 H) 6.51 (dd, J=10.57, 7.19 Hz, 1 H) 2.87 - 2.94 (m, 2 H) 2.67 - 2.77 (m, 1 H) 2.59 - 2.67 (m, 1 H) 2.47 - 2.51 (m, 3 H).

### Example 61: Chiral Resolution of Compound 172

Compound 172 (30 mg) was resolved by SFC (column: DAICEL CHIRALPAK AD (250mm*30mm,10 µm); mobile phase: [CO₂-EtOH(0.1%NH₃H₂O)];B%:15%, isocratic elution mode) to obtain compounds 293, 294, 295, and 296.

**Compound 293:** (3 mg), white solid, SFC: Rt = 1.343 min. **Compound 294:** (10 mg), white solid, SFC: Rt = 1.497 min. **Compound 295:** (8 mg), white solid, SFC: Rt = 1.703 min. **Compound 296:** (3 mg), white solid, SFC: Rt = 1.775 min. SFC analytical conditions: Column: Chiralpak AD-3 100×4.6mm I.D., 3 µm; Mobile phase: A: CO₂, B: ethanol (0.05% DEA); Gradient: from 5% to 40% of B over 3 min, hold 40% B for 0.5 min, then 5% B for 1.5 min; Flow rate: 2.8 mL/min; Column temperature: 35 °C; ABPR: 1500 psi.

### Biological Activity Test Example 1

HTRF (Homogeneous Time-Resolved Fluorescence) technology was used to evaluate the inhibitory effects of the compounds on the binding of TDG to double-stranded DNA substrates.

### Reagents and consumables

| Reagent | Supplier | Cat. No. |
|---|---|---|
| His-tagged TDG protein | In-house prepared | |
| Biotin-labeled dsDNA substrate, sequence (Forward: TCGGATGTTGTGGGTCAGUGCATGATAGTGTA-Biotin, Reverse: TACACTATCATGCGCTGACCCA CAACATCCGA) | Synthesized by Genewiz | |
| MAb Anti-6HIS-Eu cryptate Gold | Cisbio | 61HI2KLA |
| Streptavidin-XL665 | Cisbio | 610SAXLA |
| Epigeneous Binding Domain diluent buffer | Cisbio | 62DLBDDF |
| Epigeneous Binding Domain Detection buffer | Cisbio | 62DB1FDG |

### Instruments

Microplate reader (manufacturer: Perkin Elmer; model: Envision multilabel reader equipped with HTRF module) and Echo 550 (manufacturer: Labcyte; model: Echo 550).

### Experimental method

1. Preparation of compound concentration gradient: Test compounds were prepared at a starting concentration of 250 µM, with 3-fold serial dilution, 10 concentrations, and duplicate wells. In a 384-well plate, the compounds were gradient-diluted to 40× final concentration solutions at 10 different concentrations. Then 500 nl was transferred to a 384-well reaction plate using Echo 550 for later use. To the negative control wells and positive control wells, 500 nl of 100% DMSO or Biotin-unlabeled dsDNA substrate was added, respectively.
2. Add 1.5 µl/well of Epigeneous Binding Domain diluent buffer to the 384-well plate, and centrifuge at 1000 rpm for 1 min.
3. Add 4 µl of 5× final concentration of His-tagged TDG protein (final concentration 40 nM, diluted with Epigeneous Binding Domain diluent buffer) to the 384-well plate, centrifuge at 1000 rpm for 1 min, and incubate at room temperature for 10 min.
4. Add 4 µl of 5× final concentration of Biotin-labeled dsDNA substrate (final concentration 40 nM, diluted with Epigeneous Binding Domain diluent buffer) to the 384-well plate, centrifuge at 1000 rpm for 1 min, and incubate at room temperature for 30 min.
5. Add 5 µl of 4× final concentration of Streptavidin-XL665 (final concentration 5 nM, diluted with Epigeneous Binding Domain Detection buffer) to the 384-well plate, and centrifuge at 1000 rpm for 1 min.
6. Add 5 µl of 4× final concentration of MAb Anti-6HIS-Eu cryptate Gold (final concentration 0.1 µg/ml, diluted with Epigeneous Binding Domain Detection buffer) to the 384-well plate, and centrifuge at 1000 rpm for 1 min.

Incubate overnight at 4 °C, and read HTRF on the microplate reader.

### Data analysis

1. Calculation of Ratio value for each well
   Ratio = Signal 665 nm / Signal 620 nm × 104.
2. Calculation of delta Ratio value for each well
   delta Ratio = Ratio standard or sample - Ratio standard 0 (negative control).
3. IC₅₀ values were fitted using a four-parameter logistic (4PL) curve.

The biological test results are recorded in the table below. The results show that the exemplary compounds of the present invention exhibit strong inhibitory effects on the binding of TDG to double-stranded DNA substrates.
A: ${IC}_{50} \leq 500 nM ;$
B: $500 nM < {IC}_{50} \leq 5 μM ;$
C: $5 μM < {IC}_{50} \leq 50 μM ;$
D: > 50 µM.

Using the test method mentioned above, the following compound activities were obtained:

| 000 | Activity | 000 | Activity | 000 | Activity |
|---|---|---|---|---|---|
| 1 | B | 2 | C | 3 | A |
| 5 | D | 6 | C | 7 | C |
| 8 | B | 9 | A | 11 | D |
| 12 | C | 13 | B | 14 | B |
| 15 | C | 16 | C | 17 | C |
| 18 | C | 19 | B | 20 | B |
| 21 | A | 22 | A | 23 | B |
| 24 | A | 25 | B | 28 | A |
| 29 | B | 30 | B | 31 | A |
| 32 | B | 33 | B | 34 | B |
| 35 | C | 36 | C | 37 | D |
| 38 | C | 39 | A | 40 | B |
| 41 | B | 42 | A | 43 | B |
| 44 | C | 45 | B | 46 | C |
| 47 | B | 48 | B | 49 | B |
| 50 | B | 51 | B | 52 | A |
| 53 | A | 54 | C | 55 | B |
| 56 | B | 57 | B | 58 | A |
| 59 | C | 60 | A | 61 | B |
| 62 | B | 63 | B | 64 | D |
| 65 | B | 67 | A | 68 | B |
| 69 | A | 70 | B | 71 | B |
| 72 | B | 74 | C | 75 | D |
| 76 | A | 77 | A | 78 | A |
| 79 | A | 80 | A | 81 | A |
| 82 | B | 83 | B | 84 | B |
| 85 | A | 86 | B | 87 | B |
| 88 | B | 89 | B | 90 | A |
| 91 | A | 93 | D | 94 | D |
| 95 | A | 96 | B | 97 | B |
| 98 | B | 99 | A | 100 | A |
| 101 | C | 102 | B | 103 | B |
| 104 | B | 105 | B | 106 | B |
| 107 | B | 108 | B | 109 | B |
| 110 | B | 111 | B | 112 | C |
| 113 | B | 114 | B | 115 | B |
| 116 | B | 117 | A | 118 | B |
| 119 | A | 120 | B | 121 | B |
| 122 | A | 123 | B | 124 | B |
| 125 | B | 126 | A | 127 | A |
| 128 | B | 129 | B | 130 | C |
| 131 | B | 132 | C | 133 | B |
| 134 | C | 135 | D | 136 | C |
| | | 138 | B | 139 | B |
| 140 | A | 141 | B | 142 | B |
| 143 | A | 144 | A | 145 | A |
| 146 | D | 147 | A | 148 | B |
| 149 | D | 150 | B | 151 | C |
| 152 | D | 153 | A | 154 | A |
| 155 | A | 156 | A | 157 | A |
| 158 | A | 159 | C | 160 | C |
| 161 | A | 162 | A | 163 | C |
| 164 | D | 165 | C | 166 | A |
| 167 | B | 168 | A | 169 | A |
| 171 | C | 172 | C | 179 | B |
| 180 | A | 181 | B | 267 | C |
| 271 | B | 272 | B | 273 | B |
| 274 | B | 283 | B | 284 | B |
| 285 | A | 287 | A | | |

### Biological Activity Test Example 2: Inhibition of TP53 Mutant and Wild-Type Tumor Cell Proliferation by Compound 14

IC₅₀ values of compound 14 for inhibiting the proliferation of *TP53* mutant and wild-type tumor cells. The *TP53* mutant and wild-type tumor cells used in this example are all human tumor cell.

Tumor cells were plated in 384-well plates and treated with DMSO or specified concentrations of compound 14 (0.04-30 µM). After 6 days of incubation, chemiluminescence was measured using the CellTiter-Glo luminescent cell viability assay kit (Promega). Curves were fitted using GraphPad Prism software, and IC₅₀ values were calculated.

The results show that compound 14 exhibits certain inhibitory effects on various tumor cell lines including lung cancer, liver cancer, skin cancer, bladder cancer, breast cancer, colon cancer, and esophageal cancer, particularly against p53 mutant tumor cell lines (including NCI-H446, Calu-1, NCI-H1299, RERF-LC-AI, NCI-H211, NCI-H2009, NCI-H23, VMRC-LCP, NCI-H441, PC9, NCI-H520, EBC-1, NCI-H1975, NCI-H2342, NCI-H647, HCC95, SK-LU-1, SW-900, SNU423, SNU449, HuCCT1, PLC/PRF/5, SNU387, Hep3B, JHH-2, JHH7, SNU761, Huh7, CHL-1, RPMI-7951, SK-MEL-2, SK-MEL-28, MeWo, SK-MEL-3, 5637, UMUC3, RT112, HT1376, BT549, MDA-MB-468, MDA-MB-231, HS578T, EFM-19, MDA-MB-436, Colo320DM, Colo205, RKO_E6, NCI-H508, TE-15, TE-8, TE-1, TE-9, TE-10, TE-4, TE-6, TE-14, T.T) in vitro proliferation (Figure 1), indicating that the compounds of the present invention, including compound 14, possess excellent anti-tumor activity, particularly against p53 mutant tumor cells.

All documents mentioned in the present invention are incorporated by reference in this application as if each such document were individually incorporated by reference. Furthermore, it should be understood that after reading the above teachings of the present invention, those skilled in the art may make various changes or modifications to the present invention, and these equivalent forms also fall within the scope defined by the appended claims of this application.

## Claims

1. A compound of Formula I, or a deuterated derivative, stereoisomer, tautomer, or pharmaceutically acceptable salt thereof: 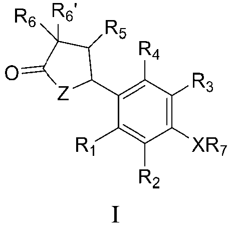
R₁ is selected from the group consisting of: OH, H, halogen, cyano, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted C₆-C₁₀ aryl, substituted or unsubstituted 3-12 membered heterocycle, substituted or unsubstituted 5-12 membered heteroaromatic ring, substituted or unsubstituted -O-3-12 membered heterocycle, substituted or unsubstituted -C₁-C₆ alkylphenyl, substituted or unsubstituted -O-phenyl, substituted or unsubstituted C₁-C₄ alkyl-C(O)-, substituted or unsubstituted C₁-C₄ alkyl-S(O)₂-, substituted or unsubstituted C₁-C₆ alkyl-NH-, (substituted or unsubstituted C₁-C₆ alkyl)₂-N-, -O(CH₂)ₛR₁₀, or -S(CH₂)ₛR₁₀; s is 0, 1, 2, or 3;
R₁₀ is selected from the group consisting of H, substituted or unsubstituted C₃₋₈ carbocycle, substituted or unsubstituted 3-8 membered heterocycle, substituted or unsubstituted C₆-C₁₀ aryl, and substituted or unsubstituted 5-12 membered heteroaromatic ring;
each R₂ is independently selected from the group consisting of H, halogen, and substituted or unsubstituted C₁-C₆ alkyl;
R₃ is selected from the group consisting of H, halogen, cyano, substituted or unsubstituted C₁-C₆ alkyl, substituted or unsubstituted C₁-C₆ alkoxy, substituted or unsubstituted C₃-C₁₂ cycloalkyl, or (C₁-C₆ alkyl)C(O)R₈, (C₁-C₆ alkyl)C(O)NHR₈, (C₁-C₆ alkyl)C(O)N(substituted or unsubstituted C₁-C₆ alkyl)R₈, (C₁-C₆ alkyl)NHC(O)R₈, and (C₁-C₆ alkyl)C(O)OR₈;
said R₈ is selected from the group consisting of H, substituted or unsubstituted C₁-C₆ alkyl, substituted or unsubstituted C₆-C₁₀ aryl, -(OCH₂CH₂)ₘ-substituted or unsubstituted C₁-C₆ alkyl, adamantyl, and substituted or unsubstituted groups selected from the group consisting of -(CH₂)ₘNHC(O)(CH₂)ₙR₁₃, -(CH₂)CHR₉NHC(O)(CH₂)ₙR₁₃, and CHR₉(CH₂)NHC(O)(CH₂)ₙR₁₃;
said R₉ is selected from the group consisting of: H, -COOH, -CONHR₁₂, - CONHCH₂R₁₂, -CONH(CH₂CH₂O)ₘ(CH₂)ₙCOOH, substituted or unsubstituted C₁-C₆ alkoxy, substituted or unsubstituted C₆-C₁₀ aryl, substituted or unsubstituted C₃-C₁₀ carbocycle, substituted or unsubstituted 3-12 membered heterocycle, and substituted or unsubstituted 5-12 membered heteroaromatic ring;
R₁₂ is selected from the group consisting of substituted or unsubstituted C₁-C₆ alkyl, substituted or unsubstituted C₃-C₈ cycloalkyl, substituted or unsubstituted C₆-C₁₀ aryl, and substituted or unsubstituted 5-12 membered heteroaromatic ring;
said R₁₃ is selected from the group consisting of substituted or unsubstituted C₃-C₁₀ carbocycle, and substituted or unsubstituted 5-12 membered heterocycle;
m and n are each independently 0, 1, 2 or 3;
R₄ is selected from the group consisting of H, halogen, cyano, substituted or unsubstituted C₁-C₆ alkoxy, substituted or unsubstituted C₁-C₆ amino, substituted or unsubstituted C₆-C₁₀ aryl, substituted or unsubstituted C₃-C₈ cycloalkyl, substituted or unsubstituted 3-12 membered heterocycle, substituted or unsubstituted 5-12 membered heteroaromatic ring, and substituted or unsubstituted -O-5-12 membered heteroaromatic ring;
or, R₃ and R₄ together with the carbon atoms to which they are attached form a structure selected from the group consisting of substituted or unsubstituted C6-C10 aromatic ring, a substituted or unsubstituted 5-10 membered heteroaromatic ring, a substituted or unsubstituted C3-C8 carbocycle, and a substituted or unsubstituted 3-10 membered heterocycle;
R₅ and R₆ are each independently selected from the group consisting of H, substituted or unsubstituted C₁-C₆ alkyl, substituted or unsubstituted C6-C10 aromatic ring, substituted or unsubstituted 5-10 membered heteroaromatic ring, substituted or unsubstituted C3-C8 carbocycle, and substituted or unsubstituted 3-10 membered heterocycle; or said R₅ and R₆ together with the carbon atom to which they are attached to form a substituted or unsubstituted 3-12 membered carbocycle;
R₆' is H, or substituted or unsubstituted C₁-C₆ alkyl;
X is O or S;
Z is O, S or NR₁₄; wherein R₁₄ is H or C₁-C₄ alkyl;
R₇ is selected from the group consisting of H, C(O)R₁₁, C(O)OR₁₁, -CH₂OC(O)OR₁₁, and -S(O)₂NHR₁₁;
said R₁₁ is selected from the group consisting of H, substituted or unsubstituted C₁-C₁₆ alkyl, substituted or unsubstituted C₆-C₁₀ aryl, -(OCH₂CH₂)ₘ-substituted or unsubstituted C₁-C₆ alkyl, and substituted or unsubstituted 5-8 membered heterocyclyl;
unless otherwise specified, in the above formulas, said heteroaromatic ring, heteroaryl, heterocycle, or heterocyclyl group contains 1, 2 or 3 heteroatoms selected from N, S or O; said aromatic ring, aryl, heteroaromatic ring, or heteroaryl can be monocyclic or fused ring; said carbocycle, cycloalkyl, heterocycle, or heterocyclyl group can be monocyclic, fused, bridged, or spiro; said carbocycle, heterocycle, or heterocyclyl can be saturated or partially unsaturated, but is not aromatic;
said "substituted" means that a hydrogen atom on the corresponding group is replaced by one or more substituents selected from the group consisting of deuterium, halogen, hydroxy, carboxy, mercapto, benzyl, C₂-C₁₂ alkoxycarbonyl, C₁-C₆ aldehyde, (C₁-C₆ alkyl)₃Si, amino, C₁-C₆ amido, nitro, cyano, unsubstituted or halogenated C₁-C₆ alkyl, C₂-C₁₀ alkenyl, C₁-C₆ alkoxy, C₃-C₆ cycloalkyl, adamantyl, C₁-C₆ alkyl-amino, C₁-C₁₂ alkylaminocarbonyl, unsubstituted or halogenated C₂-C₁₀ acyl, unsubstituted or halogenated C₁-C₄ alkyl-S(O)₂-, unsubstituted or C₁-C₄ alkyl-substituted C₁-C₄ alkyl-OC(O)NH-, unsubstituted or halogenated C₁-C₄ alkyl-SO-, unsubstituted or C₁-C₄ alkyl-substituted 5-7 membered heterocycle, and phenyl (which may have 1-5 substituents selected from halogen, C₁-C₄ alkyl, and C₁-C₄ alkoxy).

2. The compound according to claim 1, or a deuterated derivative, stereoisomer, tautomer, or pharmaceutically acceptable salt thereof, wherein said R₁ is selected from the group consisting of halogen, substituted or unsubstituted C₁-C₆ alkoxy, substituted or unsubstituted C₆-C₁₀ aryl, substituted or unsubstituted 3-12 membered heterocycle, substituted or unsubstituted 5-12 membered heteroaromatic ring, substituted or unsubstituted -O-3-12 membered heterocycle, substituted or unsubstituted C₁-C₄ alkylS(O)₂-, substituted or unsubstituted C₁-C₆ alkyl-NH-, (substituted or unsubstituted C₁-C₆ alkyl)₂-N-, and -O(CH₂)ₛR₁₀.

3. The compound according to claim 1, or a deuterated derivative, stereoisomer, tautomer, or pharmaceutically acceptable salt thereof, wherein the compound of Formula I has the structure of Formula II below: 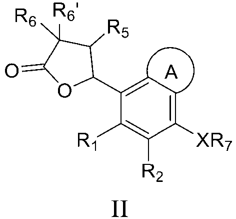 wherein ring A is a substituted or unsubstituted C6-C10 aromatic ring, a substituted or unsubstituted 5-10 membered heteroaromatic ring, a substituted or unsubstituted C3-C8 carbocycle, or a substituted or unsubstituted 3-10 membered heterocycle; preferably, ring A is a substituted or unsubstituted benzene ring, or a substituted or unsubstituted 5-7 membered heteroaromatic ring.

4. The compound according to claim 1, or a deuterated derivative, stereoisomer, tautomer, or pharmaceutically acceptable salt thereof, wherein the compound has the structure of Formula IV below: 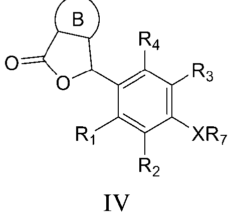 wherein ring B is a substituted or unsubstituted C3-C8 carbocycle, or a substituted or unsubstituted 3-10 membered heterocycle; preferably, ring B is a substituted or unsubstituted C3-C6 carbocycle, or a substituted or unsubstituted 3-8 membered heterocycle; wherein said carbocycle or heterocycle may be saturated or partially unsaturated.

5. The compound according to claim 1, or a deuterated derivative, stereoisomer, tautomer, or pharmaceutically acceptable salt thereof, wherein the compound has the structure of Formula V below: 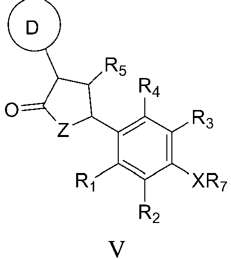 wherein ring D is a substituted or unsubstituted C6-C10 aromatic ring, a substituted or unsubstituted 5-10 membered heteroaromatic ring, a substituted or unsubstituted C3-C8 carbocycle, or a substituted or unsubstituted 3-10 membered heterocycle.

6. The compound according to claim 1, or a deuterated derivative, stereoisomer, tautomer, or pharmaceutically acceptable salt thereof, wherein the compound has the structure of Formula VI or VII below: 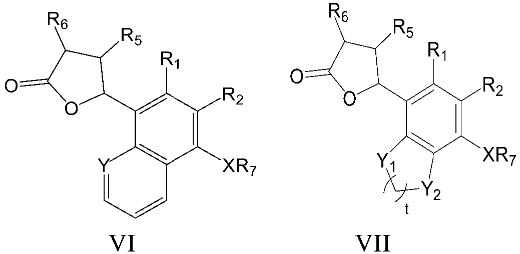 wherein Y is N or CH; Y₁ and Y₂ are each independently selected from the group consisting of CHR₁₅, NR₁₅, O and S; t is 1 or 2; wherein said R₁₅ is selected from the group consisting of deuterium, halogen, hydroxy, carboxy, mercapto, amino, nitro, cyano, unsubstituted or halogenated C₁-C₆ alkyl, and C₁-C₆ alkoxy.

7. The compound according to claim 1, or a deuterated derivative, stereoisomer, tautomer, or pharmaceutically acceptable salt thereof, wherein R₃ is selected from the group consisting of H, halogen, substituted or unsubstituted C₁-C₆ alkyl, substituted or unsubstituted C₃-C₈ cycloalkyl, and substituted or unsubstituted phenyl.

8. The compound according to claim 1, or a deuterated derivative, stereoisomer, tautomer, or pharmaceutically acceptable salt thereof, wherein R₅ and R₆ are each independently H or substituted or unsubstituted C₁-C₆ alkyl; or said R₅ and R₆ together with the carbon atom to which they are attached form a substituted or unsubstituted 3-6 membered carbocycle.

9. The compound according to claim 1, or a deuterated derivative, stereoisomer, tautomer, or pharmaceutically acceptable salt thereof, wherein R₃ is selected from the group consisting of H, halogen, cyano, substituted or unsubstituted C₁-C₆ alkyl, substituted or unsubstituted C₃-C₁₂ cycloalkyl, (C₁-C₆ alkyl)C(O)R₈, (C₁-C₆ alkyl)C(O)NHR₈, (C₁-C₆ alkyl)C(O)N(substituted or unsubstituted C₁-C₆ alkyl)R₈, (C₁-C₆ alkyl)C(O)OR₈; said R₈ is selected from the group consisting of H, substituted or unsubstituted C₁-C₆ alkyl, substituted or unsubstituted C₆-C₁₀ aryl, -(OCH₂CH₂)ₘ-substituted or unsubstituted C₁-C₆ alkyl, and a substituted or unsubstituted group selected from the group consisting of: - (CH₂)ₘNHC(O)(CH₂)ₙR₁₃, -(CH₂)CHR₉NHC(O)(CH₂)ₙR₁₃, CHR₉(CH₂)NHC(O)(CH₂)ₙR₁₃; said R₉ is selected from the group consisting of: H, -COOH, -CONHR₁₂, -CONHCH₂R₁₂, - CONH(CH₂CH₂O)ₘ(CH₂)ₙCOOH, substituted or unsubstituted C₁-C₆ alkoxy, substituted or unsubstituted C₆-C₁₀ aryl, substituted or unsubstituted C₃-C₁₀ carbocycle, substituted or unsubstituted 3-12 membered heterocycle, substituted or unsubstituted 5-12 membered heteroaromatic ring; R₁₂ is selected from the group consisting of: substituted or unsubstituted C₁-C₆ alkyl, substituted or unsubstituted C₃-C₈ cycloalkyl, substituted or unsubstituted C₆-C₁₀ aryl, substituted or unsubstituted 5-12 membered heteroaromatic ring; said R₁₃ is selected from the group consisting of: substituted or unsubstituted C₃-C₁₀ carbocycle;
m and n are each independently 0, 1, 2, or 3;
R₄ is selected from the group consisting of H, halogen, substituted or unsubstituted C₁-C₆ alkoxy, and substituted or unsubstituted -O-5-12 membered heteroaromatic ring;
or, R₃ and R₄ together with the carbon atoms to which they are attached form a substituted or unsubstituted C6-C10 aromatic ring, or a substituted or unsubstituted 5-10 membered heteroaromatic ring.

10. The compound according to claim 1, or a deuterated derivative, stereoisomer, tautomer, or pharmaceutically acceptable salt thereof, wherein the compound has the structure of Formula III below: 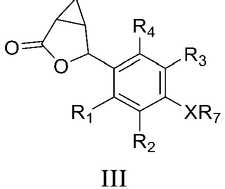
preferably, the compound has the structure of Formula III-A or III-B below: 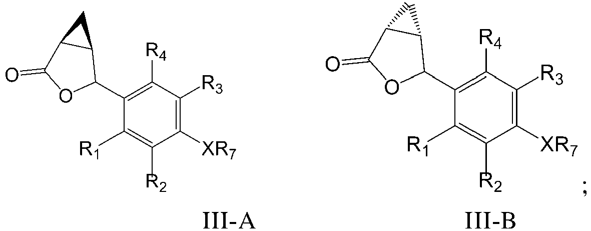
preferably, the compound has any one structure selected from the group consisting of:

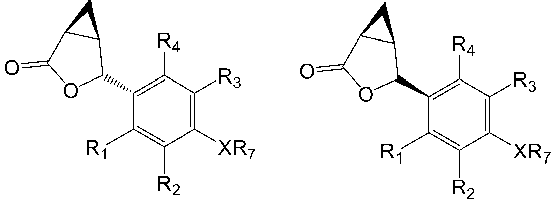

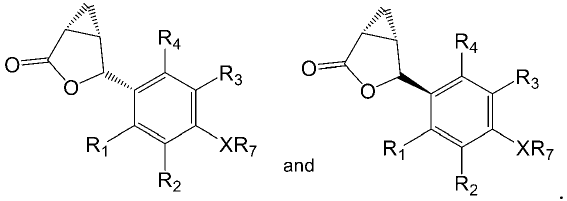

11. The compound according to claim 1, or a deuterated derivative, stereoisomer, tautomer, or pharmaceutically acceptable salt thereof, wherein the compound has the structure of Formula IX below: 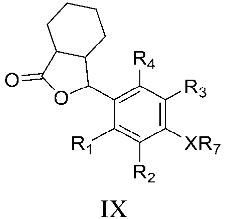
preferably, the compound has the structure of Formula IX-A or IX-B below: 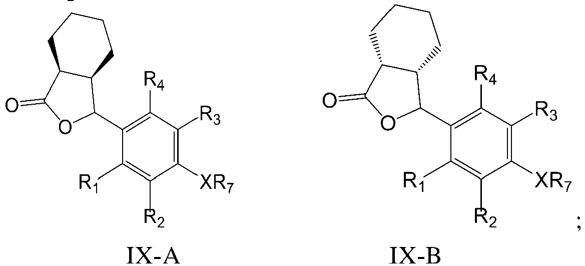
preferably, the compound has any one structure selected from the group consisting of:

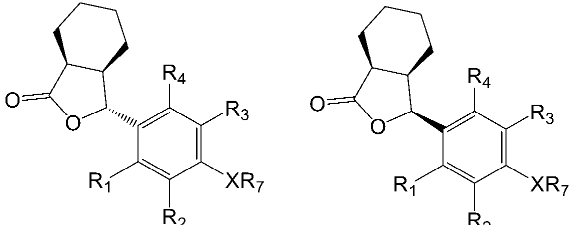

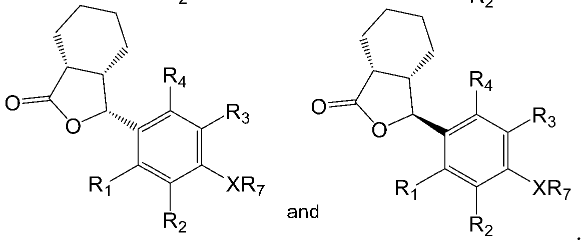

12. The compound according to claim 1, or a deuterated derivative, stereoisomer, tautomer, or pharmaceutically acceptable salt thereof, wherein the compound has the structure of Formula X below: 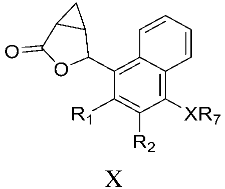
preferably, the compound has the structure of Formula X-A or X-B below: 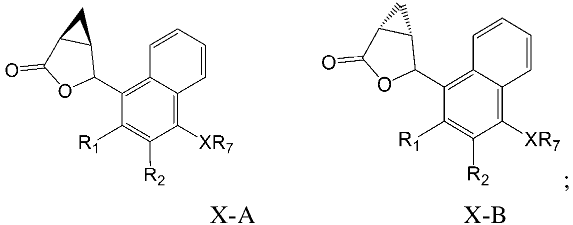
preferably, the compound has any one structure selected from the group consisting of:

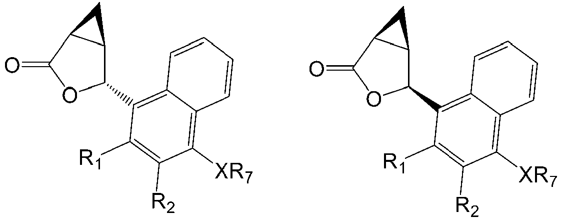

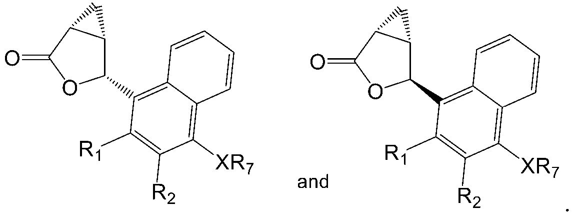

13. The compound according to any one of claims 1-12, or a deuterated derivative, stereoisomer, tautomer, or pharmaceutically acceptable salt thereof, wherein the compound is selected from the group consisting of:
| 000 | Structure | 000 | Structure |
|---|---|---|---|
| 1 | 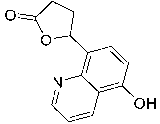 | 2 | 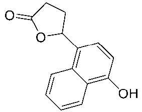 |
| 3 | 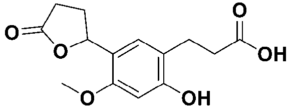 | 4 | 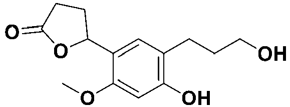 |
| 5 | 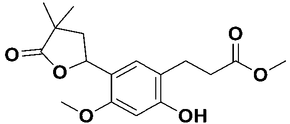 | 6 | 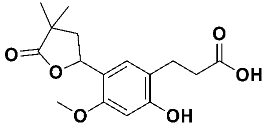 |
| 7 | 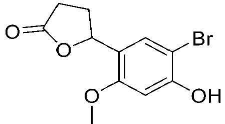 | 8 | 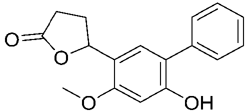 |
| 9 | 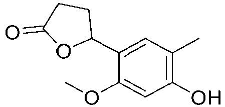 | 10 | 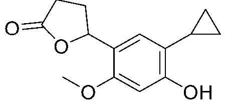 |
| 11 | 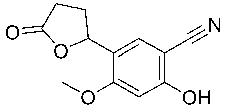 | 12 | 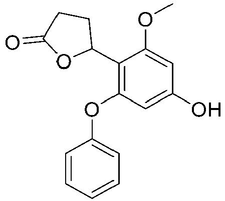 |
| 13 | 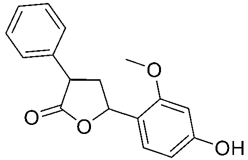 | 14 | 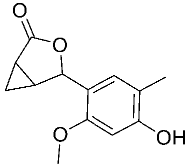 |
| 15 | 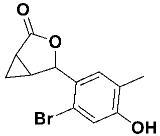 | 16 | 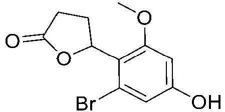 |
| 17 | 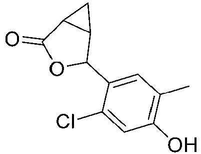 | 18 | 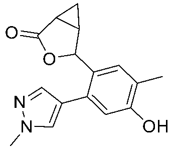 |
| 19 | 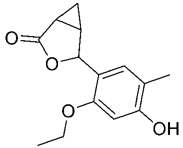 | 20 | 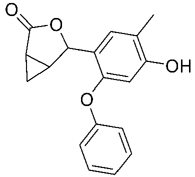 |
| 21 | 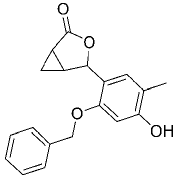 | 22 | 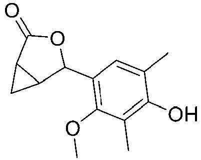 |
| 23 | 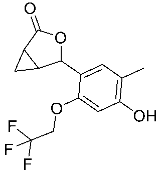 | 24 | 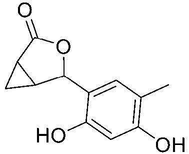 |
| 25 | 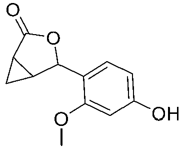 | 26, 27, 28 29 | 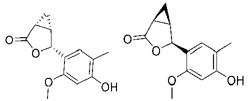 |
| | | | 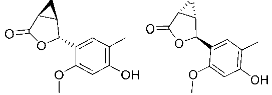 |
| 30 | 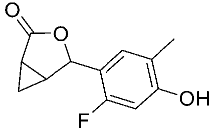 | 31 | 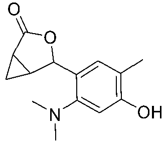 |
| 32 | 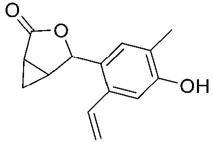 | 33 | 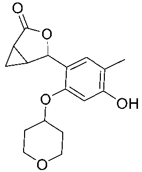 |
| 34 | 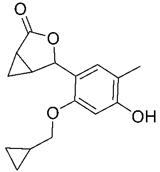 | 35 | 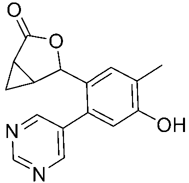 |
| 36 | 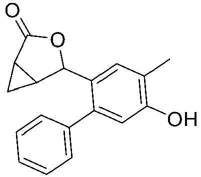 | 37 | 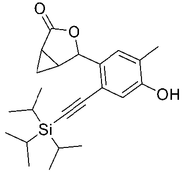 |
| 38 | 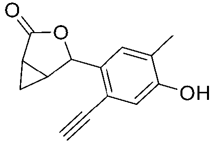 | 39 | 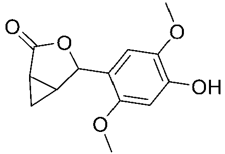 |
| 40 | 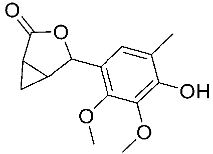 | 41 | 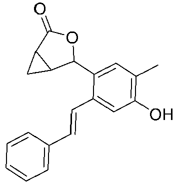 |
| 42 | 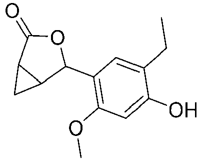 | 43 | 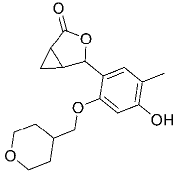 |
| 44 | 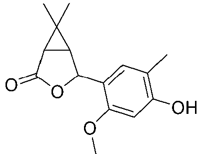 | 45 | 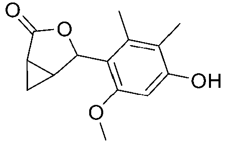 |
| 46 | 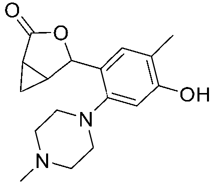 | 47 | 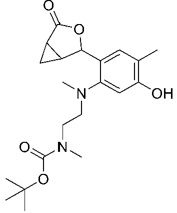 |
| 48 | 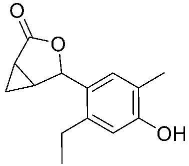 | 49 | 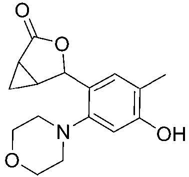 |
| 50 | 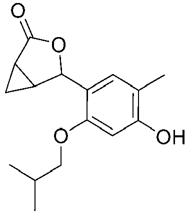 | 51 | 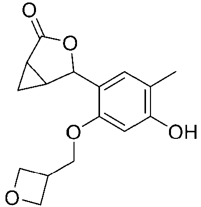 |
| 52 | 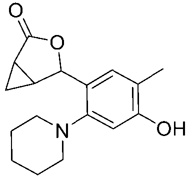 | 53 | 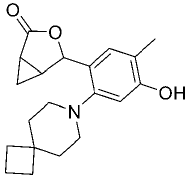 |
| 54 | 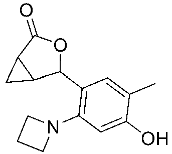 | 55 | 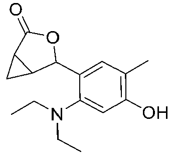 |
| 56 | 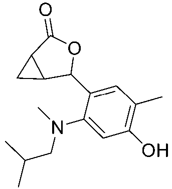 | 57 | 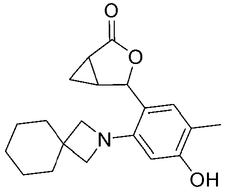 |
| 58 | 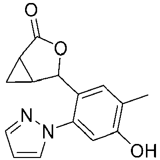 | 59 | 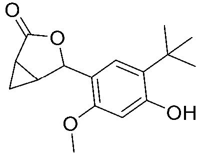 |
| 60 | 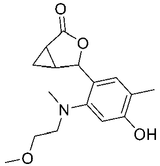 | 61 | 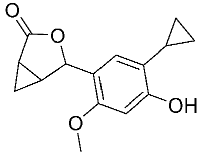 |
| 62 | 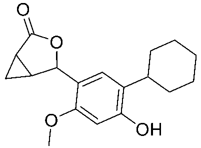 | 63 | 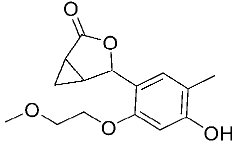 |
| 64 | 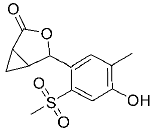 | 65 | 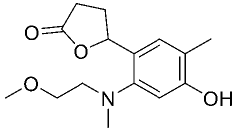 |
| 66 | 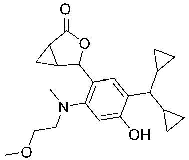 | 67 | 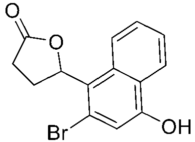 |
| 68 | 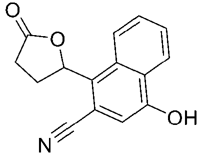 | 69 | 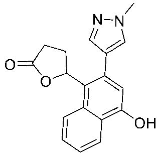 |
| 70 | 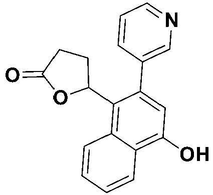 | 71, 72 | 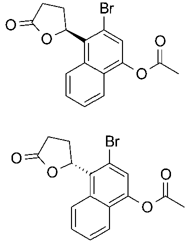 |
| 74 | 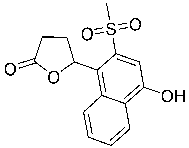 | 75 | 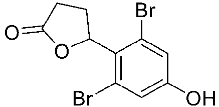 |
| 76 | 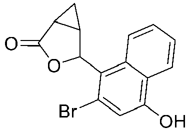 | 77 | 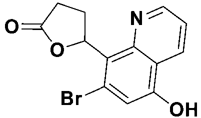 |
| 78 | 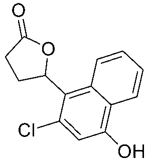 | 79 | 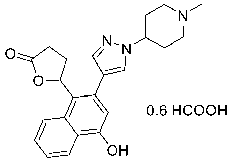 |
| 80 | 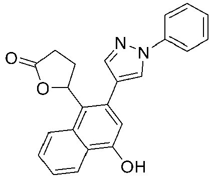 | 81 | 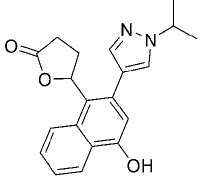 |
| 82 | 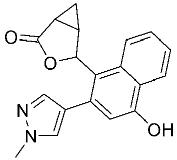 | 83 | 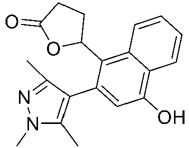 |
| 84 | 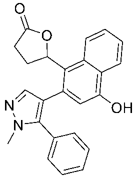 | 85 | 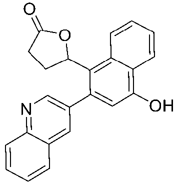 |
| 86 | 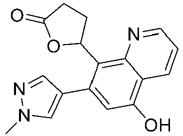 | 87 | 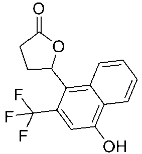 |
| 88 | 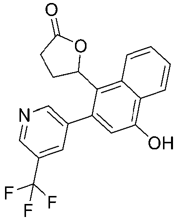 | 89 | 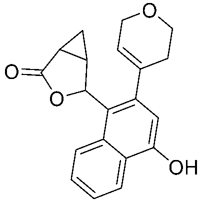 |
| 90 | 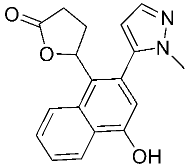 | 91 | 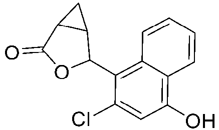 |
| 92 | 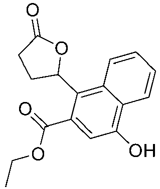 | 93 | 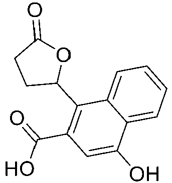 |
| 94 | 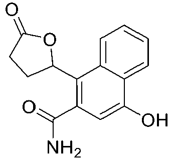 | 95 | 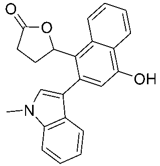 |
| 96 | 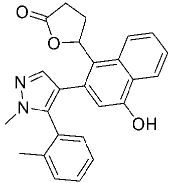 | 97 | 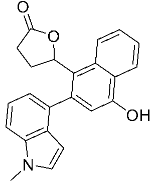 |
| 98 | 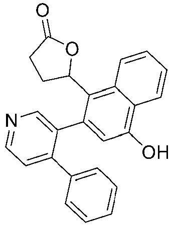 | 99 | 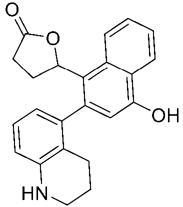 |
| 100 | 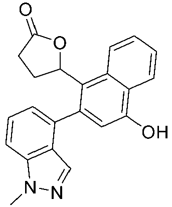 | 101 | 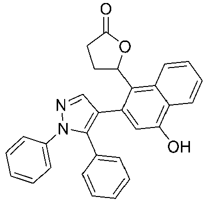 |
| 102 | 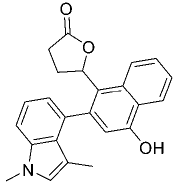 | 103 | 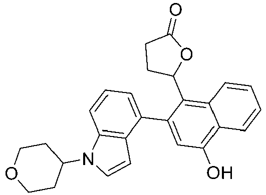 |
| 104 | 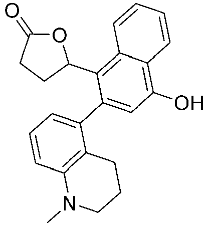 | 105 | 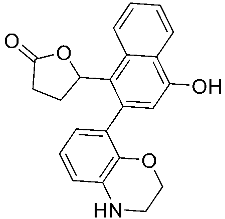 |
| 106 | 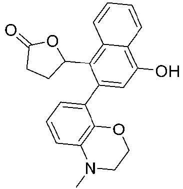 | 107 | 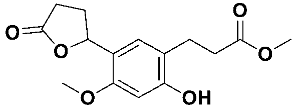 |
| 108 | 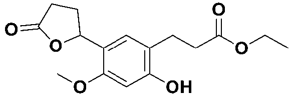 | 109 | 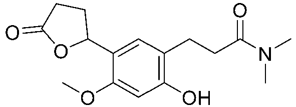 |
| 110 | 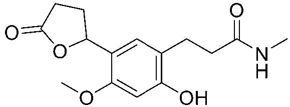 | 111 | 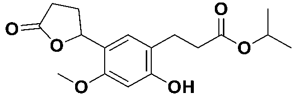 |
| 112 | 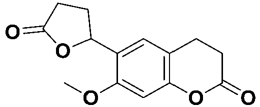 | 113 | 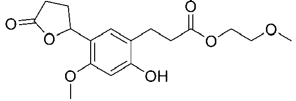 |
| 114 | 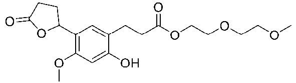 | 115 | 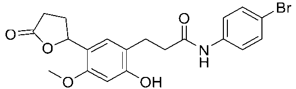 |
| 116 | 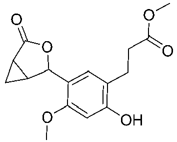 | 117 | 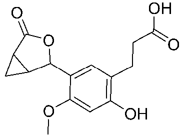 |
| 118, 119 | 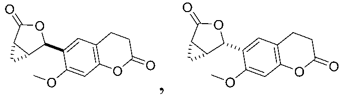 | 120 | 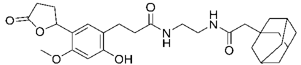 |
| 121 | 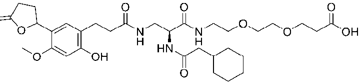 | 122 | 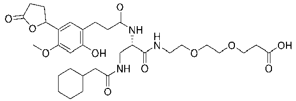 |
| 123 | 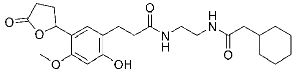 | 124 | 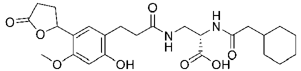 |
| 125 | 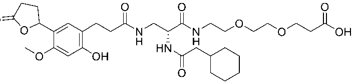 | 126 | 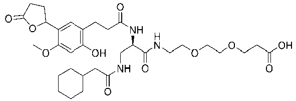 |
| 127 | 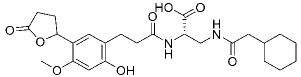 | 128 | 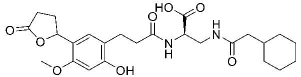 |
| 129 | 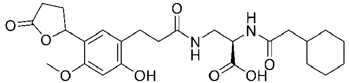 | 130 | 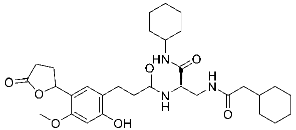 |
| 131 | 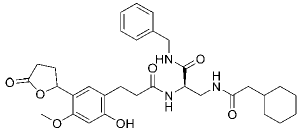 | 132 | 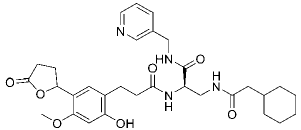 |
| 133 | 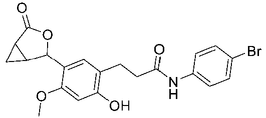 | 134 | 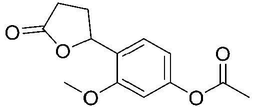 |
| 135 | 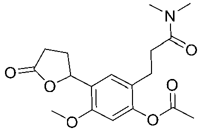 | 136 | 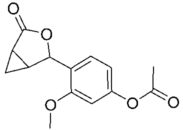 |
| 292 | 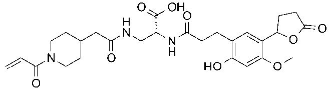 | 138 | 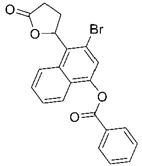 |
| 139 | 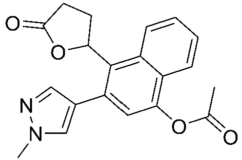 | 140 | 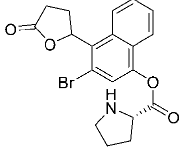 |
| 141 | 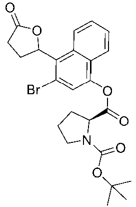 | 142 | 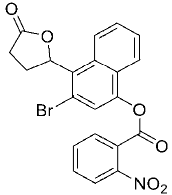 |
| 143 | 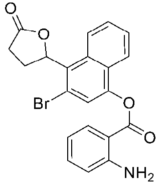 | 144 | 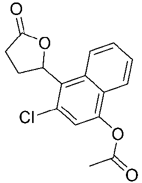 |
| 145 | 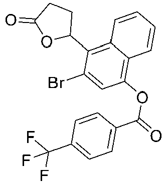 | 146 | 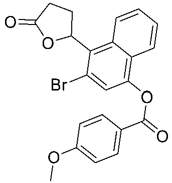 |
| 147 | 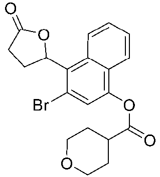 | 148 | 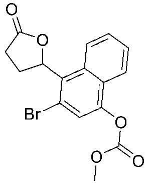 |
| 149 | 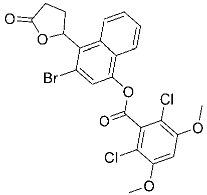 | 150 | 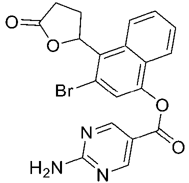 |
| 151 | 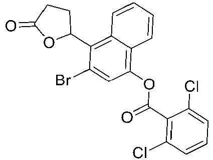 | 152 | 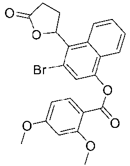 |
| 153 | 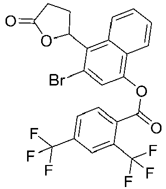 | 154 | 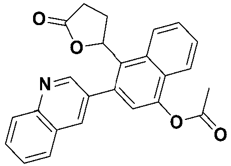 |
| 155 | 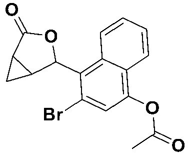 | 156 | 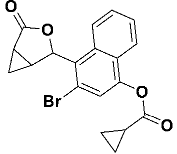 |
| 157 | 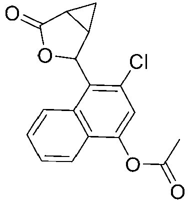 | 158 | 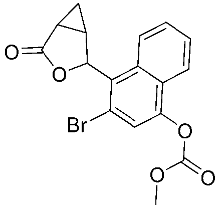 |
| 159 | 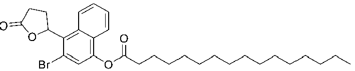 | 160 | 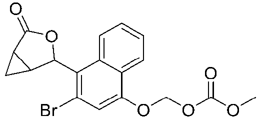 |
| 161 | 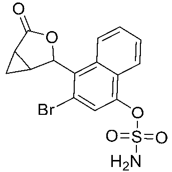 | 162 | 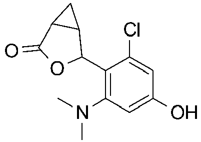 |
| 163 | 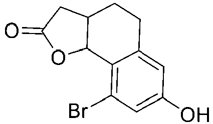 | 164 | 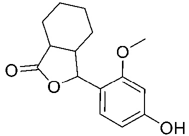 |
| 165 | 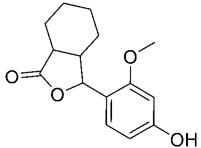 | 166 | 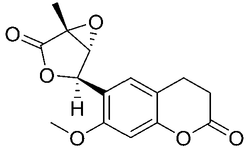 |
| 167 | 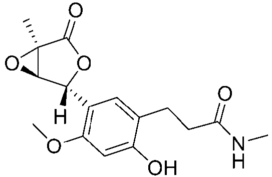 | 168 | 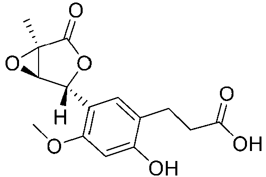 |
| 169 | 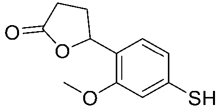 | 171 | 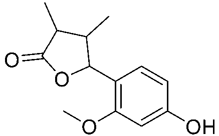 |
| 172 | 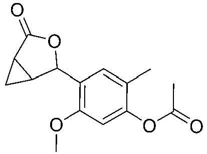 | 174 | 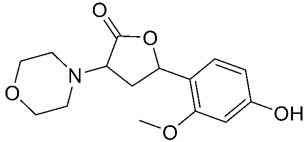 |
| 175 | 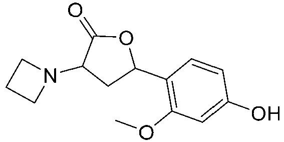 | 178 | 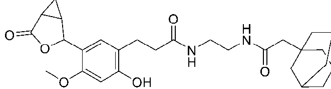 |
| 179 | 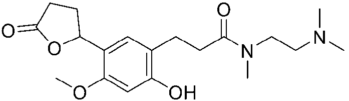 | 180 | 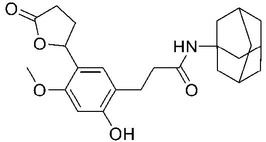 |
| 181 | 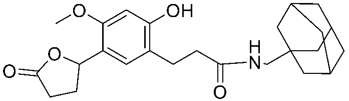 | 183 | 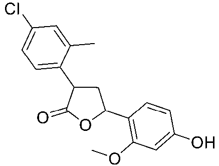 |
| 184 | 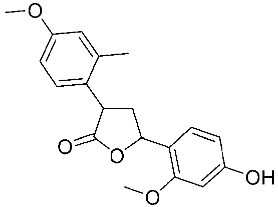 | 185 | 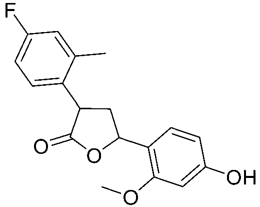 |
| 186 | 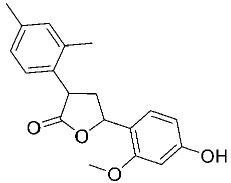 | 187 | 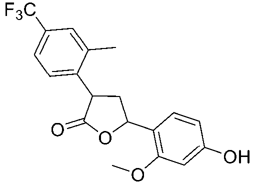 |
| 188 | 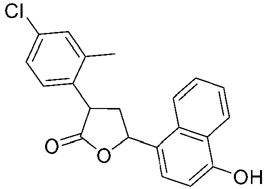 | 189 | 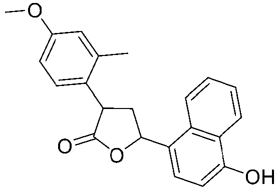 |
| 190 | 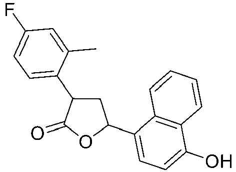 | 191 | 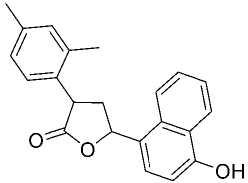 |
| 192 | 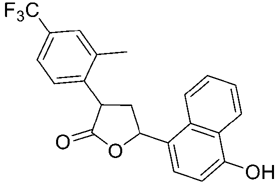 | 193 | 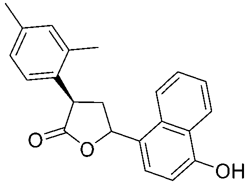 |
| 194 | 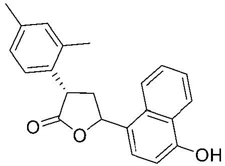 | 195 | 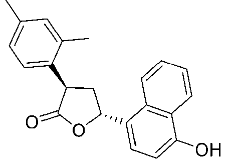 |
| 196 | 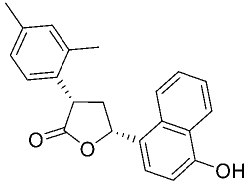 | 197 | 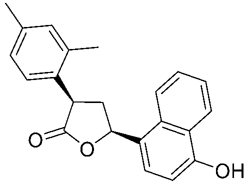 |
| 198 | 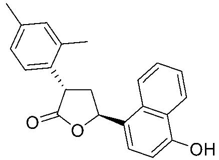 | 199 | 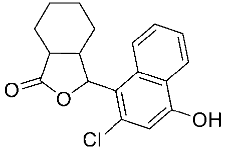 |
| 200 | 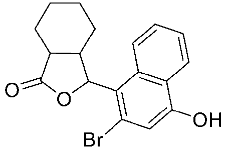 | 201 | 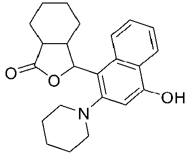 |
| 202 | 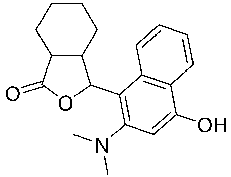 | 203 | 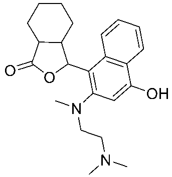 |
| 254 | 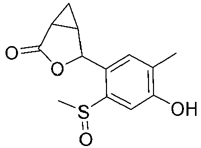 | 256 | 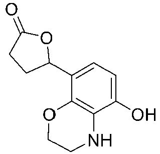 |
| 257 | 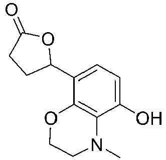 | 258 | 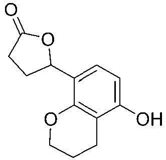 |
| 259 | 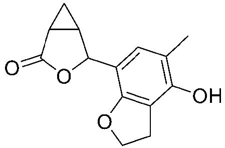 | 260 | 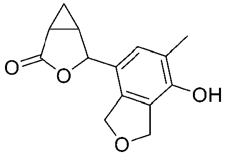 |
| 261 | 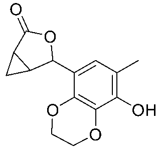 | 262 | 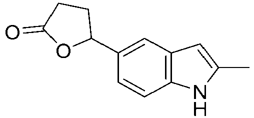 |
| 263 | 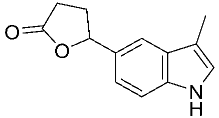 | 264 | 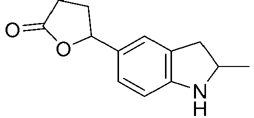 |
| 265 | 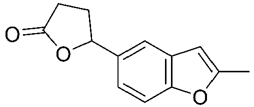 | 266 | 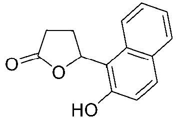 |
| 267 | 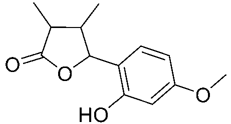 | 268, 269 | 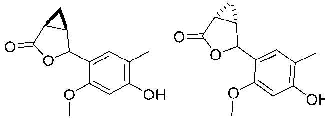 |
| 271 | 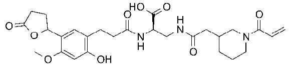 | 272 | 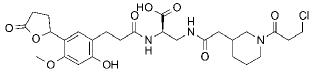 |
| 273 | 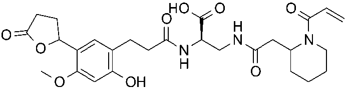 | 274 | 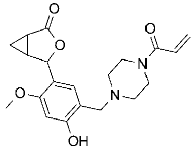 |
| 275 | 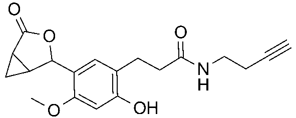 | 276 | 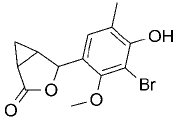 |
| 277 | 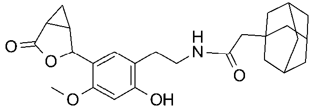 | 278 | 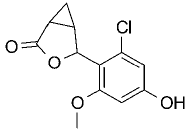 |
| 279 | 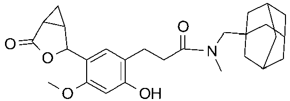 | 280 | 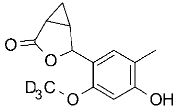 |
| 281 | 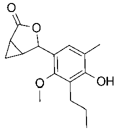 | 282 | 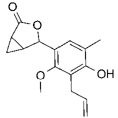 |
| 283 | 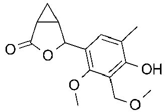 | 284 | 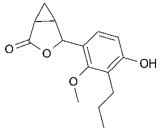 |
| 285 | 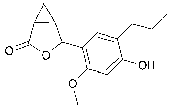 | 286 | 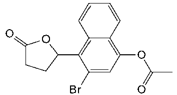 |
| 287 | 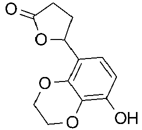 | 288 | 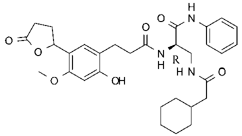 |
| 293, 294, 295, 296 | 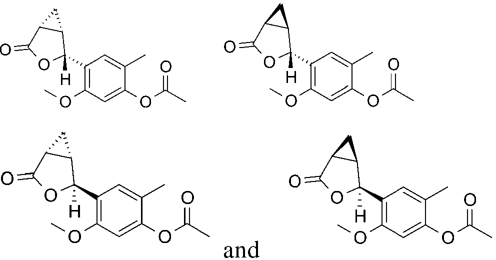 | | |

14. A pharmaceutical composition, wherein the composition comprises (i) the compound according to claim 1, or a deuterated form, stereoisomer, tautomer, or pharmaceutically acceptable salt thereof; and (ii) a pharmaceutically acceptable carrier.

15. Use of the compound according to any one of claims 1-13, or a deuterated derivative, stereoisomer, tautomer, or pharmaceutically acceptable salt thereof, for the preparation of a medicament for the treatment and/or prevention of diseases related to abnormal TDG expression; preferably, said disease is selected from the group consisting of: in another preferred embodiment, said tumor is selected from the group consisting of: lung cancer, acute leukemia, chronic leukemia, colorectal cancer, breast cancer, thyroid tumor, lymphoma, cholangiocarcinoma, liver cancer, pancreatic cancer, bronchial cancer, esophageal cancer, skin cancer, bladder cancer, oral cancer, gastric cancer, urogenital tract tumors, central and peripheral nervous system tumors, or a combination thereof; more preferably, said tumor is selected from the group consisting of: melanoma, acute myeloid leukemia, small cell lung cancer, and non-small cell lung cancer.
